# EUROPÄISCHE PATENTSCHRIFT

(11) **EP 1 994 005 B1**
(45) Veröffentlichungstag und Bekanntmachung des Hinweises auf die Patenterteilung: **20.07.2011**
(21) Anmeldenummer: 07723399.7
(22) Anmeldetag: 15.03.2007
(51) Int. Cl.: C07D 215/38, C07D 239/74, C07D 215/22, C07D 237/32, C07D 311/76, C07D 217/24, A61K 31/47, A61K 31/502, A61K 31/517, A61P 29/00, C07D 209/34, C07D 215/60, C07D 231/56, C07D 265/02

(54) **TETRAHYDRONAPHTHALINDERIVATE, VERFAHREN ZU IHRER HERSTELLUNG UND IHRE VERWENDUNG ALS ENTZÜNDUNGSHEMMER**
TETRAHYDRONAPHTHALINE DERIVATIVES, METHODS FOR THE PRODUCTION AND USE THEREOF AS ANTI-INFLAMMATORY AGENTS
DÉRIVÉS DE TÉTRAHYDRONAPHTALÈNE, PROCÉDÉS DE PRODUCTION DESDITS DÉRIVÉS ET LEUR UTILISATION COMME ANTI-INFLAMMATOIRES

(30) Priorität: 15.03.2006 EP 06090031; 22.03.2006 US 784441 P
(43) Veröffentlichungstag der Anmeldung: 26.11.2008
(73) Patentinhaber: Bayer Schering Pharma Aktiengesellschaft, 13342 Berlin (DE)
(72) Erfinder: BERGER, Markus., 13347 Berlin (DE); REHWINKEL, Hartmut., 10961 Berlin (DE); SCHAECKE, Heike., 10115 Berlin (DE); BAEURLE, Stefan., 10245 Berlin (DE); SCHMEES, Norbert., 13469 Berlin (DE)
(74) Vertreter: Seuss, Thomas
(86) Internationale Anmeldenummer: PCT/EP2007/002432
(87) Internationale Veröffentlichungsnummer: WO 2007/104582

(56) Entgegenhaltungen:
- WO-A-2005/034939
- WO-A-2006/100100
- EVANS D A ET AL: "C2-Symmetric Copper(II) Complexes as Chiral Lewis Acids. Catalytic Enantioselective Carbonyl-Ene Reactions with Glyoxylate and Pyruvate Esters" JOURNAL OF THE AMERICAN CHEMICAL SOCIETY, AMERICAN CHEMICAL SOCIETY, WASHINGTON, DC, US, Bd. 122, 2000, Seiten 7936-7943, XP002221658 ISSN: 0002-7863 in der Anmeldung erwähnt

## Beschreibung

Die vorliegende Patentanmeldung nimmt die Priorität der europäischen Patentanmeldung EP06090031.3 (Anmeldetag:15.3.2006) in Anspruch, sie beansprucht ferner alle Vorteile der Hinterlegung der US Patentanmeldung 60/784,441 (Anmeldetag:22.3.2006, US provisional application).

Die Erfindung betrifft Tetrahydronaphthalinderivate, Verfahren zu ihrer Herstellung und ihre Verwendung als Entzündungshemmer.

Aus dem Stand der Technik (WO 2005/034939) sind zyklische Entzündungshemmer der allgemeinen Formel I 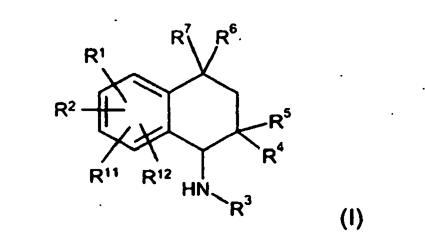 bekannt, wobei diese Verbindungen im Experiment Wirkdissoziationen zwischen antiinflammatorischen und unerwünschten metabolischen Wirkungen zeigen. Zudem weisen diese Verbindungen eine verbesserte Selektivität gegenüber anderen Steroidrezeptoren auf.

Überraschenderweise wurde nun gefunden, daß Verbindungen der Formeln (Ia) besonders dissoziiert im Hinblick auf Nebenwirkungen sind, die sich *in vitro* in Form der TAT Induktion messen lassen.

Die vorliegende Erfindung betrifft daher Verbindungen der allgemeinen Formel (Ia). 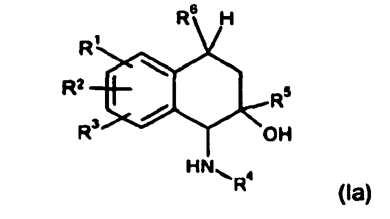 worin 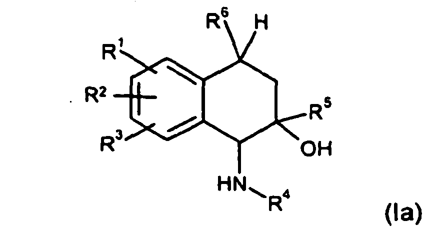 worin
I) die Reste R¹ R² und R³ unabhängig voneinander ausgewählt sind aus

   -OH, O-CH₃, Cl, F, H,
II) der Rest R⁴ ausgewählt ist aus
   2-Methylchinolin-5-yl
   2-Methylchinazolin-5-yl
   2-Ethylchinazolin-5-yl
   7-Fluor-2-methylchinazolin-5-yl,
   8-Fluor-2-methylchinazolin-5-yl,
   7,8-Difluor-2-methylchinazolin-5-yl,
   Chinolin-2(1H)-on-5-yl,
   7-Fluorchinolin-2(1H)-on-5-yl,
   8-Fluorchinolin-2(1H)-on-5-yl,
   Isochromen-1-on-5-yl
   2-Methyl-phthalazin-1-on-5-yl
   Isochinolin-2(1*H*)-on-5-y),
III) der Rest R⁵ für -CF₃ steht und
IV) der Rest R⁶ ausgewählt ist aus -CH₃, -CH₂-CH₃, -(CH₂)₂-CH₃, oder -CH=CH₂.
sowie deren Stereoisomere oder ihre Salze mit physiologisch verträglichen Anionen,
dadurch gekennzeichnet, dass die Verbindungen in der 1α, 2α, 4β-Konfiguration des 1-Amino-1,2,3,4-Tetrahydronaphthalin-2-ol Grundkörpers vorliegen oder dass die Verbindungen in der 5α,6α,8β-Konfiguration des 5,6,7,8-Tetrahydronaphtalin-1,6-diol Grundkörpers vorliegen.

Die erfindungsgemäßen Verbindungen sind dadurch gekeinzeichnet dass die enantiomeren Verbindungen in 1α, 2α, 4β-Konfiguration am 1,2,3,4-Tetrahydronaphthalin-2-ol Grundkörper vorliegen.

Aufgrund der Regeln der IUPAC-Nomenklatur entspricht dies bei 1,6-Dihydroxysubstitution des Tetrahydronaphthalins der 5α, 6α, 8β-Konfiguration des 5,6,7,8-Tetrahydronaphtalin-1,6-diol Grundkörpers (siehe Beispiele).

Eine besonders bevorzugte Untergruppe, wie sie für die vorliegende Erfindung offenbart wurden, stellen diejenigen Reste und alle ihre Unterkombinationen dar, die durch die Beispiele belegt sind.

### Definitionen:

Die Reagenzien, die während der Synthese gegebenenfalls eingesetzt werden, sind käuflich oder die publizierten Synthesen der entsprechenden Reagenzien gehören zum Stand der Technik oder publizierte Synthesen können analog angewendet werden.

Die erfindungsgemäßen Stereoisomere der allgemeinen Formel la können durch das Vorhandensein von Asymmetriezentren als Stereoisomere vorliegen. Gegenstand der vorliegenden Erfindung sind alle möglichen Stereoisomere (z.B.: RRR, RRS, RSR, SRR, RSS, SRS, SSR, SSS), sowohl als Racemate, als auch in enantiomerenreiner Form. Der Begriff Stereoisomere umfaßt auch alle möglichen Diastereomere und Regioisomere und Tautomere (z.B. Keto-Enol-Tautomere), in denen die erfindungsgemäßen Stereoisomere vorliegen können, die damit ebenfalls Gegenstand der Erfindung sind.

Als erfindungsgemäß Pc Stereoisomere der beschriebenen Verbindunges sind jene (1,2,3,4)-Tetrahydronaphthaline genannt, die (1S, 2R, 4R) oder (1S, 2R, 4S) als absolute Konfiguration am 1-Amino-1,2,3,4-tetrahydronaphthalin-2-ol-Grundkörper tragen. Aufgrund der Regeln der IUPAC-Nomenklatur entspricht dies bei 1,6-Dihydroxysubstitution des Tetrahydronaphthalins der (5S, 6R, 8R)-Konfiguration des 5,6,7,8-Tetrahydronaphtalin-1,6-diol Grundkörpers (siehe Beispiele), bzw. der (5S, 6R, 8S)-Konfiguration.

Die erfindungsgemäßen Stereoisomere können auch in Form von Salzen mit physiologisch verträglichen Anionen vorliegen, beispielsweise in der Form des Hydrochlorides, Sulfates, Nitrates, Phosphates, Pivalates, Maleates, Fumarates, Tartrates, Benzoates, Mesylates, Citrates oder Succinates.

Die erfindungsgemäßen Verbindungen werden hergestellt,
a) indem nach im Stand der Technik bekannten Methoden die offenkettigen Vorstufen der allgemeinen Formel (II) generiert werden, worin die Reste R¹, R², R³, R⁴, und R⁵ die in Anspruch 1 angegebenen Bedeutungen haben, 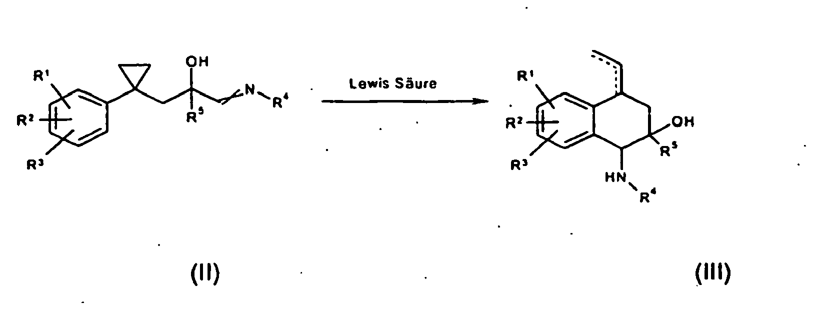 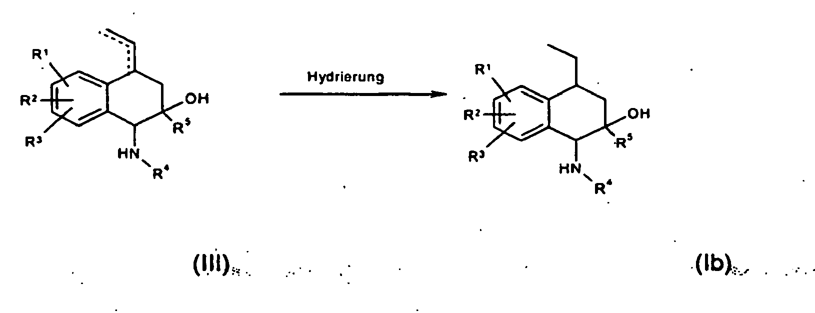 die dann entweder ohne weiteres Reagenz oder durch Zusetzen von anorganischen oder organischen Säuren oder Lewissäuren unter Temperaturen im Bereich von -70°C bis +80°C (bevorzugt im Bereich von -30°C bis +80°C) zu den Verbindungen der allgemeinen Formel (III) zyklisiert und umgelagert. werden. Eine katalytische Hydrierung, die gegebenenfalls diastereoselektiv geführt werden kann liefert dann Verbindungen der allgemeinen Formel (1b), in der R⁶ einer Ethylgruppe entspricht.
b) indem nach im Stand der Technik bekannten Methoden hergestellten Styrole der allgemeinen Formel (IV) durch eine gegebenenfalls enantioselektiv geführte En-Reaktion mit chiralen Lewis Säuren in die Verbindungen der allgemeinen Formel (V) überführt werden. Als chirale Lewis Säuren für die enantioselektive Erzeugung der quartemären Alkoholfunktion können verwendet werden: (*R*)-oder (*S*)-SEGPHOS-PdCl₂ (Mikami et al. Tetrah. Asymm. 2004, 15, 3885-89),(*R*)- oder (*S*)-BINOL-Ti(OiPr)₂ (Ding et al. Tetrah. Lett. 2004, 45, 2009-12), (*R*)- oder (*S*)-Cu *^{t}*BuBOX,), (*R*)- oder (*S*)-Cu *ⁱ*PrBOX, (*R*)- oder (*S*)-Cu PhBOX, (*R*)- oder (*S*)- Cu AdaBOX (Evans et al. J. Am. Chem. Soc. 2000, 122, 7936-43), (*R*)- oder (*S*)-ⁱPr-pybox Yb(OTf)₃, (*R*)- oder (*S*)-*^{t}*Bu-pybox Yb(OTf)₃, (*R*)-oder (*S*)-Ph-pybox Yb(OTf)₃ (Qian et al. Tetrah. Asymm. 2000, 11, 2347-57).

Durch Reduktion, Hydrierung und Aminierung wird nach dem Fachmann bekannten Methoden das Imin (VI) erzeugt, 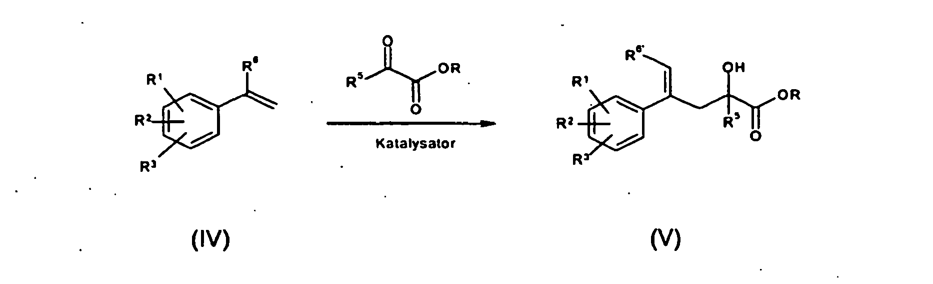 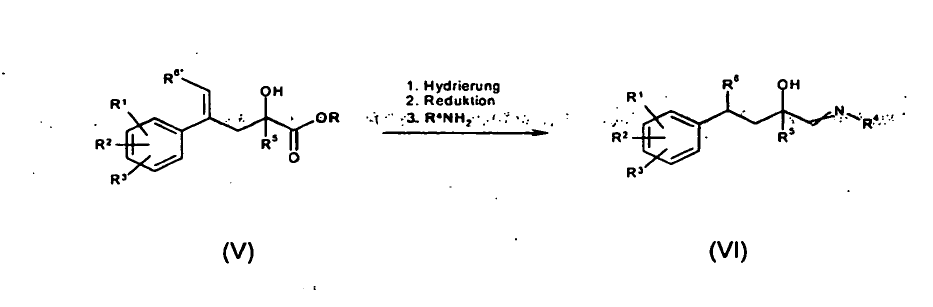 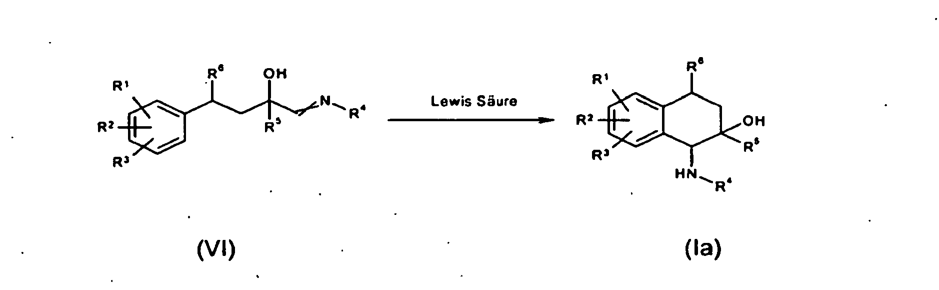 das dann entweder ohne weiteres Reagenz oder durch Zusetzen von anorganischen oder organischen Säuren oder Lewissäuren unter Temperaturen im Bereich von -70°C bis +80°C (bevorzugt im Bereich von -30°C bis +80°C) zu den Verbindungen der allgemeinen Formel (Ia) zyklisiert wird. Die allgemein in den oben aufgeführten Formeln definierten Reste R¹, R², R³, R⁴, R⁵, und R⁶ haben die in Anspruch 1 angegebenen Bedeutungen.

Gegenstand der vorliegenden Erfindung ist somit auch eine Methode zur Herstellung der Stereoisomere der allgmeinen Formel (la), die dadurch gekennzeichnet ist, dass Imine der allgemeinen Formel (VI) oder (II) entweder ohne weiteres Reagenz in einem Lösungsmittel oder konzentrierten organischen Säuren oder durch Zusetzen von anorganischen oder organischen Säuren oder Lewissäuren unter Temperaturen im Bereich von -70°C bis +80°C (bevorzugt im Bereich von -30°C bis +80°C) zu den Stereoisomere der allgemeinen Formel (Ia) oder (III) zyklisiert werden sowie deren direkte Vorstufen der Formel (V).

Die neuen Imine (IV) für die Zyklisierung sind ebenfalls Gegenstand der vorliegenden Erfindung, insbesondere die, die durch die Beispiele offenbart worden sind.

Ein besonders bevorzugtes Verfahren ist die Methode zur Herstellung von Verbindungen der allgemeinenen Formel (V), in der als Katalysatoren der enantioselektiven En-Reaktion [Cu(S,S)Bis(*tert*-butyloxazoline] (SbF₆)₂ oder [Cu(R,R)Bis(*tert*-butyloxazoline] (SbF₆)₂ eingesetzt wird und Enantiomerenüberschüsse von bis zu 95% erreicht werden, die durch Kristallisation noch weiter erhöht werden können.

Enantiomerenreine Verbindungen der Formel (V) können dann durch chromatographische Trennmethoden an Kieselgel auf der Stufe des hydrierten Aldehyds oder des Imins in die enantiomerenreinen Verbindungen der Formel (VI) überführt werden.

Diastereoselektive Hydriermethoden können alternativ zum Erhalt der enantiomerenreinen Verbindung der Formel (VI) eingesetzt werden.

Als Katalysatoren für die oben genannten katalytischen Hydrierungen kommen in Betracht:
1. Palladium auf Kohle
2. Raney-Nickel
3. Rhodium-Katalysatoren mit chiralen Liganden, wie in nachfolgenden Publikationen beschrieben:
   - Weissenstein et al., Adv. Synth.Catal. 2003, 345, 160-164
   - Imwinkelried et al., Chimia 1997, 51, 300
4. Iridium-Katalysatoren mit chiralen Liganden, wie in nachfolgenden Publikationen beschrieben:
   - Pfaltz et al., Org.Lett. 2004, 6, 2023-2026
   - Blaser et al., Chimia 1999, 53, 275
5. Ruthenium-Katalysatoen mit chiralen Liganden, wie in nachfolgender Publikation beschrieben:
   - Chirality 2000, 12, 514-522

Ein weiterer Gegenstand der Erfindung sind somit enantiomerenreine Ester der Formel (VII), 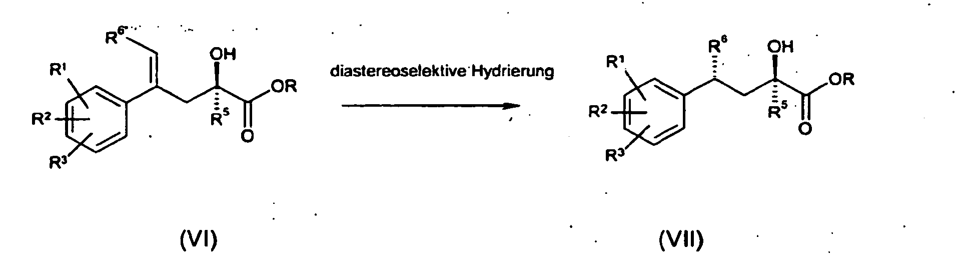 die durch diastereoselektive Hydriermethoden oder Diastereomerentrennungen erhalten werden können:

Die Bindung der Substanzen an den Glucocorticoid-Rezeptor (GR) und weitere Steroidhormon-Rezeptoren (Mineralcorticoid-Rezeptor (MR), Progesteron-Rezeptor (PR) und Androgen-Rezeptor (AR) wird mit Hilfe rekombinant hergestellter Rezeptoren überprüft. Cytosolpräparationen von Sf9 Zellen, die mit rekombinanten Baculoviren, die für den GR kodieren, infiziert worden waren, werden für die Bindungsuntersuchungen eingesetzt. Im Vergleich zur Bezugssubstanz [³H]-Dexamethason zeigen die Substanzen eine hohe Affinität zum GR.

Als wesentlicher, molekularer Mechanismus für die anti-inflammatorische Wirkung von Glucocorticoiden wird die durch den GR vermittelte Hemmung der Transkription von Cytokinen, Adhäsionsmolekülen, Enzymen und anderer pro - inflammatorischen Faktoren angesehen. Diese Hemmung wird durch eine Interaktion des GR mit anderen Transkriptionsfaktoren, z.B. AP-1 und NF-kappa-B, bewirkt (zur Übersicht siehe Cato ACB and Wade E, BioEssays 18, 371-378 1996).

Die erfindungsgemäßen Stereoisomere der allgemeinen Formel Ia hemmen die durch Lipopolysaccharid (LPS) ausgelöste Sekretion des Cytokins IL-8 in der menschlichen Monozytenzelline THP-1.

Die anti - inflammatorische Wirkung der Stereoisomere der allgemeinen Formel Ia wurde im Tierexperiment durch Testen in der Crotonöl - induzierten Entzündung in der Ratte und der Maus getestet (J. Exp. Med. (1995), 182, 99-108). Hierzu wurde den Tieren Crotonöl in ethanolischer Lösung topisch auf die Ohren appliziert. Die Testsubstanzen wurden gleichzeitig oder zwei Stunden vor dem Crotonöl ebenfalls topisch oder systemisch appliziert. Nach 16-24 Stunden wurden das Ohrgewicht als Maß für das entzündliche Ödem, die Peroxidaseaktivität als Maß für die Einwanderungen von Granulozyten und die Elastaseaktivität als Maß für die Einwanderung von neutrophilen Granulozyten gemessen. Die Stereoisomere der allgemeinen Formel Ia hemmen in diesem Test sowohl nach topischer, als auch nach systemischer Applikation die drei oben genannten Entzündungsparameter.

Eine der häufigsten unerwünschten Wirkungen einer Glucocorticoid - Therapie ist der sogenannte "Steroiddiabetes" [vgl. Hatz, HJ, Glucocorticoide: Immunologische Grundlagen, Pharmakologie und Therapierichtlinien, Wissenschaftliche Verlagsgesellschaft mbH, Stuttgart, 1998]. Ursache hierfür ist die Stimulation der Gluconeogenese in der Leber durch Induktion der hierfür verantwortlichen Enzyme und durch freie Aminosäuren, die aus dem Abbau von Proteinen (katabole Wirkung der Glucocorticoide) entstehen. Ein Schlüsseienzym des katabolen Stoffwechsels in der Leber ist die Tyrosinaminotranferase (TAT). Die Aktivität dieses Enzyms kann photometrisch aus Leberhomogenaten bestimmt werden und stellt ein gutes Maß für die unerwünschten metabolischen Wirkungen der Glucocorticoide dar. Zur Messung der TAT - Induktion werden die Tiere 8 Stunden nach Gabe der Testsubstanzen getötet, die Leber entnommen und die TAT - Aktivität im Homogenat gemessen. Die Stereoisomere der allgemeinen Formel Ia induzieren in diesem Test in Dosen, in denen sie anti - inflammatorisch wirksam sind, nicht oder nur in geringem Maße die Tyrosinaminotransferase.

Aufgrund ihrer anti-inflammatorischen und zusätzlichen anti-allergischen, immunsuppressiven und anti-proliferativen Wirkung können die erfindungsgemäßen Stereoisomere der allgemeinen Formel Ia als Medikamente zur Behandlung oder Prophylaxe folgender Krankheitszustände bei Säugetieren und Menschen Verwendung finden: Dabei steht der Begriff "ERKRANKUNG" für die folgenden Indikationen:
(i) Lungenerkrankungen, die mit entzündlichen, allergischen und / oder proliferativen Prozessen einhergehen:
   - Chronisch obstruktive Lungenerkrankungen jeglicher Genese, vor allem Asthma bronchiale
   - Bronchitis unterschiedlicher Genese
   - Alle Formen der restriktiven Lungenerkrankungen, vor allem allergische Alveolitis,
   - Alle Formen des Lungenödems, vor allem toxisches Lungenödem
   - Sarkoidosen und Granulomatosen, insbesondere Morbus Boeck
(ii) Rheumatische Erkrankungen / Autoimmunerkrankungen / Gelenkerkrankungen, die mit entzündlichen, allergischen und / oder proliferativen Prozessen einhergehen:
   - Alle Formen rheumatischer Erkrankungen, insbesondere rheumatoide Arthritis, akutes rheumatisches Fieber, Polymyalgia rheumatica
   - Reaktive Arthritis
   - Entzündliche Weichteilerkrankungen sonstiger Genese
   - Arthritische Symptome bei degenerativen Gelenkerkrankungen (Arthrosen)
   - Traumatische Arthritiden
   - Kollagenosen jeglicher Genese, z.B. systemischer Lupus erythematodes, Sklerodermie, Polymyositis, Dermatomyositis- Sjögren-Syndrom, Still-Syndrom, Felty-Syndrom
(iii) Allergien, die mit entzündlichen, und / oder proliferativen Prozessen einhergehen:
   - Alle Formen allergischer Reaktionen, z.B. Quincke Ödem, Heuschnupfen, Insektenstich, allergische Reaktionen auf Arzneimittel, Blutderivate, Kontrastmittel etc., Anaphylaktischer Schock, Urtikaria, Kontaktdermatitis
(iv) Gefäßentzündungen (Vaskulitiden)
   - Panarteriitis nodosa, Arteriitis temporalis, Erythema nodosum
(v) Dermatologische Erkrankungen, die mit entzündlichen, allergischen und / oder proliferativen Prozessen einhergehen:
   - Atopische Dermatitis (vor allem bei Kindern)
   - Psoriasis
   - Pityriasis rubra pilaris
   - Erythematöse Erkrankungen, ausgelöst durch unterschiedlichen Noxen, z.B. Strahlen, Chemikalien, Verbrennungen etc.
   - Bullöse Dermatosen
   - Erkrankungen des lichenoiden Formenkreises,
   - Pruritus (z. B. allergischer Genese)
   - Seborrhoisches Ekzem
   - Rosacea
   - Pemphigus vulgaris
   - Erythema exsudativum multiforme
   - Balanitis
   - Vulvitis
   - Haarausfall wie Alopecia areata
   - Cutane T - Zell - Lymphome
(vi) Nierenerkrankungen, die mit entzündlichen, allergischen und / oder proliferativen Prozessen einhergehen:
   - Nephrotisches Syndrom
   - Alle Nephritiden
(vii) Lebererkrankungen, die mit entzündlichen, allergischen und / oder proliferativen Prozessen einhergehen:
   - akuter Leberzellzerfall
   - akute Hepatitis unterschiedlicher Genese, z.B. viral, toxisch, arzneimittelinduziert
   - chronisch aggressive und / oder chronisch intermittierende Hepatitis
(viii) Gastrointestinale Erkrankungen, die mit entzündlichen, allergischen und / oder proliferativen Prozessen einhergehen:
   - regionale Enteritis (Morbus Crohn)
   - Colitis Ulcerosa
   - Gastritis
   - Refluxoesophagitis
   - Gastroenteritiden anderer Genese, z.B. einheimische Sprue
(ix) Proktologische Erkrankungen, die mit entzündlichen, allergischen und / oder proliferativen Prozessen einhergehen:
   - Analekzem
   - Fissuren
   - Hämorrhoiden
   - idiopathische Proktitis
(x) Augenerkrankungen, die mit entzündlichen, allergischen und / oder proliferativen Prozessen einhergehen:
   - allergische Keratitis, Uveitis, Iritis,
   - Konjunktivitis
   - Blepharitis
   - Neuritis nervi optici
   - Chorioditis
   - Ophtalmia sympathica
(xi) Erkrankungen des Hals-Nasen-Ohren-Bereiches, die mit entzündlichen, allergischen und / oder proliferativen Prozessen einhergehen:
   - allergische Rhinitis, Heuschnupfen
   - Otitis externa, z.B. bedingt durch Kontaktexem, Infektion etc.
   - Otitis media
(xii) Neurologische Erkrankungen, die mit entzündlichen, allergischen und / oder proliferativen Prozessen einhergehen:
   - Hirnödem, vor allem Tumor-bedingtes Himödem
   - Multiple Sklerose
   - akute Encephalomyelitis
   - Meningitis
   - verschieden Formen von Krampfanfällen, z.B. BNS-Krämpfe
(xiii) Bluterkrankungen, die mit entzündlichen, allergischen und / oder proliferativen Prozessen einhergehen:
   - Erworbene hämolytische Anämie
   - Idopathische Thrombocytopenia
(xiv) Tumorerkrankungen, die mit entzündlichen, allergischen und / oder proliferativen Prozessen einhergehen:
   - Akute lymphatische Leukämie
   - Maligne Lymphome
   - Lymphogranulomatosen
   - Lymphosarkome
   - Ausgedehnte Metastasierungen, vor allem bei Mamma- Bronchial- und Prostatakarzinom
(xv) Endokrine Erkrankungen, die mit entzündlichen, allergischen und / oder proliferativen Prozessen einhergehen:
   - Endokrine Orbitopathie
   - Thyreotoxische Krise
   - Thyreoiditis de Quervain
   - Hashimoto Thyreoiditis
   - Morbus Basedow
(xvi) Organ- und Gewebstransplantationen, Graft-versus-host-disease
(xvii) Schwere Schockzustände, z.B anaphylaktischer Schock , systemic inflammatory response syndrome (SIRS)
(xviii)Emesis, die mit entzündlichen, allergischen und / oder proliferativen Prozessen einhergehen:
   - z.B. in Kombination mit einem 5-HT3-Antagonisten bei Zytostika - bedingten Erbrechen.
(xix) Schmerzen bei entzündlicher Genese, z.B. Lumbago
(xx) Substitutionstherapie bei:
   - angeborene primäre Nebenniereninsuffizienz, z.B. kongenitales adrenogenitales Syndrom
   - erworbene primäre Nebenniereninsuffizienz, z.B. Morbus Addison, autoimmune Adrenalitits, postinfektiös, Tumoren, Metastasen etc.
   - angeboren sekundäre Nebeniereninsuffizienz, z.B. kongenitaler Hypopitutitarismus
   - erworbene sekundäre Nebenniereninsuffizienz, z.B. postinfektiös, Tumoren etc..

Besondere Wirksamkeit zeigen Arzneimittel, enthaltend Stereoisomere der allgemeinen Formel Ia bei den folgenden Erkrankungen:
1. Lungenerkrankungen
2. rheumatische Erkrankungen / Autoimmunerkrankungen
3. dermatologische Erkrankungen
4. degenerative Gelenkserkrankungen
5. Gefäßentzündungen
6. graft versus host disease
7. schwere Schockzustände
8. Emesis, die mit entzündlichen, allergischen und / oder proliferativen Prozessen einhergehen
9. entzündungsbedingter Schmerz

Darüber hinaus können die erfindungsgemäßen Stereoisomere der allgemeinen Formel Ia zur Therapie und Prophylaxe weiterer oben nicht genannter Krankheitszustände eingesetzt werden, für die heute synthetische Glucocorticoide verwendet werden (siehe dazu Hatz, HJ, Glucocorticoide: Immunologische Grundlagen, Pharmakologie und Therapierichtlinien, Wissenschafliche Verlagsgesellschaft mbH, Stuttgart, 1998).

Alle zuvor genannten Indikationen (i) bis (xx) sind ausführlich beschrieben in Hatz, HJ, Glucocorticoide: Immunologische Grundlagen, Pharmakologie und Therapierichtlinien, Wissenschafliche Verlagsgesellschaft mbH, Stuttgart, 1998.

Für die therapeutische Wirkungen bei den oben genannten Krankheitszuständen ist die geeignete Dosis unterschiedlich und hängt beispielsweise von der Wirkstärke der Verbindung oder allgemeinen Formel Ia, dem Wirt, der Art der Verabreichung und der Art und der Schwere der zu behandelnden Zustände, sowie der Verwendung als Prophylaktikum oder Therapeutikum ab.

Die Erfindung betrifft die Verwendung der beanspruchten Verbindungen/Stereoisomere zur Herstellung von Arzneimitteln.

Die Erfindung betrifft weiterhin
(i) die Verwendung eines der erfindungsgemäßen Stereoisomeren gemäß Formel Ia oder deren Gemischen zur Herstellung eines Medikaments zur Behandlung von einer ERKRANKUNG;
(iii) eine pharmazeutische Zusammensetzung zur Behandlung von einer ERKRANKUNG, welche Behandlung eine der erfindungsgemäßen Stereoisomere oder deren Gemische und wenigstens einen pharmazeutischen Hilfs- und/oder Trägerstoff umfaßt.

Im allgemeinen sind bei. Tieren zufriedenstellende Resultate zu erwarten, wenn die täglichen Dosen einen Bereich von 1 µg bis 100.000 µg der erfindungsgemäßen Verbindung pro kg Körpergewicht umfassen. Bei größeren Säugetieren, beispielsweise dem Menschen, liegt eine empfohlene tägliche Dosis im Bereich von 1 µg bis 100.000 µg pro kg Körpergewicht. Bevorzugt ist eine Dosis von 10 bis 30.000 µg pro kg Körpergewicht, besonders bevorzugt eine Dosis von 10 bis 10.000 µg pro kg Körpergewicht.

Zum Beispiel wird diese Dosis zweckmäßigerweise mehrmals täglich verabreicht. Zur Behandlung eines akuten Schocks (z.B. anaphylaktischer Schock) können Einzeldosen gegeben werden, die deutlich über den oben genannten Dosen liegen.

Die Formulierung der pharmazeutischen Präparate auf Basis der neuen Verbindungen erfolgt in an sich bekannter Weise, indem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskorrigentien, Färbemitteln usw., verarbeitet und in die gewünschte Applikationsform überführt. Dabei ist auf Remington's Pharmaceutical Science, 15th ed. Mack Publishing Company, East Pennsylvania (1980) hinzuweisen.

Für die orale Applikation kommen insbesondere Tabletten, Dragees, Kapseln, Pillen, Pulver, Granulate, Pastillen, Suspensionen, Emulsionen oder Lösungen in Frage.

Für die parenterale Applikation sind Injektions- und Infusionszubereitungen möglich.

Für die intraartikulären Injektion können entsprechend zubereitete Kristallsuspensionen verwendet werden.

Für die intramuskuläre Injektion können wässrige und ölige Injektionslösungen oder Suspensionen und entprechende Depotpräparationen Verwendung finden.

Für die rektale Applikation können die neuen Verbindungen in Form von Suppositorien, Kapseln, Lösungen (z.B. in Form von Klysmen) und Salben sowohl zur systemischen, als auch zur lokalen Therapie verwendet werden.

Zur pulmonalen Applikation der neuen Verbindungen können diese in Form von Aerosolen und Inhalaten verwendet werden.

Für die lokale Anwendung an Augen, äußerem Gehörgang, Mittelohr, Nasenhöhle und Nasennebenhöhlen können die neuen Verbindungen als Tropfen, Salben und Tinkturen in entsprechenden pharmazeutischen Zubereitungen verwendet werden.

Für die topische Auftragung sind Formulierungen in Gelen, Salben, Fettsalben, Cremes, Pasten, Puder, Milch und Tinkturen möglich. Die Dosierung der Verbindungen der allgemeinen Formel Ia sollte in diesen Zubereitungen 0.01 % - 20% betragen, um eine ausreichende pharmakologische Wirkung zu erzielen.

Die Erfindung umfaßt ebenfalls die erfindungsgemäßen Verbindungen der allgemeinen Formel Ia als therapeutischen Wirkstoff. Weiterhin gehört zur Erfindung die erfindungsgemäßen Verbindungen der allgemeinen Formel Ia als therapeutischen Wirkstoff zusammen mit pharmazeutisch verträglichen und annehmbaren Hilfsstoffen und Trägerstoffen.

Ebenfalls umfaßt die Erfindung eine pharmazeutische Zusammensetzung, die eine der pharmazeutisch aktiven, erfindungsgemäßen Verbindungen oder deren Gemisch oder deren pharmazeutisch verträgliches Salz und ein pharmazeutisch verträgliches Salz oder pharmazeutisch verträgliche Hilfsstoffe und Trägerstoffe enthält.

Die Erfindung betrifft ferner Verbindungen (Ia) zur Verwendung in Kombinationstherapien oder kombinierte Zusammensetzungen, worin ein Glukokortikoidrezeptor (GR) Agonist der Formel (Ia) oder ein pharmazeutisch akzeptables Salz davon, oder eine pharmazeutische Zusammensetzung enthaltend einen GR Agonisten der Formel (Ia) oder ein pharmazeutisch akzeptables Salz davon, verabreicht wird entweder gleichzeitig (gegebenenfalls in derselben Zusammensetzung) oder nacheinander zusammen mit einem oder mehreren Arzneimitteln zur Behandlung von einem der oben angesprochenen Krankheitszustände. Beispielsweise kann zur Behandlung von rheumatoider Arthritis, Osteoarthritis, COPD (chronische obstruktive Lungenerkrankung), Asthma oder allergischer Rhinitis, ein GR Agonist der vorliegenden Erfindung kombiniert werden mit einem oder mehreren Arzneimitteln zur Behandlung von einem solchen Zustand. Wo eine solche Kombination durch Inhalation verabreicht wird, kann das zu kombinierende Arzneimitel von der folgenden Liste ausgewählt werden:
- ein PDE4 Inhibitor einschließlich einem Inhibitor der Isoform PDE4D;
- ein selektiver ß.sub2. Adrenozeptor Agonist wie beispielsweise Metaproterenol, Isoproterenol, Isoprenalin, Albuterol, Salbutamol, Formoterol, Salmeterol, Terbutalin, Orciprenaline, Bitolterolmesylat, Pirbuterol oder Indacaterol;
- ein Muscarin Rezeptor Antagonist (zum Beispiel ein M1, M2 oder M3 Antagonist, wie beispielsweise ein selektiver M3 Antagonist) wie beispielsweise Ipratropiumbromid, Tiotropiumliromid, Oxitropiumbromid, Pirenzepin oder Telenzepin;
- ein Modulator der Chemokin Rezeptor Funktion (wie beispielsweise ein CCR1 Rezeptor Antagonist); oder
- ein Inhibitor der p38 Kinase Funktion.

Für einen anderen Gegenstand der vorliegenden Erfindung wird eine solche Kombination mit einem GR Agonist der Formel Ia oder einem pharmazeutisch akzeptablen Salz davon zur Behandlung von COPD, Asthma oder allergischer Rhinitis eingesetzt und kann durch Inhalation oder oral verabreicht werden in Kombination mit Xanthin (wie beispielsweise Aminophyllin oder Theophyllin), die ebenfalls durch Inhalation oder oral verabreicht werden können.

### Experimenteller Teil

### Beispiel 1

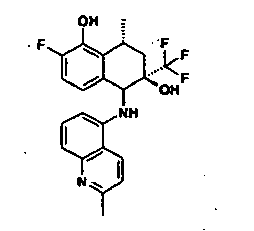

### (5α,6α,8β)-2-Fluor-8-methyl-5-[(2-methylchinolin-5-yl)amino]-6-(trifluormethyl)-5,6,7,8-tetrahydronaphthalin-1,6-diol

### 4-(3-Fluor-2-methoxyphenyl)-2-hydroxy-2-(trifluormethyl)pentanal:

41.8 g (639 mmol) Zinkstaub und 874 mg (3.1 mmol) Blei(II)-chlorid werden in 557 ml THF suspendiert und bei Raumtemperatur werden 39 ml (556 mmol) Dibrommethan zugegeben. Man rührt weitere 30 Minuten und tropft bei 0°C 68.8 ml (68.8 mmol) einer 1 M Titan(IV)-chlorid Lösung in Dichlormethan zu. Das Kühlbad wird entfernt und nach 30 Minuten bei Raumtemperatur werden 13.6 g (80.8 mmol) 1-(3-Fluor-2-methoxyphenyl)ethan-1-on *(*Chem. Commun. 2000, 14, 1323-4) in 139 ml THF zugetropft. Man rührt weitere 1.5 Stunden bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Isopropylether 0-5%) gereinigt und man erhält 9.5 g 2-Fluor-6-(1-methylenethyl)anisol.

Zu 1.56 g (5.7 mmol) 1,1'-Bi-2-naphthol werden 5.7 ml-(2.85 mmol) einer 0.5 M Titantetraisopropylat Lösung in Toluol gegeben und man rührt die rote Lösung für 2 Stunden bei Raumtemperatur. 9.5 g (57.2 mmol) 2-Fluor-6-(1-methylenethyl)anisol und 12.5 ml (95 mmol) Ethyltrifluorpyruvat werden zugegeben und man erhitzt die Mischung über 18 Stunden auf 140°C. Nach dem Abkühlen wird sofort säulenchromatographisch an Kieselgel (Hexan / Ethylacetat 0-5%) gereinigt und man erhält 8.9 g 4-(3-Fluor-2-methoxyphenyl)-2-hydroxy-2-(trifluormethyl)pent-4-ensäure-ethylester. 8.9 g (26.5 mmol) 4-(3-Fluor-2-methoxyphenyl)-2-hydroxy-2-(triftuormethyl)-pent-4-ensäure-ethylester werden in 200 ml Methanol und 2 ml Essigsäure gelöst und 890 mg Palladium auf Kohle (10%ig) werden zugegeben. Die Suspension wird 1,5 Stunden unter einer Wasserstoff Atmosphäre bei Normaldruck geschüttelt bis zu einer Wasserstoffaufnahme von 560ml. Die Mischung wird durch Celite filtriert wobei gründlich mit Ethylacetat nachgewaschen wird. Nach dem Entfernen des Lösungsmittels erhält man 8.6 g 4-(3-Fluor-2-methoxyphenyl)-2-hydroxy-2-(trifluormethyl)-pentansäure-ethylester. 8.6 g (25.4 mmol) 4-(3-Fluor-2-methoxyphenyl)-2-hydroxy-2-(trifluormethyl)-pentansäure-ethylester werden in 350 ml Diethylether auf

-30°C gekühlt und über 15 Minuten werden portionsweise 1.7 g (44.7 mmol) Lithiumaluminiumhydrid fest zugegeben. Man rührt 1.5 Stunden, wobei die Temperatur auf -45°C ansteigt, tropft dann nacheinander Ethylacetat und Wasser zu und rührt eine weitere Stunde bis sich ein gut filtrierbarer Niederschlag gebildet hat. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Diisopropylether 0-15%) liefert 3.1 g (*2R**,*4R**-4-(3-Fluor-2-methoxy-phenyl)-2-hydroxy-2-(trifluormethyl)-pentanal
¹H-NMR (300 MHz, CDCl₃); δ = 1.13 (d, 3H), 2.25 (dd, 1H), 2.58 (dd, 1H), 3.33 (qdd,1H), 3.92 (s, 3H), 3.98 (s, 1H), 6.92-6.99 (m, 3H), 9.12 (s, 1H),
0.72 g (*2R**,*4S**-4-(3-Fluor-2-methoxy-phenyl)-2-hydroxy-2-(trifluoromethyl)-pentanal ¹H-NMR (300 MHz, CDCl₃); δ= 1.21 (d, 3H), 2.33 (dd': 1H), 2.39 (dd, 1H), 3.30 (qdd, 1H), 3.74 (s, 1H) 3.94 (s, 3H), 6.90-6.99 (m, 3H), 9.71 (s, 1H) und 2.0 g Alkohol.

175 mg (0.60 mmol) (*2R**,*4R**)-4-(3-Fluor-2-methoxy-pheny)-2-hydroxy-2-(trifluoromethyl)-pentanal, 103 mg (0.63 mmol) 5-Amino-2-methylchinolin und 0.3 ml Titantetraethylat werden in 20 ml Toluol 2 h bei 100 °C gerührt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 230 mg (*2R**,*4R**)-4-(3-Fluor-2-methoxy-phenyl)-1-[(2-methychinolin-5yl)imino]-2-(trifluormethyl)pentan-2-ol als Rohprodukt. Zu 230 mg rohem Imin in 12 ml CH₂Cl₂ werden bei -30°C 6 mL (6 mmol) einer 1 M Bortribromid-Lösung getropft. Man lässt auf Raumtemperatur erwärmen und rührt 2 Stunden. Der Ansatz wird mit ges. NaHCO₃ Lösung versetzt, die Phasen werden getrennt, die wäßrige Phase mit CH₂Cl₂ extrahiert, die vereinigten organischen Phasen getrocknet (Na₂SO₄) und im Vakuum eingeengt. Säulenchromatographie an Kieselgel (Hexan/Essigester 0-75%) liefern 145 mg Produkt.
¹H-NMR (300 MHz, CDCl₃); δ = 1.39 (d, 3H), 1.82 (dd, 1H), 2.39 (dd, 1H), 2.64 (s,3H), 3.40 (m, 1H), 4.95 (s, 1H), 6.60 (s, 1H). 6.75 (m, 2H), 7.17 (d, 1H). 7.29 (d, 1H), T.45 (t, 1H), 8.20 (d, 1H).

### Beispiel 2

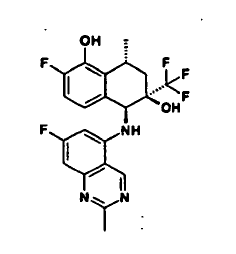

### (5α,6α,8β)-2-Fluor-5-[(7-fluor-2-methylchinazolin-5-yl)amino]-8-methyl-6-(trifluormethyl)-5,6,7,8-tetrahydronaphthalin-1,6-diol

Analog Beispiel 1 werden 250 mg (0.85 mmol) (*2R**,*4R**-4-(3-Fluor-2-methoxyphenyl)-2-hydroxy-2-(trifluormethyl)pentanal, 185 mg (1.05 mmol) 5-Amino-7-fluor-2-methylchinazolin und 0.4 ml Titantetraethylat zu (*2R**,*4R**)-4-(3-Fluor-2-methoxy-phenyl)-1-[(7-fluor-2-methychinazolin-5yl)imino]-2-(trifluoromethyl)-pentan-2-ol umgesetzt. 430 mg so erhaltenes rohes Imin werden analog Beispiel 1 bei -30°C mit 8 mL (8 mmol) 1 M Bortribromid-Lösung zum gewünschten Produkt zyklisiert. Säulenchromatographie an Kieselgel (Hexan/Essigester 0-75%) liefern 67 mg Produkt.
¹H-NMR (300 MHz, CO₃OD); δ = 1.44 (d, 3H), 1.88 (dd, 1H), 2.43 (dd, 1H), 2.74 (s, 3H), 3.40 (qdd, 1H), 5.25 (s, 1H), 6.69 (d, 1H), 6.70 (dd, 1H), 6.7.4 (d, 1H), 6.85 (dd, 1H), 9.49 (s, 1H).

### Beispiel 2A/2B

(*5*α,*6*α,*8*β)-2-Fluor-5-[(7-fluor-2-methylchinazolin-5-yl)amino]-8-methy)-6-(trifluormethyl)-5,6,7,8-tetrahydronaphthalin-1,6-diol wird mittels präparativer chiraler HPLC (Chiracel OD 5µ) in die enanantiomerenreinen Verbindungen gespalten:
(+)-Enantiomer: analytische HPLC: Rₜ = 5.5 min (Chiralcel OD 5µ, 250x4.6 mm, Hexan / Ethanol 5 =>50%(20'), 1 ml/min Fluß)
(-)-Enantiomer: analytische HPLC: Rₜ = 8.7 min (Chiralcel OD 5µ, 250x4.6 mm, Hexan/Ethanol 5=>50%(20'), 1 ml/min Fluß)

### Beispiel 3

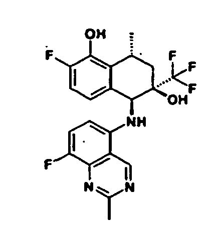

### (5α,6α,8β)-2-Fluor-5-[(8-fluor-2-methylchinazolin-5-yl)amino]8-methyl-6-(trifluormethyl)-5,6,7,8-tetrahydronaphthalin-1,6-diol

Analog Beispiel 1 werden 150 mg (0.5 mmol) (*2R**,*4R**-4-(3-Fluor-2-methoxyphenyl)-2-hydroxy-2-(trifluormethyl)pentanal, 90 mg (0.5 mmol) 5-Amino-8-fluor-2-methylchinazolin und 0.2 ml Titantetraethylat zu (*2R**,*4R**)-4-(3-Fluor-2-methoxy-phenyl)-1-[(8-fluor-2-methychinazolin-5yl)imino]-2-(trifluormethyl)-pentan-2-ol umgesetzt. 230 mg so erhaltenes rohes Imin werden analog Beispiel 1 bei -30°C mit 4 mL (4 mmol) 1 M Bortribromid-Lösung zum gewünschten Produkt zyklisiert. Säulenchromatographie an Kieselgel (Hexan/Essigester 50%) liefern 42 mg Produkt.
¹H-NMR (300 MHz, CD₃OD); δ = 1.42 (d, 3H), 1.85 (dd, 1H), 2.42 (dd, 1H), 2.80 (s, 3H), 3.41 (qdd, 1H), 5.16 (s, 1H), 6.73 - 6.80 (m, 1H), 6.83 (dd; 1H), 7.50 (dd, 1H), 9.58 (s. 1H).

### Beispiel 4

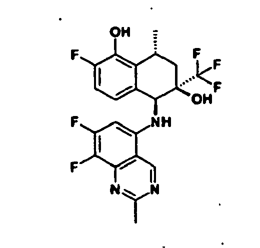

### (5α,6α,8β)-5-[(7,8-Difluor-2-methylchinazolin-5-yl)amino]-2-fluor-8-methyl-6-(trifluormethyl)-5,6,7,8-tetrahydronaphthalin-1,6-diol

Analog Beispiel 1 werden 150 mg (0.5 mmol) (*2R**,*4R**-4-(3-Fluor-2-methoxyphenyl)-2-hydroxy-2-(trifluormethyl)pentanal; 100 mg (0.51 mmol)5-Amino-7,8-difluor-2-methylchinazolin und 0.2 ml Titantetraethylat zu (*2R**,*4R**)-1-[(7,8-Difluor-2-methychinazolin-5yl)imino]-4-(3-Fluor-2-methoxy-phenyl)-2-(trifluoromethyl)-pentan-2-ol umgesetzt. 240 mg so erhaltenes rohes Imin werden analog Beispiel 1 bei -30°C mit 4 mL (4 mmol) 1 M Bortribromid-Lösung zum gewünschten Produkt zyklisiert. Säulenchromatographie an Kieselgel (Hexan/Essigester 50%) liefern 30 mg Produkt. ¹H-NMR (300 MHz, CD₃OD); δ = 1.44 (d, 3H), 1.87 (dd, 1H), 2.43 (dd, 1H), 2.79 (s, 3H), 3.40 (qdd, 1H), 5.20 (s, 1H), 6.71 (dd, 1H), 6.77 (dd, 1H), 6.85 (dd, 1H), 9.52 (s, 1H).

### Beispiel 5

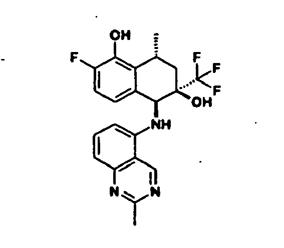

### (5α,6α,8β)-2-Fluor-8-methyl-5-[-2-methylchinazolin-5-yl)amino]-6-(trifluormethyl)-5,6,7,8-tetrahydronaphthalin-1,6-diol

Analog Beispiel 1 werden 135 mg (0.46 mmol) (*2R**,*4R**)-4-(3-Fluor-2-methoxyphenyl)-2-hydroxy-2-(trifluormethyl)pentanal, 100 mg (0.63 mmol) 5-Amino-2-methylchinazolin und 0.23 ml Titantetraethylat zu (*2R**,*4R**)-4-(3-Fluor-2-methoxy-phenyl)-1-[(2-methychinazolin-5yl)imino]-2-(trifluormethyl)pentan-2-ol umgesetzt. 260 mg so erhaltenes rohes Imin werden analog Beispiel 1 bei - 30°C mit 5 mL (5 mmol) 1 M Bortribromid-Lösung zum gewünschten Produkt zyklisiert. Säulenchromatographie an Kieselgel (Hexan/Essigester 0-75%) liefern 56 mg Produkt.
¹H-NMR (300 MHz, CD₃OD); δ = 1.44 (d, 3H), 1.87 (dd, 1H), 2.44 (dd, 1H), 2.77 (s, 3H), 3.41 (qdd, 1H), 5.23 (s, 1H), 6.73 (dd, 1H), 6.83 (dd, 1H), 6.87 (d, 1H), 7.14 (d, 1H), 7.74 (t, 1H), 9.56 (s, 1H).

### Beispiel 6

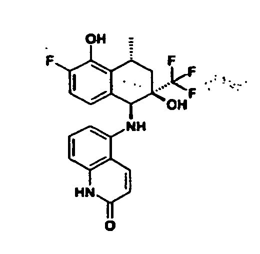

### 5-{[(5α,6α,8β)-1,6-Dihydroxy-2-fluor-8-methyl-6-(trifluormethyl)-5,6,7,8-tetrahydronaphthalin-5-yl]amino}chinolin-2(1H)-on

Analog Beispiel 1 werden 250 mg (0.46 mmol) (*2R**,*4R**)-4-(3-Fluor-2-methoxyphenyl)-2-hydroxy-2-(trifluormethyl)pentanal, 132 mg (0.63 mmol) 5-Aminochinolon und 0.4 ml Titantetraethylat zu 5-{[(*2R**,*4R**-4-(3-Fluor-2-methoxyphenyl)-2-hydroxy-2-(trifluormethyl)pentyliden]amino}chinolin-2(1*H*)-on umgesetzt. 64 mg säulenchromatographisch gereinigtes Imin (Kieselgel, Hexan/Essigester 0-75%) werden analog Beispiel 1 bei -30°C mit 1.5 mL (1.5 mmol) 1 M Bortribromid-Lösung zum gewünschten Produkt zyklisiert. Säulenchromatographie an Kieselgel (Hexan/Essigester 0-75%) liefern 52 mg Produkt.
¹H-NMR (300 MHz, CD₃oD); δ = 1.42 (d, 3H), 1.84 (dd, 1H), 2.42 (dd, 1H), 3.40 (qdd, 1H), 5.08 (s, 1H), 6.47 (d, 1H), 6.54 (d, 1H), 6.65 (d, 1H), 6.71 (dd, 1H), 6.83 (dd, 1H), 7.32 (t, 1H), 8.17 (d, 1H).

### Beispiel 7

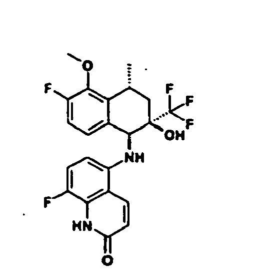

### 8-Fluor-5-{[(1α,2α,4β)-6-fluor-2-hydroxy-5-methoxy-4-methyl-2-(trifluormethyl)-1,2,3,4-tetrahydronaphthalin-1-yl]amino}-chinolin-2(1H)-on 5-Amino-8-fluorchinolin-2(1H)-on:

5,8-Difluorchinolin-2(1H)-on und 1.18 g (8.2 mmol) Cu₂O werden in 620 ml Ethylenglycol unter 8 bar mit gasf. NH₃ versetzt. Das Reaktionsgemisch wird für 19 h auf 190°C erwärmt. Nach dem Abkühlen des Reaktionsgemisches und Entfernen des Lösungsmittels wird das Rohprodukt säulenchromatographisch (Kieselgel, Hexan; CH₂Cl₂ / MeOH 0-5%) aufgereinigt. Man erhält 1.03 g 5-Amino-8-fluorchinolin-2(1*H*)-on als hellgelben Feststoff.
¹H-NMR (300 MHz, DMSO-d6); δ = 5.58 (s, 2H), 6.23 (dd, 1H), 6.31 (d, 1H), 7.03 (dd, 1H), 8.05 (dd, 1H), 11.28 (s, 1H).

Analog Beispiel 1 werden 300 mg (1.01 mmol) (*2R**,*4R**)-4-(3-Fluor-2-methoxyphenyl)-2-hydroxy-2-(trifluormethyl)pentanal, 180 mg (1.01 mmol) 5-Amino-8-fluorchinolon und 0.48 ml Titantetraisopropylat in 9 ml Xyxlol zu 8-Fluor-5-{[(*2R**,*4R**)-4-(3-Fluor-2-methoxyphenyl)-2-hydroxy-2-(trifluormethyl)-pentyliden]iamino}chinolin-2(1*H*)-on umgesetzt. 80 mg säulenchromatographisch gereinigtes Imin (Kieselgel, Hexan; CH₂Cl₂ / i-PrOH 0-5%) werden analog Beispiel 1 bei -40°C mit 1.7 ml (1.5 mmol) 1 M Bortribromid-Lösung zum gewünschten Produkt und zum ethergespaltenen Beispiel 8 zyklisiert.
¹H-NMR (300 MHz, CD₃OD); δ = 1.38 (d, 3H), 1.85 (dd, 1H), 2.39 (dd, 1H), 3.38 (m, 1H), 3.88 (s, 3H), 5.03 (s, 1H), 6.43 (dd, 1H), 6.51 (d, 1H), 6.80 - 7.05 (m, 2H), 7.15 (dd, 1H), 8.14 (dd, 1H).

### Beispiel 8

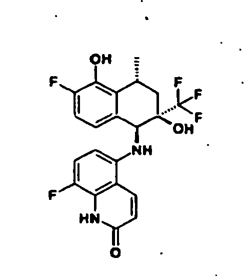

### 5-{[(1α,2α,4β)-6-fluor-2,5-dihydroxy-4-methyl-2-(trifluormethyl)-1,2,3,4-tetrahydronaphthalin-1-yl]amino}-8-fluorchinolin-2(1H)-on

Wird nach chromatographischer Reinigung aus Beispiel 7 erhalten. ¹H-NMR (300 MHz, CD₃OD); δ = 1.40 (d, 3H), 1.83 (dd, 1H), 2.40 (dd, 1H), 3.38 (m, 1H), 5.03 (s, 1H), 6.45 (dd, 1H), 6.48 (d, 1H) 6.49 (dd, 1H), 6.70 (dd, 1H), 6.82 (t, 1H), 7.07 (dd, 1H), 7.17 (dd, 1H), 8.13 (dd, 1H).

### Beispiel 8A/8B

5-{[(*1*α,*2*α,*4*β)-6-fluor-2,5-dihydroxy-4-methyl-2-(trifluormethyl)-1,2,3,4-tetrahydronaphthalin-1-yl]amino}-8-fluorchinolin-2(1H)-on wird mittels präparativer chiraler HPLC (Chiracel OD 20µ) in die enanantiomerenreinen Verbindungen gespalten:
(-)-Enantiomer: analytische HPLC: Rₜ = 7.71 min (Chirapak AD-H 5µ, 150x4.6 mm, Hexan / Ethanol 5% → 50% (20'), 1 ml/min Fluß, 25°C).
(+)-Enantiomer (ZK 376768): analytische HPLC: Rₜ = 9.53 min [Chirapak AD-H 5µ, 150x4.6 mm, Hexan / Ethanol 5% → 50% (20'), 1 ml/min Fluß, 25°C).

### Beispiel 9

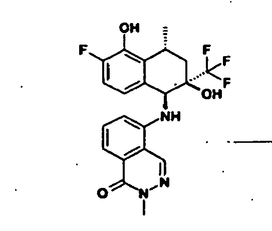

### 5-{[(1α,2α,4β)-6-fluor-2,5-dihydroxy-4-methyl-2-(trifluormethyl)-1,2,3,4-tetrahydronaphthalin-1-yl]amino}-2-methylphthalazin-1-on

Analog Beispiel 1 werden 295 mg (1.0 mmol) (*2R*,4R**)-4-(3-Fluor-2-methoxyphenyl)-2-hydroxy-2-(trifluormethyl)pentanal, 217 mg (1.0 mmol) 5-Amino-2-methylphthalazin-1-on und 0.53 ml Titantetraethylat zu 5-{[(*2R*,4R**)-4-(3-Fluor-2-methoxyphenyl)-2-hydroxy-2-(trifluoromethyl)pentyliden]amino}2-methylphthalazin-1-on umgesetzt. 590 mg so erhaltenes rohes Imin werden analog Beispiel 1 bei -30°C mit 10 mL (10 mmol) 1 M Bortribromid-Lösung zum gewünschten Produkt zyklisiert. Säulenchromatographie an Kieselgel (Hexan/Essigester 0-60%) liefern 204 mg Produkt.
¹H-NMR (300 MHz, CD₃OD); δ = 1.43 (d, 3H), 1.86 (dd, 1H), 2.43 (dd, 1H), 3.40 (qdd, 1H), 3.79 (s, 3H), 5.18 (s, 1H), 6.71 (dd, 1H), 6.84 (dd, 1H), 7.16 (d, 1H), 7.59 (t, 1H), 7.60 (d, 1H), 8.50 (s, 1H).

### Beispiel 14

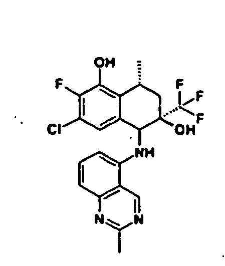

### (5α,6α,8β)-3-Chlor-2-fluor-8-methyl-5-[(2-methylchinazolin-5-yl)aminol--6-(trifluormethyl)-5,6,7,8-tetrahydronaphthalin-1,6-diol

### 4-(4-Chlor-3-fluor-2-methoxyphenyl)-2-hydroxy-2-(trifluoromethyl)pentanal:

Zu 10 g (68.2 mmol) 3-Chlor-2-fluorphenol in 68 ml Dichlormethan und 7.7 ml (98.5 mmol) Pyridin werden bei 0°C ml 5.1 ml (71.6 mmol) Acetylchlorid getropft. Man rüht die Mischung eine Stunde lang und gibt 100 ml einer 1 M Salzsäure zu. Es wird mit Dichlormethan extrahiert und mit Wasser gewaschen. Nach dem Trocknen über Natriumsulfat und dem Entfernen des Lösungsmittels im Vakuum erhält man quantitativ 13 g Essigsäure-3-chlor-2-fluorphenyl-ester. 13 g (68.2 mmol) Essigsäure-3-chlor-2-fluorphenyl-ester in 6.9 ml 1,2-Dichlorbenzol werden unter Eiskühlung zu 9.2 g (68.9 mmol) Aluminiumtrichlorid in 6.9 ml 1,2-Dichlorbenzol getropft und die Mischung wird im Anschluss 6 Stunden lang bei 100°C gerührt. Man lässt abkühlen, verdünnt mit Dichlormethan und giesst vorsichtig auf eine Mischung von 4 M Salzsäure und Eis. Die Phasen werden getrennt, es wird mit Dichlormethan extrahiert, mit gesättigter Natriumchlorid Lösung gewaschen und über Natriumsulfat getrocknet. Das Rohprodukt wird säulenchromatographisch an Kieselgel (Hexan / Ethylacetat 10-50%) gereinigt und man erhält 11.4 g 1-(4-Chlor-3-fluor-2-hydroxyphenyl)ethan-1-on und 0.74g 1-(2-Chlor-3-fluor-4-hydroxyphenyl)ethan-1-on. 11.4 g (60.4 mmol) 1-(4-Chlor-3-fluor-2-hydroxyphenyl)ethan-1-on werden in 120 ml Aceton gelöst und es werden 15.5 g (112 mmol) Kaliumcarbonat und 6.9 ml (110 mmol) Methyliodid zugegeben. Die Mischung wird 7 Stunden bei 70°C gerührt und das Lösungsmittel im Anschluss zu einem großen Teil entfernt. Man gießt den Rückstand in Wasser und extrahiert mit Methy-*t*-butylether. Es wird mit gesättigter Ammoniumchlorid Lösung gewaschen, über Natriumsulfat getrocknet und nach dem Entfernen des Lösungsmittels im Vakuum erhält man 11.3 g 1-(4-Chlor-3-fluor-2-methoxyphenyl)ethan-1-on. 27.1 g (415 mmol) Zinkstaub und 640 mg (2.3 mmol) Blei(II)chlorid werden in 400 ml THF suspendiert und bei Raumtemperatur werden 26 ml(230 mmol) Dibrommethan zugegeben. Man rührt weitere 30 Minuten und tropft bei Eisbadkühlung 46.1 ml (46.1 mmol) einer 1 M Titan(IV)-chlorid Lösung in Dichlormethan zu. Nach 30 Minuten bei 5-10°C werden 9.3 g (46.1 mmol) 1-(4-Chlor-3-fluor-2-methoxyphenyl)ethan-1-on in 92 ml THF bei 5°C zugetropft. Man rührt weitere 15 Stunden bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Ethylacetat 10-40%) gereinigt und man erhält 2.68 g 3-Chlor-2-fluor-6-(1-methylenethyl)-anisol.

Zu 760mg (2.67 mmol) 1,1'-Bi-2-naphthol werden 2.68 ml (1.34 mmol) einer 0.5 M Titantetraisopropylat Lösung in Toluol gegeben und man rührt die rote Lösung für eine Stunde bei Raumtemperatur. 5.07 g (28.1 mmol) 3-Chlor-2-fluor-6-(1-methylenethyl)-anisol und 3.25 ml (26.7 mmol) Ethyltrifluorpyruvat werden zugegeben und man erhitzt die Mischung über 17 Stunden auf 140°C. Nach dem Abkühlen wird sofort säulenchromatographisch an Kieselgel (Pentan / Diethylether 25-40%)gereinigt und man erhält 1.47 g 4-(4-Chlor-3-fluor-2-methoxyphenyl)-2-hydroxy-2-(trifluormetyl)-pent-4-ensäure-ethylester. 1.47 g (3.97 mmol) 4-(4-Chlor-3-Fluor-2-methoxyphenyl)-2-hydroxy-2-(trifluormetyl)-pent-4-ensäure-ethylester werden in 40 ml Diethylether auf -15°C gekühlt und über 10 Minuten werden portionsweise 300 mg (7.9 mmol) Lithiumaluminiumhydrid fest zugegeben. Man rührt 1 Stunden wobei sich die Mischung bis auf 0°C erwärmt und gießt in gesättigte Ammoniumchlorid Lösung. Es wird gesättigte Weinsäure Lösung zugegeben und 30 Minuten gerührt. Die Phasen werden getrennt und es wird mehrfach mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Ethylacetat 0-50%) liefert 0.38 g 4-(4-Chlor-3-fluor-2-methoxyphenyl)-2-hydroxy-2-(trifluormetyl)-pent-4-enal und 0.15 g Alkohol. 0.27 g 4-(4-Chlor-3-fluor-2-methoxyphenyl)-2-hydroxy-2-(trifluormetyl)-pent-4-enal werden in 14 ml Methanol und 0.3 ml Essigsäure gelöst und 27 mg Palladium auf Kohle (10%ig) werden zugegeben. Die Suspension wird 2 Stunden unter einer Wasserstoff Atmosphäre bei Normaldruck geschüttelt bis zu einer Wasserstoffaufnahme von 46 ml. Die Mischung wird durch Celite filtriert wobei gründlich mit Ethylacetat nachgewaschen wird. Nach dem Entfernen des Lösungsmittels erhält man 027 g 4-(4-Chlor-3-fluor-2-methoxyphenyl)-2-hydroxy-2-(trifluormetyl)pentanal als Gemisch der Diastereomeren.
¹H-NMR (300 MHz, CDCl₃); δ = 1.18 (d, 1.5H), 1.29 (d, 1.5H), 2.23 (dd, 0.5H), 2.30 (dd, 0.5H), 2.38 (dd, 0.5H), 2.55 (dd, 0.5H), 3.24 (m, 1H), 3.95 (s, 3H), 6.84 (dd, 1H), 7.05 (dd, 1H), 9.18 (s, 0.5H), 9.73 (s, 0.5H).

100 mg (0.3 mmol) 4-(4-Chlor-3-fluor-2-methoxy-phenyl)-2-hydroxy-2-(trifluoromethyl)-pentanal, 48 mg (0.3 mmol) 5-Amino-2-methylchinazolin und 0.1 ml Titantetraethylat werden in 9 ml Toluol 2 h bei 100 °C gerührt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 130 mg 4-(3-Fluor-2-methoxy-phenyl)-1-[(2-methychinolin-5yl)imino]-2-(trifluoromethyl)-pentan-2-ol als Rohprodukt. Zu 130 mg rohem Imin in 6 ml CH₂Cl₂ werden bei -30°C 3 mL (3 mmol) einer 1 M Bortribromid-Lösung getropft. Man lässt über 3 Stunden auf -5°C erwärmen. Der Ansatz wird mit ges. NaHCO₃ Lösung versetzt, die Phasen werden getrennt, die wäßrige Phase mit Ethylacetat extrahiert, die vereinigten organischen Phasen getrocknet (Na₂SO₄) und im Vakuum eingeengt. Säulenchromatographie an Kieselgel (Hexan/Essigester 0-75%) liefern 30 mg als Gemisch der Titelverbindung und (*5*α,*6*α,*8*α)-3-Chlor-2-fluor-8-methyl-5-[(2-methylchinazolin-5-yl)amino]-6-(trifluormethyl)-5,6,7,8-tetrahydronaphthalin-1,6-diol (Bsp.23). Präparative Dünnschichtchromatographie auf Aminphase (Merck) mit Ethylacetat/Methanol/Triethylamin 25:3:1 liefern 5 mg Produkt.
¹H-NMR (300 MHz, CDCl₃); δ = 1.43 (d, 3H), 1.79 (dd, 1H), 2.42 (dd, 1H), 2.82 (s, 3H), 3.44 (qdd, 1H), 5.02 (d, 1H), 6.62 (d, 1H), 6.71 (d, 1H), 6.87 (d, 1H), 7.14 (d, 1H), 7.72 (t, 1H), 9.55 (s, 1H).

### Beispiel 15

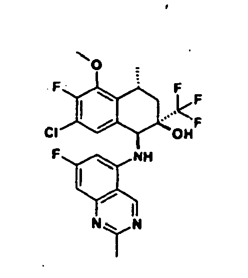

### (5α,6α,8β)-3-Chlor-2-fluor-5-[(7-fluor-2-methylchinazolin-5-yl)amino]-1-methoxy-8-methyl-6-(trifluormethyl)-5,6,7,8-tetrahydronaphthalin-6-ol

Analog Beispiel 14 werden 100 mg (0.3 mmol) 4-(4-Chlor-3-fluor-2-methoxyphenyl)-2-hydroxy-2-(trifluormethyl)pentanal, 54 mg (0.3 mmol) 5-Amino-7-fluor-2-methylchinazolin und 0.1 ml Titantetraethylat zu 4-(4-Chlor-3-fluor-2-methoxyphenyl)- 1-[(7-fluor-2-methylchinazolin-5yl)imino]-2-(trifluoromethyl)-pentan-2-ol umgesetzt. 150 mg rohes Imin werden analog Beispiel 14 bei -30°C mit 2.5 mL (2.5 mmol) 1 M Bortribromid-Lösung zum gewünschten Produkt zyklisiert. Säulenchromatographie an Kieselgel (Hexan/Essigester 50%) und anschließende präparative Dünnschichtchromatographie an Aminphase (Merck) mit Ethylacetat/Methanol/Triethylamin 25:3:1 liefern 3.4 mg Produkt und 3 mg der Dihydroxy Verbindung Bsp. 16.
¹H-NMR (300 MHz, CDCl₃); δ = 1.41 (d, 3H), 1.94 (dd, 1H), 2.45 (dd, 1H), 2.85 (s, 3H), 3.45 (ddq, 1H), 4.01 (s, 3H), 4.91 (d, 1H), 6.45 (d, 1H), 6.54 (br, 1H), 6.95 (d, 1H), 7.01 (d, 1H), 9.62 (s, 1H).

### Beispiel 16

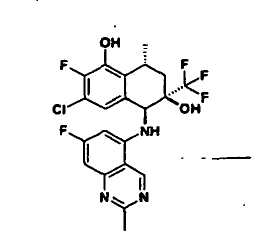

### (5α,6α,8β)-3-Chlor-2-fluor-5-[(7-fluor-2-methylchinazolin-5-yl)aminol-8-methyl-6-(trifluormethyl)-5,6,7,8-tetrahydronaphthalin-1,6-diol

Als Produkt aus Bsp. 15 nach chromatographischer Trennung erhalten:
¹H-NMR (300 MHz, CDCl₃); δ = 1.44 (d, 3H), 1.81 (dd, 1H), 2.49 (dd, 1H), 2.87 (s. 3H), 3.42 (qdd, 1H), 4.94 (d, 1H), 6.48 (d, 1H), 6.82 (d, 1H), 6.95 (d, 1H), 9.90 (s, 1H).

### Beispiel 17

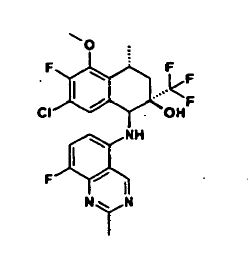

### (5α,6α,8β)-3-Chlor-2-fluor-5-[(8-fluor-2-methylchinazolin-5-yl)amino] 1-methoxy-8-methyl-6-(trifluormethyl)-5,6,7,8-tetrahydronaphthalin-6-ol

Analog Beispiel 14 werden 100 mg (0.3 mmol) 4-(4-Chlor-3-fluor-2-methoxyphenyl)-2-hydroxy-2-(trifluoromethyl)-pentanal, 53mg(0.3mmol)5-Amino-8-fluor-2-methylchinazolin und 0.1 ml Titantetraethylat zu 4-(4-Chlor-3-fluor-2-methoxy-phenyl)- 1-[(8-fluor-2-methylchinazolin-5yl)imino]-2-(trifluoromethyl)-pentan-2-ol umgesetzt. 140 mg rohes Imin werden analog Beispiel 14 bei -30°C mit 2.5 mL (2.5 mmol) 1 M Bortribromid-Lösung zum gewünschten Produkt zyklisiert. Säulenchromatographie an Kieselgel (Hexan/Essigester 50%) und anschließende präparative Dünnschicht-chromatographie an Aminphase (Merck) mit Ethylacetat/Methanol/Triethylamin 25:3:1 liefern 3 mg Produkt und 16 mg der Dihydroxy-Verbindung Bsp. 18.
¹H-NMR (300 MHz, CDCl₃); δ = 1.40 (d, 3H), 1.96 (dd, 1H), 2.42 (dd, 1H), 2.93 (s, 3H), 3.45 (ddq, 1H), 4.01 (s, 3H), 5.92 (br, 1H), 6.59 (dd, 1H), 7.10 (d, 1H), 7.50 (dd, 1H), 9.60 (s, 1H).

### Beispiel 18

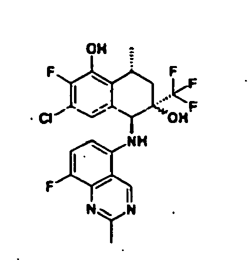

### (5α,6α,8β)-3-Chlor-2-fluor-5-[(8-fluor-2-methylchinazolin-5-yl)amino] 8-methyl-6-(trifluormethyl)-5,6,7,8-tetrahydronaphthalin-1,6-diol

Als Produkt aus Bsp. 17 nach chromatographischer Trennung erhalten:
¹H-NMR (300 MHz, CD₃OD); δ = 1.35 (d, 3H), 1.78 (dd, 1H), 2.36 (dd, 1H), 2.75 (s, 3H), 3.28 (qdd, 1H), 5.10 (s, 1H), 6.70 (dd, 1H), 6.71 (d, 1H), 7.46 (dd, 1H), 9.52 (s, 1H).

### Analog können hergestellt werden:

### Beispiel 19

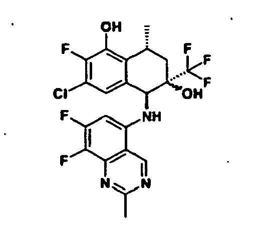

### (5α,6α,8β)-3-Chlor-5-[(7,8-difluor-2-methylchinazolin-5-yl)amino]-2-fluor-8-methyl-6-(trifluormethyl)-5,6,7,8-tetrahydronaphthalin-1,6-diol und

### Beispiel 20

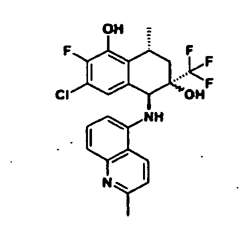

### (5α,6α,8β)-3-Chlor-2-fluor-8-methyl-5-(-2-methylchinolin-5-yl)amino]-6-(trifluormethyl)-5,6,7,8-tetrahydronaphthalin-1,6-diol

### Beispiel 21

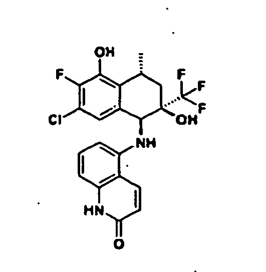

### 5-{[(1α,2α,4β)-7-Chlor-2,5-dihydroxy-6-fluor-4-methyl-2-(trifluormethyl)-1,2,3,4-tetrahydronaphthalin-1-yl]-amino}-chinolin-2(1H)-on

Analog Beispiel 14 werden 100 mg (0.3 mmol)4-(4-Chlor-3-fluor-2-methoxyphenyl)-2-hydroxy-2-(trifluoromethyl)-pentanal, 48 mg (0.3 mmol) 5-Aminochinolon und 0.1 ml Titantetraethylat zu 5-{[4-(4-Chlor-3-Fluor-2-methoxyphenyl)-2-hydoxy-2-(trifluormethyl)pentyliden]amino}chinolin-2(1*H*)-on umgesetzt. 130 mg rohes Imin werden analog Beispiel 14 bei -30°C mit 2.5 mL (2.5 mmol) 1 M Bortribromid-Lösung zum gewünschten Produkt zyklisiert. Säulenchromatographie an Kieselgel (Hexan/Essigester 50%) und anschließende präparative Dünnschicht-chromatographie an Aminphase (Merck) mit Ethylacetat/Methanol/Triethylamin 25:3:1 und präparative HPLC liefern 11 mg Produkt.
¹H-NMR (300 MHz, CD₃OD); δ = 1.38 (d, 3H), 1.84 (dd, 1H), 2.42 (dd, 1H), 3.36 (qdd, 1H), 5.09 (s, 1H), 6.48 (d, 1H), 6.54 (d, 1H), 6.68 (d, 1H), 6.70 (d, 1H), 7.34 (t, 1H), 8.19 (d, 1H).

### Beispiel 21A/21B 5-{[(1α,2α,4β)-7-Chlor-2,5-dihydroxy-6-fluor-4-methyl-2-(trifluormethyl)-1,2,3,4-tetrahydronaphthalin-1-yl]-aminol}-chinolin-2(1H)-on wird mittels präparativer chiraler HPLC (Chiracel OD 20µ) in die enanantiomerenreinen Verbindungen gespalten:

Enantiomer 1: analytische HPLC: Rₜ = 8.5 min (Chiralpak AD 5 µ, 250x4.6 mm, Hexan / Ethanol 5%=>95% in 20 min, 1 ml/min Fluß)
Enantiomer 2: analytische HPLC: Rₜ = 9.6 min (Chiralpak AD 5µ, 250x4.6 mm, Hexan / Ethanol 5% => 95% in 20 min, 1 ml/min Fluß)

### Analog kann hergestellt werden:

### Beispiel 22

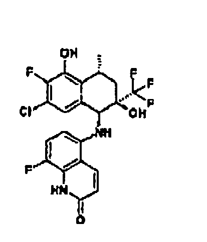

### 5-{[(1α,2α,4β)-7-Chlor-2,5-dihydroxy-6-fluor-4-methyl-2-(trifluormethyl)-1,2,3,4-tetrahydronapthalin-1-yl]-amino}-8-fluorchinolin-2(1H)-on

### Beispiel 24

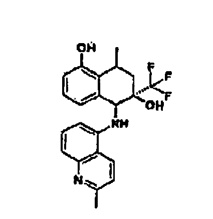

### (5α,6α,8α)-8-Methyl-5-[-2-methylchinolin-5-yl)amino]-6-(trifluormethyl)-5,6,7,8-tetrahydronaphthalin-1,6-diol (nicht erfindungsgemäß)

### 2-Hydroxy-4-(2-Methoxyphenyl)-2-(trifluormethyl)pentanal:

4-(3-Chlor-2-methoxyphenyl)-2-hydroxy-2-(trifluormethyl)pent-4-ensäureethylester kann analog Beispiel 14 aus Acetylchlorid und 2-Chlorphenol hergestellt werden. Die Reaktionsequenz mit Wasserstoff über Palladium auf Kohle und Lithiumaluminiumhydrid analog Beispiel 14 liefert in diesem Fall eine Mischung aus 2-Hydroxy-4-(2-Methoxyphenyl)-2-(trifluormethyl)pentanal und 4-(3-Chlor-2-Methoxyphenyl)-2-hydroxy-2-(trifluormethyl)pentanal, die nicht getrennt wurde.

Analog Beispiel 1 werden 160 mg des Gemisches aus 4-(3-Chlor-2-methoxyphenyl-2-hydroxy-2-(trifluormethyl)pentanal und 2-Hydroxy-4-(2-Methoxyphenyl)-2-(trifluormethyl)pentanal, 86 mg (0.55 mmol) 5-Amino-2-methylchinolin und 0.3 ml Titantetraethylat zu den entsprechenden Iminen umgesetzt. 230 mg rohes 1min werden analog Beispiel 1 bei -30°C mit 4 mL (4 mmol) 1 M Bortribromid-Lösung zu den gewünschten Produkten zyklisiert. Säulenchromatographie an Kieselgel (Hexan/Essigester 50%) und anschließend präparative Dünnschichtchromatographie an Kieselgel mit Hexan /2-Propanol 17% erlaubt die Trennung der einzelnen Produkte.
¹H-NMR (300 MHz, CDCl₃); δ = 1.61 (d, 3H), 2.24 (dd, 1H), 2.33 (dd, 1H), 2.74 (s, 3H), 3.43 (qdd, 1 H), 4.68 (br, 1H), 5.17 (br, 1H), 6.70 (d, 1H), 6.76 (d, 1H), 6.95 (m, 2H), 7.24 (d, 1H), 7.55 (m, 2H), 8.07 (d, 1H).

### Beispiel 25

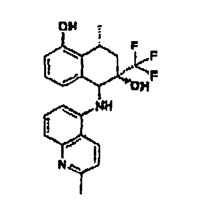

### (5α,6α,8β)-8-Methyl-5-[-2-methylchinolin-5-yl)amino]-6-(trifluormethyl)-5,6,7,8-tetrahydronaphthalin-1,6-diol

Als Produkt aus Bsp. 24 nach chromatographischer Trennung erhalten: ¹H-NMR (300 MHz, CDCl₃); δ = 1.51 (d, 3H), 1.98 (dd, 1H), 2.48 (dd, 1H), 2.77 (s, 3H), 3.47 (m, 1H), 4.69 (d, 1H), 5.17 (d, 1H), 6.71 (d, 1H), 6.74 (d, 1H), 6.94 (m, 2H), 7.25 (d, 1H), 7.56 (m, 2H), 8.12 (d, 1H).

### Belspiel 26

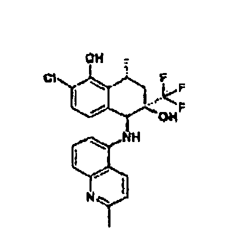

### (5α,6α,8β)-2-Chlor-8-Ethyl-2-fluoro-5-[(2-methylquinazolin-5-yl)amino]-6-(trifluoromethyl)-5,6,7,8-tetrahydronaphthalene-1,6-diol

### Als Produkt aus Bsp. 24 nach chromatographischer Trennung erhalten:

¹H-NMR (300 MHz, CDCl₃); δ 1.47 (d, 3H), 2.00 (dd, 1H), 2.47 (dd, 1H), 2.73 (s, 3H), 3.45 (m, 1H), 5.03 (d, 1H), 5.82 (br, 1H), 6.70 (d, 1H), 6.90 (d, 1H), 7.13 (d, 1H), 7.22 (d. 1H), 7.45 (d, 1H), 7.55 (t, 1H), 8.06 (d, 1H).

### Beispiel 27

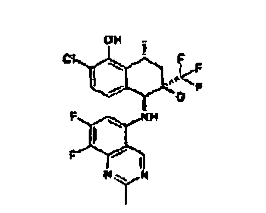

### (5α,6α,8β)-8-Methyl-5-[2-methylchinolin-5-yl)amino]-6-trifluormethyl)-5,6,7,8-tetrahydronaphthalin-1,6-diol

Analog Beispiel 1 werden 100 mg des Gernisches aus 4-(3-Chlor-2-methoxyphenyl)-2-hydroxy-2-(trifluormethyl)pentanal und 2-Hydroxy-4-(2-Methoxyphenyl)2-(trifluormethyl)pentanal, 80 mg (0.42 mmol) 5-Amino-7,8-difluor-2-methylchinazolin und 0.3 ml Titantetraethylat zu den entsprechenden Iminen umgesetzt. 180 mg rohes Imin werden analog Beispiel 1 bei -30°C mit 4 mL (3 mmol) 1 M Bortribromid-Lösung zu den gewünschten Produkten zyklistert. Säulenchromatographie an Kieseigel (Hexan/Essigesler 50%) und anschließende präparative Dünnschichtchromatographie an Kieselgel mit Hexan / 2-Propanol 17% liefert 7 mg Produkt.
¹H-NMR (300 MHz, CDCl₃); δ 1.45 (d, 3H), 1.94 (dd, 1H), 2.45 (dd, 1H), 2.91 (s, 3H), 3.46 (m, 1H), 4.85 (d, 1H), 5.70 (d, 1H), 6.48 (dd, 1H), 6.83 (d, 1H), 7.09 (d, 1H), 9.23 (s, 1H).

### Analog kann hergestellt werden:

### Beispiel 28

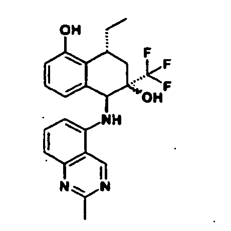

### (5α,6α,8β)-8-Ethyl-5-[2-methylchinazolin-5-yl)amino]-6-(trifluormethyl)-5,6,7,8-tetrahydronaphthalin-1,6-diol

### Beispiel 29

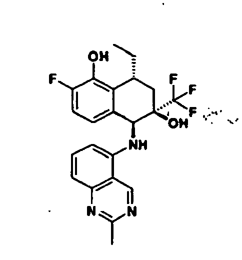

### (5α,6α,8β)-8-Ethyl-2-fluor-5-[(2-methylchinazolin-5-yl)amino]-6-(trifluormethyl)-5,6,7,8-tetrahydronaphthalin-1,6-diol

### 4-(3-Fluor-2-methoxyphenyl)-2-hydroxy-2-(trifluoromethyl)hexanal:

29.04 g (3-Fluor-2-methoxy-phenyl)-boronsäure, 25 g 2-Brom-1-buten und Tetrakis (triphenylphosphine) palladium werden in 174 ml Toluen und 17.4 ml 1-Propanol gelöst. Die Mischung wird über 5 Stunden in einem geschlossenen Gefäß auf 120°C erhitzt und nach dem Abkühlen in Wasser gegeben. Die wäßrige Phase wird dreimal mit Diethylether extrahiert, die vereinigten organischen Phasen werden mit gesättigter Natriumchlorid Lösung gewaschen und über Na₂SO₄ getrocknet Nach vorsichtigem entfernen des Lösungsmittels wird der Rückstand säulenchromatographisch an Kieselgel gereinigt (Hexan / Diethylether). Man erhält 16.6g (49.7%) 6-(But-1-en-2-yl)-2-fluoroanisol. Zu 4.0 g (22.2 mmol) 6-(But-1-en-2-yl)-2-fluoroanisol and 2.8 g Molekularsieb in 5.85 ml (44.4 mmol) Ethyltrifluorpyruvat werden bei 0°C über 30 Minuten 1005 mg (1.1 mmol) [Cu(S,S)-bisphenyl-oxazoline)(H₂O)₂]((SbF₆)₂, in 56 ml Dichlormethan getropft. Man rührt die Reaktionsmischung 16 Stunden bei 0°C und reinigt die Reaktionsmischung mittels Säulenchomatographie an Kieselgel (Hexan / Ethylacetat). Man erhält 7.2 g (92.6%) des enantiomer angereicherten (R)-4-(3-Fluor-2-methoxy-phenyl)-2-hydroxy-2-(trifluoromethyl)-hex-4-ensäureethylesters als E/Z Mischung (E/Z Verhältnis 2 :1, E : ca. 9%ee, Z : ca. 58%ee). 9.3 g (26.6 mmol) (E/Z)-4-(3-Fluor-2-methoxy-phenyl)-2-hydroxy-2-(trifluoromethyl)-hex-4-ensäureethylester werden in 300 ml Diethylether unter Argon gelöst und auf -15°C gekühlt. 2.02 g Lithiumaluminumhydride warden portionsweise über 30 Minuten als Feststoff zugegeben und es wird eine weitere Stunde gerüht wobei die Temperatur auf -5°C ansteigt. Nach weiteren 30 Minuten bei -5°C werden 4 ml Ethylacetate zugetropft und die Mischung wird weitere 10 Minuten gerührt. Man gießt auf eine Mischung von Eis und gesättigter Ammoniumchlorid Lösung und rührt heftig.Man trennt die Phasen und extrahiert mehrfach mit Ethylacetat und Diethylether. Die vereinten organischen Extrakte werden mit gesättigter Natriumchlorid Lösung gewaschen und über Na₂SO₄ getrocknet. Das Lösungsmittel wird abdestilliert und der Rückstand säulenchromatographisch an Kieselgel gereinigt (Hexan / Ethylacetat). Man erhält 5.9 g (72.6%) (E/Z)-4-(3-Fluor-2-methoxyphenyl)-2-hydroxy-2-trifluoromethyl-hex-4-enal und 2.0 g (E/Z)-4-(3-Fluoro-2-methoxy-phenyl)-2-trifluoromethyl-hex-4-en-1,2-diol. 1.51 g (4.9 mmol) (E/Z)-4-(3-Fluor-2-methoxy-phenyl)-2-hydroxy-2-(trifluoromethyl)hex-4-enal werden in 40 ml Methanol and 1.2 ml Essigsäure gelöst and 80 mg Palladium auf Kohle (10%ig) werden zugegeben. Die Suspension wird unter einer Wasserstoff Atmosphäre bei Normaldruck bis zur vollständigen Umsetzung geschüttelt. Die Mischung wird durch Celite filtriert wobei gründlich mit Ethylacetat nachgewaschen wird. Nach dem Entfernen des Lösungsmittels und säulenchromatographischer Trennung an Kieselgel (Hexan / Diisopropylether 10-25%) erhält man 530 mg enantiomer angereichertes (*2R*,4R**)-4-(3-Fluor-2-methoxy-phenyl)-2-hydroxy-2-(trifluormethyl)hexanal ¹H-NMR (300 MHz, CDCl₃); δ = 0.77 (d, 3H), 1.65 (m, 2H), 2.32 (dd, 1H), 2.55 (dd, 1H), 3.14 (m, 1H), 3.91 (s, 3H), 3.97 (s, 1H), 6.86 (dd, 1H), 6.95-6.99 (m, 2H), 8.99 (s, 1H) und 620 mg enantiomer angereichertes (*2R*,4S**)-4-(3-Fluor-2-methoxyphenyl)-2-hydroxy-2-(trifluoromethyl)hexanal
¹H-NMR (300 MHz, CDCl₃); δ = 0.73 (d, 3H), 1.60 (m, 2H), 2.35 (dd, 1H), 2.43 (dd, 1H), 2.96 (m, 1H), 3.63 (s, 1H), 3.92 (s, 3H), 6.84 (dd, 1H),6.93-6.99 (m, 2H), 9.67 (s, 1H).

113 mg (0.37 mmol) (*2R*,4R**)-4-(3-Fluor-2-methoxypheny)-2-hydroxy-2-(trifluoromethyl)hexanal, 103 mg (0.37 mmol) 5-Amino-2-methylchinazolin und 0.2 ml Titantetraethylat werden in 15 ml Toluol 1.5 h bei 100 °C gerührt. Nach dem Abkühlen gießt man auf Wasser und rührt heftig nach. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 178 mg (*2R*,4R**)-4-(3-Fluor-2-methoxy-phenyl)-1-[(2-methychinazolin-5yl)imino]-2-(trifluoromethyl)hexan-2-ol als Rohprodukt. Zu 178 mg rohem Imin in 20 ml CH₂Cl₂ werden bei -20°C 1.6 mL (1.6 mmol) einer 1 M Bortribromid-Lösung getropft. Man lässt auf Raumtemperatur erwärmen und rührt 1.5 Stunden. Man gießt in ges. NaHCO₃ Lösung, trennt die Phasen, extrahiert die wäßrige Phase mit CH₂Cl₂, trocknet die vereinigten organischen Phasen (Na₂SO₄) und engt im Vakuum ein. Säulenchromatographie an Kieselgel (Hexan/Essigester 50%) liefern 20 mg Produkt.
¹H-NMR (300MHz, CD₃OD); δ = 0.99 (t, 3H), 1.81 (ddq, 1H), 2.04 (m, 2H), 2.43 (dd, 1H), 2.82 (s, 3H), 3.43 (dddd, 1H), 5.14 (s, 1H), 6.73 (dd, 1H), 6.82 (m, 2H), 7.20 (d, 1H), 7.77 (t, 1H), 9.63 (s, 1H).

### Beispiel 29A/29B (5α,6α,8β)-8-Ethyl-2-fluor-5-[(2-methylchinazolin-5-yl)amino]-6-(trifluormethyl)-5,6,7,8-tetrahydronaphthalin-1,6-diol wird mittels präparativer chiraler HPLC (Chiralpak AD 5µ) in die enanantiomerenreinen Verbindungen gespalten:

(-)-Enantiomer: analytische HPLC: Rₜ = 2.58 min (Chiralpak AD 5µ, 250x4.6 mm, Hexan / Ethanol 25%, 1 ml/min Fluß)
(+)-Enantiomer analytische HPLC: Rₜ = 5.53 min (Chiralpak AD 5µ, 250x4.6 mm, Hexan / Ethanol 25%, 1 ml/min Fluß)

### Beispiel 30A

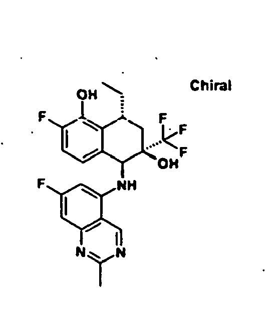

### (5S,6R,8R)-8-Ethyl-2-fluor-5-[(7-fluor-2-methylchinazolin-5-yl)amino]-6-(trifluoromethyl)-5,6,7,8-tetrahydronaphthalen-1,6-diol

### (R,R)-4-(3-Fluor-2-methoxyphenyl)-2-hydroxy-2-(tritluoromethyl)hexanal:

Zu 4.0 g (22.2 mmol) 6-(But-1-en-2-yl)-2-fluoroanisol and 2.8 g Molekularsieb in 5.85 ml (44.4 mmol) Ethyltrifluorpyruvat werden bei 0°C über 30 Minuten 1005 mg (1.1 mmol) [Cu(*R,R*)-2,2-bis(4,5-dihydro-4-*tert*-butyloxazolin-2-yl)propan (H2O)₂]((SbF₆)₂, Komplex (J. Org. Chem. 1998, 63, 4541-4544) in 56 ml Dichlormethan getropft. Man rührt die Reaktionsmischung 16 Stunden bei 0°C und reinigt die Reaktionsmischung mittels Säulenchomatographie an Kieselgel (Hexan / Ethylacetat). Man erhält 7.2 g (%) (*R*)-4-(3-Fluor-2-methoxyphenyl)-2-hydroxy-2-(trifluoromethyl)-hex-4-ensäureethylesters mit einem Enantiomerenüberschuß von größer 90% als E/Z Mischung. Analog Beispiel 29 wird der so erhaltene ungesättigte Ester mittels Lithiumaluminiumhydrid und Wasserstoff unter Palladiumkatalyse in die nahezu enantiomerenreinen Aldehyde überführt.

120 mg (0.4 mmol) (*R,R*)-4-(3-Fluor-2-methoxy-phenyl)-2-hydroxy-2-(trifluoromethyl)hexanal und 75 mg (0.42 mmol) 5-Amino-7-fluor-2-methylchinazolin werden in 10 ml Toluol gelöst und 0.1 ml (0.42 mmol) Titanethylat werden zugeben. Man erhitzt die Reaktionsmischung 2 Stunden auf 100°, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man trocknet über Natriumsulfat und entfernt das Lösungsmittel im Vakuum und erhält 205 mg (2*R*,4*R*)-4-(3-Fluor-2-methoxy-phenyl)-1-[(7-fluor-2-methychinolin-5yl)imino]-2-(trifluoromethyl)-hexan-2-ol als Rohprodukt. Das rohe Imin wird in 18 ml CH₂Cl₂ gelöst und auf -40°C gekühlt. 3.4 ml (3.4 mmol) einer 1 M BBr₃ Lösung in Dichlormethan werden über 5 min langsam zugetropft und man läßt über 1 Stunde auf 0°C erwärmen nach 30 Minuten bei 0°C wird auf eine Mischung aus ges. NaHCO₃ und Eis gegossen. Man extrahiert mehrfach mit Ethylacetat, wäscht mit gesättigter NaCl Lösung und trocknet über Na₂SO₄. Säulenchromatographische Reinigung an Kieselgel (150 ml) mit Ethylacetat ergeben 37 mg Produkt (analytische HPLC: Rₜ = 9.2 min (Chiralcel OD 5µ, 250x4.6 mm, Hexan / Ethanol 10%, 1 ml/min Fluß)) als (-)-Enantiomer. ¹H-NMR (300MHz, CD₃OD); δ = 0.96 (t, 3H), 1.76 (ddq, 1H), 2.02 (dd, 1H), 2.06 (ddq, 1H), 2.41 (dd, 1H), 2.77 (s, 3H), 3.39 (m, 1H), 5.13 (s, 1H), 6.58 (d, 1H), 6.73 (dd, 1H), 6.77 (d, 1H), 6.88 (dd, 1H), 9.51 (s, 1H).

### Beispiel 30B

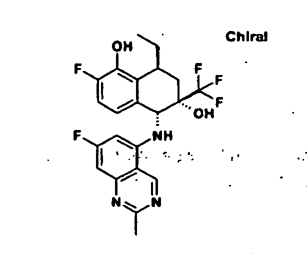

### (5R,6S,8S)-8-Ethyl-2-fluor-5-[(7-fluoro-2-methylquinazolin-5-yl)amino]-6-(trifluoromethyl)-5,6,7,8-tetrahydronaphthalene-1,6-diol

### 3-[1-(3-Fluor-2-methoxyphenyl)-cyclopropyl]-2-oxopropionsäureethylester.

Zu 26 g (180 mmol) 2,6-Difluoranisol und 14,6 ml (198 mmol) Cyclopropylcyanid in 500 mL Toluen werden bei 0°C über 40 min 396 ml einer.0,5 molaren (198 mmol) Lösung von Bis-(trimethylsilyl)-kaliumamid in Toluen getropft. Man rührt 18 Stunden bei Raumtemperatur und versetzt unter Eiskühlung mit Wasser und 1 M Schwefelsäure.

Die organische Phase wird abgetrennt und die wässrige Phase mehrfach mit Ethylacetat extrahiert. Es wird mit Sole gewaschen, mit Natriumsulfat getrocknet und im Vakuum eingeengt. Nach chromatographischer Reinigung an Kieselgel (Hexan / Ethylacetat 10%-20%) erhält man 12,7 g 1-(3-Fluor-2-methoxyphenyl)-cyclopropylnitril. 12.7 g (66.1 mmol) des Nitrils werden in Toluol bei - 78°C langsam mit 82.7 ml (99.2 mmol) Diisobutylaluminiumhydrid Lösung (20% in Toluol) versetzt und nach 3 h bei -78°C wurden 11.1 ml Isopropanol zugetropft. Man lässt auf -5°C erwärmen und gibt 150 ml einer 10%igen wässrigen Weinsäure Lösung zu. Nach Verdünnen mit Ether wird kräftig gerührt, die organische Phase wird abgetrennt und die wässrige Phase mehrfach mit Ethylacetat extrahiert. Es wird mit Sole gewaschen, mit Natriumsulfat getrocknet und im Vakuum eingeengt. Man erhält 11.8 g Aldehyd als gelbes Öl. Eine Lösung von 16.3 g (60.7 mmol) 2-Diethylphosphono-2-ethoxyessigsäure-ethylester in 60 ml Tetrahydrofuran wird unter Eiskühlung innerhalb von 20 Minuten mit 33.4 ml (66.8 mmol) einer 2 M Lösung von Lithiumdiisopropylamid in Tetrahydrofuran-Heptan-Toluol versetzt und 30 Minuten bei 0 °C gerührt. Innerhalb von 30 Minuten wird eine Lösung von 11.8 g (60.7 mmol) I in 61 ml Tetrahydrofuran bei 0°C zugetropft. Nach 20 Stunden bei RT wird Eiswasser zugegeben und mehrfach mit Ether und Ethylacetat extrahiert. Man wäscht mit gesättigter Ammoniumchlorid Lösung, trocknet über Natriumsulfat und engt ein. Das Rohprodukt wird mit 170 ml 2 M Natronlauge in 170 ml Ethanol über 15 Stunden bei Raumtemperatur verseift. Man erhält 13,9 g Säure, die mit 87 ml 2 N Schwefelsäure bei 90°C über 16 Stunden gerührt werden. Nach dem Abkühlen stellt man mit Kaliumcarbonat basisch, wäscht mit Ether und säuert mit Salzsäure an. Nach Extraktion mit Ethylacetat, Waschen mit gesättigter Natriumchlorid Lösung und Entfernen des Lösungsmittels werden 10.2 g der rohen Ketosäure erhalten. 10.2 g (40.6 mmol) *3-[1-(3-Fluor-2-methoxyphenyl)-cyclopropyl]-2-oxopropionsäure* und 4.5 ml (85.3 mmol) Schwefelsäure (96%ig) werden in 200 ml Ethanol eine Stunde unter Rückfluss erhitzt. Der Ansatz wird im Vakuum eingeengt, der Rückstand in Eiswasser gegeben und mit gesättigter Natriumhydrogencarbonat Lösung basisch gestellt. Es wird mehrfach mit Essigester extrahiert, mit gesättigter Natriumchlorid Lösung gewaschen, getrocknet (Natriumsulfat) und im Vakuum eingeengt. Nach chromatographischer Reinigung an Kieselgel (Hexan / Ethylacetat 20%) erhält man 9.6 g 3-[1-(3-Fluor-2-methoxyphenyl)-cyclopropyl]-2-oxopropionsäure-ethylester.
¹H-NMR (CDCl₃): δ = 0.90 (m, 4H), 1.29 (t, 3H), 3.09 (s, 2H), 3.99 (d, 3H), 4.20 (q, 2H), 6.87 (ddd, 1H), 6.95 (ddd, 1H), 7.07 (d, 1H).

### 3-[1-(3-Fluor-2-methoxyphenyl)-cyclopropyl]-2-hydroxy-2-(trifluormethyl)propanal

9.6 g (34.3 mmol) 3-[1-(3-Fluor-2-methoxyphenyl)-cyclopropyl]-2-oxopropionsäure-ethylester und 34.5 ml (233mmol) (Trifluormethyl)-trimethylsilan in 343 ml DMF werden mit 46.9 g Cäsiumcarbonat bei 0°C versetzt. Es wird 2 h bei 0°C gerührt und anschließend wird die Reaktionsmischung auf Wasser gegeben. Es wird mehrfach mit Essigester extrahiert, mit gesättigter Natriumchlorid Lösung gewaschen, mit Natriumsulfat getrocknet und im Vakuum eingeengt. Nach chromatographischer Reinigung an Kieselgel (Hexan / Ethylacetat 10%-40%) erhält man 10.4 g 3-[1-(3-Fluor-2-methoxyphenyl)-cyclopropyl]-2-hydroxy-2-(trifluormethyl)-propansäure-ethylester als gelbes Öl. Dieses Öl wird in 297 ml Diethylether bei 0°C mit 2.25 g (59.4 mmol) Lithiumaluminiumhydrid versetzt und noch 1 Stunden bei RT gerührt. Zum Ansatz werden bei 0°C vorsichtig 20 ml gesättigte Ammoniumchlorid Lösung gegeben und es wird 15 Minuten kräftig nachgerührt. Man extrahiert mehrfach mit Diethylether, wäscht mit gesättigter Natriumchlorid Lösung, trocknet mit Natriumsulfat und engt im Vakuum ein. Nach chromatographischer Reinigung an Kieselgel (Hexan / Ethylacetat 10%-50%) erhält man 5.6 g 3-[1-(3-Fluor-2-methoxyphenyl)-cyclopropyl]-2-(trifluormethyl)-propan -1,2-diol. Zu 5.6 g (18.1 mmol) Diol in 100 ml Dichlormethan und 61 ml DMSO gibt man 12.4 ml (89 mmol) Triethylamin und portionsweise über 10 min 11 g (70 mmol) Pyridin SO₃ Komplex. Man rührt über 3 Stunden und gibt gesättigte Ammoniumchlorid Lösung zu. Die Mischung wird weitere 15 min gerührt, die Phasen getrennt und es wird mit Dichlormethan extrahiert. Man wäscht mit Wasser und trocknet über Natriumsulfat. Das Lösungsmittel wird im Vakuum entfernt und nach chromatographischer Reinigung an Kieselgel (Hexan / Ethylacetat, 0-50%) erhält man 5.9 g Produkt.
¹H-NMR (CDCl₃): δ = 0.68-0.76 (m, 2H), 0.90-1.02 (m, 2H), 2.03 (d, 1H), 2.91 (d, 1H), 3.85 (s, 1H), 4.03 (s, 3H), 6.80 (d, 1H), 6.87 (ddd, 1H), 6.98 (dd, 1H), 9.26 (s, 1H).

Analog Beispiel 29 wird aus 800 mg (2.61 mmol) 3-[1-(3-Fluor-2-methoxyphenyl)-cyclopropyl]-2-hydroxy-2-(trifluormethyl)propanal und 500 mg (2.82 mmol) 5-Amino-7-fluor-2-methylchinazolin quantitativ 1-[(7-Fluor-2-methyl-chinazol-5-yl)imino]-3-[1-(3-fluor-2-methoxyphenyl)-cyclopropyl]-2-(trifluormethyl)propan-2-ol hergestellt. Die Behandlung mit 24 ml (24 mmol) BBr₃ Lösung und anschliessendes Refluxieren über 14 Stunden ergeben 55 mg (*1R,2S, Z*) 4-Ethylen 6-Fluor-1-[(7-fluor-2-methylchinazolin-5-yl)amino]-2-(trifluormethyl)-1,2,3,4-tetrahydronaphthalin-2,5-diol nach Chromatographie an Kieselgel (Hexan / Ethylacetat 25 bis 50%) und präparativer chiraler HPLC an Chiracel OD-H 5µ (analytische HPLC: Rₜ = 10.4 min (Chiralcel OD 10µ, 250x4.6 mm, Hexan / Ethanol 7%, 1 ml/min Fluß) als (+)-Enantiomer. ¹H-NMR (300MHz, CD₃0D); δ = 1.77 (d, 3H), 2.57 (d, 1H), 2,80 (s, 3H), 3.14 (d, 1H). 4.64 (s, 1H), 5.86 (q,1 1H), 6.26 (dd, 1H), 6.77- 6.97 (m, 2H), 7.02 (dd, 1H), 9.57 (s, 1H). 20 mg (*1R*, *2S*, *Z*) 4-Ethyliden-6-fluor-1-[(7-fluor-2-methylchinazolin-5-yl)amino]--2-(trifluormethyl)-1,2,3,4-tetrahydronaphthalin-2,5-diol werden bei RT unter N₂ in 1 ml Ethylacetat und 0,07 ml Et₃N gelöst und mit 2 mg Pd-C (10%) versetzt. Man schüttelt die Mischung 2h unter einer Wasserstoff Atmosphäre; (H₂-Aufnahme: 17ml) und filtriert,die Reaktionsmischung über Celite, wobei gründlich mit EE nachgespült wird. Man engt auf ca. 4 ml ein und rührt 3 Stunden mit 40 mg aktiviertem MnO₂.Die Reaktionsmischung wird über Celite filtriert, mit Ethylacetat nachgespült und eingeengt. Präparative Dünnschicht-chromatographie an Kieselgel (Hexan / 2-Propanol 15%) liefert 2 mg gewünschtes Produkt als gelbes ÖI.
¹H-NMR (300MHz, CD₃OD); δ = 0.96 (t, 3H), 1.76 (ddq, 1H). 2.02 (dd, 1H). 2.06 (ddq, 1H), 2.41 (dd, 1H), 2.77 (s, 3H), 3.39 (m, 1H), 5.13 (s, 1H), 6.58 (d,1H), 6.73 (dd, 1H), 6.77 (d, 1H), 6.88 (dd, 1H), 9.51 (s, 1H) und 1 mg des anderen Diastereomeren.

### Beispiel 31

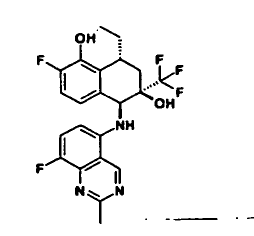

### (5α,6α,8β)-8-Ethyl-2-fluor-5-[(8-fluor-2-methylchinazolin-5-yl)aminol-6-(trifluormethyl)-5,6,7,8-tetrahydronaphthalin-1,6-diol

Analog Beispiel 29 werden 123 mg (0.40 mmol) (*2R**,*4R**)-4-(3-Fluor-2-methoxy-phenyl)-2-hydroxy-2-(trifluormethyl)hexanal, 72 mg (0.60 mmol) 5-Amino-8-fluor-2-methylchinazolin und 0.22 ml Titantetraethylat zu *(2R*,4R**)-4-(3-Fluor-2-methoxy-phenyl)-1-[(8-fluor-2-methylchinazolin-5yl)imino]-2-(trifluormethyl)hexan-2-ol umgesetzt. 170 mg rohes Imin werden analog Beispiel 29 bei -30°C mit 2.8 mL (2.8 mmol) 1 M Bortribromid-Lösung zum gewünschten Produkt zyklisiert. Nach chromatographischer Reinigung an Kieselgel (Dichlormethan / Ethylacetat 0-40%) erhält man 21 mg Produkt.
¹H-NMR (300MHz, CD₃OD); δ = 0.97 (t, 3H), 1.77 (m, 1H). 2.03 (dd, 1H), 2.04 (m, 1H). 2.42 (dd, 1H). 2.84 (s, 3H), 3.42 (dddd, 1H), 5.10 (s, 1H), 6.71 (dd,1H), 6.78 (dd, 1H), 6.90 (dd, 1H), 7.56 (dd, 1H), 9.63 (s, 1H).

### Beispiel 31A/31B (5α,6α,8β-8-Ethyl-2-fluor-5-[(8-fluor-2-methylchinazolin-5-yl)amino]-6-(trifluormethyl)-5,6,7,8-tetrahydronaphthalin-1,6-diol wird mittels präparativer chiraler HPLC (Chiralcel OD-H 5µ) in die enanantiomerenreinen Verbindungen gespalten:

(+)-Enantiomer: analytische HPLC: Rₜ = 4.13 min (Chiralcel OD-H 5µ, 250x4.6 mm, Hexan / Ethanol 10%, 1 ml/min Fluß)
(-)-Enantiomer: analytische HPLC: Rₜ = 10.28 min (Chiralcel OD-H 5µ, 250x4.6 mm, Hexan / Ethanol 10%, 1 ml/min Fluß)

### Beispiel 32

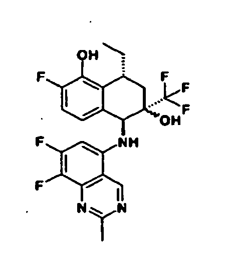

### (5α,6α,8β-5-[(7,8-Difluor-2-methylchinazolin-5-yl)aminol-8-ethyl-2-fluor-6-(trifluormethyl)-5,6.7,8-tetrahydronaphthalin-1,6-diol

Analog Beispiel 29 werden 138 mg (0.45 mmol) (*2R**,*4S**)-4-(3-Fluor-2-methoxyphenyl)-2-hydroxy-2-(trifluormethyl)-hextanal, 89 mg (0.45 mmol) 5-Amino-7,8-difluor-2-methylchinazolin und 0.24 ml Titantetraethylat zu (*2R**,*4S**)-1-[(7.8-Difluor-2-methylchinazolin-5yl)imino]-4-(3-fluor-2-methoxyphenyl)-2-(trifluormethyl)-pentan-2-ol umgesetzt. 210 mg rohes Imin werden analog Beispiel 29 bei -30°C mit 3.5 mL (3.5 mmol) 1 M Bortribromid-Lösung zum gewünschten Produkt zyklisiert. Nach chromatographischer Reinigung an Kieselgel (Dichlormethan / Ethylacetat 0-40%) erhält man 21 mg Produkt.
¹H-NMR (300MHz, CD₃OD); δ =0.97 (t, 3H), 1.78 (m, 1H), 2.04 (dd, 1H), 2.06 (m, 1H), 2.42 (dd, 1H), 2.84 (s, 3H), 3.39 (m, 1H), 5.13 (s, 1H), 6.72 (dd, 1H), 6.76 (dd, 1H), 6.90 (dd, 1H), 9.56 (s, 1H).

### Beispiel 33

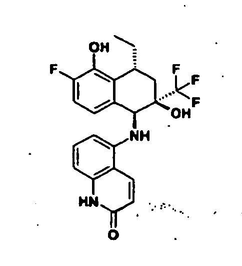

### 5-{[1α,2α,4β)-4-Ethyl-6-fluor-2,5-dihydroxy-2-(trifluormethyl)-1,2,3,4-tetrahydronaphthalin-1-yl]amino}-chinolin-2(1H)-on

Analog Beispiel 29 werden 300 mg (0.97 mmol) (*2R**,*4S**)-4-(3-Fluor-2-methoxyphenyl)-2-hydroxy-2-(trifluormethyl)-hextanal, 132 mg (0.97 mmol) 5-Amino-chinol-2(H1)-on und 0.44 ml Titantetraethylat zu 5-{[(*2R**,*4S**-4-(3-Fluor-2-methoxy-phenyl)-2-hydroxy-2-(trifluormethyl)hexyliden]amino}chinolin-2(1*H*)-on umgesetzt. 250 mg rohes Imin werden analog Beispiel 29 bei -20°C mit 2 mL (2 mmol) 1 M Bortribromid-Lösung zum gewünschten Produkt zyklisiert. Präparative Dünnschicht-chromatographie an Kieselgel (Hexan / 2-Propanol 17%) liefert 11.5 mg gewünschtes Produkt
¹H-NMR (300 MHz, CD₃OD); δ = 0.97 (t, 3H), 1.78 (m, 1H), 1.96-2.15 (m, 2H), 2.40 (dd, 1H), 3.42 (dddd, 1H), 5.03 (s, 1H), 6.49 (d, 1H), 6.53 (d, 1H), 6.70 (d, 1H), 6.75 (dd, 1H), 6.88 (dd, 1H), 7.36 (t, 1H), 8.23 (d, 1H).

### Beispiel 34

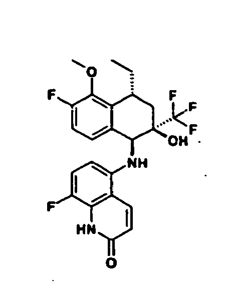

### 5-{ [(1α,2α,4β)-4-Ethyl-6-fluor-2-hydroxy-5-methoxy-2-(trifluormethyl)-1,2,3,4-tetrahydronaohthalin-1 -yl]amino}-8-fluorchinolin-2(1H)-on

Analog Beispiel 29 wird 4-(3-Fluor-2-methoxyphenyl)-2-hydroxy-2-(trifluormethyl)hexanal und 5-Amino-8-fluorchinolin-2(1H)-on zu 8-Fluor-5-{[4-(3-fluor-2-methoxyphenyl)-2-hydroxy-2-(trifluormethyl)hexyliden]amino}chinolin-2(1*H*)-on kondensiert. Die Umsetzung mit 1 M Bortribromid-Lösung liefert nach Säulenchromatographie an Kieselgel (Hexan/Ethylacetat 33-100%) und präparativer HPLC liefert sowohl das gewünschte Produkt als auch die Methylether gespaltenen Verbindungen Beispiel 35 und 44.
¹H-NMR (400 MHz, CD₃OD); δ = 0.92 (t, 3H), 1.70 (m, 1H), 1.86 (m, 1H), 2.02 (dd, 1H), 2.32 (dd, 1H), 3.65 (m, 1H), 3.90 (s, 3H), 4.94 (s, 1H), 6.33 (dd, 1H), 6.54 (d, 1H), 6.90 - 7.00 (m, 2H), 7.17 (dd, 1H), 8.17 (d, 1H).

### Beispiel 35

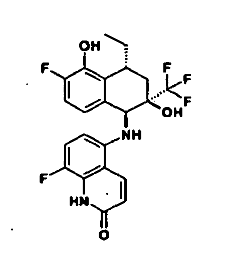

### 5-{[(1α,2α,4β)-4-Ethyl-6-fluor-2.5-dihydroxy-2-(trifluormethyl)-1,2,3,4-tetrahydronaphthalin-1-yl}aminol-8-fluorchinolin-2(1H)-on

Als Produkt aus Bsp. 34 nach chromatographischer Trennung erhalten:
¹H-NMR (400 MHz, CD₃OD); δ = 0.93 (t, 3H), 1.73 (m, 1H), 1.90 - 2.02 (m, 2H), 2.85 (dd, 1H), 3.60 (m, 1H), 4.92 (s, 1H), 6.34 (dd, 1H), 6.53 (d, 1H), 6.70 (dd, 1H), 6.84 (dd, 1H), 7.17 (dd, 1H), 8.17 (dd, 1H).

### Beispiel 35A/35B 5-{[(1α,2α,4β-4-Ethyl-6-fluor-2,5-dihydroxy-2-(trifluormethyl)-1,2,3,4-tetrahydronaphthalin-1-yl]amino}-8-fluorchinolin-2(1H)-on wird mittels präparativer chiraler HPLC (Chiralcel OD-H 5µ) in die enanantiomerenreinen Verbindungen gespalten:

(-)-Enantiomer: analytische HPLC: Rₜ = 7.29 min (Chiralpak AD-H 5µ, 250x4.6 mm, Hexan / Ethanol 5 => 50% (20'), 1 ml/min Fluß)
(+)-Enantiomer: analytische HPLC: Rₜ = 8.90 min (Chiralpak AD-H 5µ, 250x4.6 mm, Hexan / Ethanol 5 => 50% (20'), 1 ml/min Fluß)

### Beispiel 36

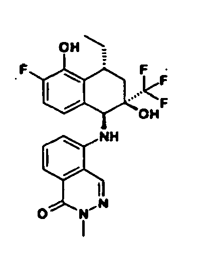

### 5-{[(1α,2α,4β)-4-Ethyl-6.fluor-2,5-dihydroxy-2-(trifluormethyl)-1,2,3,4-tetrahydronaphthalin-1-yl]amino}-2-methylphthalazin-1-on

Analog Beispiel 29 werden 265 mg (0.86 mmol) 4-(3-Fluor-2-methoxyphenyl)-2-hydroxy-2-(trifluormethyl)hexanal, 150 mg (0.86 mmol) 5-Amino-2-methylphthalazin-1-on und 0.42 ml Titantetraethylat zu 5-{[4-(3-Fluor-2-methoxyphenyl)-2-hydoxy-2-(trifluormethyl)hexyliden]amino}-2-methylphthalazin-1-on umgesetzt. 410 mg rohes Imin werden analog Beispiel 29 bei -20°C mit 3.5 mL (3.5 mmol) 1 M Bortribromid-Lösung zum gewünschten Produkt zyklisiert. Säulenchromatographie an Kieselgel (Hexan/Essigester 50%) und anschließende präparative Dünnschichtchromatographie an Kieselgel mit Dichlormethan / Methanol 9 : 1 liefern 10 mg Produkt und 8.6 mg der 8α Verbindung (Beispiel 45).
¹H-NMR (300 MHz, CD₃OD); δ = 0.85 (t, 3H), 1.66 (m, 1H), 1.90 (dd, 1H), 1.91 (m, 1H), 2.29 (dd, 1H), 3.29 (dddd, 1H), 3.72 (s,3H), 4.89 (s, 1H), 6.63 (dd, 1H), 6.77 (dd, 1H), 6.98 (t, 1H), 7.51 (m, 2H), 8.43 (s, 1H).

### Analog können hergestellt werden:

### Belsplel 37

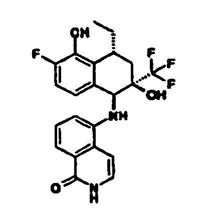

### 5-{[(1α,2α.4β)-4-Ethyl-6-fluor-2.5-dihydroxy-2-(trifluormethyl)-1,2,3,4-tetrahydronaphthalin-1-yl]namino}-isochinolin-(2H)-on

### und Beispiel 38

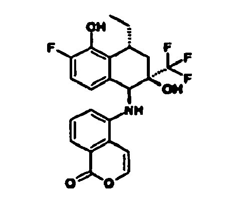

### 5-{[(1α,2α,4β)-4-Ethyl-6-fluor-2,5-dihydroxy-2-(trifluormethyl)-1,2,3,4-tetrahydranaphthalin-1-yl]amino}isochromen-1-on

### Beispiel 48

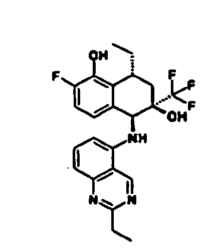

### (5α,6α,8β)-5-[(2-Ethylchinazolin-5-yl)amino]-2-ftuor-8-propyl-6-(trifluormethyl)-5.6.7.8-tetrahydronaohthalin-1.6-diol

Analog Beispiel 31 werden 112 mg (0.37 mmol) (4-(3-Fluor-2-methoxyphenyl)-2-hydroxy-2-(trifluormethyl)hexanal, 60 mg (0.37 mmol) 5-Amino-2-ethylchinazolin und 0.2 ml Titantetraethylat zu 1-[(2-Ethylchinazolin-5yl)imino]-4-(3-Fluor-2-methoxyphenyl)-2-(trifluormethyl)hexan-2-ol umgesetzt. 280 mg rohes Imin werden analog Beispiel 31 bei -20°C mit 2.4 mL (2.4 mmol) 1 M Bortribromid-Lösung zum gewünschten Produkt zyklisiert. Nach chromatographischer Reinigung an Kieselgel (Ethylacetat) erhält man 4 mg gewünschtes Produkt und 11 mg der 8a Verbindung (Beispiel 49).
¹H-NMR (300 MHz, CD₃OD); δ = 0.99 (t, 3H), 1.46 (t,3H), 1.80 (m, 1H), 2.04 (dd, 1H), 2.05 (m, 1H), 2.43 (dd, 1H), 3.08 (q, 2H), 3.42 (dddd, 1H), 5.17 (s, 1H), 6.77 (dd, 1H), 6.81 (d, 1H), 6.89 (dd, 1H), 7.22 (d, 1H), 7.78 (t, 1H), 9.64 (s, 1H).

### Beispiel 50

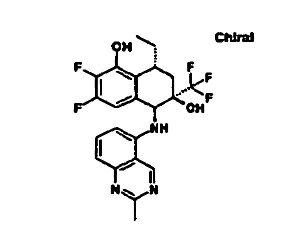

### (5S,6R,8R)-8-Ethyl-2,3-difluor-5-[(2-methylchinazolin-5-yl)amino]-6-(trifluormethyl)-5,6,7,8-tetrahydronaphthalin-1,6-diol

### 4-(3, 4-Difluor-2-methoxy-phenyl)-2-hydroxy-2-(trifluoromethyl)-hexanal:

Zu 20 g (153.7 mmol) 2,3-Diftuorphenol in 150 ml Dichlormethan und 17.5 ml Pyridin werden bei 0°C 14 ml (161 mmol) Propionsäurechlorid getropft. Man rührt die Mischung zwei Stunden lang und gibt 100 ml einer 2 M Salzsäure zu. Es wird mit Dichlormethan extrahiert und mit Wasser gewaschen. Nach dem Trocknen über Natriumsulfat und dem Entfernen des Lösungsmittels im Vakuum erhält man 30.1 g Propionsäure-2,3-difluorphenylester. 30.1 g (161 mmol) Propionsäure-2,3-difluorphenylester in 16 ml 1,2-Dichlorbenzol werden zu 21.5 g (161 mmol) Aluminiumtrichlorid in 16 ml 1,2-Dichlorbenzol getropft und die Mischung wird im Anschluss 6 Stunden lang bei 100°C gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit gesättigter Natriumchlorid Lösung gewaschen und über Natriumsulfat getrocknet. Das Rohprodukt wird säulenchromatographisch an Kieselgel (Hexan / Ethylacetat 10-20%) gereinigt und man erhält 21.5 g 1-(3,4-Difluor-2-hydroxyphenyl)propan-1-on. 21.4 g (115 mmol) 1-(3,4-Difluor-2-hydroxyphenyl)-propan-1-on werden in 170 ml Aceton gelöst und es werden 29.5 g Kaliumcarbonat und 13 ml (209 mmol) Methyliodid zugegeben. Die Mischung wird 4 Stunden unter Rückfluß gekocht, 12 Stunden bei Raumtemperatur gerührt und das Lösungsmittel im Anschluss zu einem großen Teil entfernt. Man gießt den Rückstand in Wasser und extrahiert mit Diethylether. Es wird mit Wasser gewaschen, über Natriumsulfat getrocknet und nach dem Entfernen des Lösungsmittels im Vakuum erhält man 21.2 g 1-(3,4-Difluor-2-methoxyphenyl)propan-1-on.

31,2 g (476 mmol) Zinkstaub und 740mg (2.65 mmol) Blei(II)-chlorid werden in 320 ml THF suspendiert und bei 0°C werden 30 ml (265 mmol) Dibrommethan zugegeben. Man rührt weitere 30 Minuten bei Raumtemperatur und tropft bei 0°C 53 ml (53 mmol) einer 1 M Titan(IV)-chlorid Lösung in Dichlormethan zu. Das Kühlbad wird entfernt und nach einer Stunde wird die Reaktionsmischung wieder auf 0°C gekühlt. 10.6 g (53 mmol) 1-(3,4-Difluor-2-methoxyphenyl)-propan-1-on in 106 ml THF zugetropft. Man rührt eine weitere Stunde bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit gesättigter Natriumchlorid Lösung, trocknet über Natriumsulfat und entfernt das Lösungsmittel. Das Rohprodukt wird säulenchromatographisch an Kieselgel (Hexan / Diisopropylether 20-40%) gereinigt und man erhält 4.3 g 2,3-Difluor-6-(1 -methylenpropyl)anisol.

Zu 23.6 g (119 mmol) 2,3-Difluor-6-(1-methylenpropyl)anisol, 31.4 ml (238 mmol) Ethyltrifluorpyruvat und 10 g Molekularsieb werden bei 0°C über 30 Minuten 2.58 g (2.98 mmol) [Cu(R,R)-bis-*tert*-butyl-oxazoline)(H₂O)₂]((SbF₆)₂, in 85 ml Dichlormethan getropft. Man rührt die Reaktionsmischung 16 Stunden bei 0°C und reinigt die Reaktionsmischung mittels Säulenchomatographie an Kieselgel (Hexan / Ethylacetat 0-10%). Man erhält 16.7 g (*R*)-4-(3,4-Difluor-2-methoxyphenyl)-2-hydroxy-2-(trifluormetyl)-hex-4-ensäureethylester als E/Z Gemisch mit einem Enantiomerenüberschuß von größer 80%. 16.7g.(45.3 mmol) *E*/*Z*-4-(3,4-Diluor-2-methoxyphenyl)-2-hydroxy-2-(trifluormetyl)-hex-4-ensäure-ethylester werden in 600 ml Diethylether auf -5°C gekühlt und über 10 Minuten werden portionsweise 3.44 mg (90.7 mmol) Lithiumaluminiumhydrid fest zugegeben. Man rührt 2 Stunden bei Raumtemperatur und gießt in gesättigte Ammoniumchlorid Lösung. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält so 13.9 rohes *E*/*Z*-4-(3,4-Difluor-2-methoxyphenyl)-2-(trifluormethyl)-hex-4-en-1,2-diol. 16 g (49 mmol) (E/Z-4-(3,4-Difluor-2-methoxyphenyl)-2-(trifluormethyl)-hex-4-en-1,2-diol) werden in 680 ml Methanol and 9.4 ml Essigsäure gelöst and 1.07g Palladium auf Kohle (10%ig) werden zugegeben Die Suspension wird unter einer Wasserstoff Atmosphäre bei Normaldruck bis zur vollständigen Umsetzung geschüttelt. Die Mischung wird durch Celite filtriert wobei gründlich mit Ethylacetat nachgewaschen wird. Nach dem Entfernen des Lösungsmittels erhält man 16.1 g rohes 4-(3,4-Difluor-2-methoxyphenyl)-2-(trifluormethyl)-hexan-1,2-diol als Gemisch der Diastereomeren. Zu 16.1 g (49 mmol) 4-(3,4-Difluor-2-methoxyphenyl)-2-(trifluormethyl)-hexan-1,2-diol in 600 ml Dichlormethan und 220 ml DMSO gibt man 33.5 ml (242 mmol) Triethylamin und portionsweise über 10 min 29.8 g (188 mmol) Pyridin SO₃ Komplex. Man rührt über 3 Stunden und gibt gesättigte Ammoniumchlorid Lösung zu. Die Mischung wird weitere 5 Minuten gerührt, die Phasen getrennt und es wird mit Dichlormethan extrahiert. Man wäscht mit Wasser und trocknet über Natriumsulfat. Das Lösungsmittel wird im Vakuum entfernt und nach Säulenchomatographie an Kieselgel (Hexan / Diisopropylether 0-30%). Man erhält 2.7 g (*2R*,*4R*)-4-(3,4-Difluor-2-methoxyphenyl)-2-hydroxy-2-(trifluormetyl)hexanal
¹H-NMR (300 MHz, CDCl₃); δ = 0.75 (t, 3H), 1.55 - 1.73 (m, 2H), 2.30 (dd, 1H), 2.54 (dd, 1H), 3.06 (m,1H), 3.92 (s, 1H), 3.96 (s, 3H), 6.75 - 6.84 (m, 2H), 9.02 (s, 1H).und 3.9 g (*2R*,*4S*)-4-(3,4-Difluor-2-methoxyphenyl)-2-hydroxy-2-(trifluormetyl)hexanal -
¹H-NMR (300 MHz, CDCl₃); δ 0.71 (t, 3H), 1.50 - 1.70 (m, 2H), 2.33 (dd, 1H), 2.41 (dd, 1H), 2.87 (m,1H), 3.60 (s, 1H), 3.95 (s, 3H), 6.75 - 6.86 (m, 2H), 9.69 (s,1H).

300 mg (0.92 mmol) (*2R*,*4R*)-4-(3,4-Difluor-2-methoxy-phenyl)-2-hydroxy-2-(trifluoromethyl)hexanal und 146 mg (0.92 mmol) 5-Amino-2-methylchinazolin werden in 20 ml Toluol gelöst und 0.29 ml (0.92 mmol) Titantert-butylat und 0.1 ml Essigsäure werden zugegeben. Man erhitzt die Reaktionsmischung 2 Stunden auf 100°, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält so 349 mg (*2R*,*4R*)-4-(3,4-Difluor-2-methoxyphenyl)-1-[(2-methylchinazolin-5-yl)imino]-2-(trifluoromethyl)hexan-2-ol-als-Rohprodukt. Das rohe Imin wird in 35 ml CH₂Cl₂ gelöst und auf -30°C gekühlt. 6 ml (6 mmol) einer 1 M BBr₃ Lösung in Dichlormethan werden über 5 min langsam zugetropft und man läßt über 16 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säulenchromatographische Reinigung an Kieselgel (Hexan / iso-Propanol 0- 10%) und anschließende HPLC Trennung an chiraler stationärer Phase ergeben 70 mg Produkt (analytische HPLC: Rₜ = 8.36 min (Chiralcel OD 5µ, 250x4.6 mm, Hexan / Ethanol 5%, 1 ml/min Fluß)) als (-)- Enantiomer.
¹H-NMR (300MHz, CDₛOD): δ = 0.93 (t, 3H), 1.74 (ddq, 1H), 1.96 (m, 1H), 1.99 (dd, 1H), 2.38 (dd, 1H), 2.78 (s, 3H), 3.30 (m, 1H), 5.08 (s, 1H), 6.59 (dd,1H), 6.77 (d, 1H), 7.17 (d, 1H), 7.74 (t, 1H), 9.57-(s, 1H).

### Beispiel 51

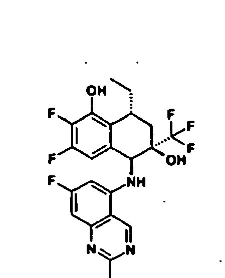

### (5α,6α,8β)-8-Ethyl-2,3-difluor-5-[(7-fluor-2-methylchinazolin-5-yl)aminol-6-(trifluormethyl)-5,6,7,8-tetrahydronaphthalin-1,6-diol

Analog Beispiel 50 werden 137 mg (0.42 mmol) (*2R**,*4R**)-4-(3,4-Difluor-2-methoxyphenyl)-2-hydroxy-2-(trifluoromethyl)hexanal, 78 mg (0.44 mmol) 5-Amino-7-fluor-2-methylchinazolin und 0.2 ml Titantetraethylat zu 5-([(*2R**,*4R**)-4-(3,4-Difluor-2-methoxyphenyl)-1-[(7-fluor-2-methylchinolin-5-yl)imino]-2-(trifluoromethyl)hexan-2-ol umgesetzt. 121 mg chromatographisch gereinigtes Imin werden analog Beispiel 29 bei -40°C mit 2.5 mL (2.5-mmol) 1M Bortribromid-Lösung zum gewünschten Produkt zyklisiert. Präparative Dünnschichtchromatographie an Kieselgel (Hexan / 2-Propanol 15%) liefert 25 mg gewünschtes Produkt
¹H-NMR (300 MHz, CD₃OD); δ = 0.99 (t, 3H), 1.78 (ddq, 1H), 2.04 (dd, 1H), 2.06 (m, 1H), 2.44 (dd, 1H), 2.81 (s, 3H), 3.32 (m, 1H), 5.17 (s, 1H), 6.63 (dd,1H), 6.66 (d, 1H), 6.83 (d, 1H), 9.56 (s, 1H)

### Beispiel 51A / 51B

### (5α,6α,8β)-8-Ethyl-2,3-difluor-5-[(7-fluor-2-methylchinazolin-5-yl)amino]-6-(trifluormethyl)-5,6,7,8-tetrahydronaphthalin-1,6-diol wird mittels präparativer chiraler HPLC (Chiralcel OD-H 5µ) in die enanantiomerenreinen Verbindungen gespalten:

- (+)-Enantiomer: analytische HPLC: Rₜ= 5.14 min (Chiralpcel OD-H 5µ, 250x4.6 mm, Hexan / Ethanol 5% => 20% (20'), 1 ml/min Fluß)
(-)-Enantiomer: analytische HPLC: Rₜ = 8.56 min (Chiralcel OD-H 5µ, 250x4.6 mm, Hexan / Ethanol 5%.=> 20% (20'), 1 ml/min Fluß)

### Analog können hergestellt werden:

### Beispiel 52

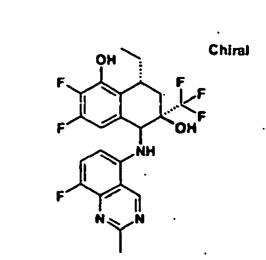

### (5S,6R,8R)-8-Ethyl-2,3-difluor-5-[(8-fluor-2-methylchinazolin-5-yl)amino]-6-(trifluormethyl)-5,6,7,8-tetrahydronaphthalin-1,6-diol

### Beispiel 53

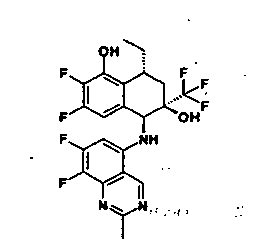

### (5S,6R,8R)-8-Ethyl-2,3-difluor-5-[(7,8-difluor-2-methylchinazolin-5-yl)aminol-6-(trifluormethyl)-5,6,7,8-tetrahydronaphthalin-1,6-diol

### Beispiel 54

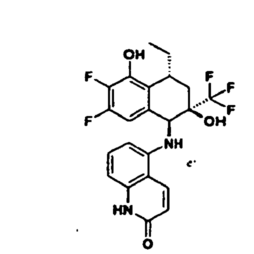

### 5-{[(1α,2α,4β)-4-ethyl-6,7-difluor-2,5-dihydroxy-2-(trifluormethyl)-1,2,3,4-tetrahydrohaphthalin-1-yl]amino}chinolin-2(1H)-on

Analog Beispiel 29 werden 210 mg (0.64 mmol) (*2R**,*4R**)-4-(3,4-Difluor-2-methoxyphenyl)-2-hydroxy-2-(trifluoromethyl)hexanal, 132 mg (0.97 mmol) 5-Aminochinolin-2(1H)-on und 0.27 ml Titantetraethylat zu 5-{[(*2R**,*4R**)-4-(3,4-Difluor-2-methoxy-phenyl)-2-hydroxy-2-(trifluormethyl)hexyliden]amino}chinolin-2(1H)-on umgesetzt. 234 mg rohes Imin werden analog Beispiel 29 bei -40°C, mit 5 mL (5 mmol) 1 M Bortribromid-Lösung zum gewünschten Produkt zyklisiert. Säulenchromatographie an Kieselgel (Hexan / Ethylacetat 33-100%) liefert 25 mg gewünschtes Produkt.
¹H-NMR (300 MHz, CD₃OD): δ = 0.92 (t, 3H), 1.71 (ddq, 1H), 1.94 (m, 1H), 1.96 (dd, 1H), 2.34 (dd, 1H), 3.28 (m, 1H), 4.94 (s, 1H), 6.43 (d, 1H), 6.50 (d, 1H), z6.58 (dd, 1H), 6.68 (d, 1H), 7.33 (t, 1H), 8.18 (d, 1H).

### Beispiel 54A 154B

### 5-{[(1α,2α,4β)-4-Ethyl-6,7-difluor-2,5-dihydroxy-6,7-difluor-2-(trifluormethyl)-1,2,3,4-tetrahydronaphthalin-1-yl]amino}chinolin-2(1H)-on wird mittels präparativer chiraler HPLC (Chiralpak OD-H 5µ) in die enanantiomerenreinen Verbindungen gespalten:

(-)-Enantiomer: analytische HPLC: Rₜ = 7.68 min (Chiralpak IA 5µ, 250x4.6 mm, Hexan / Ethanol 10%, 1 ml/min Fluß)
(+)-Enantiomer analytische HPLC: Rₜ = 9.35 min (Chiralcel IA 5µ; 250x4.6mm, Hexan / Ethanol 10%, 1 ml/min Fluß)

### Beispiel 55

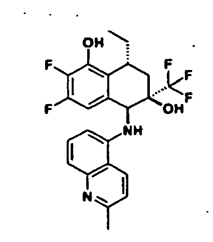

### (5α,6α,8β)-8-Ethyl-2,3-difluor-5-[(2-methylchinolin-5-yl)amino]-6-(trifluormethyl)-5,6,7,-tetrahydronaohthalin-1,6-diol

Analog Beispiel 29 werden 210 mg (0.64 mmol) (*2R**,*4R**)-4-(3,4-Difluor-2-methoxyphenyl)-2-hydroxy-2-(trifluoromethyl)hexanal, 132 mg (0.97 mmol) 5-Aminochinolin-2(1H)-on und 0.27 ml Titantetraethylat zu -{[(*2R**,*4R**-4-(3,4-Difluor-2-methoxy-phenyl)-2-hydroxy-2-(trifluormethyl)hexyliden]amino}chinolin-2(1*H*)-on umgesetzt. 234 mg rohes Imin werden analog Beispiel 29 bei-20°C mit 2 mL (2 mmol) 1 M Bortribromid-Lösung zum gewünschten Produkt zyklisiert. Präparative Dünnschichtchromatographie an Kieselgel (Hexan / 2-Propanol 17%) liefert 15.4 mg gewünschtes Produkt ¹H-NMR (300MHz, CD₃OD); δ = 0.92 (t, 3H), 1.76 (ddq, 1H), 1.95 (m, 1H), 1.99 (dd, 1H), 2.38 (dd, 1H), 2.78 (s. 3H), 3.30 (m, 1H), 5.09 (s, 1H), 6.59 (dd, 1H). 6.69 (d, 1H), 7.38 (m, 2H), 7.56 (t, 1H), 8.46 (d, 1H).

### Beispiel 56

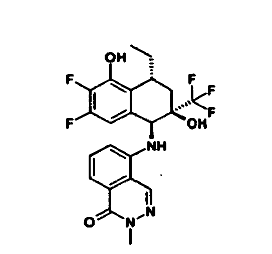

### 5-{[(1α,2α,4β-4-ethyl-6,7-difluor-2,5-dihydroxy-2-(trifluormethyl)-1,2,3.4-tetrahydronaphthalin-1-yl]amino]phthalazin-1-on

Analog Beispiel 29 werden 372 mg (1.14 mmol) (*2R**,*4R**)-4-(3,4-Difluor-2-methoxyphenyl)-2-hydroxy-2-(trifluormethyl)hexanal, 200 mg (1.14 mmol) 5-Aminophthalazin-1-on und 0.36 ml Titantetratert.-butylat zu 5-{[(*2R**,*4R**)-4-(3,4-Difluor-2-methoxy-phenyl)-2-hydroxy-2-(trifluormethyl)hexyliden]amino} phthalazin-l-on umgesetzt. 560 mg rohes Imin werden analog Beispiel 29 bei - 30°C mit 11.8 mL (11.8 mmol) 1 M Bortribromid-Lösung zum gewünschten Produkt zyklisiert. Präparative Dünnschichtchromatographie an Kieselgel (Hexan / 2-Propanol 17%) liefert 249 mg gewünschtes Produkt ¹H-NMR (300 MHz, CD₃OD); δ = 0.92 (t, 3H), 1.70 (ddq, 1H), 1.95 (m, 1H), 1.96 (dd, 1H), 2.37 (dd, 1H), 3.29 (m, 1H), 3.81 (s, 3H), 5.03 (s, 1H), 6.58 (dd, 1H), 7.07 (dd, 1H), 7.61 (d, 1H), 7.62 (d, 1H), 8.51 (s, 1H).

### Analog können hergestellt werden aus 3-Chlor-2-fluorphenol:

### Beispiel 57

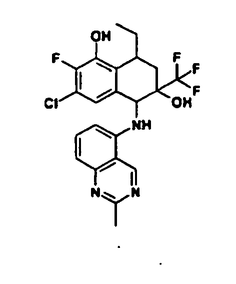

### 3-Chlor-8-ethyl-2-fluor-5-[(2-methylchinazolin-5-yl)amino]-6-(trifluormethyl-5,6,7,8-tetrahydronaphthalin-1,6-diol

### und Beispiel 58

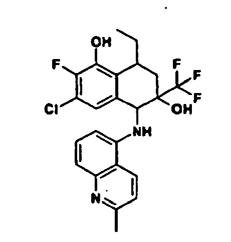

### 3-Chlor-8-ethyl-2-fluor-5-[(7-fluoro-2-methylquinazolin-5-yllaminol-6-(trifluormethyl)-5,6,7,8-tetrahydronaphthalene-1,6-diol

### und Beispiel 59

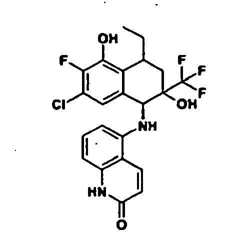

### 5-{[-7-Chlor-4-ethyl-6-fluor-2,5-dihydroxy-2-(trifluormethyl)-1,2,3,4-tetrahydronaphthalin-1-yl]amino}chinolin-2(1H)-on

### und Beispiel 60

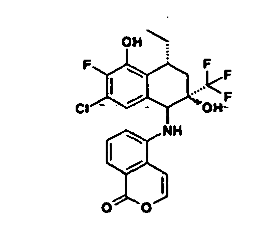

### 5-{[-7-Chlor-4-ethyl-6-fluor-2,5-dihydroxy-2-(trifluormethyl)-1,2,3,4-tetrahydronaphthalin-1-yl]amino}isochromen-1-on

### und Beispiel 61

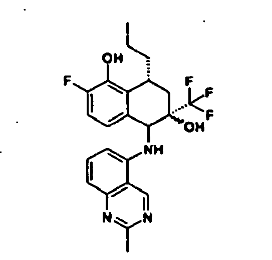

### (5α,6α,8β)-2-fluor-5-[(2-methylchinazolin-5-yl)amino]-8-prop-1-yl-6-(trifluormethyl)-5,6,7,8-tetrahydronaphthalin-1,6-diol

### 4-(3-Fluor 2-methoxy phenyl)-2-hydroxy-2-(trifluoromethyl)heptanal:

Zu 25 g (213 mmol) 2-Fluorphenol in 150 ml Dichlormethan und 24 ml Pyridin werden bei 0°C 21 ml (210 mmol) Buttersäurechlorid getropft. Man rührt die Mischung zwei Stunden lang und gibt 100 ml einer 2 M Salzsäure zu. Es wird mit Dichlormethan extrahiert und mit Wasser gewaschen. Nach dem Trocknen. über Natriumsulfat und dem Entfernen des Lösungsmittels im Vakuum erhält man 40 g Buttersäure-2-fluorphenylester. 40 g (213 mmol) Buttersäure-2-fluorphenylester in 22 ml 1,2-Dichlorbenzol werden zu 28 g (213 mmol) Aluminiumtrichlorid in 25 ml 1,2-Dichlorbenzol getropft und die Mischung wird im Anschluss 20 Stunden lang bei 100°C gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit gesättigter Natriumchlorid Lösung gewaschen und über Natriumsulfat getrocknet. Das Rohprodukt wird säulenchromatographisch an Kieselgel (Hexan / Ethylacetat 0-10%) gereinigt und man erhält 20.7 g 1-(3-Fluor-2-hydroxyphenyl)butan-1-on. 20.7 g (114 mmol) 1-(3-Fluor-2-hydroxyphenyl)-butan-1-on werden in 200 ml Aceton gelöst und es werden 31.5 g Kaliumcarbonat und 14 ml (230 mmol) Methyliodid zugegeben. Die Mischung wird 6 Stunden bei 70°C, 12 Stunden bei Raumtemperatur gerührt und das Lösungsmittel im Anschluss zu einem großen Teil entfernt. Man gießt den Rückstand in Wasser und extrahiert mit Diethylether. Es wird mit Wasser gewaschen, über Natriumsulfat getrocknet und nach dem Entfernen des Lösungsmittels im Vakuum erhält man 20.7 g 1-(3-Fluor-2- - methoxyphenyl)butan-1-on. 31.7 g Zinkstaub und 660 mg Blei(II)-chlorid werden in 330 ml THF suspendiert und bei Raumtemperatur werden 29.5 ml Dibrommethan zugegeben. Man rührt weitere 30 Minuten und tropft bei 0°C ml 56 ml(56 mmol) einer 1 M Titan(IV)-chlorid Lösung in Dichlormethan zu. Das Kühlbad wird entfernt und nach 30 Minuten bei Raumtemperatur werden 10.3 g (52.5 mmol) 1-(3-Fluor-2-methoxyphenyl)butan-1-on in 50 ml THF zugetropft. Man rührt eine weitere Stunde bei Raumtemperatur. Es wird mit Dietylether verdünnt und das Reaktionsgemisch wird vorsichtig auf eine Mischung von 4 M Salzsäure und Eis gegeben. Man trennt die Phasen, extrahiert mit Diethylether, wäscht mit Wasser, trocknet über Natriumsulfat und entfernt das Lösungsmittel. Das Rohprodukt wird säulenchromatographisch an Kieselgel (Hexan / Diisopropylether 0-10%) gereinigt und man erhält 4.26 g 2-Fluor-6-(1-methylenbutyl)anisol.

Zu 698 mg (2.56 mmol) 1,1'-Bi-2-naphthol werden 2.56 ml (1.28 mmol) einer 0.5 M Titantetraisopropylat Lösung in Toluol gegeben und man rührt die rote Lösung für 2 Stunden bei Raumtemperatur. 4.26 g (21.9 mmol) 2-Fluor-6-(1-methylenbutyl)anisol und 5.7 ml (44 mmol) Ethyltrifluorpyruvat werden zugegeben und man erhitzt die Mischung über 18 Stunden auf 140°C. Nach dem Abkühlen wird sofort säulenchromatographisch an Kieselgel (Hexan / Ethylacetat 0-15%)gereinigt und man erhält 5.82 g 4-(3-Fluor-2-methoxyphenyl)-2-hydroxy-2-(trifluormethyl)hept-4-ensäureethylester. 2.6 g (7.1 mmol) 4-(3-Fluor-2-methoxyphenyl)-2-hydroxy-2-(trifluormethyl)hept-4-ensäure-ethylester werden in 65 ml Methanol gelöst und 260 mg Palladium auf Kohle (10%ig) werden zugegeben. Die Suspension wird 4 Stunden unter einer Wasserstoff Atmosphäre bei Normaldruck geschüttelt bis zu einer Wasserstoffaufnahme von 155 ml. Die Mischung wird durch Celite filtriert wobei gründlich mit Ethylacetat nachgewaschen wird. Nach dem Entfernen des Lösungsmittels erhält man 2.6 g 4-(3-Fluor-2-methoxyphenyl)-2-hydroxy-2-(trifluormethyl)heptansäureethylester. 2.6 g (7.1 mmol) 4-(3-Fluor-2-methoxyphenyl)-2-hydroxy-2-(trifluormethyl)-heptansäureethylester werden in 150 ml Diethylether auf -10°C gekühlt und über 15 Minuten werden portionsweise 520 g (14.2 mmol) Lithiumaluminiumhydrid fest zugegeben. Man rührt 1.5 Stunden bei -15°C, tropft dann nacheinander Ethylacetat und Wasser zu und rührt eine weitere Stunde bis sich ein gut filtrierbarer Niederschlag gebildet hat. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat und entfernt das Lösungsmittel im Vakuum. Die säulenchromatographische Trennung an Kieselgel (Hexan / Diisopropylether 0-15%) liefert 2.1 g 4-(3-Fluor-2-methoxyphenyl)-2-hydroxy-2-(trifluoromethyl)heptanal als Gemisch der Diastereomeren.
¹H-NMR (300 MHz, CDCl₃): δ = 0.70 (m, 3H), 0.95-1.60 (m, 4H), 1.95 - 2.20 (m, 2H), 2.32 (dd, 0.5H), 2.48 (dd, 0.5H), 2.94 (m, 0.5H), 3.26 (m, 0.5H), 3.59 (s, 0.5H) 3.84 (s, 0.5H), 3.89 (s, 3H), 6.74 - 6.92 (m, 3H), 8.93 (s, 0.5H), 9.62 (s, 0.5H)

300 mg (0.97 mmol) 4-(3-Fluor-2-methoxy-phenyl)-2-hydroxy-2-(trifluoromethyl)heptanal und 138 mg (0.87 mmol) 5-Amino-2-methylchinazolin werden in 28 ml Toluol gelöst und 0.48 ml Titantetraethylat werden zugegeben. Man erhitzt die Reaktionsmischung 2 Stunden auf 100°, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält so 350 mg rohes 4-(3-Fluor-2-methoxyphenyl)-1-[(2-methylchinazolin-5-yl)imino]-2-(trifluoromethyl)heptan-2-ol als Rohprodukt. Das rohe Imin wird in 35 ml CH₂Cl₂ gelöst und auf -20°C gekühlt. 5.8 ml (5.8 mmol) einer 1 M BBr₃ Lösung in Dichlormethan werden über 5 min langsam zugetropft und man läßt über 1.5 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säulenchromatographische Reinigung an Kieselgel (Hexan / iso-Propanol 0 - 15%) und anschließende HPLC Trennung an chiraler stationärer Phase ergeben 16 mg Produkt und 26 mg der entsprechenden (5α,6α,8α)-Verbindung (Beispiel 62).
¹H-NMR (300 MHz, CD₃OD); δ = 1.00 (t, 3H), 1.42 (m, 2H), 1.68 (m, 1H), 2.00 (m, 1H), 2.04 (dd, 1H), 2.42 (dd, 1H), 2.81 (s, 3H), 3.48 (m, 1H), 5.16 (s, 1H), 6.75 (dd, 1H), 6.80 (d, 1H), 6.87 (dd, 1H), 7.18 (d, 1H), 7.77 (t, 1H), 9.60 (s, 1H).

### Beispiel 65

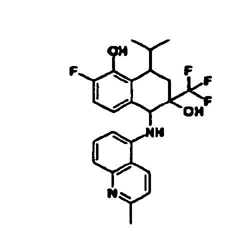

### 2-Fluor-5-[(2-methylchinolin-5-yl)amino]-8-(prop-2-yl)-6-(trifluormethyl)-5,6,7,8-tetrahydronanhthalin-1,6-diol

### 4-(3-Fluor-2-mothoxy-phonyl)-2-hydroxy-5-methyl-2-(trifluormethyl)-hexanat.,

2-Fluor-6-(2-methyl-1-methylenpropyl)anisol kann analog Beispiel 61 aus iso-Buttersäurechlorid und 2-Fluorphenol hergestellt werden. Zu 788 mg Ytterbium(III)triftuormethansulfonat werden 1,8 ml Ethyltrifluorpyruvat und 2.5 g (12.9 mmol) 2-Fluor-6-(2-methyl-1-methylenpropyl)anisol in 5 ml Dichlorethan gegeben. Die Reaktionsmischung wird 16 Stunden auf 100°C erhitzt und nach dem Abkühlen sofort säulenchromatographisch an Kieselgel (Hexan / Diisopropylether 0 - 8%) gereinigt. Man erhält 2.55 g 4-(3-Fluor-2-methoxyphenyl)-2-hydroxy-5-methyl-2-(trifluormethyl)hex-4-ensäureethylester die analog Beispiel 61 zum Diastereomerengemisch von 4-(3-Fluor-2-methoxyphenyl)-2 -hydroxy-5-methyl-2-(trifluormethyl)hexanal umgesetzt werden.
¹H-NMR (300 MHz, CDCl₃); δ = 0.69 (d, 1.5H), 0.72 (d, 1.5H), 0.96 (d, 1.5H), 0.98 (d, 1.5H), 1.55 - 2.24 (m, 3H), 3.10- 3.30 (m, 1H), 3.89 (s, 3H), 6.78 - 7.08 (m, 3H), 9.05 (s, 0.5H), 9.65 (s, 0.5H).

Analog Beispiel 61 werden 200 mg (0.62 mmol) 4-(3-Fluor-2-methoxyphenyl)-2-hydroxy-5-methyl-2-(trifluormethyl)hexanal, 125 mg (0.80 mmol) 5-Amino-2-methylchinolin und 0.3 ml Titantetraethylat zu 4-(3-Fluor-2-methoxyphenyl)-1-[(2-methychinolin-5yl)imino]-5-methyl-2-(trifluormethyl)hexan-2-ol umgesetzt. 229 mg säulenchromatographisch (Kieselgel, Hexan / Ethylacetat 0 - 35%) gereinigtes Imin werden analog Beispiel 61 bei -20°C mit 5 mL (5 mmol) 1 M Bortribromid-Lösung in Dichlormethan zum gewünschten Produkt zyklisiert. Säulenchromatographie an Kieselgel (Hexan /2-Propanol 0-15%) und anschließende präparative HPLC (SunFire C18, Wasser / Methanol) liefern 11 mg Produkt.
¹H-NMR (300 MHz, CDCl₃); δ = 0.84 (d, 3H), 1.02 (d, 3H), 2.15 (dd, 1H), 2.37 (dd, 1H), 2.54 (m, 1H), 2.73 (s, 3H), 3.40 (m, 1H), 4.89 (d, 1H), 5.19 (br,1H), 6.50 (d, 1H), 6.88 (m, 2H), 7.24 (d, 1H), 7.42 (d, 1H), 7.49 (t, 1H), 8.16 (d, 1H).

### Beispiel 66

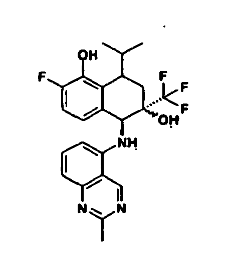

### 2-Fluor-5-[(2-methylchinazolin-5-yl)amino]-8-prop-2-yl-6-(trifluormethyl)-5,6,7,8-tetrahydronaphthalin-1,6-diol

Analog Beispiel 61 werden 280 mg (0.87 mol) 4-(3-Fluor-2-methoxyphenyl)-2-hydroxy-5-methyl-2-(trifluoromethyl)hexanal, 175 mg (1.1 mmol) 5-Amino-2-methylchinazolin und 0.45 ml Titantetraethylat zu 4-(3-Fluor-2-methoxyphenyl)-1-[(2-methylchinazolin-5yl)imino]-5-methyl-2-(trifluoromethyl)hexan-2-ol umgesetzt. 360 mg so erhaltenes rohes Imin werden analog Beispiel 61 bei -30°C mit 6 mL (6 mmol) 1 M Bortribromid-Lösung in Dichlormethan zum gewünschten Produkt zyklisiert. Säulenchromatographie an Kieselgel (Hexan / Ethylacetat 0-70%) und anschließende präparative HPLC (SunFire C18, Wasser / Methanol) liefern 6 mg Produkt.
¹H-NMR (300 MHz, CD₃OD); δ = 0.82 (d, 3H), 1.06 (d, 3H), 2.12 (dd, 1H), 2.21 (dd, 1H), 2.69 (m, 1H), 2.83 (s, 3H), 3.51 (m, 1H), 5.00 (s, 1H), 6.69 (d, 1H), 6.75 (dd, 1H), 6.89 (dd, 1H), 7.20 (d, 1H), 7.77 (t, 1H), 9.63 (s, 1H).

### Beispiel 67

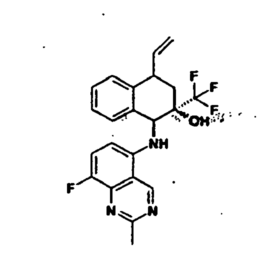

### 4-Ethenyl-1-[(8-fluoro-2-methylquinazolin-5-yl)amino]-2-(trifluoromethyl)-1,2,3,4-tetrahydronaphthalene-2-ol

Analog Beispiel 30B werden 50 mg (0.12 mmol) *cis*-4'-[(8-Fluor-2-methylchinazolin-5-yl)amino]-3,4'-dihydro-3'-(trifluomethyl)spiro[cyclopropan-1,1'(2'H)-naphthalin]-3'-ol (WO 2005/034939) in 1.2 ml Dichlormethan mit 0.6 ml (0.6 mmol) 1 M BBr₃ Lösung versetzt. Anschließendes Refluxieren über 4 Stunden ergibt nach präparativer Dünnschichtchromatographie an Kieselgel (Hexan / 2-Propanol 10%) 15 mg Produkt.
¹H-NMR (300 MHz, CDCl₃): δ = 2.08 (dd, 1H), 2.43 (dd, 1H), 2.93 (s, 3H), 3.93 (m, 1H), 5.14 (d, 1H), 5.33 (d, 1H), 5.37 (d, 1H), 5.50 (d, 1H), 5.83 (ddd, 1H), 6.77 (dd, 1H), 7.17 - 7.26 (m, 3H), 7.35 (d, 1H), 7.51 (dd, 1H), 9.32 (s, 1H).

### Beispiel 68

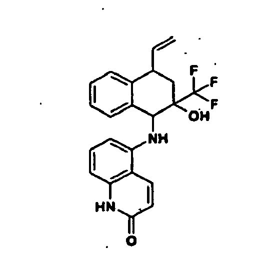

### 5-{[4-Ethenyl-2-hydroxy-2-fluor-2-(trifluormethyl)-1,2,3,4-tetrahydronaphthalin-1-yl]amino}chinolin-2(1H)-on

Analog Beispiel 30B werden 50 mg (0.12 mmol) 5-{3',4'-Dihydro-3'-hydroxy-3'-(trifluormethyl)spiro[cyclohexan-1,1'(2'H)-naphthalin-4'-yl]amino}chinolin-2(1H)-on (WO 2005/034939) in 1.2 ml Dichlormethan mit 0.6 ml (0.6 mmol) 1 M BBr₃ Lösung versetzt. Anschließendes Refluxieren über 4 Stunden ergibt nach präparativer Dünnschichtchromatographie an Kieselgel (Hexan / 2-Propanol 10%) 19 mg Produkt.
¹H-NMR (300 MHz, CD₃OD): δ = 2.01 (dd, 1H), 2.43 (dd, 3H), 3.91 (m, 1H), 5.23 (d, 1H), 5.29 (d, 1H), 5.33 (d, 1H), 5.38 (d, 1H), 5.82 (ddd, 1H), 6.56 (m, 3H), 7.14 - 7.37 (m, 5H), 8.07 (d, 1H).

### Beispiel 69

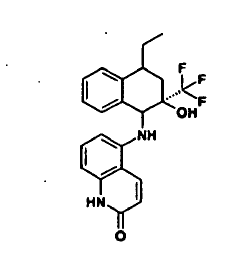

### 5-{[4-Ethyl-2-hydroxy-2-fluor-2-(trifluormethyl)-1,2,3,4-tetrahydronaphthalin-1-yl]-amino}-chinolin-2(1H)-on

13.6 mg (34µmol) 5-{[4-Ethenyl-2-hydroxy -2-fluor-2-(trifluormethyl)-1,2,3,4-tetrahydronaphthalin-1-yl]amino}chinolin-2(1H)-on werden bei RT unter N₂ in 2 ml Methanol gelöst und mit 3 mg Pd-C (10%) versetzt. Man schüttelt die Mischung 1.5 Stunden unter einer Wasserstoff Atmosphäre; (H₂-Aufnahme: 15 ml), filtriert,die Reaktionsmischung über Celite, wobei gründlich mit Methanol nachgespült wird, und erhält so 6 mg Produkt.
¹H-NMR (300 MHz, CD₃OD); δ = 0.99 (t, 1.5H), 1.19 (t, 1.5H), 1.61 (dd, 0.5H), 1.75-2.20 (m, 2H), 1.92 (dd, 0.5H), 2.39 (dd, 0.5H), 2.60 (dd, 0.5H), 2.77 (m, 0.5H), 3.23 (m, 0.5H), 5.24 (s, 1H), 6.51 (d, 1H), 6.42 (d, 0.5H), 6.51 (d, 0.5H), 6.55 (d, 0.5H), 6.67 (d, 0.5H), 6.70 (d, 1H), 7.11 (t, 0.5H), 7.13 (t, 0.5H), 7.24-7.42 (m, 4H), 8.21 (d, 0.5H), 8.26 (d, 0.5H).

### Analog können hergestellt werden:

### Beispiel 70

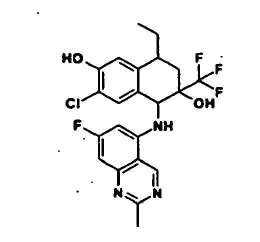

### 3-Chlor-8-ethyl-5-[(7-fluor-2-methylchinazolin-5-yl)aminol-6-(trifluormethyl)-5,6,7,8-tetrahydronaphthalin-2,6-diol

### und Beispiel 71

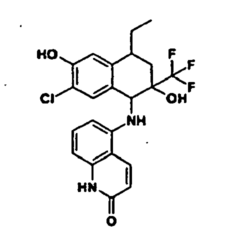

### 5-{[7-Chlor-2,6-dihydroxy-4-ethyl-2-(trifluormethyl)-1,2,3,4-tetrahydronaphthalin-1-yl]-amino}chinolin-2(1H)-on

### und Beispiel 72

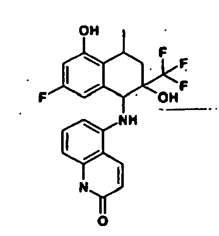

### 5-{[2,5-Dihydroxy-7-fluor-4-methyl-2-(trifluormethyl)-1,2,3,4-tetrahydronaphthalin-1-yl]amino}chinolin-2(1H)-on

### und Beispiel 73

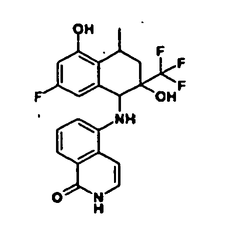

### 5-{[2,5-Dihydroxy-7-fluor-4-methyl-2-(trifluormethyl)-1,2,3,4-tetrahydronaphthalin-1-yl]lamino}isochinolin-1(2H)-on

### und Beispiel 74

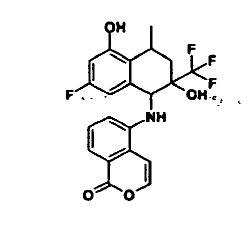

### 5-{[2,5-Dihydroxy-7-fluor-4-methyl-2-(trifluormethyl)-1,2,3,4-tetrahydronaphthalin-1-yl]amino}isochromen-1-on

### und Beispiel 75

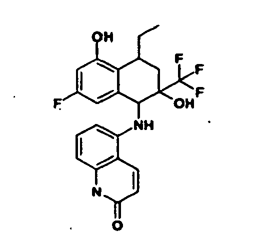

### 5-{[4-Ethyl-7-fluor-2,5-dihydroxy-2-(trifluormethyl)-1,2,3,4-tetrahydronaphthalin-1-yl]aminol}chinolin-2(1H)-on

### und Beispiel 76

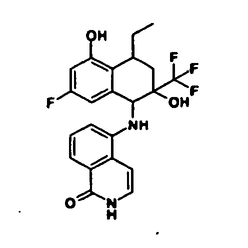

### 5-{[4-Ethyl-7-fluor-2,5-dihydroxy-2-(trifluormethyl)-1,2,3,4-tetrahydronaphthalin-1-yl]amino}isochinolin-1(2H)-on

### und Beispiel 77

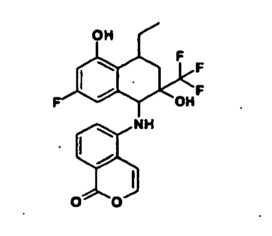

### 5-{[4-Ethyl-7-fluor-2,5-dihydroxy-2-(trifluormethyl)-1,2,3,4-tetrahydronaphthalin-1-yl]amino}isochromen-1-on

### und Beispiel 78

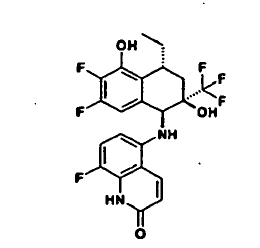

### 5-{[(1α,2α,4β)-4-Ethyl-6,7-difluor-2,5-dihydroxy-2-(trifluormethyl)-1,2,3,4-tetrahydronaphthalin-1-yl]amino}chinolin-2(1H)-on

### und Beispiel 79

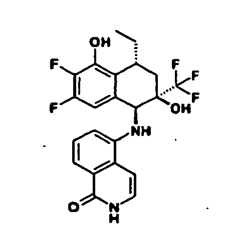

### 5-{[(1α,2α,4β)-4-Ethyl-6,7-difluor-2,5-dihydroxy-2-(trifluormethyl)-1,2,3,4-tetrahydronaphthalin-1-yl]amino}isochinolin-1(2H)-on

### Beispiel 80

**Erfindungsgemäße Stereoisomere**

| **MOLSTRUCTURE** | **Bsp. Nr.** | **IL8 IC50** | **IL8 eff** | **TAT EC50** | **TAT eff** | **Diss. (TAT_{EC50}/** IL8_{IC50}) |
|---|---|---|---|---|---|---|
| 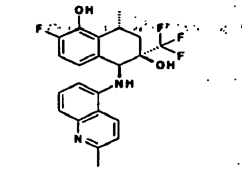 | **1** | **22 nM** | **79%** | **1 µM** | **62%** | **45,5** |
| 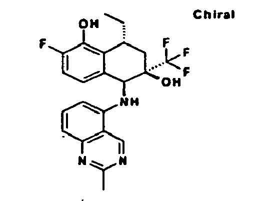 | **29A** | **16 nM** | **77%** | **120 nM** | **95%** | **7,5** |
| 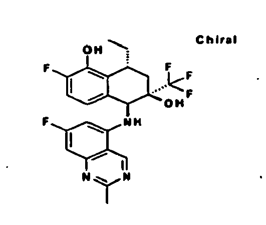 | **30A** | **6,6 nM** | **89%** | **17 nM** | **95%** | **2,8** |
| 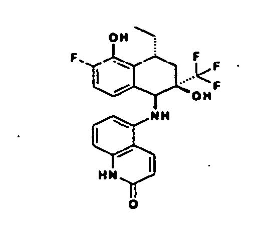 | **43** | **12 nM** | **88%** | **43 nM** | **100%** | **3,6** |
| 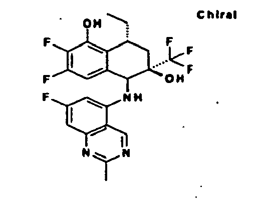 | **51A** | **13 nM** | **72%** | **150 nM** | **81%** | **11,5** |
| 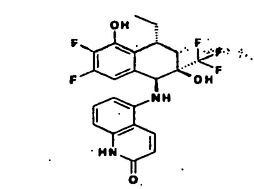 | **54** | **5,7 nM** | **78%** | **490 nM** | **76%** | **86** |
| 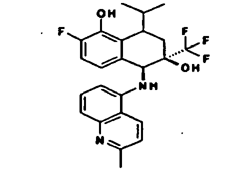 | **65** | **38 nM** | **58%** | **1 µm** | **73%** | **26,3** |
| 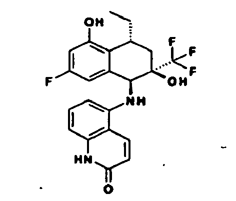 | **75** | **7,1 nM** | **68%** | **120 nM** | **89%** | **16,9** |
| 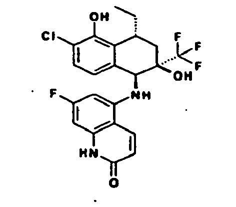 | **101** | **1,8 nM** | **92%** | **74nM** | **97%** | **41** |
| 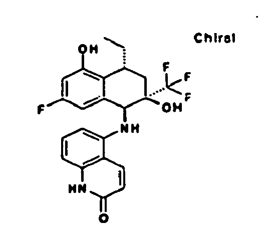 | **104B** | **11nM** | **73%** | **300 nM** | **80%** | **27** |
| 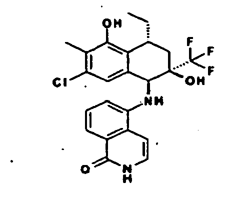 | **121B** | **3,7 nM** | **88%** | **300 nM** | **99%** | **81** |

**Verbindungen des Standes der Technik (WO2005/034939)**

| **MOLSTRUCTURE** | **IL8 IC50** | **IL8 eff** | **TAT EC50** | **TAT eff** | **Diss. (TAT_{EC50}/ IL8_{IC50})** |
|---|---|---|---|---|---|
| 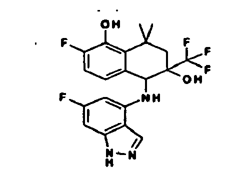 | **20 nM** | **88%** | **4,3 nM** | **100%** | **0,22** |
| 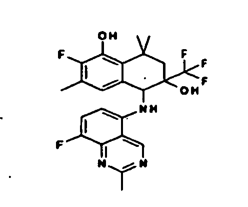 | **40nM** | **79%** | **6,5 nM** | **100%** | **0,16** |
| 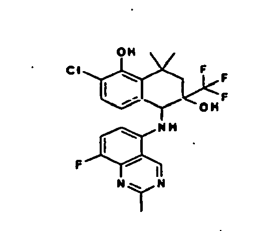 | **22 nM** | **73%** | **3 nM** | **98%** | **0,14** |
| 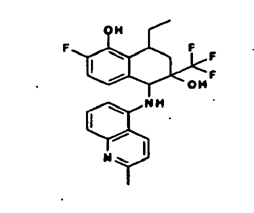 | **220 nM** | **69%** | **140 nM** | **88%** | **0,64** |
| 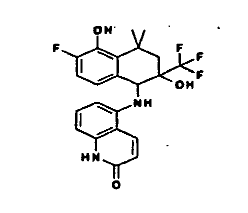 | **14 nM** | **68%** | **3,8 nM** | **92%** | **0,27** |
| 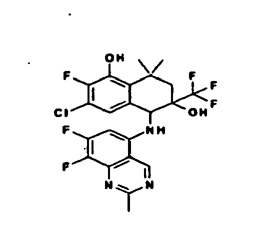 | **31 nM** | **96%** | **4,2 nM** | **100%** | **0,14** |

Die hervorragenden Eigenschaften der erfindungsgemäßen Stereoisomer werden durch die beispielhafte Auswahl von Verbindungen der vorliegenden Erfindung im Vergleich mit einer Auswahl von Verbindungen des Standes der Technik (WO 2005/034939) überzeugend dargelegt: Die *in-vitro* Dissoziation der IL-8 Hemmung von der TAT-Inhibition wurde in allen Fällen um mindestens den Faktor 5 gesteigert.

### Beispiele 81A und 81B

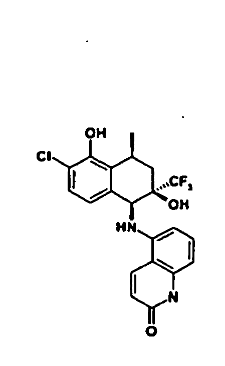

### 5-{[(1R,2S,4R)-6-Chlor-2,5-dihydroxy-4-methyl-2-(trifluormethyl)-1,2,3,4-tetrahydronaphthalin-1-yl]amino}-1H-chinolin-2-on und 5-{[(1S,2R,4S)-6-Chlor-2,5-dihydroxy-4-methyl-2-(trifluormethyl)-1,2,3,4-tetrahydronaphthalin-1-yl]amino}-1H-chinolin-2-on

5-{[4-(3-Chlor-2-methoxyphenyl)-2-hydroxy-2-, trifluormethyl)pentyliden]amino}1H-chinolin-2-on (554,1 mg, 1,16 mmol), hergestellt in Analogie zu beschriebenen Verfahren unter Verwendung des entsprechenden Aldehyds, wird in 5.8 mL Dichlormethan gelöst und bei 0 °C tropfenweise mit 12,81 mL einer 1 M Lösung von Bortribromid in Dichlormethan versetzt. Nach dreieinhalbstündigem Rühren bei 5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie (Flashmaster, unterschiedliche Laufmittelsysteme) werden 31,2 mg (5,6%) des unpolaren Diastereomers und 74,9 mg (13,3%) des polaren Diastereomers erhalten (jeweils als Racemate). Letzteres wird in den Beispielen 82A und 82B beschrieben.

Das unpolare Diastereomer (23,9 mg) wird mittels chiraler HPLC (Chiralcel OD-H 5 µ, Laufmittel: Hexan/ Ethanol) in seine Enantiomeren getrennt. Erhalten werden 8,4 mg (35,2%) des (-)-Enantiomers ([α]_{D} = -6,4°, MeOH) und 10,5 mg (43,9%) des (+)-Enantiomers
([α]_{D} = +8,8°, MeOH). Über die absolute Stereochemie kann natürlich keine Aussage gemacht werden.

### Beispiele 82A und 82B

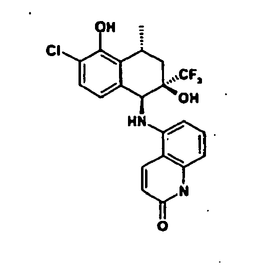

### 5-{[(1R,2S,4S)-6-Chlor-2, 5-dihyrdroxy-4-methyl-2-(trifluormethyl)-1,2,3,4-tetrahydronaphthalin-1-yl]amino}-1H-chinolin-2-on und 5-{[(1S,2R_{,}4R)-6-Chlor-2,5-dihydroxy-4-methyl-2-(trifluormethyl)-1,2,3,4-tetrahydronaphthalin-1-yl]amino}-1H-chinolin-2-on

65,3 mg des in den Beispielen 81A und 81B beschriebenen polaren racemischen Diastereomers werden mittels chiraler HPLC (Chiralpak AD-H 5 µ, Laufmittel: Hexan/ Ethanol) in seine Enantiomeren getrennt. Erhalten werden 31,9 mg (48,9%) des (-)-Enantiomers ([α]_{D} = -93,3°, MeOH) und 32,4 mg (49,6%) des (+)- Enantiomers ([α]_{D} = +97,9°, MeOH). Über die absolute Stereochemie kann natürlich keine Aussage gemacht werden.

### Beispiele 83A und 83B

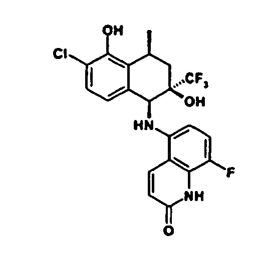

### 5-{[(1R,2S,4R)-6-Chlor-2, 5-dihydroxy-4-methyl-2-(trifluormethyl)-1,2,3,4-tetrahydronaphthalin-1-yl]amino}-8-fluor-1H-chinolin-2-on und 5-{[(1S,2R,4S)-6-Chlor-2,5-dihydroxy-4-methyl-2-(trifluormethyl)-1,2,3,4-tetrahydronaphthalin-1-yl]amino}-8-fluor-1H-chinolin-2-on

5-{[4-(3-Chlor-2-methoxyphenyl)-2-hydroxy-2-trifluormethyl)pentyliden]amino}8-fluor-1H-chinolin-2-on (485 mg, 1,03 mmol), hergestellt in Analogie zu beschriebenen Verfahren unter Verwendung des entsprechenden Aldehyds, wird in 4.8 mL Dichlormethan gelöst und bei 0 °C tropfenweise mit 10,3 mL einer 1 M Lösung von Bortribromid in Dichlormethan versetzt. Nach drei Stunden Rühren bei 5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie (Flashmaster, unterschiedliche Laufmittelsysteme) werden 87,7 mg (18,6%) des unpolaren Diastereomers und 62,9 mg (13,4%) des polaren Diastereomers erhalten, beide als Racemate. Die Racematspaltung des letzteren wird in den Beispielen 84A und 84B beschrieben. Das unpolare Diastereomer (77 mg) wird mittels chiraler HPLC (Chiralcel OD-H 5 µ, Laufmittel: Hexan/ Ethanol) in seine Enantiomeren getrennt. Erhalten werden 38,2 mg (49.6%) des (-)-Enantiomers ([α]_{D} = -8,5°, MeOH) und 35,9 mg (46,6%) des (+)-Enantiomers ([α]_{D} = +9,4°, MeOH). Über die absolute Stereochemie kann natürlich keine Aussage gemacht werden.

### Beispiele 84A und 84B

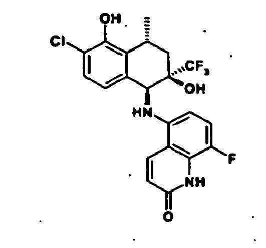

### 5-{[(1R,2S,4S)-6-Chlor-2, 5-dihydroxy-4-methyl-2-(trifluormethyl)-1,2,3,4-tetrahydronaphthalin-1-yl]amino}-8-fluor-1H-chinolin-2-on und 5-{[(1S,2R,4R)-6-Chlor-2,5-dihydroxy-4-methyl-2-(trifluormethyl)-1,2,3,4-tetrahydronaphthalin-1-yl]amino}-8-fluor-1H-chinolin-2-on

56.9 mg des in den Beispielen 83A und 83B beschriebenen polaren racemischen Diastereomers werden mittels chiraler HPLC (Chiralpak AD-H 5 µ, Laufmittel: Hexan/ Ethanol) in seine Enantiomeren getrennt. Erhalten werden 26,3 mg (46,2%) des
(-)-Enantiomers ([α]_{D} = -100,9°, MeOH) und 26,6 mg (46,8%) des (+)-Enantiomers
([α]_{D} = +98,7°, MeOH). Über die absolute Stereochemie kann natürlich keine Aussage gemacht werden.

### Beispiele 85A und 85B

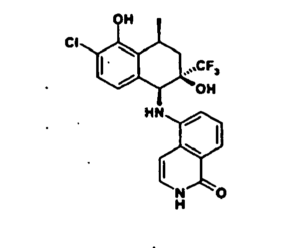

### 5-{[(1R,2S,4R)-6-Chlor-2, 5-dihydroxy-4-methyl-2-(trifluormethyl)-1,2,3,4-tetrahydronaphthalin-1-yl]amino}-2H-chinolin-1-on und 5-{[(1S,2R,4S)-6-Chlor-2,5-dihydroxy-4-methyl-2-(trifluormethyl)-1,2,3,4-tetrahydronaphthalin-1-yl]amino}-2H-chinolin-1-on

5-{[4-(3-Chlor-2-methoxyphenyl)-2-hydroxy-2-frifluormethyl)pentyliden]amino}2*H*-chinolin-1-on (590 mg, 1,3 mmol), hergestellt in Analogie zu beschriebenen Verfahren unter Verwendung des entsprechenden Aldehyds und Amins, wird in 5.9 mL Dichlormethan gelöst und bei 0 °C tropfenweise mit 12,8 mL einer 1 M Lösung von Bortribromid in Dichlormethan versetzt. Nach drei Stunden Rühren bei 5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie (Flashmaster, unterschiedliche Laufmittelsysteme) werden 55,1 mg (9,6%) des unpolaren Diastereomers und 27,9 mg (4,9%) des polaren Diastereomers erhalten, beide als Racemate. Die Racematspaltung des letzteren wird in den folgenden Beispielen 86A und 86B beschrieben.

Das unpolare Diastereomer (49,6 mg) wird mittels chiraler HPLC (Chiralpak AD-H 5 µ, Laufmittel: Hexan/ Ethanol) in seine Enantiomeren getrennt. Erhalten werden 22,4 mg (45,2%) des (+)-Enantiomers ([α]_{D} = +96,6°, MeOH) und 20,9 mg (42,1%) des
(-)-Enantiomers ([α]_{D} = -95,5°, MeOH). Über die absolute Stereochemie kann natürlich keine Aussage gemacht werden.

### Beispiele 86A und 86B

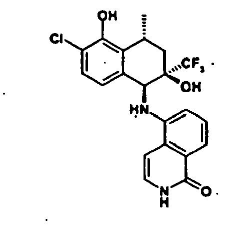

### 5-{[(1R,2S,4S)-6-Chlor-2,5-dihydroxy-4-methyl-2-(trifluormethyl)-1,2,3,4-tetrahydronaphthalin-1-yl]amino}-2H-chinolin-1-on und 5-{[(1S,2R,4R)-6-Chlor-2,5-dihydroxy-4-methyl-2-(trifluormethyl)-1,2,3,4-tetrahydronaphthatin-1-yl]amino}-2H-chinolin-1-on

21,6 mg des in den Beispielen 85A und 85B beschriebenen polaren racemischen Diastereomers werden mittels chiraler HPLC (Chiralpak AD-H 5 µ, Laufmittel: Hexan/ Ethanol) in seine Enantiomeren getrennt. Erhalten werden 9,2 mg (42,6%) des
(-)-Enantiomers ([α]_{D} = -0,9°, MeOH) und 9,5 mg (44%) des (+)-Enantiomers ([α]_{D} = +2,7°, MeOH). Über die absolute Stereochemie kann natürlich keine Aussage gemacht werden.

### Beispiele 87A und 87B

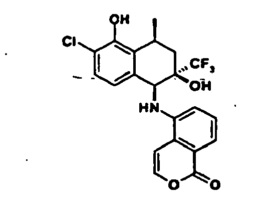

### 5-{[(1R,2S,4R)-6-Chlor-2, 5-dihydroxy-4-methyl-2-(trifluormethyl)-1,2,3,4-tetrahydronaphthalin-1-yl]amino}-isochromen-1-on und

### 5-{[(1S,2R,4S)-6-Chlor-2,5-dihydroxy-4-methyl-2-(trifluormethyl)-1,2,3,4-tetrahydronaphthalin-1-yl]amino}-isochromen-1-on

5-{[4-(3-Chlor-2-methoxyphenyl)-2-hydroxy-2-trifluormethyl)pentyliden]amino}-isochromen-1-on (510 mg, 1,12 mmol), hergestellt in Analogie zu beschriebenen Verfahren unter Verwendung des entsprechenden Aldehyds und Amins, wird in 5.1 mL Dichlormethan gelöst und bei 0 °C tropfenweise mit 12,8 mL einer 1 M Lösung von Bortribromid in Dichlormethan versetzt. Nach drei Stunden Rühren bei 5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie (Flashmaster, unterschiedliche Laufmittelsysteme) werden 108,7 mg (22%) des unpolaren Diastereomers und 113,9 mg (23,1 %) des polaren Diastereomers erhalten, beide als Racemate. Die Racematspaltung des letzteren wird in den folgenden Beispielen 88A und 88B beschrieben.

Das unpolare Diastereomer (90 mg) wird mittels chiraler HPLC (Chiralpak AD-H 5 µ, Laufmittel: Hexan/ Ethanol) in seine Enantiomeren getrennt. Erhalten werden 43,1 mg (47,8%) des (+)-Enantiomers ([α]_{D} = +103,3°, MeOH) und 40,4 mg (44,8%) des
(-)-Enantiomers ([α]_{D} = -104,4°, MeOH). Über die absolute Stereochemie kann natürlich keine Aussage gemacht werden.

### Beispiele 88A und 88B

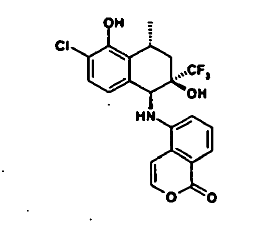

### 5-{[(1R,2S,4S)-6-Chlor-2,5-dihydroxy-4-methyl-2-(trifluormethyl)-1,2,3,4-tetrahydronaphthalin-1-yl]amino}-isochromen-1-on und 5-{[(1S,2R,4R)-6-Chlor-2,5-dihydroxy-4-methyl-2-(trifluormethyl)-1,2,3,4-tetrahydronaphthalin-1-yl]amino}-isochromen-1-on

82,2 mg des in den Beispielen 87A und 87B beschriebenen polaren racemischen Diastereomers werden mittels chiraler HPLC (Chiralpak AD-H5 µ, Laufmittel: Hexan/ Ethanol) in seine Enantiomeren getrennt. Erhalten werden 37,5 mg (45,6%) des
(-)-Enantiomers ([α]_{D} = -104,4°, MeOH) und 40,8 mg (49,6%) des (+)-Enantiomers
([α]_{D} = +103,3°, MeOH). Über die absolute Stereochemie kann natürlich keine Aussage gemacht werden.

### Beispiele 89A und 89B

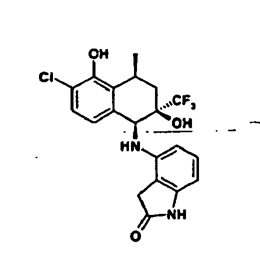

### 4-{[(1R,2S,4R)-6-Chlor-2, 5-dihydroxy-4-methyl-2-trifluormethyl)-1,2,3,4-tetrahydronaphthalin-1-yl]amino}-1,3-dihydroindol-2-on und 4-{[(1S,2R,4S)-6-Chlor-2,5-dihydroxy-4-methyl-2-(trifluormethyl)-1,2,3,4-tetrahydronaphthalin-1-yl]amino}-1.3-dihydroindol-2-on

4-{[4-(3-Chlor-2-methoxyphenyl)-2-hydroxy-2-trifluormethyl)pentyliden]amino}-1,3-dihydroindol-2-on (590 mg, 1,34 mmol), hergestellt in Analogie zu beschriebenen Verfahren unter Verwendung des entsprechenden Aldehyds und Amins, wird in 5.8 mL Dichlormethan gelöst und bei 0 °C tropfenweise mit 13,4 mL einer 1M Lösung von Bortribromid in Dichlormethan versetzt. Nach drei Stunden Rühren bei 5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie (Flashmaster, unterschiedliche Laufmittelsysteme) werden 36,3 mg (6,4%) des unpolaren Diastereomers und 60,7 mg (10,6%) des polaren Diastereomers erhalten, beide als Racemate. Die Racematspaltung des polaren Diastereomers wird in den folgenden Beispielen 90A und 90B beschrieben.

Das unpolare Diastereomer (30,8 mg) wird mittels chiraler HPLC (Chiralpak AD-H 5 µ, Laufmittel: Methanol/ Ethanol) in seine Enantiomeren getrennt. Erhalten werden 15,2 mg (49,4%) des (-)-Enantiomers ([α]_{D} = -1,7°, MeOH, stärker gefärbte Lösung als beim (+)-Enantiomer) und 14,3 mg (46,4%) des (+)- Enantiomers ([α]_{D} = +6.5°, MeOH). Über die absolute Stereochemie kann natürlich keine Aussage gemacht werden.

### Beispiele 90A und 90B

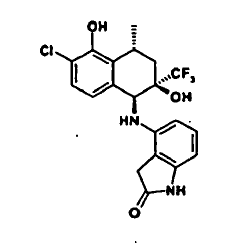

### 4-{[(1R,2S,4S)-6-Chlor-2, 5-dihydroxy-4-methyl-2-(trifluormethyl)-1,2,3,4-tetrahydronaphthalin-1-yl]amino}-1,3-dihydroindol-2-on und 4-{[(1S,2R,4R)-6-Chlor-2,5-dihydroxy-4-methyl-2-(trifluormethyl)-1,2,3,4-tetrahydronaphthalin-1-yl]amino}-1,3-dihydroindol-2-on

46,2 mg des in den Beispielen 89A und 89B beschriebenen polaren racernischen Diastereomers werden mittels chiraler HPLC (Chiralpak AD-H 5 µ, Laufmittel: Hexan/ Ethanol) in seine Enantiomeren getrennt. Erhalten werden 22,5 mg (48,7%) des
(-)-Enantiomers ([α]_{D} = -94,1°, MeOH) und 17,4 mg (37,7%) des (+)- Enantiomers
([α]_{D} = +110,9°, MeOH). Über die absolute Stereochemie kann natürlich keine Aussage gemacht werden.

### Beispiel 92

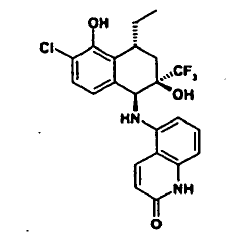

### 5-{[(1α,2α,4β)-6-Chlor-2, 5-dihydroxy-4-ethyl-2-(trifluormethyl)-1,2,3,4-tetrahydronaphthalin-1-yl)amino}-1H-chinolin-2-on

Die Synthese dieser Verbindung wurde in Beispiel 91 beschrieben. Es wurden 62,9 mg (15,8%) der gewünschten Verbindung isoliert.

### Beispiele 92A und 92B

### 5-{[(1R,2S,4S)-6-Chlor-2, 5-dihydroxy-4-ethyl-2-(trifluormethyl)-1,2,3,4-tetrahydronaphthalin-1-yl]aminol-1H-chinolin-2-on und 5-{[(1S,2R,4R)-6-Chlor-2,5-dihydroxy-4-ethyl-2-(trifluormethyl)-1,2,3,4-tetrahydronaphthalin-1-yl]amino}-1H-chinolin-2-on

Das in Beispiel 92 aufgeführte polare Diastereomer (58 mg) wird mittels chiraler HPLC (Chiralpak AD-H 5 µ, Laufmittel: Hexan/ Ethanol) in seine Enantiomeren getrennt. Erhalten werden 28,1 mg (48,5%) des (-)-Enantiomers ([α]_{D} = -97,3°, MeOH) und 23,6 mg (40,7%) des (+)-Enantiomers ([α]_{D} = +110,2°, MeOH). Über die absolute Stereochemie kann natürlich keine Aussage gemacht werden.

### Beispiel 93

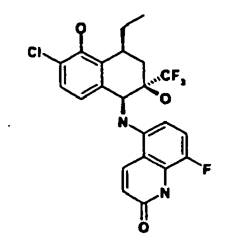

### 5-{[(1α,2α,4α)-6-Chlor-2,5-dihydroxy-4-ethyl-2-(trifluormethyl)-1,2,3,4-tetrahydronaphthalin-1-yl]amino}-8-fluor-1H-chinolin-2-on

5-{[4-(3-Chlor-2-methoxyphenyl)-2-hydroxy-2-trifluormethyl)hexyliden]amino}-8-fluor-1H-chinolin-2-on (490 mg, 1,01 mmol), hergestellt aus dem in Beispiel 91 beschriebenen Aldehyd und 5-Amino-8-fluor-1H-chinolin-2-on, wird in 4.4 mL Dichlormethan gelöst und bei 0 °C tropfenweise mit 10,1 mL einer 1 M Lösung von Bortribromid in Dichlormethan versetzt. Nach dreistündigem Rühren zwischen 0 und 5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie (Flashmaster, unterschiedliche Laufmittelsysteme) werden 53,9 mg (11,3%) des unpolaren Diastereomers und 63 mg (13,2%) des polaren Diastereomers (die Charakterisierung erfolgt in Beispiel 94) erhalten (jeweils als Racemat).

### Beispiele 93A und 93B

### 5-{[(1R,2S,4R)-6-Chlor-2,5-dihydroxy-4-ethyl-2-(trifluormethyl)-1,2,3,4-tetrahydronaphthalin-1-yl]amino}-8-fluor-1H-chinolin-2-on und 5-{[(1S,2R,4S)-6-Chlor-2,5-dihvdroxy-4-ethyl-2-(trifluormethyl)-1,2,3,4-tetrahydronaphthalin-1-yl]amino}-8-fluor-1H-chinolin-2-on

Das in Beispiel 93 beschriebene unpolare Diastereomer (41,2 mg) wird mittels chiraler HPLC (Chiralpak IB 5 µ, Laufmittel: Hexan/ Ethanol) in seine Enantiomeren getrennt. Erhalten werden 18 mg (43,7%) des (-)-Enantiomers ([α]_{D} = -21,2°, MeOH) und 18,7 mg (45,4%) des (+)-Enantiomers ([α]_{D} = +22,3°C MeOH). Über die absolute Stereochemie kann natürlich keine Aussage gemacht werden.

### Beispiel 94

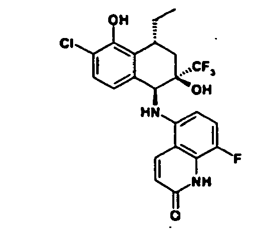

### 5-{[(1α,2α,4β)-6-Chlor-4-ethyl-2,5-dihydroxy-2-(trifluormethyl)-1,2,3,4-tetrahydronaphthalin-1-yl]amino}-8-fluor-1H-chinolin-2-on

Die Synthese dieser Verbindung wurde in Beispiel 93 beschrieben. Es wurden 63 mg (13,2%) der gewünschten Verbindung isoliert.

### Beispiele 94A und 94B

### 5-{[(1R,2S,4S)-6-Chlor-4-ethyl-2,5-dihydroxy-2-(trifluormethyl)-1,2,3,4-tetrahydronaphthalin-1-yl]amino}-8-fluor-1H-chinolin-2-on und 5-{[(1S,2R,4R)-6-Chlor-4-ethyl-2,5-dihydroxy-2-(trifluormethyl)-1,2,3,4-tetrahydronaphthalin-1-yl]amino}-8-fluor-1H-chinolin-2-on

Das in Beispiel 94 aufgeführte polare Diastereomer (53 mg) wird mittels chiraler HPLC (Chiralpak IB 5 µ, Laufmittel: Hexan/ Ethanol) in seine Enantiomeren getrennt. Erhalten werden 22,7 mg (42,9%) des (+)-Enantiomers ([α]_{D} = +109,3°, MeOH) und 24,5 mg (46,3%) des (-)-Enantiomers ([α]_{D} = -111.8°, MeOH). Über die absolute Stereochemie kann natürlich keine Aussage gemacht werden.

### Beispiel 96

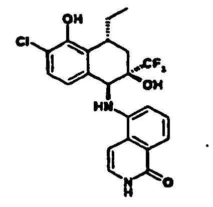

### 5-{[(1α,2α,4β)-6-Chlor-4-ethyl-2,5-dihydroxy-2-(trifluormethyl)-1,2,3,4-tetrahydronaphthalin-1-yl]amino}-2H-chinolin-1-on

Die Synthese dieser Verbindung wurde in Beispiel 95 beschrieben. Es wurden 104,6 mg (18.3%) der gewünschten Verbindung isoliert.

### Beispiele 96A und 96B

### 5-{[(1R,2S,4S)-6-Chlor-4-ethyl-2,5-hydroxy-2-(trifluormethyl)-1,2,3,4-tetrahydronaphthalin-1-yl]amino}-2H-chinolin-1-on und 5-{[(1S,2R,4R)-6-Chlor-4-ethyl-2,5-dihydroxy-2-(trifluormethyl)-1,2,3,4-tetrahydronaphthalin-1-yl]amino}-2H-chinolin-1-on

Das in Beispiel 96 aufgeführte polare Diastereomer (94,4 mg) wird mittels chiraler HPLC (Chiralpak AD-H 5 µ, Laufmittel: Hexan/ Ethanol) in seine Enantiomeren getrennt. Erhalten werden 40,4 mg (42.8%) des (-)-Enantiomers ([α]_{D} = -14,2°, MeOH) und 48,6 mg (48,6%) des (+)-Enantiomers ([α]_{D} = +14,1°, MeOH). Über die absolute Stereochemie kann natürlich keine Aussage gemacht werden.

### Beisplel 98

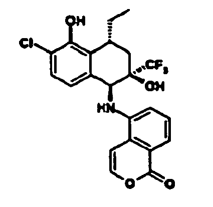

### 5-{[(1α,2α,4β)-6-Chlor-4-ethyl-2,5-dihydroxy-2-(trifluormethyl)-1,2,3,4-tetrahydronaphthalin-1-yl]amino}isochromen-1-on

Die Synthese dieser Verbindung wurde in Beispiel 97 beschrieben. Es wurden 214,1 mg (34%) der gewünschten Verbindung isoliert.

### Beispiele 98A und 98B

### 5-{[(1R,2S,4S)-6-Chlor-4-ethyl-2,5-dihydroxy-2-(trifluormethy)-1,2,3,4-tetrahydronaphthalin-1-yl]amino}-isochromen-1-on und 5-{[(1S,2R,4R)-6-Chlor-2,5-dihydroxy-4-2-(trifluormethyl)-1,2,3,4-tetrahydronaphthalin-1-yl]amino}-isochromen-1-on

Das in Beispiel 98 aufgeführte polare Diastereomer (189 mg) wird mittels chiraler HPLC (Chiralpak IA 5 µ, Laufmittel: Hexan/ Ethanol) in seine Enantiomeren getrennt. Erhalten werden 87,6 mg (46,4%) des (-)-Enantiomers ([α]_{D} = -25,4°, MeOH) und 86,9 mg (46.0%) des (+)-Enantiomers ([α]_{D} = +29,4°, MeOH). Über die absolute Stereochemie kann natürlich keine Aussage gemacht werden.

### Beispiel 100

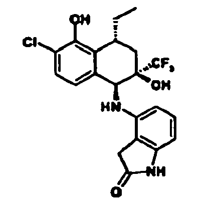

### 5-{[(1α,2α,4β)-6-Chlor-4-ethyl-2,5-dihydroxy-2-(trifluormethyl)-1,2,3,4-tetrahydronaphthalin-1-yl]amino}-1,3-dihydroindol-2-on

Die Synthese dieser Verbindung wurde in Beispiel 99 beschrieben. Es wurden 125,5 mg (24,2%) der gewünschten Verbindung isoliert.

### Beispiele 100A und 100B

### 5-{[(1R,2S,4S)-6-Chlor-2,5-dihydroxy-4-ethyl-2-(trifluormethyl)-1,2,3,4-tetrahydranphthalin-1-yl]amino}-1,3-dihydroindol-2-on und 4-{[(1S,2R,4R)-6-Chlor-2,5-dihydroxy-4-ethyl-2-(trifluormethyl)-1,2,3,4-tetrahydronaphthalin-1-yl]amino}-1,3-dihydroindol-2-on

Das in Beispiel 100 aufgeführte polare Diastereomer (115,6 mg) wird mittels chiraler HPLC (Chiralpak IB 5 µ, Laufmittel: Hexan/ Ethanol) in seine Enantiomeren getrennt. Erhalten werden 46,8 mg (40.5%) des (+)-Enantiomers ([α]_{D} = +110,8°, MeOH) und 54,6 mg (47.2%) des (-)-Enantiomers ([α]_{D} = -84,7°, MeOH). Die stark differierenden Drehwerte können durch die unterschiedliche Färbung der Messlösungen erklärt werden. Über die absolute Stereochemie kann natürlich keine Aussage gemacht werden.

### Beispiel 102

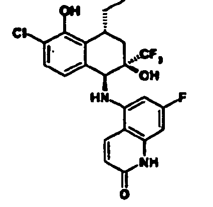

### 5-{[(1α,2α,4β)-6-Chlor-2,5-dihydroxy-4-ethyl-2-(trifluormethyl)-1,2,3,4-tetrahydronaphthalin-1-yl]amino}-7-fluor-1H-chinolin-2-on

Die Synthese dieser Verbindung wurde in Beispiel 101 beschrieben. Es wurden 70,1 mg (18.5%) der gewünschten Verbindung isoliert.

### Beispiele 102A und 102B

### (1R,2S,4S)-5-{[6-Chlor-4-ethyl-2,5-dihydroxy-2-(trifluormethyl)-1,2,3,4-tetrahydronaphthalin-1-yl]amino}-7-fluor-1H-chinolin-2-on und 5-{[(1S,2R,4R)-6-Chlor-2,5-dihydroxy-4-ethyl-2-(trifluormethyl)-1,2,3,4-tetrahydronaphthalin-1-yl]amino}-7-fluor-1H-chinolin-2-on

Das in Beispiel 102 beschriebene Diastereomer (120 mg) wird mittels chiraler HPLC (Chiralpak IB 5 µ, Laufmittel: Hexan/ Ethanol) in seine Enantiomeren getrennt. Erhalten werden 53,3 mg (44,3%) des (+)-Enantiomers ([α]_{D} = +78,2°, MeOH) und 52,7 mg (43,8%) des (-)-Enantiomers ([α]_{D} = -79,3°, MeOH). Über die absolute Stereochemie kann natürlich keine Aussage gemacht werden.

### Beispiel 103

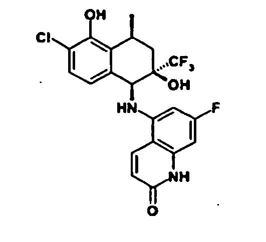

### 5-{[(1α,2α,4α)-6-Chlor-2,5-dihydroxy-4-methyl-2-(trifluormethyl)-1,2,3,4-tetrahydronaphthalin-1-yl]lamino}-7-fluor-1H-chinolin-2-on

5-{[4-(3-Chlor-2-methoxyphenyl)-2-hydroxy-2-trifluormethyl)pentyliden]amino}-7-fluor-1H-chinolin-2-on (382 mg, 0,81 mmol), hergestellt aus dem entsprechenden Aldehyd und 5-Amino-7-fluor-1*H-*chinolin-2-on, werden in 8,1 mL Dichlormethan gelöst und bei -50 °C tropfenweise mit 8,1 mL einer 1 M Lösung von Bortribromid in Dichlormethan versetzt (innerhalb von 20 Minuten). Nach drei Stunden Rühren bei -40 °C, zwei Stunden zwischen -40 und 0 °C, eine Stunde zwischen 0 °C und Raumtemperatur und 18 Stunden bei Raumtemperatur wird der Ansatz auf ein Gemisch aus gesättigter Natriumhydrogencarbonatlösung und Eis gegossen, mit 150 mL Ethylacetat verdünnt und anschließend 20 Minuten kräftig gerührt. Die Ethylacetatphase wird abgetrennt und die wässrige Phase ein weiteres Mal mit Ethylacetat (100 mL) extrahiert. Die vereinigten organischen Extrakte werden zweimal mit Wasser (je 40 mL) und einmal mit Sole (40 mL) gewaschen, getrocknet und das Lösungsmittel abrotiert. Nach Chromatographie am Flashmaster (Laufmittel: Dichlormethan/ Methanol) werden 330,7 mg eines Diastereomerengemisches erhalten, das aus der gewünschten Verbindung und der in Position 4 epimeren Verbindung besteht. Die Diastereomeren werden mittels HPLC getrennt (Chiralpak AD-H 5µ, Laufmittel: Hexan/ Ethanol). Erhalten werden 191,8 mg (51,8%) der gewünschten Verbindung und 95,9 mg (24,9%) der in Position 4 epimeren Verbindung (die Charakterisierung erfolgt in Beispiel 104), jeweils als Racemat.

### Beispiele 103A und 103B

### 5-{[(1R,2S,4R)-6-Chlor-2,5-dihydroxy-4-methyl-2-(trifluormethyl)-1,2,3,4-tetrahydronaphthalin-1-yl]amino}-7-fluor-1H-chinolin-2-on und 5-{[(1S,2R,4S)-6-Chlor-2,5-dihydroxy-4-methyl-2-(trifluormethyl)-1,2,3,4-tetrahydronaphthalin-1-yl]amino}-7-fluor-1H-chinolin-2-on

Das in Beispiel 103 beschriebene Diastereomer (160 mg) wird mittels chiraler HPLC (Chiralcel OD-H, Laufmittel: Hexan/ Ethanol) in seine Enantiomeren getrennt. Erhalten werden 81 mg (50,6%) des (+)-Enantiomers ([α]_{D} = +25,8°, MeOH) und 79,6 mg (49,8%) des (-)-Enantiomers ([α]_{D} = -28,4°, MeOH). Über die absolute Stereochemie kann natürlich keine Aussage gemacht werden.

### Beispiel 104

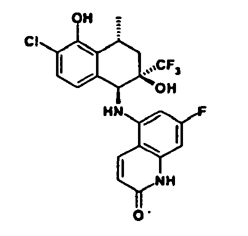

### 5-{[(1α,2a,4β)-6-Chlor-2,5-dihydroxy-4-methyl-2-(trifluormethyl)-1,2,3,4-tetrahydronaphthalin-1-yl]amino}-7-fluor-1H-chinolin-2-on

Die Synthese dieser Verbindung wurde in Beispiel 103 beschrieben. Es wurden 95,9 mg (24,9%) der gewünschten Verbindung isoliert.

### Beispiele 104A und 104B

### 5-{[(1R,2S,4S)-6-Chlor-2,5-dihydroxy-4-methyl-2-(trifluormethyl)-1,2,3,4-tetrahydronaphthalin-1-yl]amino}-7-fluor-1H-chinolin-2-on und 5-{[(1S,2R,4R)-6-Chlor-2,5-dihydroxy-4-ethyl-2-(trifluormethyl)-1,2,3,4-tetrahydronaphthalin-1-yl]amino}-7-fluor-1H-chinolin-2-on

Das in Beispiel 104 beschriebene Diastereomer (80 mg) wird mittels chiraler HPLC (Chiralcel OD-H 5 µ, Laufmittel: Hexan/ Ethanol) in seine Enantiomeren getrennt. Erhalten werden 40,1 mg (50,1%) des (+)-Enantiomers ([α]_{D} = +70,2°, MeOH) und 39,2 mg (49%) des (-)-Enantiomers ([α]_{D} = -67,2°, MeOH). Über die absolute Stereochemie kann natürlich keine Aussage gemacht werden.

### Beispiel 106

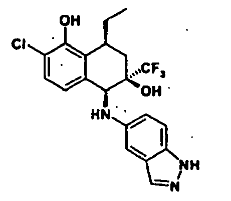

### (5α,6α,8α)-2-Chlor-8-ethyl-5-[(indazol-5-yl)amino]-6-(trifluormethyl)-5,6,7,8-tetrahydronaphthalin-1,6-diol

1,1,1-Trifluor-2-[(indazol-5-ylimino)methyl]-4-(3-chlor-2-methoxyphenyl)hexan-2-ol
(760 mg, 1,73 mmol), hergestellt aus dem entsprechenden Aldehyd und 5-Amino-indazol, werden in 7,6 mL Dichlormethan gelöst und bei -50 °C tropfenweise mit 17,28 mL einer 1 M Lösung von Bortribromid in Dichlormethan versetzt (innerhalb von 20 Minuten). Nach drei Stunden Rühren bei -40 °C, zwei Stunden zwischen -40 und 0 °C, einer Stunde zwischen
0 °C und Raumtemperatur und 18 Stunden bei Raumtemperatur wird der Ansatz auf ein Gemisch aus gesättigter Natriumhydrogencarbonatlösung und Eis gegossen, mit 150 mL Ethylacetat verdünnt und anschließend 20 Minuten kräftig gerührt. Die Ethylacetatphase wird abgetrennt und die wässrige Phase ein weiteres Mal mit Ethylacetat (100 mL) extrahiert. Die vereinigten organischen Extrakte werden zweimal mit Wasser (je 40 mL) und einmal mit Sole (40 mL) gewaschen, getrocknet und das Lösungsmittel abrotiert. Nach mehrfacher Chromatographie am Flashmaster (Laufmittel: Dichlormethan/ Methanol) werden 74,3 mg (10,1%) der gewünschten Verbindung und 236,4 mg (32,1%) der in Position 4 epimeren Verbindung erhalten (jeweils als Racemat). Die Charakterisierung der letzteren Verbindung erfolgt in Beispiel 107.
¹H-NMR (400 MHz, CD₃OD): δ = 1,06 (3H), 1,86 (1H), 1,95-2,11 (2H), 2,32 (1H), 3,05 (1H), 4,80 (1H), 6,75 (1H), 6,95-7,07 (3H), 7,32 (1H), 7,80 (1H).

### Beispiel 107

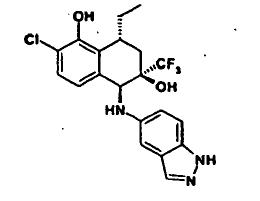

### (5α,6α,8β)-2-Chlor-8-ethyl-5-[(indazol-5-yl)amino]-6-(trifluormethyl)-5,6,7,8-tetrahydronaphthalin-1,6-diol

Die Synthese der Verbindung wurde in Beispiel 106 beschrieben. Erhalten wurden 236.4 mg (32,1%).
¹H-NMR (400 MHz, CD₃OD): δ = 0,92 (3H), 1,73 (1H), 1,87-2,051 (2H), 2,32 (1H), 3,33 (1H), 4,80 (1H), 6,75 (1H), 6,91 (2H), 7,06 (1H), 7,34 (1H), 7,74 (1H).

### Beispiel 108

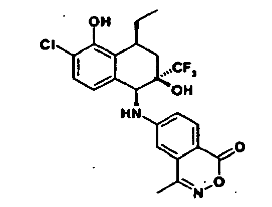

### 6-{[(1α,2α,4α)-6-Chlor-2,5-dihydroxy-4-ethyl-2-(trifluormethyl)-1,2,3,4-tetrahydronaphthalin-1-yl]amino}-4-methylbenzo[d][1,2]oxazin-1-on

6-{[4-(3-Chlor-2-methoxyphenyl)-2-hydroxy-2-trifluormethyl)hexyliden]amino}4-methylbenzo[d][1,2]oxazin-1-on (170 mg, 0,35 mmol), hergestellt aus dem in Beispiel 91 beschriebenen Aldehyd und 6-Amino-4-methylbenzo[d][1,2]oxazin-1-on, werden in 3,5 mL Dichlormethan gelöst und bei -50 °C tropfenweise mit 3,52 mL einer 1 M Lösung von Bortribromid in Dichlormethan versetzt (innerhalb von 20 Minuten). Nach zwei Stunden Rühren bei -40 °C, einer Stunde zwischen -40 und -20 °C, einer Stunde zwischen -20 und -10 °C und einer Stunde zwischen-10 und 0 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden zweimal mit Wasser (je 20 mL) und einmal mit Sole (20 mL) gewaschen, getrocknet und das Lösungsmittel abrotiert. Nach Chromatographie am Flashmaster (Isolute NH₂, Laufmittel: Dichlormethan/ Methanol) werden 10,2 mg (6.2%) der gewünschten Verbindung (leicht verunreinigt) und 26,4 mg (16%) der in Position 4 epimeren Verbindung erhalten (jeweils als Racemat). Die Charakterisierung der letzteren Verbindung erfolgt in Beispiel 109.
¹H-NMR (300 MHz, CD₃OD): δ = 1,13 (3H), 1,75-2,28 (3H), 2,38 (1H), 2,49 (1H), 3,11 (1H) 5.24 (1H), 6,78 (1H), 7,06 (1H), 7,15 (1H), 7,30 (1H), 8,05 (1H).

### Beispiel 109

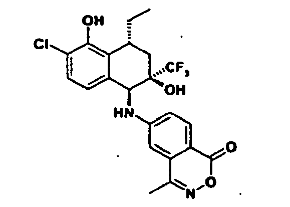

### 6-{[(1α,2α,4β)-6-Chlor-2,5-dihydroxy-4-ethyl-2-(trifluormethyl)-1,2,3,4-tetrahydronaphthalin-1-yl]amino}-4-methylbenzo[d][1,2]oxazin-1-on

Die Synthese der Verbindung wurde in Beispiel 108 beschrieben. Erhalten wurden 26,4 mg (16%).
¹H-NMR (300 MHz, CD₃OD): δ = 0,99 (3H), 1,78 (1H), 1,98-2,15 (2H), 2,36-2,51 (4H), 3,37 (1H, liegt teilweise unter dem Löungsmittelsignal), 5,22 (1H), 6,81 (1H), 7,02 (1H), 7,15 (1H), 7,25 (1H), 8,08 (1H).

### Beispiel 111

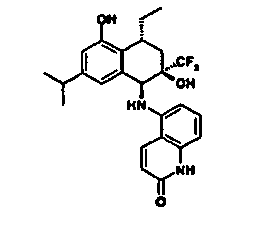

### 5-{[(1α,2α,4β)-7-Isoapropyl-2,5-dihydroxy-4-ethyl-2-(trifluormethyl)-1,2,3,4-tetrahydronaphthalin-1-yl]amino}-1H-chinolin-2-on

*5-*{*[4-(4-Isopropyl-2-methoxyphenyl)-2-hydroxy-2-trifluormethyl)hexyliden]amino}-1H-chinolin-2-on*

Das Gemisch aus 4-(4-Isopropyl-2-methoxyphenyl)-2-hydroxy-2-(trifluormethyl)-hexanal und 4-(4-Isopropyl-2-methoxyphenyl)-2-hydroxy-3-methyl-2-(trifluormethyl)-pentanal (600 mg, 1,8 mmol), 5-Amino-1H-chinolin-2-on (287,4 mg, 1,79 mmol) und 2,67 mL Essigsäure werden 11 Tage bei Raumtemperatur gerührt. Das Reaktionsgemisch wird dreimal mit Toluol abgezogen und der Rückstand chromatographiert (Flashmaster, Laufmittel: Ethylacetat/ Hexan). Isoliert werden 554,1 mg (65,1%) des gewünschten Imins, und zwar als Gemisch von 5-{[4-(4-Isopropyl-2-methoxyphenyl)-2-hydroxy-2-trifluormethyl)hexyliden]amino}-1H-chinolin-2-on und 5-{[4-(4-Isopropyl-2-methoxyphenyl)-2-hydroxy-3-methyl-2-trifluormethyl)pentyliden]amino}-1H-chinolin-2-on.

Das Gemisch aus 5-{[4-(4-Isopropyl-2-methoxyphenyl)-2-hydroxy-2-trifluormethyl)hexyliden]amino}-1H-chinolin-2-on und 5-{[4-(4-Isopropyl-2-methoxyphenyl)-2-hydroxy-3-methyl-2-trifluormethyl)pentyliden]amino}-1H-chinolin-2-on (554,1 mg, 1,16 mmol) wird in 5,1 mL Dichlormethan gelöst und bei -20 °C tropfenweise mit 11,68 ml einer 1 M Lösung von Bortribromid in Dichlormethan versetzt. Nach dreieinhalbstündigem Rühren im Temperaturbereich zwischen -20 und 0 °C wird der Ansatz auf ein Gemisch aus gesättigte Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser(20 mL) und Sole (20 mL) gewaschen getrocknet und das Lösungsmittel abrotiert. Nach mehrmaliger Chromatographie (Flashmaster, unterschiedliche Laufmittelsysteme) werden 30,1 mg (11,25%) der gewünschten Verbindung erhalten.
1H-NMR (300 MHz, CD₃OD): δ = 0,91 (3H), 1,00-1,09 (6H), 1,69 (1H), 1,88-2,04 (2H), 2,33 (1H). 2,62 (1H), 3,24 (1H, liegt zum Teil unter dem Lösungsmittelsignal), 4,99 (1H), 6,44-6,51 (2H), 6,58 (1H), 6,60-6,70 (2H), 7,32 (1H), 8,20 (1H).

### Beispiel 112

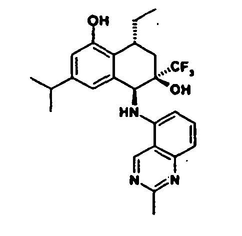

### (5α.6α.8β)-8-Ethyl-3-isopropyl-5-[2-methylchinazolin-5-ylamino]-6-(trifluormethyl)-5,6,7, 8-tetrahydronaphthalin-1,6-diol

Das Gemisch aus 1,1,1-Trifluor-2-[(2-methylchinazolin-5-ylimino)-methyl-]-4-(4-isopropyl-2-methoxyphenyl)-hexan-2-ol und 1,1,1-Trifluor-3-methyl-2-[(2-methylchinazolin-5-ylimino)-methyl-]-4-(4-isopropyl-2-methoxyphenyl)-pentan-2-ol (558,2 mg, 1,18 mmol), hergestellt aus dem Gemisch der in Beispiel 110 beschriebenen Aldehyde und 5-Amino-2-methylchinazolin, wird in 5,1 mL Dichlormethan gelöst und bei -20 °C tropfenweise mit 11,79 ml einer 1 M Lösung von Bortribromid in Dichlormethan versetzt. Nach dreieinhalbstündigem Rühren im Temperaturbereich zwischen -20 und +5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie (Flashmaster, unterschiedliche Phasen, Laufmittel: Dichlormethan/ Methanol) werden 30,5 mg (11,3%) der gewünschten Verbindung erhalten.
1H-NMR (400 MHz, CD₃OD) δ = 0,92 (3H), 1,00-1,08 (6H), 1,70 (1H), 1,91-2,08 (2H), 2,38 (1H), 2,60 (1H), 3,29 (1H, liegt zum Teil unter dem Lösungsmittelsignal), 5,12 (1H), 6,55-6,65 (2H), 6,78 (1H), 7,13 (1H), 7,74 (1H), 9,59 (1H).

### Beispiel 113

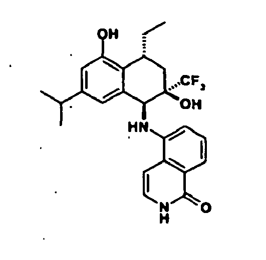

### 5-{(1α.2α.4β)-7-Isopropyl-2,5-dihydroxy-4-ethyl-2-(trifluormethyl)-1,2,3,4-tetrahydronaphthalin-1-ylamino}-2H-chinolin-1-on

Das Gemisch aus 5-{[4-(4-Isopropyl-2-methoxyphenyl)-2-hydroxy-2-trifluormethyl)hexyliden]amino}-2H-chinolin-1-on und 5-{[4-(4-Isopropyl-2-methoxyphenyl)-2-hydroxy-3-methyl-2-trifluormethyl)pentyliden]amino}-2H-chinolin-1-on (500,8 mg,
1,06 mmol), hergestellt aus dem Gemisch der in Beispiel 110 beschriebenen Aldehyde und 5-Amino-2H-chinolin-1-on, wird in 4,6 ml Dichlormethan gelöst und bei -20 °C tropfenweise mit 10,6 mL einer 1 M Lösung von Bortribromid in Dichlormethan versetzt. Nach dreieinhalbstündigem Rühren im Temperaturbereich zwischen -20 und +5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach Chromatographie am Ftashmaster (NH₂-Phase, Laufmittel: Dichlormethan/ Methanol) werden 29,1 mg (12%) der gewünschten Verbindung erhalten.
1H-NMR (300 MHz, CD₃OD): δ = 0,91 (3H), 0,96-1,08 (6H), 1,69 (1H), 1,91-2,04 (2H), 2,33 (1H), 2,60 (1H). 3,25 (1H, liegt zum Teil unter dem Lösungsmittelsignal), 4,98 (1H), 6,58 (1H), 6,62 (1H), 6,81-6,93 (2H), 7,13 (1H), 7,32 (1H), 7,62 (1H).

### Beispiel 114

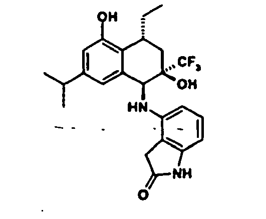

### 4-{[(1α.2α,4β)-7-Isopropyl-2,5-dihydroxy-4-ethyl-2-(trifluormethyl)-1,2,3,4-tetrahydronaphthalin-1-y]lamino}-r1,3-dihydroindol-2-on

Das Gemisch aus 5-{[4-(4-Isopropyl-2-methoxyphenyl)-2-hydroxy-2-trifluormethyl)hexyliden]amino}-1,3-dihydroindol-2-on und 5-{[4-(4-Isopropyl-2-methoxyphenyl)-2-hydroxy-3-methyl-2-trifluormethyl)pentyliden]amino}-1,3-dihydroindol-2-on (578,6 mg, 1,26 mmol), hergestellt aus dem Gemisch der in Beispiel 110 beschriebenen Aldehyde und 4-Amino-1,3-dihydroindol-2-on, wird in 5,5 mL Dichlormethan gelöst und bei
-20 °C tropfenweise mit 12,5 mL einer 1 M Lösung von Bortribromid in dichlormethan versetzt. Nach dreieinhalbstündigem Rühren im Temperaturbereich zwischen -20 und +5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach Chromatographie am Flashmaster (NH₂-Phase, Laufmittel: Dichlormethan/Methanol) werden 38,6 mg (13,8%) der gewünschten Verbindung erhalten.
1H-NMR (400 MHz, CD₃oD): δ = 0,90 (3H), 1.03-1.11 (6H), 1,68 (1H), 1,83-2,00 (2H), 2,30 (1H), 2,65 (1H), 3,19-3,40 (3H, liegen zum Teil unter dem Wassersignal), 4,89 (1H), 6,29, (1H), 6,35 (1H), 6,55 (1H), 6,69 (1H), 7,03 (1H).

### Beispiel 115

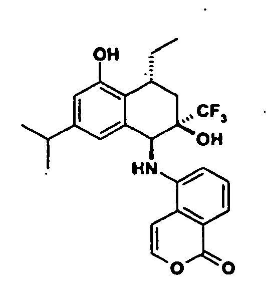

### 5-{[(1α,2α,4β)-7-Isopropyl-2,5-dihydroxy-4-ethyl-2-(trifluormethyl)-1,2,3,4-tetrahydronaphthalin-1-yl]amino}-isochromen-1-on

Das Gemisch aus 5-{[4-(4-Isopropyl-2-methoxyphenyl)-2-hydroxy-2-trifluormethyl)hexyliden]amino}-isochromen-1-on und 5-{[4-(4-Isopropyl-2-methoxyphenyl)-2-hydroxy-3-methyl-2-trifluormethyl)pentyliden]amino}-isochromen-1-on (678,4 mg,
1,42 mmol), hergestellt aus dem Gemisch der in Beispiel 110 beschriebenen Aldehyde und 5-Amino-isochromen-1-on, wird in 6,22 mL Dichlormethan gelöst und bei -20 °C tropfenweise mit 14,3 mL einer 1 M Lösung von Bortribromid in Dichlormethan versetzt. Nach dreieinhalbstündigem Rühren im Temperaturbereich zwischen -20 und +5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrfacher Chromatographie am (NH₂-Phase, Laufmittel: Dichlormethan/ Methanol) werden 9,2 mg (2,8%) der gewünschten Verbindung erhalten.
1H-NMR (400 MHz, CD₃OD): δ = 0,91 (3H), 0,98-1,11 (6H), 1,67 (1H), 1,99-2,05 (2H), 2,32 (1H), 2,62 (1H), 3,25 (1H, liegt zum Teil unter dem Lösungsmittelsignal), 4,97 (1H), 6,56- 6,62 (2H), 6,87 (1H), 7,02 (1H), 7,31-7,45 (2H), 7,58 (1H).

### Beispiel 116

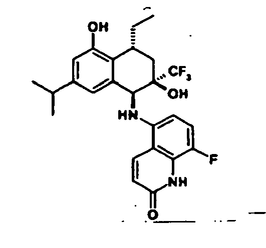

### 5-{[(1α.2α.4β)-7-Isoropyl-2,5-dihydroxy-4-ethyl-2-(trifluormethyl)-1,2,3,4-tetrahydronaphthalin-1-yl]amino}-8-fluor-1H-chinolin-2-on

Das Gemisch aus 5-{[4-(4-Isopropyl-2-methoxyphenyl)-2-hydroxy-2-trifluormethyl)hexyliden]amino}-8-fluor-1H-chinolin-2-on und 5-{[4-(4-Isopropyl-2-methoxyphenyl)-2-hydroxy-3-methyl-2-trifluormethyl)pentyliden]amino}-8-fluor-1H-chinolin-2-on (680 mg, 1,38 mmol), hergestellt aus dem in Beispiel 110 beschriebene Gemisch der Aldehyde und 5-Amino-8-fluor-1H-chinolin-2-on, wird in 6,8 mL Dichlormethan gelöst und bei -10 °C tropfenweise mit 13,81 ml einer 1 M Lösung von Bortribromid in Dichlormethan versetzt. Nach dreieinhalbstündigem Rühren im Temperaturbereich zwischen -10 und +5 °C wird der Ansatz auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, getrocknet und das Lösungsmittel abrotiert. Nach mehrmaliger Chromatographie inklusive HPLC (verschiedene Phasen, unterschiedliche Laufmittel) werden 31 mg (9,4%) der gewünschten Verbindung und 9,9 mg (3%) der in Position 4 epimeren Verbindung erhalten (Charakterisierung erfolgt in Beispiel 117).
1H-NMR (300 MHz, CD₃OD): δ = 0,91 (3H), 1,00-1,10 (6H), 1,68 (1H), 1,88-2,03 (2H), 2,32 (1H), 2,62 (1H), 3,25 (1H, liegt zum Teil unter dem Lösungsmittelsignal), 4,92 (1H), 6,39 (1H), 6,49-6,63 (3H), 7,19 (1H), 8,19 (1H).

### Beispiel 117

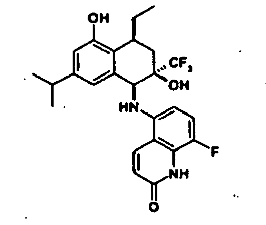

### 5-{[(1α,2α,4β)-7-Isopropyl-2,5-dihydroxy-4-ethyl-2-(trifluormethyl)-1,2,3;4-tetrahydroriaphthalin-1-yl]}amino}-8-fluor-1H-chinolin-2-on

Die Synthese der Verbindung wurde in Beispiel 116 beschrieben. Erhalten werden 9,9 mg (3%) der gewünschten Verbindung.
1H-NMR (300 MHz, CD₃OD): δ = 1,05-1,18 (9H), 1,91 (1H), 2,04-2,20 (2H), 2,40 (1H), 2,67 (1H), 2,99 (1H), 4,99 (1H), 6,50-6,69 (4H), 7,28 (1H), 8,20 (1H).

### Beispiel 118

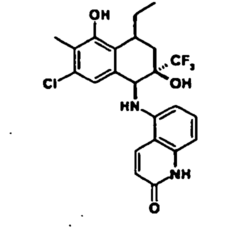

### 5-{[(1α,2α,4α)-7-Chlor-2,5-dihydroxy-4-ethyl-6-methyl-2-etrifluormethyl}-1,2,3,4-tetrahydronaphthalin-1-yl]amino}-1H-chinolin-2-on

*4-(4-Chlor-3-methyl-2-methoxyphenyl)-2-hydroxy-2-(trifluormethyl)-hexanal* 1 g (2,93 mmol) 4-(4-Chlor-3-methyl-2-methoxyphenyl)-2-(trifluormethyl)-hexan-1,2-diol (hergestellt in Analogie zu der in Beispiel 110 beschriebenen Sequenz: Acetylierung des entsprechenden Phenols, Friesscbe Verschiebung der Acetylgruppe, Veretherung des Phenols, Wittig-Umsetzung, En-Reaktion, Reduktion des Esters zum Alkohol) werden in der üblichen Weise mit SO₃/ Pyridin Komplex umgesetzt. Nach dreistündigem Rühren bei Raumtemperatur wird die Reaktionsmischung auf ein Gemisch aus gesättigter NH₄CL- Lösung und Eis gegossen. Es wird dreimal mit Methyl-tert.butylether extrahiert. Die vereinigten organischen Extrakte werden zweimal mit Sole gewaschen und getrocknet (Na₂SO₄). Nach dem Abrotieren des Lösungsmittels wird der Rückstand mittels Flashchromatographie gereinigt (Kieselgel, Laufmittel: Hexan/ Ethylacetat). Erhalten werden 290 mg (29,2%) des gewünschten Aldehyds als Gemisch der beiden Diastereomeren (jeweils als Racemat).

*5-{[4-(4-Chlor-3-methyl-2-methoxyphenyl)-2-hydroxy-2-(trifluormethyl)hexyliden]amino}-1H-chinolin-2-on* 4-(4-Chlor-3-methyl-2-methoxyphenyl)-2-hydroxy-2-(tritluormethyl)-hexanal (290 mg, 0.86 mmol), 5-Amino-1H-chinolin-2-on (137.4 mg, 0.86 mmol) und 3 mL Essigsäure werden zwei Tage bei Raumtemperatur gerührt. Das Reaktionsgemisch wird zweimal mit Toluol und Dichlormethan abgezogen und der Rückstand chromatographiert (Flashmaster, Laufmittel: Dichlormethan/ Methanol). Isoliert werden 327,8 mg (79,5%) des gewünschten Imins, 5-{[4-(4-Chlor-3-methyl-2-methoxyphenyl)-2-hydroxy-2-trifluormethyl)hexyliden]amino}-1H-chinolin-2-on als Gemisch der beiden Diastereomeren (jeweils als Racemat). Das im vorigen Abschnitt beschriebene Imin, 5-{[4-(4-Chlor-3-methyl-2-methoxyphenyl)-2-hydroxy-2-trifluormethyl)hexyliden]amino}-1H-chinolin-2-on (327,8 mg (0,68 mmol) wird in 3,2 mL Dichlormethan vorgelegt und bei -40 °C tropfenweise mit 6,82 mL einer 1 M Lösung von BBr₃ in Dichlormethan versetzt. Nach drei Stunden Rühren im Temperaturbereich zwischen -40 und +10 °C ist kein Ausgangsmaterial mehr vorhanden. Die Reaktionsmischung wird vorsichtig auf eine Mischung aus gesättigter NaHCO₃ -Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels chromatographiert. Isoliert werden nach mehrmaliger Chroamtographie (unterschiedliche Systeme, Laufmittel: Dichlonnethan/Methanol) 9,3 mg (2,9%) der gewünschten Verbindung (verunreinigt) und 39,2 mg (12,3%) der in der Position 4 epimeren Verbindung (siehe Beispiel 119).
1H-NMR (300 MHz, CD₃OD): δ = 1,08 (3H), 1,85-2,10 (3H), 2,22 (3H), 2,40 (1H), 3,01 (1H), 5,08 (1H), 6,48 (1H), 6,59 (1H), 6,68 (1H), 6,82 (1H), 7,38 (1H), 8,19 (1H).

### Beispiel 119

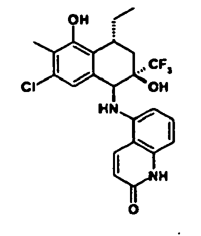

### 5-{[(1α,2α,4β)-7-Chlor-2,5-dihydroxy-4-ethyl-6-methyl-2-(trifluormethyl)-1,2,3,4-tetrahydronaphthalin-1-yl]amino}-1H-chinolin-2-on

39,2 mg (12,3%) der im Titel genannten Verbindung wurden erhalten (siehe Beispiel 118), und zwar als Racemat. Die Racemätspaltung wird im folgenden Beispiel beschrieben.

### Beispiele 119A und 11 9B

### 5-{[(1R, 2S,4S)-7-Chlor-2,5-dihydroxy-6-methyl-4-ethyl-2-(trifluormethyl)-1,2,3,4-tetrahydronaphthalin-1yl]amino}-1H-chinolin-2-on und 5-{[(1S,2R,4R)-7-Chlor-2,5-dihydroxy-6-methyl-4-ethyl-2-(trifluormethyl)-1,2,3,4-tetrahydronaphthalin-1-yl]amino}-1H-chinolin-2-on

Die in Beispiel 119 aufgeführte Verbindung (104 mg) wird mittels chiraler HPLC (Chiralpak IA 5 µ, Laufmittel Hexan/ Ethanol) in ihre Enantiomeren getrennt. Erhalten werden 43,2 mg (41,5%) des einen Enantiomers (Retentionszeit: 10,5 - 12,7 Minuten) und 40,7 mg (45,2%) des anderen Enantiomers (Retentionszeit: 15,1-18 Minuten). Über die absolute Stereochemie kann natürlich keine Aussage gemacht werden.

### Beispiel 120

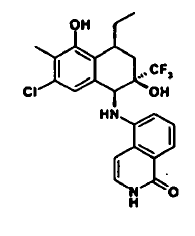

### 5-{[(1α,2α,4α-)-7-Chlor-2,5-dihydroxy-6-methyl-4-ethyl-2-(trifluormethyl)-1,2,3,4-tetrahydronaphthalin-1-yl]amino}-2H-chinolin-1-on

5-{[4-(4-Chlor-3-methyl-2-methoxyphenyl)-2-hydroxy-2-trifluormethyl)hexyliden]amino}-2H-chinolin-1-on (366 mg, 0,76 mmol), hergestellt aus dem in-Beispiel 118 beschriebenen Aldehyd und 5-Amino-2H-chinolin-1-on, wird in 3,7 mL Dichlormethan gelöst und bei -40 °C tropfenweise mit 7,62 mL einer 1 M Lösung von Bortribromid in Dichlormethan versetzt. Nach drei Stunden Rühren im Temperaturbereich zwischen -40 und +10 °C ist kein Ausgangsmaterial mehr vorhanden. Die Reaktionsmischung wird vorsichtig auf eine Mischung aus gesättigter NaHCO₃ -Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels mehrfach chromatographiert. Isoliert werden 10,5 mg (3%) der gewünschten Verbindung (ein wenig verunreinigt) und 12,7 mg (3,6%) der in Position 4 epimeren Verbindung (siehe Beispiel 121).
1H-NMR (300 MHz, CD₃OD): δ = 1,09 (3H), 1,82-2,11 (3H), 2,22 (3H), 2,39 (1H), 3,02 (1H), 5,03 (1H), 6,79-6,83 (2H), 7,07 (1H), 7,13 (1H), 7,38 (1H), 7,69 (1H).

### Beispiel 121

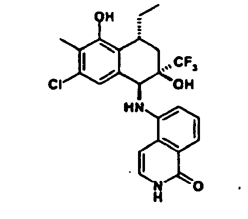

### 5-{[1α,2α,4β)-7-Chlor-2,5-dihydroxy-6-methyl)-4-ethyl-2-(trifluormethyl-1,2,3,4-tetrahydronaphthalin-1-yl]amino}-2H-chinolin-1-on

12,7 mg (3,6%) der im Titel genannten Verbindung wurden erhalten (siehe Beispiel 120), und zwar als Racemat. Die Racematspaltung wird im folgenden Beispiel beschrieben.

### Beispiele 121A und 121B

### 5-{[(1R,2S,4S)-7-Chlor-2,5-dihydroxy-6-methyl-4-ethyl-2-(trifluormethyl)-1,2,3,4-tetrahydronaphthalin-1-yl]amino}-2H-chinolin-1-on und 5-{[(1S,2R,4R)-7-Chlor-2,5-dihydroxy-6-methyl-4-ethyl-2-(trifluormethyl)-1,2,3,4-tetrahydronaphthalin-1-yl]amino}-2H-chinolin-1-on

Die in Beispiel 121 aufgeführte racemische Verbindung (64 mg) wird mittels chiraler HPLC (Chiralpak IA 5 µ, (Laufmittel Hexan/ Ethanol) in ihre Enantiomeren getrennt. Erhalten werden 28,1 mg (43,9%) des einen Enantiomers (Retentionszeit: 13,8 -16 Minuten) und
33,3 mg (52%) des anderen Enantiomers (Retentionszeit: 16-20 Minuten, enthält noch 13,6% einer Verunreinigung und 1,6% des Enantiomers mit der kürzeren Retentionszeit). Über die absolute Stereochemie kann natürlich keine Aussage gemacht werden.

### Beispiel 123

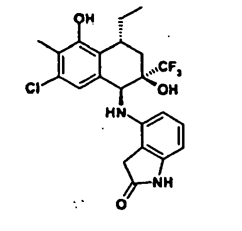

### 4-{[(1α,2α,4β)-7-Chlor-2,5-dihydroxy-6-methyl-4-ethyl-2-(trifluormethyl)-1,2,3,4-tetrahydronaphthalin-1-yl]amino}-1,3-dihydroindol-2-on

5-{[4-(4-Chlor-3-methyl-2-methoxyphenyl)-2-hydroxy-2-trifluormethyl)hexyliden]amino}1,3-dihydtoindol-2-on (295,8 mg, 0,63 mmol), hergestellt aus dem in Beispiel 118 beschriebenen Aldehyd und 5-Amino-1,3-dihydroindol-2-on, wird in 3 mL Dichlormethan gelöst und bei -40°C. tropfenweise mit 6,3 mL einer 1 M Lösung von Bortribromid in Dichlormethan versetzt. Nach drei Stunden Rühren im Temperaturbereich zwischen -40 und +10°C ist kein Ausgangsmaterial mehr vorhanden. Die Reaktionsmischung wird vorsichtig auf eine Mischung aus gesättigter NaHCO₃ -Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels mehrfach chromatographiert. Isoliert werden 19,1 mg (6,6%) der Titelverbindung.
1H-NMR (300 MHz, CD₃OD): δ = 0,90 (3H), 1,69 (1H), 1,80-2,00 (2H), 2,22 (3H), 2,30 (1H), 3,25-3,43. (3H, die Signale liegen teilweise unter dem Lösungsmittelsignal), 4,89 (1H), 6,28-6,32 (2H), 6,89 (1H), 7,05 (1H).

### Beispiel 124

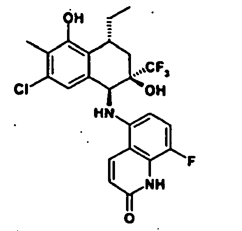

### 5-{[(1α,2α,4β)-7-Chlor-2,5-dihydroxy-4-ethyl-6-methyl-2-(trifluormethyl)-1,2,3,4-tetrahydronaphthalin-1-yl]amino}-8-fluor-1H-chinolin-2-on

5-{[4-(4-Chlor-3-methyl-2-methoxyphenyl)-2-hydroxy-2-trifluormethyl)hexyliden]amino}-8-fluor-1H-chinolin-2-on (628,8 mg, 1,26 mmol), hergestellt aus dem in Beispiel 118 beschriebenen Aldehyd und 5-Amino-8-fluor-1H-chinolin-2-on, wird in 7,3 mL Dichlormethan gelöst und bei -40 °C tropfenweise mit 12,6 mL einer 1M Lösung von Bortribromid in Dichlormethan versetzt. Nach drei Stunden Rühren im Temperaturbereich zwischen -40 und +10 °C ist kein Ausgangsmaterial mehr vorhanden. Die Reaktionsmischung wird vorsichtig auf eine Mischung aus gesättigter NaHCO₃-Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels zweimal chromatographiert (Flashmaster, Laufmittel: Dichlormethan/ Methanol). Isoliert werden 52,8 mg (8,6%) der gewünschten Verbindung.

### Beispiele 124A und 124B

### 5-{[(1R, 2S,4S)-7-Chlor-2,5-dihydroxy-4-ethyl-6-methyl-2-(trifluormethyl)-1,2,3,4-tetrahydronaphthalin-1-yl]amino}-8-fluor-1H-chinolin-2-on und 5-{[(1S,2R,4R)-7-Chlor-2,5-dihydroxy-4-ethyl-6-methyl-2-(trifluormethyl)-1,2,3,4-tetrahydronaphthalin-1-yl]amino}-8-fluor-1H-chinolin-2-on

Die in Beispiel 124 aufgeführte racemische Verbindung (35,4 mg) wird mittels chiraler HPLC (Chiralpak IA 5 µ, Laufmittel Hexan/ Ethanol) in seine Enantiomeren getrennt. Erhalten werden 14,5 mg (41%) des einen Enantiomers (Retentionszeit: 9,1 -10,4 Minuten) und 17,5 mg (49,4%) des anderen Enantiomers (Retentionszeit: 10,9-13,2 Minuten). Über die absolute Stereochemie kann natürlich keine Aussage gemacht werden.

### Beispiel 127

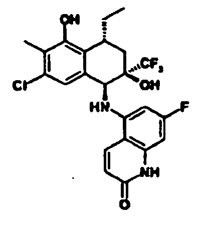

### 5-{[(1α,2a,4β)-7-Chlor-2,5-dihydroxy-4-ethyl-6-methyl-2-(trifluormethyl)-1,2,3,4-tetrahydronaphthalin-1 -yl]amino}-7-fluor-1H-chinolin-2-on

38,7 mg (36,9%) der im Titel genannten Verbindung wurden erhalten (siehe Beispiel 126), und zwar als Racemat. Die Racematspaltung wird im folgenden Beispiel beschrieben.

### Beispiele 127A und 1278

### 5-{[(1R,2S,4S)-7-Chlor-2,5-dihydroxy-4-ethyl-6-methyl-2-(trifluormethyl)-1,2,3,4-tetrahydronaphthalin-1-yl]amino}-7-fluor-1H-chinolin-2-on und 5-{[(1S,2R,4R)-7-Chlor-2,5-dihydroxy-ethyl-6-methyl-2-(trifluormethyl)-1,2,3,4-tetrahydronaphthalin-1-yl]amino}-7-fluor-1H-chinotin-2-on

27,2 mg der in Beispiel 127 aufgeführten Verbindung werden mittels chiraler HPLC (Chiralpak IA 5 µ, Laufmittel Hexan/ Ethanol) in ihre Enantiomeren getrennt. Erhalten werden 7,7 mg (28,3%) des einen Enantiomers (Retentionszeit: 11,2 -14,5 Minuten) und 10,4 mg (38,2%) des anderen Enantiomers (Retentionszeit: 14,5-17,9 Minuten). Über die absolute Stereochemie kann natürlich keine Aussage gemacht werden.

### Beispiel 129

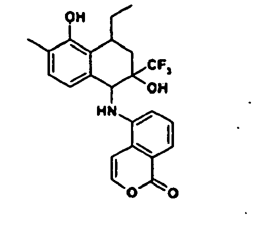

### 5-{[4-Ethyl)-2,5-dihydroxy-6-methyl-2-(trifluormethyl)-1,2,3,4-tetrahydronaphthalin-1-yl]amino}isochromen-1-on

5-{[4-(4-Chlor-3-methyl-2-methoxyphenyl)-2-hydroxy-2-trifluormethyl)hexyliden]amino}isochromen-1-on (408,6 mg, 0,85 mmol), hergestellt aus dem in Beispiel 118 beschriebenen Aldehyd und 5-Amino-isochromen-1-on, wird in 4 mL Dichlormethan vorgelegt und bei -40 °C tröpfenweise mit 8,48 mL einer 1M Lösung von BBr₃ in Dichlormethan versetzt. Nach dreistündigem Rühren im Temperaturbereich zwischen -40 und +10 °C wird der Ansatz auf ein Gemisch aus gesättigter
Natriumhydrogencarbonatlösung und Eis gegossen und dreimal mit Ethylacetat extrahiert. Nach dem Waschen der vereinigten organischen Extrakte mit Sole wird getrocknet (Na₂SO₄) und das Lösungsmittel abrotiert. Nach mehrfacher Chromatographie (Flashmaster, unterschiedliche Phasen, Laufmittel: Dichlormethan/ Methanol) werden 13,3 mg (3,6%) der Titelverbindung als Stereoisomerengemisch erhalten. Diese Deschloroverbindung ist während der Herstellung des Aldehyds entstanden, wahrscheinlich bei einem der Reduktionsschritte.

### Beispiel 130

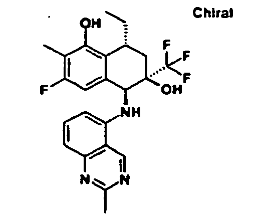

### (5S,6R,8R)-8-Ethyl-2,3-difluor-5-[(2-methilchinazolin-5-yl)amine 6-(trifluormethyl)-5,6,7,8-tetrahydronaphthalin-1,6-diol

### 4-(4-Fluor 2-methoxy-3-methylphenyl)-2-hydroxy-2-(trifluormethyl)hexanal:

Zu 18.6 g (147 mmol) 3-Fluor-2-methylphenol (Lock et al. Chem. Ber. 1936, 69, 2253-55) in 150 ml Dichlormethan und 16.6 ml Pyridin werden bei 0°C 13.5 ml (155 mmol) Propionsäurechlorid getropft. Man rührt die Mischung zwei Stunden lang und gibt 100 ml einer 2 M Salzsäure zu. Es wird mit Dichlormethan extrahiert und mit Wasser gewaschen. Nach dem Trocknen über Natriumsulfat und dem Entfernen des Lösungsmittels im Vakuum erhält man 20.6 g Propionsäure-3-fluor-2-methylphenylester. 20.6 g (113 mmol) Propionsäure-3-fluor-2-methylphenylester in 12 ml 1,2-Dichlorbenzol werden zu 15.1 g (113 mmol) Aluminiumtrichlorid in 12 ml 1,2-Dichlorbenzol getropft und die Mischung wird im Anschluss 6 Stunden lang bei 100°C gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit gesättigter Natriumchlorid Lösung gewaschen und über Natriumsulfat getrocknet. Das Rohprodukt wird säulenchromatographisch an Kieselgel (Hexan / Ethylacetat 10-20%) gereinigt und man erhält 19 g 1-(4-Fluor-2-hydroxy-3-methylphenyl)propan-1-on. 7.96 g (43.8 mmol) 1-(4-Fluor-2-hydroxy-3-methylphenyl)-propan-1-on werden in 65 ml Aceton gelöst und es werden 11.3 g Kaliumcarbonat und 4.96 ml (79 mmol) Methyliodid zugegeben. Die Mischung wird 20Stunden unter Rückfluß gekocht, wobei nach 16 Stunden weitere 3 g Kaliumcarbonat und 1.5 ml (22 mmol) Methyliodid nachgegeben werden. Das Lösungsmittel wird zu einem größen Teil entfernt, man gießt den Rückstand in Wasser und extrahiert mit Diethylether. Es wird mit Wasser gewaschen, über Natriumsulfat getrocknet und nach dem Entfernen des Lösungsmittels im Vakuum erhält man 7.9 g 1-(4-Fluor-2-methoxy-3-methylphenyl)propan-1-on. 27,3 g (416 mmol) Zinkstaub und 570 mg (2.05 mmol) Blei(II)-chlorid werden in 211 ml THF suspendiert und bei Raumtemperatur werden 25.4 ml (364 mmol) Dibrommethan zugegeben. Man rührt weitere 30 Minuten bei Raumtemperatur und tropft bei 0°C 49 ml (49 mol) einer 1 M Titan(IV)-chlorid Lösung in Dichlormethan zu. Das Kühlbad wird entfernt und nach einer Stunde wird die Reaktionsmischung wieder auf 0°C gekühlt. 9.5 g (48 mmol) 1-(4-Fluor-2-methoxy-3-methylphenyl)propan-1-on in 28 ml THF zugetropft. Man rührt zwei weitere Stunden bei Raumtemperatur. Es wird mit Diethylether verdünnt und das Reaktionsgemisch wird vorsichtig auf eine Mischung von 4 M Salzsäure und Eis gegeben. Man trennt die Phasen, extrahiert mit Diethylether, wäscht mit gesättigter Natriumchlorid Lösung, trocknet über Natriumsulfat und entfernt das Lösungsmittel. Das Rohprodukt wird säulenchromatographisch an Kieselgel (Hexan / Diisopropylether 0-20%) gereinigt und man erhält 5.7 g 3-Fluor-2-methyl-6-(1-methylenpropyl)anisol.
¹H-NMR (300 MHz, CDCl₃); δ = 1.01 (t, 3H), 2.20 (d, 1H), 2.47 (qdd, 2H), 3.68 (s, 1H), 5.02 (ddd, 1H), 5.14 (ddd,1H), 6.76 (dd, 1H), 6.94 (dd, 1H). Zu 5.7 g (29.3 mmol) 3-Fluor-2-methyl-6-(1-methylenpropyl)anisol, 7.7 ml (170 mmol) Ethyltrifluorpyruvat und 15 g Molekularsieb werden bei 0°C über 30 Minuten 0.76 g (0.88 mmol) [Cu(R,R)-2,2-bis(4,5-dihydro-4-tert-butyloxazolin-2-yl)propan)(H₂O)₂]((SbF₆)₂. in 38 ml Dichlormethan getropft. Man rührt die Reaktionsmischung 16 Stunden bei 0°C und reinigt die Reaktionsmischung mittels Säulenchomatographie an Kieselgel (Hexan/Ethytacetat 0-20%); Man erhält 8.7 g (*R*)-4-(4-Fluor-2-methoxy-3-methylphenyl)-2-hydroxy-2-(trifluormethyl)-hex-ensäureethylester als E/Z Gemisch mit einem Enantiomerenüberschuß von größer 80%, und erhält so 13.9 rohes E/Z-4=(4-Fluor-2-methoxy-3-methylphenyl)-2-(trifluormethyl)-hex-4-en-1,2-diol. 4.0 g (11 mmol) (2R, 4E/Z)-4-(4-Fluor-2-methoxy-3-methylphenyl)-2-hydroxy-2-(trifluormethyl)-hex-4-ensäureethylester werden in 180 ml 2,2,2-Trifluorethanol gelöst and 400 mg Palladium auf Kohle (10%ig) werden zugegeben. Die Suspension wird unter einer Wasserstoff Atmosphäre bei 100 bar bis zur vollständigen Umsetzung geschüttelt. Die Mischung wird durch Celite filtriert wobei gründlich mit Ethylacetat nachgewaschen wird. Das eingeengte Rohprodukt wird säulenchromatographisch-an Kieselgel (Hexan / Ethylacetat 15%) gereinigt und man erhält 3.7 g (10 mmol) (*2R,4R*/*S*)-4-(4-Fluor-2-methoxy-3-methylphenyl)-2-hydroxy-2-(trifluormethyl)-hexansäure-ethylester als Diastereomerengemisch. 3.7 g (10 mmol) (*2R,4R*/*S*)-4-(4-Fluor-2-methoxy-3-methylphenyl)-2-hydroxy-2-(trifluormethyl)-hexansäure-ethylester werden in 132 ml Diethylether auf -15°C gekühlt und über 10 Minuten werden portionsweise 1.03 g (27.3 mmol) Lithiumaluminiumhydrid fest zugegeben. Man rührt 3 . Stunden bei -10°C und gibt vorsichtig Ethylacetat und Wasser zu. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung und trocknet über Natriumsulfat. Nach dem Entfernen des Lösungsmittels erhält man 3.4 g rohes 4-(4-Fluor-2-methoxy-3-methylphenyl)-2-hydroxy-2-(trifluormethyl)-hexanal als Gemisch der Diastereomeren. Säulenchomatographie an Kieselgel (Hexan /Diisopropylether 0-30%) liefert 1.04 g (*2R,4R*)-4-(4-Fluor-2-methoxy-3-methylphenyl)-2-hydroxy-2-(trifluormethyl)hexanal
¹H-NMR (300 MHz, CDCl₃); δ = 0.82 (t, 3H), 1.55 - 1.73 (m, 2H), 2.33 (dd, 1H), 2.36 (dd, 1H), 3.07 (m,1H), 3.72 (s, 3H), 4.20 (s, 1H), 6.81 (dd, 1H), 6.94 (dd, 1H), 8.89 (s, 1H).
und 1.13g (*2R,4S*)-4-(4-Fluor-2-methoxy-3-methylphenyl)-2-hydroxy-2-(trifluormethyl)hexanal.
¹H=NMR (300 MHz;CDCl₃) δ 0.71 (t, 3H), 1.50 -1.70 (m, 2H); 2.34 (dd, 1H), 2.39 (dd, 1H), 2.93 (m, 1H), 3.73 (s, 3H), 3.89 (s, 1H), 6.81 (dd, 1H), 6.93 (dd; 2H), 9.69 (s, 1H).

300 mg (0.93 mmol) (2R,4R)-4-(4-Fluor-2-methoxy-3-methylphenyl)-2-hydroxy-2-(trifluormethyl)hexanal und 165 mg (1.04 mmol) 5-Amino-2-methylchinazolin werden in 20 ml Toluol gelöst und 0.56 ml (1.86 mmol) Titantert-butylat und 0.11 ml Essigsäure werden zugegeben. Man erhitzt die Reaktionsmischung 2 Stunden auf 100°, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält 530 mg (*2R,4R*)-4-(3,4-Difluor-2-methoxyphenyl)-1-[(2-methylchinazolin-5-yl)imino]-2-(trifluormethyl)hexan-2-ol als Rohprodukt. Die säulenchromatographische Reinigung an Kieselgel (Hexan / Ethylacetat 0-50%) liefert 250 mg des reinen Imins, die in 22 ml CH₂Cl₂ gelöst und auf -30°C gekühlt werden. 4.3 ml (4.3 mmol) einer 1 M BBr₃ Lösung in Dichlormethan werden über 5 min langsam zugetropft und man läßt über 22 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säulenchromatographische Reinigung an Kieselgel (Hexan /Ethylacetat 0-100%) 147 mg Produkt.
¹H-NMR (300MHz, CD₃OD); δ =0.92 (t, 3H), 1.68 (ddq, 1H), 1.92 (m, 1H), 2.01 (dd, 1H), 2.08 (d, 3H), 2.35 (dd, 1H). 2.78 (s, 3H), 3.33 (m, 1H), 5.08 (s, 1H), 6.50 (d, 1H), 6.74 (d,1H), 7.17 (d, 1H), 7.74 (t, 1H), 9.57 (s, 1H):

### Beispiel 131

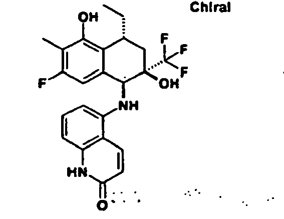

### 5-{[(1S,2R,4R)-4-Ethyl-7-fluor-2,5-dihydroxy-6-methyl-2-(trifluormethyl)-1,2,3,4-tetrahydronaphthalin-1-yl]amino}chinolin-2(1H)-on

Analog Beispiel 130 werden 300 mg (0.93 mmol) (*2R,4R*)-4-(4-Fluor-2-methoxy-3-methylphenyl)-2-hydroxy-2-(trifluormethyl)hexanal, 149 mg (0.93 mmol) 5-Aminochinolin-2(1H)-on.und 0.27 ml Titan*tert-*butylat zu 5-{[(*2R,4R*)-4-(4-Fluor-2-methoxy-3-methylphenyl)-2-hydroxy-2-(trifluormethyl)hexyliden)amino)chinolin-2(1*H*)-on umgesetzt. 210 mg chomatographisch gereinigtes Imin (Kieselgel, Hexan / Ethylacetat 0-80%) werden analog Beispiel 130 bei -30°C mit 3.6 mL (3.6 mmol) 1 M Bortribromid-Lösung zum gewünschten Produkt zyklisiert. Säulenchromatographie an Kieselgel (Hexan / Ethylacetat 0-100%) liefert 203 mg gewünschtes Produkt.
¹H-NMR(300 MHz, CD₃OD); δ = 0.90 (t, 3H), 1.66 (ddq, 1H), 1.89 (m, 1H), 1.99 (dd, 1H), 2.08 (d, 3H), 2.32 (dd, 1H), 3.33 (m, 1H), 4.95 (s, 1H), 6.42 (d, 1H), 6.47 (d, 1H), 6.49 (d, 1H), 6.67 (d, 1H), 7.32 (t, 1H), 8.19 (d, 1H).

### Beispiel132

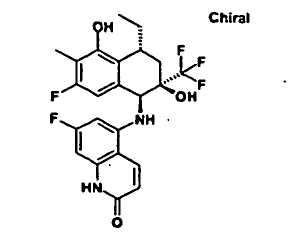

### 5-{[(1S,2R,4R)-4-Ethyl-7-fluor-2,5-dihydroxy-6-methyl-2-(trifluormethyl)-1,2,3,4-tetrahydronaphthalin-1-yl]}amino}7-fluorchirlolin-2(1H)-on

Analog Beispiel 130 werden 271 mg (0.51 mmol) (*2R,4R*)-4-(4-Fluor-2-methoxy-3-methylphenyl)-2-hydroxy-2-(trifluormethyl)hexanal, 90 mg (0.51 mmol) 5-Amino-7-fluorchinolin-2(1H)-on und 0.32 ml (1.02 mmol) Titan*tert*-butylat zu 7-Fluor-5-{[(*2R,4R*)-4-(4-Fluor-2-methoxy-3-methylphenyl)-2-hydroxy-2-(trifluormethyl)hexyliden]amino}chinolin-2(1*H*)-on umgesetzt. 320 mg rohes Imin werden analog Beispiel 130 bei -30°C mit 4.1 mL (4.1 mmol) 1 M Bortribromid-Lösung zum gewünschten Produkt zyklisiert. Säulenchromatographie an Kieselgel (Hexan / Ethylacetat 50%) liefert 165 mg gewünschtes Produkt.
¹H-NMR (300 MHz. CD₃OD); δ = 0.91 (t, 3H), 1.68 (ddq, 1H), 1.90 (m, 1H), 1.99 (dd, 1H); 2.09 (s, 3H), 2.33 (dd, 1H), 3.32 (m, 1H), 4.93 (s, 1H), 6.21 (d, 1H), 6.38 (d, 1H), 6.42 (d, 1H), 6.45 (d, 1H), 8.13 (d, 1H).

### Beispiel 133

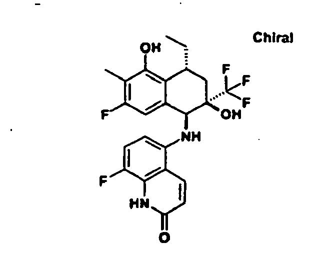

### 5-{[(1S,2R,4R)-4-Ethyl-7-fluor-2,5-dihydroxy-6-methyl-2-(trifluormethyl)-1,2,3,4-tetrahydronaphthalin-1-yl]amino}-8-fluorchinolin-2(1H)-on

Analog Beispiel 130 werden 300 mg (0.93 mmol) (*2R,4R*)-4-(4-Fluor-2-methoxy-3-methylphenyl)-2-hydroxy-2-(trifluormethyl)hexanal, 165 mg (0.93 mmol) 5= Amino-8-fluorchinolin-2(1*H*)-on und 0.58 ml Titan*tert*-butylat zu 8-Fluor-5-{[(*2R,4R*)-4-(4-fluor-2-methoxy-3-methylphenyl)-2-hydroxy-2-(trifluormethyl)hexyliden]amino}chinolin-2(1*H*)-on umgesetzt. 280 mg chomatographisch gereinigtes Imin (Kieselgel, Hexan / Ethylacetat 0-75%) werden analog Beispiel 130 bei -30°C mit 4.6 mL (4.6 mmol) 1 M Bortribromid-Lösung zum gewünschten Produkt zyklisiert. Säulenchromatographie an Kieselgel (Hexan / Ethylacetat 0-100%) liefert 230 mg gewünschtes Produkt.
¹H-NMR (300 MHz, CD₃OD) δ = 0.90 (t, 3H), 1.67 (ddq, 1H), 1.90 (m, 1H), 1.97 (dd, 1H), 2.08 (s, 3H), 2.31 (dd, 1H), 3.32 (m, 1H); 4.89 (s, 1H), 6.32 (dd, 1H), 6.49 (d, 1H), 6.54 (d, 1H), 7.17 (t, 1H), 8.18 (d, 1H).

### Beispiel 134

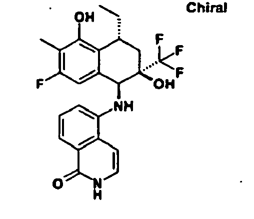

### 5-{[(1S,2R,4R)-4-Ethyl-7-fluor-2,5-dihydroxy-6-methyl-2-(trifluormethyl)-1,2,3,4,-tetrahydronaphthalin-1-yl]lamino}isochinolin-1(2H)-on

Analog Beispiel 130 werden 200 mg (0.62 mmol) (*2R,4R*)-4-(4-Fluor-2-methoxy-3-methylphenyl)-2-hydroxy-2-(trifluormethyl)hexanal, 100 mg (0.62 mmol) 5-Aminoisochinolin-1 (2H)-on und 0.39 ml (1.25 mmol) Titantert-butylat zu 5-{[(*2R,4R*)-4-(4-Fluor-2-methoxy-3-methylphenyl)-2-hydroxy-2-(trifluormethyl)hexyliden]amino}isochinolin-1(2*H*)-on umgesetzt. 460 mg rohes Imin werden analog Beispiel 130 bei -30°C mit 7.9 mL (7.9 mmol) 1 M Bortribromid-Lösurig zum gewünschten Produkt zyklisiert. Säulenchromatographie an Kieselgel (Hexan / Ethylacetat 50-100%) liefert 223 mg gewünschtes Produkt.
¹H-NMR (300 MHz, CD₃OD): δ = 0.91 (t, 3H), 1.69 (ddq, 1H). 1.90 (m, 1H), 2.02 (dd, 1H). 2.08 (s., 3H), 2.32 (dd, 1H), 3.31 (m, 1H), 4.95 (s, 1H), 6.48 (d, 1H), 6.82 (d, 1H), 6.85 (d, 1H), 7.15 (d, 1H), 7.33 (t, 1H), 7.65 (d, 1H).

### Beispiel 135 (5S,6R,8R)-8-Ethyl-3-fluor-5-[(7-fluor-2-methytchinazolin-5-yl)aminol-2-methyl-6-trifluormethyl)5,6,7,8-tetrahydronaphthalin-1,6-diol

Analog Beispiel 130 werden 300 mg (0.93 mmol) (*2R,4R*)-4-(4-Fluor-2-methoxy-3-methylphenyl)-2-hydroxy-2-(trifluormethyl)hexanal, 183 mg (1.03 mmol) 5-Amino-7-fluor-2-methylchinazolin und 0.58 ml (1.86 mmol) Titan*tert-*butylat zu 5-{[(*2R,4R*)-4-(4-Fluor-2-methoxy-3-methylphenyl)-1-[(7-fluor-2-methylchinazolin-5-yl)imino]-2-(trifluormethyl)hexan-2-ol umgesetzt 460 mg rohes Imin werden analog Beispiel 130 bei -30°C mit 7.6 mL (7.6 mmol) 1 M Bortribromid-Lösung zum gewünschten Produkt zyklisiert. Säulenchromatographie an Kieselgel (Hexan / Ethylacetat 0-65%) liefert 135 mg gewünschtes Produkt.
¹H-NMR (300 MHz, CDCl₃); δ = 0.90 (t, 3H), 1.64 (ddq, 1H), 1.87 (ddq, 1H), 1.96 (s, 3H), 2.03 (dd, 1H), 2.39 (dd, 1H), 2.84 (s, 3H), 3.29 (m, 1H), 4.80 (d, 1H), 6.19 (d,1H), 6.34 (d, 1H), 6.62 (d, 1H), 6.87 (d, 1H), 9.31 (s, 1H).

### Beispiel 136

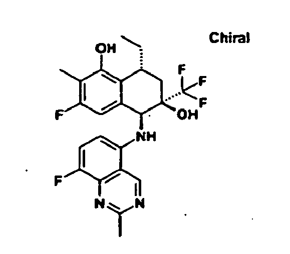

### (5S,6R,8R)-8-Ethyl-3-fluor-5-[(8-fluor-2-methylchinazolin-5-yl)amino]-2-methyl-6-(trifluormthyl)-5,6,7,8-tetrahydronaohthalin-1,6-diol

Analog Beispiel 130 werden 300 mg (0.93 mmol) (*2R,4R*)-4-(4-Fluor-2-methoxy-3-methylphenyl)-2-hydroxy-2-(trifluormethyl)hexanal, 183 mg (1.03 mmol) 5-Amino-8-fluor-2-methylchinazolin und 0.58 ml (1.86 mmol) Titantert-butylat zu 5-{[(2*R*,4*R*)-4-(4-Fluor-2-methoxy-3-methylphenyl)-1-[(8-fluor-2-methylchinazolin-5-yl)imino]-2-(trifluormethyl)hexan-2-ol umgesetzt. 450 mg rohes Imin werden analog Beispiel 130 bei -30°C mit 7.6 mL (7.6 mmol) 1 M Bortribromid-Lösung zum gewünschten Produkt zyklisiert. Säulenchromatographie an Kieselgel (Hexan / Ethylacetat 0-65%) liefert 77 mg gewünschtes Produkt.
¹H-NMR (300 MHz, CDCl₃); δ = 0.88 (t, 3H), 1.62 (ddq, 1H), 1.86 (ddq, 1H), 1.94 (s, 3H), 2.02 (dd, 1H), 2.37 (dd, 1H), 2.83 (s, 3H), 3.26 (m, 1H), 4.79 (d, 1H), 5.83 (d,1H), 6.49 (dd, 1H), 6.65 (d, 1H), 7.46 (dd, 1H), 9.44 (s, 1H).

### Beispiel 137

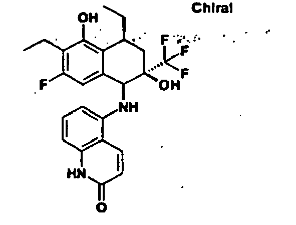

### 5-{[(1S,2R,4S)-4,6-Diethyl-7-fluor-2,5-dihvdroxy-2-(trifluormethyl)-1,2',3,4-tetrahydronaphthalin-1-yl]amino}chinolin-2(1H)-on

### 4-(3-Ethyl-4-fluor-2-methoxyphenyl)-2-hydroxy-2-(tflfluomJethyl)hexanal:

Zu 615 mg (4 mmol) 2'-Fluor-6-hydroxyacetophenon in Diethylenglycol werden 224 mg (4 mmol) Kaliumhydroxid und 240 mg (4.8 mmol) Hydrazinhydrat gegeben und man erhitzt 3 Stunden auf 160°C. Die Badtemperatur wir anschließend für 3 Stunden auf 210°C erhöht wobei das Reaktionsgefäß nun mit einer Destillationbrücke verschlossen wurde. Nach dem abkühlen gießt man in gesättigte Ammoniumchlorid Lösung, extrahiert mit Diethylether, wäscht mit Wasser und gesättigter Natriumchlorid Lösung und trocknet über Natriumsulfat. Nach Entfernen des Lösungsmittels erhält man 600 mg rohes 2-Ethyl-3-fluorphenol. Zu 600 mg (4 mmol) 2-Ethyl-3-fluorphenol in 10 ml Dichlormethan und 0.64 ml Pyridin werden bei 0°C 0.48 ml (5.5 mmol) Propionsäurechlorid getropft. Man rührt die Mischung 4 Stunden lang und gibt 20 ml einer 2 M Salzsäure zu. Es wird mit Dichlormethan extrahiert und mit Wasser gewaschen. Nach dem Trocknen über Natriumsulfat, Entfernen des Lösungsmittels im Vakuum und Chomatographie an Kieselgel (Hexan / Ethylacetat 25%) erhält man 580 mg Propionsäure-2-ethyl-3-fiuorphenylester. 580 mg (3 mmol) Propionsäure-2-ethyl-3-fluorphenytester in 2 ml 1,2-Dichlorbenzol werden zu 0.4 g (3 mmol) Aluminiumtrichlorid in 3 ml 1,2-Dichlorbenzol getropft und die Mischung wird im Anschluss 3 Stunden lang bei 100°C und 16 Stunden bei Raumtemperatur gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit Wasser und gesättigter Natriumchlorid Lösung gewaschen und über Natriumsulfat getrocknet. Das Rohprodukt wird säulenchromatographisch an Kieselgel (Hexan / Ethylacetat 10%) gereinigt und man erhält 580 mg 1-(3-Ethyl-4-fluor- 2-hydroxyphenyl)propan-1-on. 580 mg (3 mmol) 1-(3-Ethyl-4-fluor-2-hydroxyphenyl)propan-1-on werden in 4.6 ml Aceton gelöst und es werden 0.77 g (5.4 mmol) Kaliumcarbonat und 0.33 ml (5.4 mmol) Methyliodid zugegeben. Die Mischung wird 4 Stunden auf 70°C erhitzt, 12 Stunden bei Raumtemperatur gerührt und das Lösungsmittel im Anschluss zu einem großen Teil entfernt. Man gießt den Rückstand in Wasser und extrahiert mit Diethylether. Es wird mit Wasser gewaschen, über Natriumsulfat getrocknet und nach dem Entfernen des Lösungsmittels im Vakuum erhält man 700 mg 1-(3-Ethyl-4-fluor-2-methoxyphenyl)propan-1-on als Rohprodukt. 2.66 g (40 mmol) Zinkstaub und 56 mg (0.2 mmol) Blei(II)-chlorid werden in 20 ml THF suspendiert und bei 0°C werden 2.5 ml (35 mmol) Dibrommethan zugegeben. Man rührt weitere 30 Minuten bei Raumtemperatur und tropft bei 0°C 4.7 ml (4.7 mmol) einer 1 M Titan(IV)-chlorid Lösung in Dichlormethan zu. Das Kühlbad wird entfernt und nach einer Stunde wird die Reaktionsmischung wieder auf 0°C gekühlt. 700 mg (3.3 mmol) 1-(3-Ethyl-4-fluor-2-methoxyphenyl)propan-1-on in 3 ml THF zugetropft. Man rührt eine weitere Stunde bei Raumtemperatur. Es wird mit Dietylether verdünnt und das Reaktionsgemisch wird vorsichtig auf eine Mischung von 4 M Salzsäure und Eis gegeben wobei die Temperatur 5°C nicht übersteigt. Man trennt die Phasen, extrahiert mit Diethylether, wäscht mit Wasser und gesättigter Natriumchlorid Lösung, trocknet über Natriumsulfat und entfernt das Lösungsmittel. Das Rohprodukt wird säulenchromatographisch an Kieselgel (Hexan / Diisopropylether 0-20%) gereinigt und man erhält 650 mg 2-ethyl-3-fluor-6-(1-methylenpropyl)anisol. Zu 650 mg (3,2 mmol) 2-Ethyl-3-fluor-6-(1-methylenpropyl)anisol, 1,3 ml (9,6 mmol) Ethyltrifluorpyruvat und 2 g Molekularsieb werden bei 0°C über 30 Minuten 23 mg (0.026 mmol) [Cu(R,R)-2,2-bis(4,5-dihydro-4-*tert*-butyloxazolin-2-yl)propan)(H₂O)₂]((SbF₆)₂, in 85 ml Dichlormethan getropft. Man rührt die Reaktionsmischung 16 Stunden bei 0°C und reinigt die Reaktionsmischung mittels Säulenchomatographie an Kieselgel (Hexan / Ethylacetat 0-10%). Man erhält 600 mg (R)-4-(3-Ethyl-4-fluor-2-methoxyphenyl)-2-hydroxy-2-(trifluormethyl)hex-4-ensäureethylester als E/Z Gemisch mit einem Enantiomerenüberschuß von größer 80%. 200 mg (0.53 mmol) *E*/*Z-*4-(3,4-Difluor-2-methoxyphenyl)-2-hydroxy-2-(trifluormethyl)-hex-4-ensäureethylester werden in 8 ml Diethylether auf -5°C gekühlt und es werden portionsweise 40 mg (1.06 mmol) Lithiumaluminiumhydrid fest zugegeben. Man rührt eine Stunde bei Raumtemperatur und gießt in gesättigte Ammoniumchlorid Lösung. Die Phasen werden getrennt und es wird mehrfach mit Ethylacetat extrahiert. Man wäscht mit gesättigter Natriumchlorid Lösung, trocknet über Natriumsulfat, entfernt das Lösungsmittel im Vakuum und erhält nach chromatographie an Kieselgel (Hexan / Diisopropylether, 25%) (*2R,4E*/*Z*)*-4-*(3-Ethyl-4-fluor-2-methoxyphenyl)-2hydroxy-2-(trifluormethyl)hex-4-enal. 102 mg (*2R, 4E*/*Z*)=4-(3-Ethyl-4-fluor-2-methoxyphenyl)-2hydroxy-2-(trifluormethyl)hex-4-enal werden in 5 ml Trifluorethanol gelöst land 50 mg Palladium auf Kohle (10%ig) werden zugegeben. Die Suspension wird unter einer Wasserstoff Atmosphäre bei Normaldruck bis zur vollständigen Umsetzung geschüttelt. Die Mischung wird durch Celite filtriert wobei gründlich mit Ethylacetat nachgewaschen wird. Nach dem Entfernen des Lösungsmittels erhält man 16.1 88 mg rohes (*2R*,*4R*/*S*)-4-(3-Ethyl-4-fluor-2-methoxyphenyl)-2-hydroxy-2-(trifluormethyl)hexanal als Gemisch der Diastereomeren.

45 mg (0.13 mmol) (2*R*,*4R*/*S*)-4-(3-Ethyl-4-fluor-2-methoxyphenyl)-2-hydroxy-2-(trifluormethyl)hexanal und 21.4 mg (0.13 mmol) 5-Aminochinolin-2(1*H*)-on werden in 20 ml Toluol gelöst und 0.56 ml (1.86 mmol) Titan*tert*-butylat und 0.11 ml Essigsäure werden zugegeben. Man erhitzt die Reaktionsmischung 2 Stunden auf 100°, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält 80 mg (5-{[(*2R*,*4R*/*S*)-4-(3-Ethyl-4-fluor-2-methoxy-phenyl)-2-hydroxy-2-(trifluormethyl)hexyliden]amino)chinolin-2(1*H*)-on als Rohprodukt. Das Imin wird in 4.4 ml CH₂Cl₂ gelöst und auf -30°C gekühlt. 1.5 ml (1.5 mmol) einer 1 M BBr₃ Lösung in Dichlormethan werden über 15 min langsam zugetropft und man läßt über 22 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säulenchromatographische Reinigung an Kieselgel (Hexan / *iso*-Propanol 16%) und anschließende präparative

Dünnschichtchromatographie an einer Aminphase (Merck NH₂F₂₅₄, Ethylacetat/ Triethylamin, 2%) liefern 8 mg der Titelverbindung und 6 mg des entsprechenden *8R*-Diastereomeren.
¹H-NMR (300 MHz, CD₃OD); δ = 1.07 (t, 3H), 1.08 (t, 3H), 1.92 (m, 2H), 2.03 (dd, 1H), 2.41 (dd, 1H), 2.62 (q, 2H), 2.99 (m, 1H), 5.06 (s, 1H), 6.47 (d, 2H), 6.59 (d, 1H), 6.67 (d, 1H), 7.34 (t, 1H), 8.20 (d, 1H).

### Beispiel 138

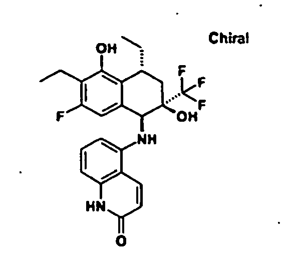

### 5-{[(1S,2R,4R)-4,6-Diethyl-7-fluor-2,5-dihydroxy-2-(trifluormethyl)-1,2,3,4-tetrahydronaphthalin-1-yl]amino}chinolin-2(1H)-on

6 mg des gewünschten Produktes werden wie in Beispiel 137 beschrieben nach präparativer Dünnschichtchromatographie an Kieselgel und Aminphase erhalten.
¹H-NMR (300 MHz, CD₃OD); δ = 0.91 (t, 3H), 1.08 (t, 3H), 1.67 (ddq, 1H), 1.89 (m, 1H), 2.00 (dd, 1H), 2.32 (dd, 1H), 2.63 (q, 2H), 3.32 (m, 1H), 4.95 (s, 1H), 6.42 (d, 1H), 6.447 (d, 1H), 6.50 (d, 1H), 6.67 (d, 1H), 7.32 (t, 1H), 8.19 (d, 1H).

### Beispiel 139

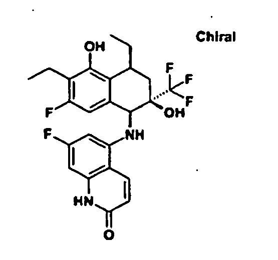

### 5-{[1S,2R,4S)-4,6-Diethyl-7-fluor-2,5-dihydroxy-2-(trifluormethyl)-1,2,3,4-tetrahydronaphthalin-1-lyl]amino}-7-fluorchinolin-2(1H)-on

Analog Beispiel 137 werden 45 mg (0.13 mmol) (*2R*,*4R*/*S*)-4-(3Ethyl-4-fluor-2-methoxyphenyl)-2-hydroxy-2-(trifluormethyl)hexanal, 23 mg (0.13 mmol) 5-Amino-7-fluorchinolin-2(1H)-on und 0.09 ml (0.27 mmol) Titan*tert*-butylat zu 5-{[(*2R,4R*/*S*)-4-(3-Ethyl-4-fluor-2-methoxy-phenyl)-2-hydroxy-2-(trifluormethyl)hexyliden]amino}-7-fluorchinolin-2(1*H*)-on umgesetzt. 80 mg rohes Imin werden analog Beispiel 137 bei -30°C mit 1.3 mL (1.3 mmol) 1 M Bortribromid-Lösung zyklisiert. Säulenchromatographische Reinigung an Kieselgel (Hexan / *iso*-Propanol 16%) und anschließende präparative Dünnschichtchromatographie an einer Aminphase (Merck NH₂F₂₅₄, Ethylacetat / Triethylamin, 2%) liefern 7 mg der Titelverbindung und 5 mg des entsprechenden (8*R*)-Diastereomeren. ¹H-NMR (300 MHz, CD₃OD); δ = 1.07 (t, 3H), 1.08 (t, 3H), 1.92 (m, 2H), 2.06 (dd, 1H), 2.39 (dd, 1H), 2.63 (q, 2H), 2.99 (m, 1H), 5.04 (s, 1H), 6.38 (d, 2H), 6.40 (d, 1H), 6.45 (d, 1H), 8.15 (d, 1H).

### Beispiel 140

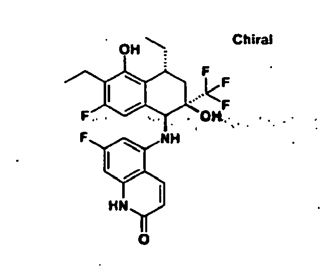

### 5-{[(1S,2R,4R)-4,6-Diethyl-7-fluor-2,5-dihydroxy-2-(trifluormethyl)-1,2,3,4-tetrahydronaphthalin-1-yl]amino}-7-fluorchinolin-2(1H)-on

5 mg des gewünschten Produktes werden wie in Beispiel 139 beschrieben nach präparativer Dünnschichtchromatographie an Kieselgel und Aminphase erhalten.
¹H-NMR (300 MHz, CD₃OD); δ = 0:91 (t, 3H), 1.09 (t, 3H), 1.67 (ddq, 1H), 1.91 (m, 1H), 1.99 (dd, 1H), 2.33 (dd, 1H), 2.64 (q, 2H), 3.31 (m, 1H), 4.93 (s, 1H), 6.20 (d, 1H), 6.38 (d, 1H), 6.42 (d, 1H), 6.45 (d, 1H), 8.13 (d, 1H).

### Beispiel 141

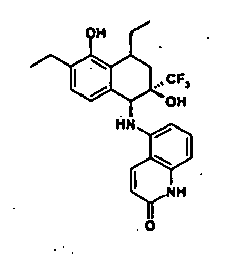

### 5-{[(1α,2α,4α)-4,6-Diethyl-2,5-dihydroxy-2-(trifluormethyl)-1,2,3,4-tetrahydronaphthalin-1-yl]amino}-1H-chinolin-2-on

### 4-(3-Ethyl-2-methoxyphenyl)-2-hydroxy-2-(trifluormethyl)-hexanal

2,28 (7,12 mmol) eines Gemisches aus 4-(3-Ethyl-2-methoxyphenyl)-2-(trifluormethyl)-hexan-1,2-diol und 4-(3-Ethyl-2-methoxyphenyl)-3-methyl-2-(trifluormethyl)-pentan-1,2-diol (hergestellt in Analogie zu der in Beispiel 110 beschriebenen Sequenz: Acetylierung des entsprechenden Phenols, Friessche Verschiebung der Acetylgruppe, Veretherung des Phenols, Wittig-Umsetzung, En-Reaktion, Reduktion des Esters zum Alkohol) werden in der üblichen Weise mit SO₃/ Pyridin Komplex (3,4 g, 21,35 mmol) umgesetzt. Nach dreistündigem Rühren bei Raumtemperatur wird die Reaktionsmischung auf ein Gemisch aus gesättigte NH₄CL- Lösung und Eis gegossen. Es wird dreimal mit Diethylether extrahiert. Die vereinigten organischen Extrakte werden zweimal mit Sole gewaschen, getrocknet (Na₂SO₄). Nach dem Abrotieren des Lösungsmittels wird der Rückstand mittels Chromatographie gereinigt (Kieselgel, Laufmittel Hexan/ Ethylacetat). Erhalten werden
2,07 g (91,4%) eines Gemischs der beiden Aldehyde.

### 5-{[4-(3-Ethyl-2-methoxyphenyl)-2-hydroxy-2-(trifluomethyl)hexyliden]amino}-1H-chinolin-2-on

Das Gemisch aus 4-(3-Ethyl-2-methoxyphenyl)-2-hydroxy-2-(trifluormethyl)-hexanal und 4-(3-Ethyl-2-methoxyphenyl)-2-hydroxy-3-methyl-2-(trifluormethyl)-pentanal (690 mg, 2,16 mmol), 5-Amino-1H-chinolin-2-on (347,2 mg, 2,17 mmol) und 3 mL Essigsäure werden zwei Tage bei Raumtemperatur gerührt. Das Reaktionsgemisch wird zweimal mit Toluol und Dichlormethan abgezogen und der Rückstand chromatographiert (Flashmaster, Laufmittel: Dichlormethan/ Methanol). Isoliert werden 709,7 mg (71,1 %) eines Gemisches der beiden Imine.

Das im vorigen Abschnitt beschriebene Gemisch aus 5-{[4-(3-Ethyl-2-methoxyphenyl)-2-hydroxy-2-(trifluormethyl)hexyliden]amino}-1H-chinolin-2-on und 5-{[4-(3-Ethyl-2-methoxyphenyl)-2-hydroxy-3-methyl-2-(trifluormethyl)pentyliden]amino}-1H-chinolin-2-on (709,7 mg, 1,54 mmol) wird in 7,1 mL Dichlormethan vorgelegt und bei -40 °C tropfenweise mit 15,41 mL einer 1 M Lösung von BBr₃ in Dichlormethan versetzt. Nach drei Stunden Rühren im Temperaturbereich zwischen -40 und +10 °C ist kein Ausgangsmaterial mehr vorhanden. Die Reaktionsmischung wird vorsichtig auf eine Mischung aus gesättigter NaHCO₃-Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels chromatographiert (NH₂ Flash, Laufmittel: Dichlormethan/ Methanol). Dabei wird ein Gemisch aus der gewünschten Verbindung und dem entsprechenden 3,4-Dimethyltetrahydronaphthalinderivat erhalten, das per HPLC (Kromasil 18 5 µ, Laufmittel Wasser/ Methanol) in die Konstitutionsisomeren getrennt wird. Isoliert werden 83,6 mg (33,6%) der Titelverbindung. Es wird ein weiteres Gemisch erhalten, das aus dem in der Position 4 epimeren Ethylderivat und einem weiteren 3,4 Dimethylderivat besteht. Das epimere Ethylderivat wird in Beispiel 142 beschrieben.

### Beispiele 141A und 141B

### 5-{[(1R,2S,i)-4,6-Diethyl-2,5-dihydroxy-2-(trifluormethyl)-1,2,3,4-tetrahydronaphthalin-1-yl]amino}-1H-chinolin-2-on und 5-ff(1S,2R,4S)-4,6-Diethyl-2,5-dihydroxy-2-(trifluormethyl)-1,2,3,4-tetrahydronaphthalin-1-yl]amino}-1H-chinolin-2-on

66,4 mg des in Beispiel 141 beschriebenen Racemats werden mittels chiraler HPLC (Chiralpak AD-H 5 µ, Laufmittel Hexan/ Ethanol) in seine Enantiomeren getrennt. Es werden 32,4 mg (48,5%) des einen Enantiomers (Retentionszeit 8,6-9,8 Minuten) und 32,4 mg (48,5%) des anderen Enantiomers (Retentionszeit 11,3 -12,8 Minuten) isoliert. Über die absolute Konfiguration der Verbindungen kann natürlich keine Aussage gemacht werden.

### Beispiel 142

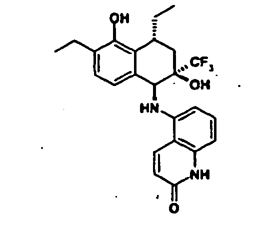

### 5-{[(1α,2α,4β)-4,6-Diethyl-2,5-dihydroxy-2-(trifluormethyl)-1,2,3,4-tetrahydronaphthalin-1-yl]amino}-1H-chinolin-2-on

Das in Beispiel 141 beschriebene Gemisch (160 mg) aus dem epimeren Ethyl-und dem 3,4-Dimethylderivat wird per HPLC getrennt (Kromasil 18 5µ, Laufmittel Wasser/ Acetonitril). erhalten werden 75,8 mg (47,4%) der Titelverbindung und 65,9 mg (41,2%) des entsprechenden 3,4-Dimethyltetrahydronaphthalinderivats.

### Beispiele 142A und 142B

### 5-{[(1R,2S,4S)-4,6-Diethyl-2,5-dihydroxy-2-(trifluormethyl)-1,2,3,4-tetrahydronaphthalin-1-yl]amino}1H-chinolin-2-on und 5-{[(1S,2R,4R)-4,6-Diethyl-2,5-dihydroxy-2-(trifluormethyl)-1,2,3,4-tetrahydronaphthalin-1-yl]amino}-1H-chinolin-2-on

53,9 mg des in Beispiel 142 beschriebenen Racemats werden mittels chiraler HPLC (Chiralpak AD-H 5 µ, Laufmittel Hexan/ Ethanol) in seine Enantiomeren getrennt. Es werden 29 mg (> 50%) des einen Enantiomers (Retentionszeit 10,6-12,2 Minuten) und 28 mg
(> 50%) des anderen Enantiomers (Retentionszeit 13,5 -15,7 Minuten) isoliert. Über die absolute Konfiguration der Verbindungen kann natürlich keine Aussage gemacht werden.

### Beispiel 143

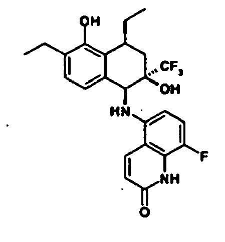

### 5-{[(1α,2α,4α)-4,6-Diethyl-2,5-dihydroxy-2-(trifluormethyl)-1,2,3,4-tetrahydronaohthalin-1-yl]amino}-8-fluor-1H-chinolin-2-on

Das Gemisch aus 5-{[4-(3-Ethyl-2-methoxyphenyl)-2-hydroxy-2-(trifluormethyl)hexyliden]amino}-8-fluor-1H-chinolin-2-on und 5-{[4-(3-Ethyl-2-methoxyphenyl)-2-hydroxy-3-methyl-2-(trifiuormethyl)pentyliden]amino}-8-fluor-1H-chinolin-2-on (721,6 mg, 1,5 mmol), hergestellt aus dem in Beispiel 142 beschriebenen Gemisch der beiden Aldehyde und 5-Amino-8-fluor-1H-chinolin-2-on, wird in 7,2 mL Dichlormethan vorgelegt und bei -40 °C tropfenweise mit 15,1 mL einer 1 M Lösung von BBr₃ in Dichlormethan versetzt. Nach drei Stunden Rühren im Temperaturbereich zwischen -40 und +10 °C ist kein Ausgangsmaterial mehr vorhanden. Die Reaktionsmischung wird vorsichtig auf eine Mischung aus gesättigter NaHCO₃ -Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels chromatographiert (NH₂ Flash, Laufmittel: Dichlormethan/ Methanol). Dabei wird ein Gemisch aus der gewünschten Verbindung und dem entsprechenden
3,4-Dimethyltetrahydronaphthalinderivat erhalten, das per HPLC (Kromasil 18 5 µ, Laufmittel: Wasser/ Acetonitril) in die Konstitutionsisomeren getrennt wird. Isoliert werden 98,9 mg (36,2%) der Titelverbindung. Es wird ein weiteres Gemisch erhalten, das aus dem in der Position 4 epimeren Ethylderivat und einem weiteren 3,4 Dimethylderivat besteht. Das epimere Ethylderivat wird in Beispiel 145 beschrieben.

### Beispiele 143A und 143B

### 5-{[(1R,2S,4R)-4,6-Diethyl-2,5-dihydroxy-2-(trifluomnethy)-1,2,3,4-tetrahydronaphthalin-1-yl]amino}-8-fluor-1H-chinolin-2-on und 5-{[(1S,2R,4S)-4,6-Diethyl-2,5-dihydroxy-2-(trifluormethyl)-1,2,3,4-tetrahydronaphthalin-1-yl]amino}-8-fluor-1H-chinolin-2-on

81,4 mg des in Beispiel 143 beschriebenen Racemats werden mittels chiraler HPLC (Chiralpak AD-H 5 µ, Laufmittel: Hexan/ Ethanol) in seine Enantiomeren getrennt. Es werden 40 mg (49,4%) des einen Enantiomers (Retentionszeit 9,4-10,8 Minuten) und 40 mg (49,4%) des anderen Enantiomers (Retentionszeit 13,3 -15,8 Minuten) isoliert. Über die absolute Konfiguration der Verbindungen kann natürlich keine Aussage gemacht werden.

### Beispiel 144

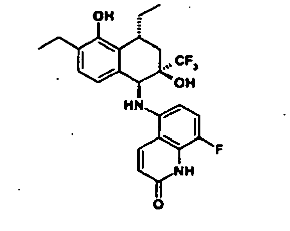

### 5-{[(1α,2α,4β)-4,6-Diethyl-2,5-dihydroxy-2-(trifluormethyl)-1,2,3,4-tetrahydronaphthalin-1-yl]amino}-8-fluor-1H-chinolin-2-on

Das in Beispiel 143 beschriebene Gemisch (200 mg) aus dem epimeren Ethyl-und dem 3,4-Dimethylderivat wird per HPLC getrennt (Kromasil 18 5µ, Laufmittel: Wasser/ Acetonitril). Erhalten werden 72,7 mg (36%) der Titelverbindung.

### Beispiele 144A und 144B

### 5-{[(1R,2S,4S)-4,6-Diethyl-2 5-dihydroxy-2-(trifluormethyl)-1,2,3,4-tetrahydronaphthalin-1-yl]amino}-8-fluor-1H-chinolin-2-on und 5-{[(1S,2R,4R)- 4,6-Diethyl-2,5-dihvdroxy-2-(trifluormethyl)-1,2,3,4-tetrahydronaphthalin-1-yl]amino}-8-fluor-1H-chinolin-2-on

59 mg des in Beispiel 144 beschriebenen Racemats werden mittels chiraler HPLC (Chiralpak AD-H 5 µ, Laufmittel: Hexan/ Ethanol) in seine Enantiomeren getrennt. Es werden 26 mg (44,1%) des einen Enantiomers (Retentionszeit 7,8-8,8 Minuten) und 26 mg
(44,1%) des anderen Enantiomers (Retentionszeit 16,5 -18 Minuten) isoliert. Über die absolute Konfiguration der Verbindungen kann natürlich keine Aussage gemacht werden.

### Beispiel 146

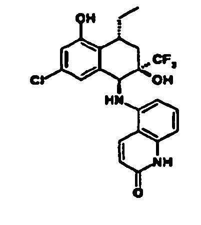

### 5-{[(1α,2α,4β)-7-Chlor-2,5-dihydroxy-4-ethyl-2-(trifluormethyl)-1,2,3,4-tetrahydronaphthalin-1-yl}aminol-1H-chinolin-2-on

Das Gemisch aus 5-{[4-(4-Chlor-2-methoxyphenyl)-2-hydroxy-2-(trifluormethyl)hexyliden]amino}-1H-chinotin-2-on und 5-{[4-(4-Chlor-2-methoxyphenyl)-2-hydroxy-3-methyl-2-(trifluormethyl)pentylidenlamino}-1H-chinolin-2-on (448,7 mg, 0,96 mmol), hergestellt aus der Reaktion des Gemischs der entsprechenden Aldehyde mit 5-Amino-1H-chinolin-2-on, wird in 4,5 mL Dichlormethan vorgelegt und bei -40°C tropfenweise mit 9,6 mL einer 1 M Lösung von BBr₃ in Dichlormethan versetzt. Nach vier Stunden Rühren im Temperaturbereich zwischen -40 °C und Raumtemperatur ist kein Ausgangsmaterial mehr vorhanden. Die Reaktionsmischung wird vorsichtig auf eine Mischung aus gesättigter NaHCO₃ -Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels chromatographiert (NH₂ Flash, Laufmittel Dichlormethan/ Methanol). Dabei wird ein Gemisch aus der gewünschten Verbindung und dem entsprechenden 3,4-Dimethyltetrahydronaphthalinderivat erhalten, das per HPLC (Kromasil 18 5 µ, Laufmittel: Wasser/Methanol) in die Konstitutionsisomeren getrennt wird. Isoliert werden 7 mg (3,2%) der Titelverbindung.
1H-NMR (400 MHz, CD₃OD): δ = 0,84 (3H), 1,60 (1H), 1,80-1,91 (2H), 2,29 (1H), 3,20 (1H, das Signal liegt teilweise unter dem Lösungsmittelsignal), 4,90 (1H), 6,38 (1H), 6,43 (1H), 6,58-6,67 (3H), 7,28 (1H), 8,12 (1H).

### Beispiel 147

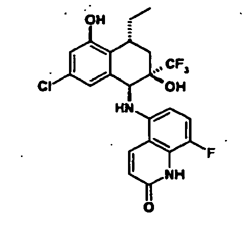

### 5-{[(1α,2α.4β)-7-Chlor-2,5-dihydroxy-4-ethyl-2-(trifluomethyl)-1,2,3,4-tetrahydronaphthalin-1-yl]aminol-8-fluor-1H-chinolin-2-on

Das Gemisch aus 5-{[4-(4-Chlor-2-methoxyphenyl)-2-hydroxy-2-(trifluormethyl)hexyliden]amino}-8-fluor-1H-chinolin-2-on und 5-{[4-(4-Chlor-2-methoxyphenyl)-2-hydroxy-3-methyl-2-(trifluormethyl)pentyliden]amino}-8-fluor-1H-chinolin-2-on (467,6 mg, 0,96 mmol), hergestellt aus der Reaktion des Gemischs der entsprechenden Aldehyde mit 5-Amino-8-fluor-1H-chinolin-2-on, wird in 4,8 mL Dichlormethan vorgelegt und bei -40 °C tropfenweise mit 9,6 mL einer 1 M Lösung von BBr₃ in Dichlormethan versetzt. Nach vier Stunden Rühren im Temperaturbereich zwischen -40 °C und Raumtemperatur ist kein Ausgangsmaterial mehr vorhanden. Die Reaktionsmischung wird vorsichtig auf eine Mischung aus gesättigter NaHCO₃ -Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels chromatographiert (NH₂ Flash, Laufmittel Dichlormethan/ Methanol). Dabei wird ein Gemisch aus der gewünschten Verbindung und dem entsprechenden 3,4-Dimethyltetrahydronaphthalinderivat erhalten, das per HPLC (Kromasil 18 5 µ, Laufmittel: Wasser/ Acetonitril) in die Konstitutionsisomeren getrennt wird. Isoliert werden 9,7 mg (4,3%) der Titelverbindung.
1H-NMR (400 MHz, CD₃OD): δ = 0,92 (3H), 1,69 (1H), 1,88-2,00 (2H), 2,34 (1H), 3,27 (1H, das Signal liegt teilweise unter dem Lösungsmittelsignal), 4,91 (1H), 6,37 (1H), 6,55 (1H), 6,68-6,73 (2H), 7,19 (1H), 8,18 (1H).

### Beispiel 148

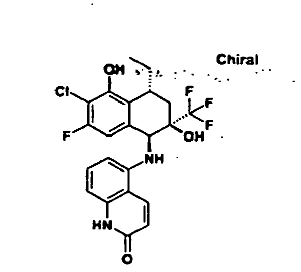

### 5-{[(1S,2R,4R)-6-Chlor-4-ethyl-2,5-dihydroxy-7-fluor-2-(trifluormethyl)-1,2,3,4-tetrahydronaphthalin-1-yl]amino}chinolin-2(1H)-on

### 4-(3-Chlor-4-fluor-2-methoxyphenyl)-2-hydroxy-2-(trifluormethyl)hexanal:

Zu 21.5 g (146.7 mmol) 2-Chlor-3-fluorphenol (R. Sanz et al. J. Org. Chem. 2005, 70, 6548-51) in 97 ml Dichlormethan und 16.5 ml Pyridin werden bei 0°C 14 ml (161 mmol) Propionsäurechlorid getropft. Man rührt die Mischung 16 Stunden lang und gibt 100 ml einer 2 M Salzsäure zu. Es wird mit Dichlormethan extrahiert und mit Wasser gewaschen. Nach dem Trocknen über Natriumsulfat und dem Entfernen des Lösungsmittels im Vakuum erhält man 27.1 g Propionsäure-2-chlor-3-fluorphenylester. 30.1 g (133.7 mmol) Propionsäure-2-chlor-3-fluorphenylester in 42 ml 1,2-Dichlorbenzol werden zu 19.6 g (133 mmol) Aluminiumtrichlorid in 42 ml 1,2-Dichlorbenzol getropft und die Mischung wird im Anschluss 18 Stunden lang bei 100°C gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit Wasser und gesättigter Natriumchlorid Lösung gewaschen und über Natriumsulfat getrocknet. Das Rohprodukt wird säulenchromatographisch an Kieselgel (Hexan / Ethylacetat 0-10%) gereinigt und man erhält 22.8 g 1-(3-Chlor-4-fluor-2-hydroxyphenyl)propän-1-on. 22.8 g (112 mmol) 1-(3-Chlor-4-fluor-2-hydroxyphenyl)propan-1-on werden in 170 ml Aceton gelöst und es werden 28.4 g (205 mmol) Kaliumcarbonat und 12.3 ml (198 mmol) Methyliodid zugegeben. Die Mischung wird 4 Stunden unter Rückfluß gekocht, 12 Stunden bei Raumtemperatur gerührt und das Lösungsmittel im Anschluss zu einem großen Teil entfernt. Man gießt den Rückstand in Wasser und extrahiert mit Diethylether. Es wird mit Wasser gewaschen, über Natriumsulfat getrocknet und nach dem Entfernen des Lösungsmittels im Vakuum erhält man 24.4 g 1-(3-Chlor-4-fluor-2-methoxyphenyl)propan-1-on. 63,3 g (969 mmol) zinkstaub und 1.33 g (4.77 mmol) Blei(II)-chlorid werden in 490 ml THF suspendiert und bei 0°C werden 59 ml (846 mmol) Dibrommethan zugegeben. Man rührt weitere 30 Minuten bei Raumtemperatur und tropft bei 0°C 113 ml 113 mmol) einer 1 M Titan(IV)-chlorid Lösung in Dichlormethan zu. Das Kühlbad wird entfernt und nach einer Stunde wird die Reaktionsmischung wieder auf 0°C gekühlt. 124.4 g (113 mmol) 1-(3-Chlor-4-fluor-2-methoxyphenyl)propan-1-on in 65 ml THF zugetropft. Man rührt eine weitere Stunde bei Raumtemperatur. Es wird mit Dietylether verdünnt und das Reaktionsgemisch wird vorsichtig auf eine Mischung von 4 M Salzsäure und Eis gegeben wobei die Temperatur 5°C nicht übersteigt. Man trennt die Phasen, extrahiert mit Diethylether, wäscht mit Wasser und gesättigter Natriumchlorid Lösung, trocknet über Natriumsulfat und entfernt das Lösungsmittel. Das Rohprodukt wird säulenchromatographisch an Kieselgel (Hexan / Diisopropylether 0-20%) gereinigt und man erhält 12.7 g 2-Chlor-3-fluor -6-(1-methylenpropyl)anisol
1H-NMR (300 MHz, CDCl3); δ = 1.01 (t, 3H), 2.46 (q, 2H), 3.79 (s, 3H), 5.05 (s,1H), 5.18 (s,1H), 6.89 (dd, 1H), 7.02 (dd, 1H),

Zu 2 g (9.3 mmol) 2-Chlor-3-fluor -6-(1-methylenpropyl)anisol, 2.46 ml (18.6 mmol) Ethyltrifluorpyruvat und 5 g Molekularsieb werden bei 0°C über 30 Minuten 440 mg (0.5 mmol) [Cu(R,R)-2,2-bis(4,5-dihydro-4-tert-butyloxazolin-2-yl)propan)(H₂O)₂]((SbF₆)₂, in 12 ml Dichlormethan getropft. Man rührt die Reaktionsmischung 16 Stunden bei 0°C und reinigt die Reaktionsmischung mittels Säulenchomatographie an Kieselgel (Hexan / Diisopropylether 20%). Man erhält 1.04 g (R)-4-(3-Chlor-4-fluor-2-methoxyphenyl)-2-hydroxy-2-(trifluormethyl)-hex-4-ensäureethylester als E/Z Gemisch mit einem Enantiomerenüberschuß von größer 80%. 1.14g (1.09 mmol) (S)-(S)-(3,5-Me-4-MeOPh)₂PPhFc-CH(CH₃)-P(3,5-CF₃Ph)₂ und 389 mg (104 mmol) (Rh(nbd)₂]BF₄ werden unter Argon in 60 ml entgastem 2,2,2-Trifluorethanol gelöst und 10 Minuten gerührt. Die so hergestellte Katalysatorlösung und eine Lösung von 10 g (26 mmol) (2R, 4E/Z)-4-(3-Chlor-4-fluor-2-methoxyphényl)-2-hydroxy-2-(trifluormethyl)-hex-4-ensäureethylester in 340 ml entgastem 2,2,2-Trifluorethanol werden unter Argon in einen Stahlautoklaven überführt und über 20 Stunden bei 80°C einem Wasserstoffdruck von 80 bar ausgesetzt. Nach dem Abkühlen wird die Reaktionsmischung eingeengt und mittels Säulenchomatographie an Kieselgel (Hexan / Ethylacetat 0-15%) aufgereinigt. Man erhält 9.2 g 4-(3-Chlor-4-fluor-2-methoxyphenyl)-2-hydroxy-2-(trifluormethyl)-hexansäureethylester. 9.2 g (24 mmol) 4-(3-Chlor-4-fluor-2-methoxyphenyl)-2-hydroxy-2-(trifluormethyl)hexansäureethylester werden in 315 ml Diethylether auf -10°C gekühlt und über 10 Minuten werden portionsweise 2.46 g (38 mmol) Lithiumaluminiumhydrid fest zugegeben. Man rührt 2 Stunden wobei sich die Reaktionsmischung auf 0°C erwärmt, man gibt 5 ml Ethylacetat zu und gießt in gesättigte Ammoniumchlorid Lösung und Eis. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält so nach Säulenchomatographie an Kieselgel (Hexan / Diisopropylether 0-30%) 1.3 g (2R,4R)-4-(3-Chlor-4-fluor-2-methoxyphenyl)-2-hydroxy-2-(trifluormethyl)hexanal.
¹H-NMR (300 MHz, COCl₃); δ = 0.75 (t, 3H), 1.55 - 1.73 (m, 2H), 2.30 (dd, 1H). ' 2.54 (dd, 1H), 3.06 (m,1H), 3.92 (s, 1H), 3.96 (s, 3H), 6.75 - 6.84 (m, 2H), 9.02 (s, 1H).
und 2.3 g (*2R,4R*/*S*)-4-(,4-Fluor-2-methoxyphenyl)-2-hydroxy-2-(trifluormethyl)hexanal

300 mg (0.88 mmol) (*2R, 4R*)-4-(3-Chlor-4-fluor-2-methoxyphenyl)-2-hydroxy-2-(trifluormethyl)hexanal und 140 mg (0.88 mmol) 5-Aminochinolin-2(1H)-on werden in 19 ml Toluol gelöst und 0.55 ml (1.75 mmol) Titantert-butylat und 0.1 ml Essigsäure werden zugegeben. Man erhitzt die Reaktionsmischung 2 Stunden auf 100°C, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält 539 mg (5-{[(2R,4R)-4-(3-Chlor-4-fluor-2-methoxyphenyl)-2-hydroxy-2-(trifluormethyl)hexyliden]amino}chinolin-2(1H)-on als Rohprodukt. Das Imin wird in 44 ml CH₂Cl₂ gelöst und auf -30°C gekühlt 8.9 ml (8.9 mmol) **einer 1M** BBr₃ Lösung in Dichlormethan werden über 15 min langsam zugetropft und man läßt über 22 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säulenchromatographische Reinigung an Kieselgel (Hexan / Ethylacetaat 0-100%) liefern 134 mg der Titelverbindung.
¹H-NMR (300 MHz, CD₃OD); δ = 0.92 (t, 3H), 1.70 (ddq, 1H), 1.19 (ddq, 1H), 1.99 (dd, 1H), 2.36 (dd, 1H), 3.32 (m, 1H), 4.97 (s, 1H), 6.44 (d, 1H), 6.50 (d, 1H), 6.63 (d, 1H), 6.68 (d, 1H), 7.33 (t, 1H), 8.18 (d, 1H).

### Beispiel 149

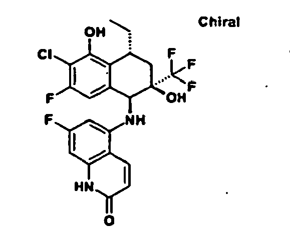

### 5-{[(1S,2R,4R)-6-Chlor-4-ethyl-2,5-dihydroxy-7-fluor-2-(trifluormethyl)-1,2,3,4-tetrahydronaphthalin-1-yl]amino}-7-fluorchinolin-2(1H)-on

Analog Beispiel 148 werden 173 mg (0.51 mmol) (*2R,4R*)-4-(3-Chlor-4-fluor-2-methoxyphenyl)-2-hydroxy-2-(trifluormethyl)hexanal, 90 mg (0.51 mmol) 5-Amino-7-fluorchinolin-2(1H)-on und 0.32 ml Titantert-butylat zu 5-{[(*2R,4R*)-4-(3-Chlor-4-fluor-2-methoxy-phenyl)-2-hydroxy-2-(trifluormethyl)hexyliden]amino}-7-fluorchinolin-2(1*H*)-on umgesetzt. 270 mg rohes Imin werden analog Beispiel 148 bei -30°C mit 7.6 mL (7.6 mol) 1 M Bortribromid-Lösung zum gewünschten Produkt zyklisiert. Säulenchromatographie an Kieselgel (Hexan / Ethylacetat 0-70%) liefert 26 mg gewünschtes Produkt.
¹H-NMR (300 MHz, CD₃OD); δ = 0.92 (t, 3H), 1.69 (ddq, 1H), 1.96 (m, 1H), 1.99 (dd, 1H), 2.37 (dd, 1H), 3:31 (m, 1H), 4.97 (s, 1H), 6.26 (d, 1H), 6.40 (d, 1H), 6.43 (d, 1H), 6.60 (d,1H), 8.13 (d, 1H).

### Beispiel 150

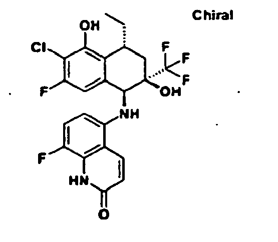

### 5-{[(1S,2R,4R)-6-Chlor-4-ethyl-2,5-dihydroxy-7-fluor-2-(trifluormethyl)-1,2,3,4-tetrahydronaphthalin-1-yl]amino}-8-fluorchinolin-2(1H)-on

Analog Beispiel 148 werden 300 mg (0.88 mmol) (*2R, 4R*)-4-(3-Chlor-4-fluor-2-methoxyphenyl)-2-hydroxy-2-(trifluormethyl)hexanal, 156 mg (0.88 mmol) 5-Amino-8-fluorchinolin-2(1H)-on und 0.55 ml Titan*tert*-butylat zu 5-{[(*2R,4R*)-4-(3-Chlor-4-fluor-2-methoxy-phenyl)-2-hydroxy-2-(trifluormethyl)hexyliden]amino}-8-fluorchinolin-2(1*H*)-on umgesetzt. 480 mg rohes Imin werden analog Beispiel 148 bei -30°C, mit 7.6 mL (7.6 mmol) 1 M Bortribromid-Lösung zum gewünschten Produkt zyklisiert. Säulenchromatographie an Kieselgel (Hexan / Ethylacetat 0-70%) liefert 119 mg gewünschtes Produkt.
¹H-NMR (300 MHz, CD₃OD); δ = 0.91 (t, 3H), 1.69 (ddq, 1H), 1.93 (m, 1H), 1.98 (dd, 1H), 2.35 (dd, 1H), 3.32 (m, 1H), 4.91 (s, 1H), 6.36 (dd, 1H), 6.54 (d, 1H), 6.65 (dd, 1H), 7.18 (dd, 1H), 8.17 (d, 1H).

### Beispiel 151

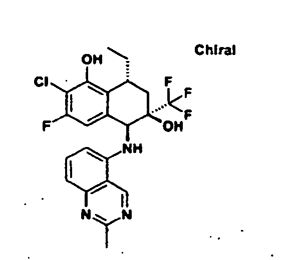

### (5S,6R,8R)-2-Chlor-8-ethyl-3-fluor-5[(2-methylchinazolin-5-yl)amino]-6-(trifluormethyl)-5,6,7,8-tetrahydronaphthalin-1,6-diol

Analog Beispiel 48 werden 100 mg (0.29 mmol) (*2R,4R*)-4-(3-Chlor-4-fluor-2-methoxyphenyl)-2-hydroxy-2-(trifluormethyl)hexanal, 52 mg (0.33 mmol) 5-Aminochinazolinn und 0.27 ml Titantert-butylat zu 5-{[(*2R,4R*)-4-(3-Chlor-4-fluor-2-methoxyphenyl)-1-[(2-methylchinazolin-5-yl)imino]-2-(trifluormethyl)hexan-2-ol umgesetzt. 130 mg rohes Imin werden analog Beispiel 148 bei -30°C mit 5 mL (5 mmol) 1 M Bortribromid-Lösung zum gewünschten Produkt zyklisiert. präparative Dünnschichtchromatographie an Kieselgel (Hexan / Ethylacetat 50%) liefert 19 mg gewünschtes Produkt.
¹H-NMR (300 MHz, CDCl₃); δ = 0.92 (t, 3H), 1.69 (ddq, 1H), 1.93 (m, 1H), 2.04 (dd, 1H), 2.39 (dd, 1H), 2.91 (s, 3H), 3.34 (m, 1H), 4.88 (d, 1H), 6.18 (d, 1H), 6.64 (d, 1H), 6.79 (d, 1H), 7.31 (d, 1H), 7.75 (t, 1H), 9.57 (s, 1H).

### Beispiel 152

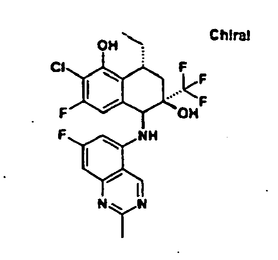

### (5S,6R,8R)-2-Chlor-8-ethyt-3-fluor-5-[(7-fluor-2-methylchinazolin-5-yl)amino]-6-(trifluormethyl)-5,6,7,8-tetrahydronahthalin-1,6-diol

Analog Beispiel 148 werden 300 mg (0.88 mmol (*2R*,*4R*)-4-(3-Chlor-4-fluor-2-methoxyphenyl)-2-hydroxy-2-(trifluormethyl)hexanal, 172 mg (0.97 mmol) 5-Amino-7-fluor-2-methylchinazolin und 0.55 ml Titantert-butylat zu 5-{[(*2R*,*4R*)-4-(3-Chlor-4-fluor-2-methoxyphenyl)-1-[(7-fluor-2-methylchinazolin-5-yl)imino]-2-(trifluormethyl)hexan-2-ol umgesetzt. 450 mg rohes Imin werden analog Beispiel 148 bei -30°C mit 2.5 mL (2.5 mmol) 1 M Bortribromid-Lösung zum gewünschten Produkt zyklisiert. Säulenchromatographie an Kieselgel (Hexan / Ethylacetat 0-50%) liefert 56 mg gewünschtes Produkt.
¹H-NMR (300 MHz, CD₃OD); δ = 0.93 (t, 3H), 1.71 (ddq, 1H), 1.98 (m, 1H), 2.01 (dd, 1H), 2.41 (dd, 1H), 2.75 (s, 3H), 3.31 (m, 1H), 5.14 (s, 1H), 6.62 (d, 1H), 6.63 (d, 1H); 6.78 (d, 1H), 9.50 (s, 1H).

### Beispiel 153

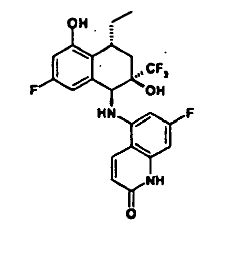

### 5-{[(1α,2α,4β)-7-Fluor-2,5-dihydroxy-4-ethy)-2-(trifluormethyl-1,2,3,4-tetrahydronaphthalin-1-yl]aminol}-7-fluor-1H-chinolin-2-on

5-{[4-(4-Fluor-2-methoxyphenyl)-2-hydroxy-2-(trifluorhethyl)hexyliden]amino)-7-fluor-1H-chinolin-2-on (163,1 mg, 0,35 mmol), hergestellt aus dem in Beispiel 148 beschriebenen Aldehyd und 5-Amino-7-fluor-1H-chinolin-2-on, wird in 3,5 mL Dichlormethan vorgelegt und bei -20°C tropfenweise mit 3,5 mL einer 1M Lösung von BBr₃ in Dichlormethan versetzt. Nach drei Stunden Rühren im Temperaturbereich zwischen -20 und + 10 °C ist kein Ausgangsmaterial mehr vorhanden. Die Reaktionsmischung wird vorsichtig auf eine Mischung aus gesättigter NaHCO₃-Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels mehrfach chromatographiert (NH₂ Flash, Laufmittel: Dichlormethan/ Methanol). Dabei werden 12,6 mg (8%) der Titelverbindung erhalten.
1H-NMR (300 MHz, CD₃OD): δ = 0,93 (3H), 1,68 (1H), 1,89-2,06 (2H), 2,35 (1H), 3,25 (1H, das Signal liegt teilweise unter dem Lösungsmittelsignal), 4,96 (1H), 6,22 (1H), 6,32-6,50 (4H), 8,13 (1H).

### Beispiel 154

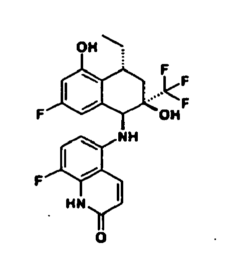

### 5-{[(5S,6R,8R)-8-Ethyl-3-fluor-1,6-dihydroxy-6-(trifluormethyl)-5,6,7,8-tetrahydronaphthalin-5-yllamino}-8-fluorchinolin-2(1H)-on

Wird als zusätzliches Produkt neben Beispiel 150 isoliert.
¹H-NMR (300 MHz, CD₃OD); δ = 0.90 (t, 3H), 1.67 (ddq, 1H), 1.92 (m, 1H), 1.95 (dd, 1H), 2.33 (dd, 1H), 3.25 (m, 1H), 4.91 (s, 1H), 6.34 (dd, 1H), 6.44 (m, 2H), 6.54 (d, 1H), 7.18 (dd, 1H), 8.18 (d, 1H).

### Beispiel 155

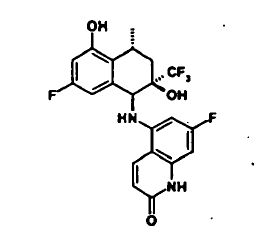

### 5-{[(1α,2α,4β)-7-Fluor-2,5-dihydroxy-4-methyl-2-(trifluormethyl)-1,2,3,4-tetrahydronaphthalin-1-yl]amino}-7-fluor-1H-chinolin-2-on

5-{[4-(4-Fluor-2-methoxyphenyl)-2-hydroxy-2-(trifluormethyt)pentytiden]amino}-7-fluor-1H-chinolin-2-on (241 mg, 0,53 mmol), hergestellt aus dem auf übliche Weise synthetisierten Atdehyd und 5-Amino-7-fluor-1H-chinolin-2-on, wird in 3 mL Dichlormethan vorgelegt und bei -40 °C tropfenweise mit 5,31 mL einer 1M Lösung von BBr₃ in Dichlormethan versetzt. Nach drei Stunden Rühren bei -40 °C lässt man den Ansatz auf Raumtemperatur kommen. Nach dem Rühren über Nacht bei Raumtemperatur wird die Reaktionsmischung vorsichtig auf eine Mischung aus gesättigter NaHCO₃-Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels chromatographiert (NH₂ Flash, Laufmittel: Dichlormethan/ Methanol). Dabei werden 82,4 g (35,2%) der Titelverbindung erhalten.
¹H-NMR (300 MHz, CD₃OD): δ = 1,45 (3H), 1,91 (1H), 2,47 (1H), 3,30 (1H, das Signal liegt praktisch unter dem Lösirngsmittelsignal), 5,11 (1H), 6,33-6,52 (5H), 8,18 (1H).

### Beispiel 156

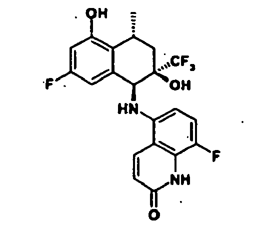

### 5-{[(1α,2α,4β)-7-Fluor-2,5-dihydroxy-4-methyl-2-(trifluormethyl)-1,2,3,4-tetrahydronaphthalin-1-yl]amino}-8-fluor-1H-chinolin-2-on

5-{[4-(4-Fluor-2-methoxyphenyl)-2-hydroxy-2-(trifluormethyl)pentyliden]amino}-8-fluor-1H-chinolin-2-on (481,6 mg, 1,06 mmol), hergestellt aus dem auf übliche Weise synthetisierten Aldehyd und 5-Amino-8-fluor-1H-chinolin-2-on, wird in 5 mL Dichlormethan vorgelegt und bei -40°C tropfenweise mit 10,6 mL einer 1M Lösung von BBr₃ in Dichlormethan versetzt. Nach drei Stunden Rühren bei -40 °C lässt man den Ansatz auf Raumtemperatur kommen. Nach dem Rühren über Nacht bei Raumtemperatur wird die Reaktionsmischung vorsichtig auf eine Mischung aus gesättigter NaHCO₃-Lösung und Eis gegeben und dreimal mit Ethylacetat extrahiert. Die vereinigten organischen Extrakte werden mit Sole gewaschen, über Na₂SO₄ getrocknet und der Rückstand nach dem Abrotieren des Lösungsmittels chromatographiert (Flash, Laufmittel: Dichlormethan/ Methanol). Dabei werden 196,7 g (42,1%) der Titelverbindung erhalten.
1H-NMR (300 MHz, CD₃OD): δ = 1,39 (3H), 1,82 (1H), 2,39 (1H), 3,30 (1H, das Signal liegt praktisch unter dem Lösungsmittelsignal), 5,01 (1H), 6,38-6,50 (3H), 6,53 (1H), 7,19 (1H), 8,17 (1H).

### Beispiel 157

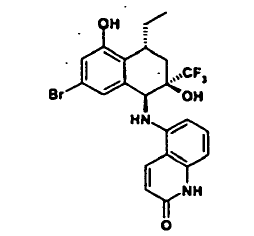

### 5-{[(1α,2α,4β)-7-Brom-2,5-dihydroxy-4-ethyl-2-(trifluormethyl)-1,2,3,4-tetrahydronaphthalin-1-yl]amino}-1H-chinolin-2-on

5-{[4-(4-Brom-2-methoxyphenyl)-2-hydroxy-2-(trifluormethyl)hexyliden]amino}-1H-chinolin-2-on (530 mg, 1,04 mmol), hergestellt aus dem in analoger Weise zu Beispiel 91 synthetisierten Aldehyd und 5-Amino-1H-chinolin-2-on, wird in 10 mL Dichlormethan gelöst und bei -50 °C tropfenweise mit 10,4 mL einer 1 M Lösung von BBr₃ in Dichlormethan versetzt. Nach drei Stunden Rühren bei -40 °C, zwei Stunden Rühren zwischen -40 °C und 0 °C, eine Stunde zwischen 0 °C und Raumtemperatur und 18 Stunden bei Raumtemperatur wird die Reaktionsmischung vorsichtig auf eine Mischung aus gesättigter NaHCO₃-Lösung und Eis gegeben. Nach Zugabe von 150 mL Ethylacetat wird 20 Minuten kräftig gerührt. Die Ethylacetatphase wird abgetrennt und die wässrige Phase ein weiters Mal mit Ethylacetat extrahiert. Die vereinigten organischen Extrakte werden mit Wasser (zweimal je 40 mL) und Sole (einmal 40 mL) gewaschen, über Na₂SO₄ getrocknet und der Rückstand, nach dem Abrotieren des Lösungsmittels chromatographiert (Flash, Laufmittel: Dichlormethan/ Methanol). Dabei werden 216,6 g (42%) der Titelverbindung erhalten.
1H-NMR (400 MHz, CD₃OD): δ = 0,90 (3H), 1,70 (1H), 1,89-2,02 (2H), 2,35 (1H), 3,26 (1H, das Signal liegt teilweise unter dem Lösungsmittelsignal), 4,98 (1H), 6,45 (1H), 6,49 (1H), 6,69 (1H), 6,87 (2H), 7,33 (1H), 8,19 (1H).

### Beispiel 158

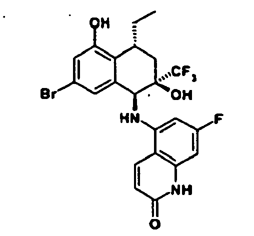

### 5-{[(1α,2α,4β)-7-Brom-2,5-dihydroxy-4-ethyl-2-trifluormethyl)-1,2,3,4-tetrahydronaphthalin-1-yl]amino}-7-fluor-1H-chinolin-2-on

5-{[4-(4-Brom-2-methoxyphenyl)-2-hydroxy-2-(trifluormethyl)hexyliden]amino}-7-fluor-1H-chinolin-2-on (500 mg, 0,94 mmol), hergestellt aus dem in analoger Weise zu Beispiel 91 synthetisierten Aldehyd und 5-Amino-7-fluor-1H-chinolin-2-on, wird in 9,1 mL Dichlormethan gelöst und bei -50 °C tropfenweise mit 9,45 mL einer 1M Lösung von BBr₃ in Dichlormethan versetzt Nach drei Stunden Rühren bei -40 °C, zwei Stunden Rühren zwischen -40 °C und 0 °C, eine Stunde zwischen 0 °C und Raumtemperatur und 18 Stunden bei Raumtemperatur wird die Reaktionsmischung vorsichtig auf eine Mischung aus gesättigter NaHCO₃-Lösung und Eis gegeben. Nach Zugabe von 150 mL Ethylacetat wird 20 Minuten kräftig gerührt. Die Ethylacetatphase wird abgetrennt und die wässrige Phase ein weiters Mal mit Ethylacetat (100 mL) extrahiert. Die vereinigten organischen Extrakte werden mit Wasser (zweimal je 40 mL) und Sole (einmal 40 mL) gewaschen, über Na₂SO₄ getrocknet und der Rückstand nach dem Abrotieren des Lösungsmittels chromatographiert (Flash, Laufmittel: Dichlormethan/ Methanol). Dabei werden 171,7 g (35,3%) der Titelverbindung erhalten.

### Beispiele 158A und 158B

### 5-{[(1R,2S,4S)-7-Brom-2,5-dihydroxy-4-ethyl-2-trifluormethyl)-1,2,3,4-tetrahydronaphthalin-1-yl]amino}-7-fluor-1H-chinolin-2-on und 5-{[(1S,2R,4R)-7-Brom-2,5-dihydroxy-4-ethyl-2-(trifluormethyl)-1,2,3,4-tetrahydronaphthalin-1-yl]amino}-7-fluor-1H-chinolin-2-on

Das in Beispiel 158 beschriebene Racemat wird mittels chiraler HPLC (Chiralpak AD-H 5µ, Laufmittel Hexan/ Ethanol) in seine Enantiomeren getrennt. Erhalten werden 85,2 mg des (+)-Enantiomer ([α]_{D} = +93,7°, MeOH) und 79 mg des (-)-Enantiomers ([α]_{D} = -95,9°, MeOH). Über die absolute Stereochemie kann natürlich keine Aussage gemacht werden.

### Beispiel 159

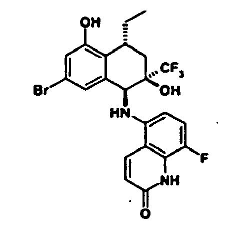

### 5-{[(1α,2α,4β)-7-Brom-2,5-dihydroxy-4-ethyl-2-(trifluormethyl)-1,2,3,4-tetrahydronaphthalin-1-yl]amino}-8-fluor-1H-chinolin-2-on

5-{[4-(4-Brom-2-methoxyphenyl)-2-hydroxy-2-(trifluormethyl)hexyliden]amino}-8-fluor-1H-chinolin-2-on (550 mg, 1,04 mmol), hergestellt aus dem in analoger Weise zu Beispiel 91 synthetisierten Aldehyd und 5-Amino-8-fluor-1H-chinolin-2-on, wird in 10 mL Dichlormethan gelöst und bei -50 °C tropfenweise mit 10,4 mL einer 1 M Lösung von BBr₃ in Dichlormethan versetzt. Nach drei Stunden Rühren bei -40 °C, zwei Stunden Rühren zwischen -40 °C und
0 °C, eine Stunde zwischen 0 °C und Raumtemperatur und 18 Stunden bei Raumtemperatur wird die Reaktionsmischung vorsichtig auf eine Mischung aus gesättigter NaHCO₃-Lösung und Eis gegeben. Nach Zugabe von 150 mL Ethylacetat wird 20 Minuten kräftig gerührt. Die Ethylacetatphase wird abgetrennt und die wässrige Phase ein weiters Mal mit Ethylacetat (100 mL) extrahiert. Die vereinigten organischen Extrakte werden mit Wasser (zweimal je 40 mL) und Sole (einmal 40 mL) gewaschen, über Na₂SO₄ getrocknet und der
Rückstand nach dem Abrotieren des Lösungsmittels chromatographiert (NH₂ Flash, Laufmittel: Dichlormethan/ Methanol). Dabei werden 209,4 g (39,1%) der Titelverbindung erhalten.
1H-NMR (300 MHz, CD₃OD): δ = 0,92 (3H), 1,69 (1H), 1,86-2,01 (2H), 2.33 (1H), 3,25 (1H, das Signal liegt teilweise unter dem Lösungsmittelsignal), 4,92 (1H), 6.36 (1H). 6,53 (1H), 6,86 (2H), 7,19 (1H), 8,19 (1H).

### Beispiel 160

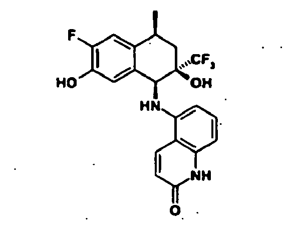

### 5-{[(1α,2α,4α)-6-Fluor-2,7-dihydroxy-4-methyl-2-(trifluormethyl)-1,2,3,4-tetrahydronaphthalin-1-yl]amino}-1H-chinolin-2-on

5-{[4-(3-Fluor-4-methoxyphenyl)-2-hydroxy-2-(trifluormethyl)pentyliden]amino}-1H-chinolin-2-on (160 mg, 0,37 mmol), hergestellt aus dem entsprechenden Aldehyd und 5-Amino-1H-chinolin-2-on, wird in 3,5 mL Dichlormethan gelöst und bei -20 °C tropfenweise mit 3,7 mL einer 1 M Lösung von BBr₃ in Dichlormethan versetzt. Nach drei Stunden Rühren zwischen -20 und +5 °C wird die Reaktionsmischung vorsichtig auf eine Mischung aus gesättigter NaHCO₃-Lösung und Eis gegeben. Nach Zugabe von 100 mL Ethylacetat wird 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden mit Wasser (20 mL) und Sole (20 mL) gewaschen, über Na₂SO₄ getrocknet und der Rückstand nach dem Abrotieren des Lösungsmittels chromatographiert (Isolute NH₂, Laufmittel Dichlormethan/ Methanol). Dabei wird ein Diastereomerengemisch (145 mg) erhalten, das über HPLC (Kromasil C18, 5 µ, Laufmittel: Wasser/ Methanol) in die reinen Diastereomeren getrennt wird. Es werden 55,3 mg (35,7%) der Titelverbindung erhalten und 89,4 mg (57.7%) der in Position 4 epimeren Verbindung, die in Beispiel 162 beschrieben wird.
1H-NMR (300 MHz, DMSO-d₆): δ = 1,32 (3H), 1,80 (1H), 2,18 (1H), 3,09 (1H), 5,28 (1H), 6,10 (1H), 6,25 (1H), 6,39 (1H), 6,55-6,63 (2H), 6,82 (1H), 7,10 (1H), 7,28 (1H), 8,18 (1H), 9,57 (1H), 11,53 (1H).

### Beispiel 161

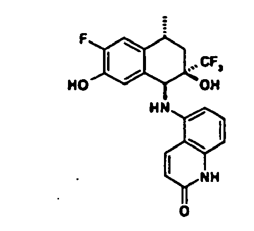

### 5-{[(1α,2α,4β)-6-Fluor-2,7-dihydroxy-4-methyl-2-(trifluormethyl)-1,2,3,4-tetrahydronaphthalin-1-yl]aminol-1H-chinolin-2-on

Nach Diastereomerentrennung, beschrieben in Beispiel 160, werden 89,4 mg (57,7%) der Titelverbindung erhalten.
1H-NMR (300 MHz, DMSO-d6): δ = 1,39 (3H), 1,59 (1H), 2,39 (1H), 2,93 (1H), 4,90 (1H), 6,10 (1H), 6,12 (1H), 6,35-6,48 (2H), 6,59,(1H), 6,80 (1H), 7.06 (1H), 7,22 (1H), 8,23 (1H), 9,52 (1H), 11,58 (1H).

### Beispiel 162

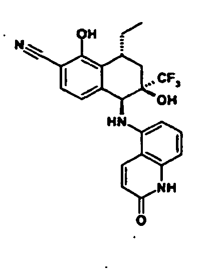

### (5α,6α,8β)-8-Ethyl-1,6-dihydroxy-5-(2-oxo-1,2-dihydrochinolin-5-ylamino)-6-(trifluormethyl-5,6,7,8-tetrahydronaphthalin-2-carbonitril

5-{[(*1*α,*2*α,*4*β-6-Chlor-2, 5-dihydroxy-4-ethyl-2-(trifluormethyl)-1,2,3,4-tetrahydronaphthalin-1-yl]amino}-1H-chinolin-2-on, beschrieben in den Beispielen 92A und 92B, (16,7 mg, 0,037 mmol), Natriumcyanid (3,6 mg, 0,074 mmol) und Nickel(I)bromid
(8,17 mg, 0,037 mmol) werden in 0,33 mL N-Methylpyrrolidinon gegeben und in der Mikrowelle bei einem Druck von 20 Bar und einer Temperatur von 200 °C umgesetzt. Anschließend wird die Reaktionsmischung mit fünf mL Ethylacetat verdünnt. Nach Zugabe von zwei mL Wasser wird für 15 Minuten kräftig gerührt. Es werden weitere 50 mL Ethylacetat zugegeben und die organische Phase zweimal mit Wasser (je 10 mL) und einmal mit Sole (10 mL) geschüttelt. Nach dem Trocknen über Na₂SO₄ wird das Lösungsmittel abrotiert und der Rückstand an Kieselgel (Laufmittel Dichlormethan/ Methanol) chromatographiert. Isoliert werden 7,7 mg (47,7%) der gewünschten Verbindung. IR (Diamant): 2225 cm⁻¹.

### Beispiel 163

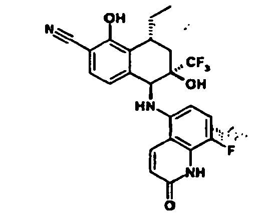

### ((5α,6α,8β)-8-Ethyl-1,6-dihydroxy-5-(8-fluor-2-oxo-1,2-dihydrochinolin-5-ylamino)-6-(trifluormethyl)-5,6,7,8-tetrahydronaphthalin-2-carbonitril

5-{[(*1*α,*2*α,*4*β)-6-Chlor-4-ethyl-2,5-dihydroxy-2-(trifluormethyl)-1,2,3,4-tetrahydronaphthalin-1-yl]amino}-8-fluor-1*H*-chinolin-2-on, beschrieben in den Beispielen 94A und 94B, (13,8 mg, 0,029 mmol), Natriumcyanid (2,87 mg, 0,059 mmol) und Nickel(I)bromid (6,4 mg, 0,029 mmol) werden in 0,26 mL N-Methylpyrrolidinon gegeben und in der Mikrowelle bei einem Druck von 20 Bar und einer Temperatur von 200 °C umgesetzt. Anschließend wird die Reaktionsmischung mit fünf mL Ethylacetat verdünnt. Nach Zugabe von zwei mL Wasser wird für 15 Minuten kräftig gerührt. Es werden weitere 50 mL Ethylacetat zugegeben und die organische Phase zweimal mit Wasser (je 10 mL) und einmal mit Sole (10 mL) geschüttelt. Nach dem Trocknen über Na₂SO₄ wird das Lösungsmittel abrotiert und der Rückstand an Kieselgel (Laufmittel Dichlormethan/ Methanol) chromatographiert. Isoliert werden 10,8 mg (79,9%) der gewünschten Verbindung. IR (Diamant): 2230 cm⁻¹.

### Beispiel 164

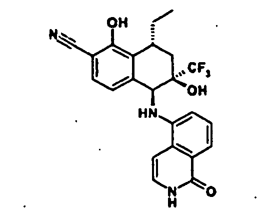

### (5α,6α,8β)-8-Ethyl-1,6-dihydroxy-5-[(1-oxo-1,2-dihydroisochiholin-5-yl)aminol-6-(trifluormethyl)-5,6,7,8-tetrahydronaphthalin-2-carbonitril

5-{[(*1*α,*2*α,*4*β)-6-Chlor-2,5-dihydroxy-4-ethyl-2-(trifluormethyl)-1,2,3,4-tetrahydronaphthalin-1-yl]amino}-2H-chinolin-1-on, beschrieben in den Beispielen 96A und 96B, (34,8 mg, 0,077 mmol), Natriumcyanid (7,53 mg, 0,15 mmol) und Nickel(I)bromid
(16,8 mg, 0,077 mmol) werden in 0,68 mL N-Methylpyrrolidinon gegeben und in der Mikrowelle bei einem Druck von 20 Bar und einer Temperatur von 200 °C umgesetzt. Anschließend wird die Reaktionsmischung mit fünf mL Ethylacetat verdünnt. Nach Zugabe von zwei mL Wasser wird für 15 Minuten kräftig gerührt. Es werden weitere 50 mL Ethylacetat zugegeben und die organische Phase zweimal mit Wasser (je 10 mL) und einmal mit Sole (10 mL) geschüttelt. Nach dem Trocknen über Na₂SO₄ wird das Lösungsmittel abrotiert und der Rückstand an Kieselgel (Laufmittel Dichlormethan/ Methanol) chromatographiert. Isoliert werden 18,8 mg (55,2%) der gewünschten Verbindung.
IR (Diamant): 2230 cm⁻¹.

### Beispiel 165

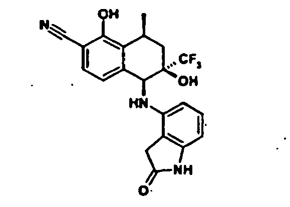

### (5α,6α,8α)-8-Methyl-1,6-dihydroxy-5-(2-oxo-2,3-dihydro-1H-indol-4-ylamino)-6-(trifluormethyl)-5,6,7,8-tetrahydronaphthalin-2-carbonitril

4-{[6-Chlor-2,5-dihydroxy-4-methyl-2-(trifluormethyl)-1,2,3,4-tetrahydronaphthalin-1-yl]amino}-1,3-dihydroindol-2-on (70 mg, 0,16 mmol), beschrieben in den Beispielen 89A und 89B, und zwar eine Fraktion, die beide Diastereomere als Racemat enthält, Natriumcyanid (16,1 mg, 0,33 mmol) und Nickel(I)bromid (35,8 mg, 0,16 mmol) werden in 1,45 mL N-Methylpyrrolidinon gegeben und in der Mikrowelle bei einem Druck von 20 Bar und einer Temperatur von 200 °C umgesetzt. Anschließend wird die Reaktionsmischung mit fünf mL Ethylacetat verdünnt. Nach Zugabe von zwei mL Wasser wird für 15 Minuten kräftig gerührt. Es werden weitere 50 mL Ethylacetat zugegeben und die organische Phase zweimal mit Wasser (je 10 mL) und einmal mit Sole (10 mL) geschüttelt. Nach dem Trocknen über Na₂SO₄ wird das Lösungsmittel abrotiert und der Rückstand an Kieselgel (Laufmittel: Dichlormethan/ Methanol) chromatographiert. Isoliert werden 15,9 mg (23.2%) der Titelverbindung und 9,1 mg (13,3%) der in Position 8 epimeren Verbindung, die in Beispiel 167 charakterisiert wird.
IR (diamant): 2225 cm⁻¹.

### Beispiel 166

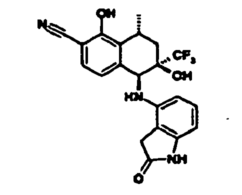

### (5α,6α,8β)-1,6-Dihydroxy-8-methyl-5-[(2-oxo-2,3-dihydro-1H-indol-4-yl)aminol-6-(trifluormethyl)-5,6,7.8-tetrahydronaphthalin-2-carbonitril

9,1 mg der Titelverbindung werden nach Reaktion und Chromatographie, beschrieben in Beispiel 165, erhalten.
IR (KBr): 2225 cm⁻¹.

### Beispiel 168

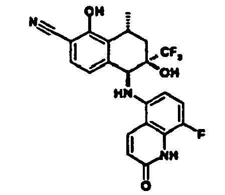

### (5α,6α,8β)-5-[(8-Fluor-2-oxo-1,2-dihydrochinolin-5-yl)amino]-1,6-dihydroxy-8-methyl-6-(trifluormethyl)-5,6,7,8-tetrahydronaphthalin-2-carbonitril

5-{[(*1*α,*2*α,*4*β)-6-Chlor-2,5-dihydroxy-4-methyl-2-(trifluormethyl)-1,2,3,4-tetrahydronaphthalin-1-yl]amino-8-fluor-1H-chinolin-2-on, beschrieben in den Beispielen 84A und 848, (21,4 mg, 0,047 mmol), Natriumcyanid (4,6 mg, 0,094 mmol) und Nickel(I)bromid (10,2 mg, 0,047 mmol) werden in 0,41 mL N-Methylpyrrolidinon gegeben und in der Mikrowelle bei einem Druck von 20 Bar und einer Temperatur von 200 °C umgesetzt. Nach der schon mehrfach beschriebenen Aufarbeitung und Chromatographie unter identischen Bedingungen werden 15,4 mg (73,5%) der gewünschten Verbindung isoliert. IR (Diamant): 2230 cm⁻¹.

### Beispiel 169

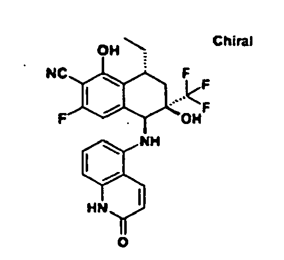

### 5-{[(5S,6R,8R)-8-Ethyl-1,6-dihydroxy-3-fluor-5-[(2-oxo-1,2-dihydrochinolin-5-yl)amino]-6-(trifluormethyl)-5,6,7,8-tetrahydronaphthalin-2-carbonitril

20 mg (0.04 mmol) 5-{[(*5S*,*6R*,*8R*)-2-Chlor-8-ethyl-3-fluor-1,6-dihydroxy-2-6-(trifluormethyl)-5,6,7,8-tetrahydronaphthalin-5-yl]amino}chinolin-2(1H)-on 4.2 mg (0.08 mmol) Natriumcyanid und 9.3 mg (0.04 mmol) Nickel(II)bromid werden in 1.4 ml N-Methyl-2-pyrrolidinon über 20 min mit Mikrowellen bestrahlt (CEM Discover® , max. Temperatur 200°C, Energie 120 W, max. Druck 20 bar). Die Reaktionsmischung wird mit Ethylacetat und wasser verdünnt und kräftig gerührt. Man trennt die Phasen und wäscht mit Wasser und gesättigter Natriumchlorid Lösung. Nach Trocknen über Natriumsulfat und Entfernen des Lösungsmittels liefert präparative Dünnschichtchromatographie an einer Aminphase (Merck NH₂F₂₅₄, Ethylacetat / Methanol / Triethylamin 15:3:1) 7 mg gewünschtes Produkt.
¹H-NMR (300 MHz, CD₃OD); δ = 0.90 (t, 3H), 1.66 (ddq, 1H), 1.86 (m, 1H), 1.98 (dd, 1H), 2.33 (dd, 1H), 3.28 (m, 1H), 4.99 (s, 1H), 5.97 (d, 1H), 6.41 (d, 1H), 6.50 (d, 1H), 6.66 (d, 1H), 7.32 (t, 1H), 8.18 (d, 1H).

### Beispiel 170

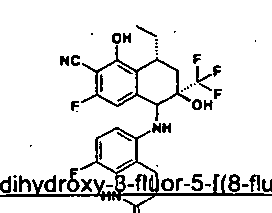

### (5α,6α,8β)-8-Ethyl-1,6-dihydroxy-3-fluor-5-[(8-fluor-2-oxo-1,2-dihydrochinolin-5-yl)amino]-6-(trifuormethyl)-5,6,7,8-tetrahydronaphthalin-2-carbonitril

15 mg (0.03 mmol) 5-{[(*5S*,*6R*,*8R*)-2-Chlor-8-ethyl-3-fluor-1,6-dihydroxy-2-6-(trifluormethyl)-5,6,7,8-tetrahydronaphthalin-5-yl]amino}-8-fluorchinolin-2(*1H*)-on 3.0 mg (0.06 mmol) Natriumcyanid und 6.7 mg (0.03 mmol) Nickel(II)bromid werden in 1 ml N-Methyl-2-pyrrolidinon über 20 min mit Mikrowellen bestrahlt (CEM Dicover®, max. Temperatur 200°C, Energie 120 W, max. Druck 20 bar). Die Reaktionsmischung wird mit Ethylacetat und Wasser verdünnt und kräftig gerührt. Man trennt die Phasen und wäscht mit Wasser und gesättigter Natriumchlorid Lösung. Nach Trocknen über Natriumsulfat und Entfernen des Lösungsmittels liefert präparative Dünnschichtchromatographie an Kieselgel (Ethylacetat) 8 mg gewünschtes Produkt.
¹H-NMR (300 MHz, CD₃OD); δ = 0.90 (t, 3H), 1.66 (ddq, 1H), 1.86 (m, 1H), 1.98 (dd, 1H), 2.33 (dd, 1H), 3.28 (m, 1H), 4.99 (s, 1H), 5.97 (d, 1H), 6.41 (d, 1H), 6.50 (d, 1H), 6.66 (d, 1H), 7.32 (t, 1H), 8.18 (d, 1H).

### Beispiel 171

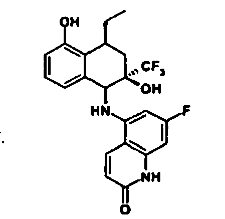

### 5-{[(1α,2α,4α)-2,5-Dihydroxy-4-ethyl-2-(trifluormethyl)-1,2,3,4-tetrahydronaphthalin-1-yl]amino}-fluor-1H-chinolin-2-on

5-{[4-(2-Methoxyphenyl)-2-hydroxy-2-(trifluormethyl)hexyliden]amino}-7-fluor-1H-chinolin-2-on (670 mg, 1,49 mmol), hergestellt aus 4-(2-Methoxyphenyl)-2-hydroxy-2-(trifluormethyl)-hexanal (synthetisiert in Analogie zu dem in Beispiel 91 beschriebenen Verfahren) und 5-Amino-7-fluor-1H-chinolin-2-on, wird in 14,9 mL Dichlormethan gelöst und bei -50 °C tropfenweise mit 14,87 mL einer 1 M Lösung von Bortribromid in Dichlormethan versetzt. Nach zweistündigem Rühren bei -40 °C, einstündigem Rühren zwischen -40 und -20 °C einstündigem Rühren zwischen -20 und -10 °C, einstündigem Rühren zwischen -10 °C und Raumtemperatur werden 18 Stunden bei Raumtemperatur nachgerührt. Der Ansatz wird auf ein Gemisch aus gesättigter Natriumhydrogencarbonatlösung und Eis gegossen, mit 150 mL Ethylacetat verdünnt und anschließend 20 Minuten kräftig gerührt. Die Ethylacetatphase wird abgetrennt und die wässrige Phase ein weiteres Mal mit Ethylacetat (100 mL) extrahiert. Die vereinigten organischen Extrakte werden zweimal mit Wasser (je 40 mL) und einmal mit Sole (40 mL) gewaschen, getrocknet und das Lösungsmittel abrotiert. Nach mehrmaliger Chromatographie (Flashmaster, Laufmittel Dichlormethan/ Methanol) werden 97,4 mg der Titelverbindung und 178,4 mg des in Position 4 epimeren Diastereomers (Charakterisierung in Beispiel 172) erhalten (jeweils als Racemat). Desweiteren wird eine leicht verunreinigte Fraktion der Titelverbindung erhalten (118,6 mg).
1H-NMR (400 MHz, CD₃OD): δ = 1,09 (3H), 1,89 (1H), 2,02-2,13 (2H), 2,39 (1H), 3,00 (1H), 5,07 (1H), 6,32-6,42 (3H), 6,55-6,73 (2H), 6,96 (1H), 8,13 (1H).

### Beispiel 172

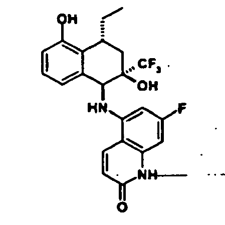

### 5-{[(1α,2α,4β)-2,5-Dihydroxy-4-ethyl-2-(trifluormethyl)-1,2,3,4-tetrahydronanhthalin-1-yl]amino}-7-fluo-1H-chinolin-2-on

Es werden 178,4 mg der im Titel beschriebenen Verbindung isoliert, deren Herstellung im Beipiel 171 beschrieben worden ist.
1H-NMR (400 MHz, CD₃OD): δ = 0,93 (3H), 1,70 (1H), 1,92-2,05 (2H), 2,37 (1H), 3,30 (1H, das Signal liegt praktisch unter dem Lösungsmitteisignal), 4,99 (1H), 6,22 (1H), 6,37 (1H), 6,42 (1H), 6,69 (2H), 6,95 (1H), 8,12 (1H).

### Beispiel 173

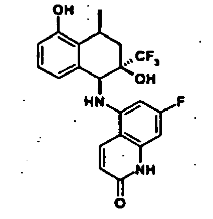

### 5-{[(1α,2α,4β)-2,5-Dihydroxy-4-methyl-2-(trifluormethyl)-1,2,3,4-tetrahydronaphthalin-1-yl]amino}-7-fluor-1H-chinolin-2-on

5-{[4-(2-Methoxyphenyl)-2-hydroxy-2-(trifluormethyl)pentyliden]amino}-7-fluor-1H-chinolin-2-on (290 mg, 0,66 mmol), hergestellt aus 4-(2-Methoxyphenyl)-2-hydroxy-2-(trifluormethyl)-pentanal und 5-Amino-7-fluor-1H-chinolin-2-on nach dem Titanatverfahren, wird in 6,6 mL Dichlormethan gelöst und bei -50 °C tropfenweise mit 6,65 mL einer 1 M Lösung von Bortribromid in Dichlormethan versetzt. Nach der in Beispiel 172 beschriebenen Reaktionsdurchführung (Temperaturprogramm) und Aufarbeitung wird der Rückstand mehrfach chromatographiert (Flashmaster NH₂ Phase, Laufmittel: Dichlormethan/ Methanol). Es werden 37,8 mg (13,5%) der Titelverbindung und 79,3 mg (28,3%) des in Position 4 epimeren Diastereomers (Charakterisierung in Beispiel 175) erhalten (jeweils als Racemat).

### Beispiele 173A und 173B

### 5-{[(1R,2S,4R)-2,5-Dihydroxy-4-methyl-2-(trifluormethyl)-1,2,3,4-tetrahydronanhthalin-1-yl]amino}-7-fluor-1H-chinolin-2-on und 5-{[(1S,2R,4S)-2,5-Dihydroxy-4-methyl-2-(trifluormethyl)-1,2,3,4-tetrahydronaphthalin-1-yl]amino}-7-fluor-1H-chinolin-2-on

Das in Beispiel 173 beschriebene Diastereomer (69,5 mg) wird mittels chiraler HPLC (Chiralcel OD 5 µ, Laufmittel Hexan/ Ethanol) in seine Enantiomeren getrennt. Erhalten werden 13,9 mg (20%) des (-)-Enantiomers ([α]_{D} = -46.6° MeOH) und 14,1 mg (20,3%) des (+)-Enantiomers ([α]_{D} = +46,9°, MeOH). Über die absolute Stereochemie kann natürlich keine Aussage gemacht werden.

### Beispiel 174

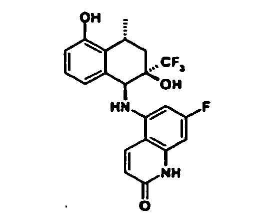

### 5-{[(1α,2α,4β)-2,5-Dihydroxy-4-methyl-2-(trifluormethyl)-1,2,3,4-tetrahydronaphthalin-1-yl]amino}7-fluor-1H-chinolin-2-on

79,3 mg (28,3%) der Titelverbindung werden durch die in Beispiel 174 beschriebene Reaktion erhalten.

### Beispiele 174A und 174B

### 5-{[(1R,2S,4S)-2,5-Dihydroxy-4-methyl-2-(trifluormethyl)-1,2,3,4-tetrahydronaphthalin-1-yl]amino}-7-fluor-1H-chinolin-2-on und 5-{[(1S,2R,4R)-2,5-Dihydroxy-4-methyl-2-(trifluormethyl)-1,2,3,4-tetrahydronaphthalin-1-yl]amino}-7-fluor-1H-chinolin-2-on

Das in Beispiel 174 beschriebene Diastereomer (67,4 mg) wird mittels chiraler HPLC (Chiralcel OD 5 µ, Laufmittel Hexan/ Ethanol) in seine Enantiomeren getrennt. Erhalten werden 33,1 mg (49,1 %) des (+)-Enantiomers ([α]_{D} = +39,9°, MeOH) und 27;2 mg (40,4%) des (-)-Enantiomers ([α]_{D} =-48,7°, MeOH). Über die absolute Stereochemie kann natürlich keine Aussage gemacht werden.

### Beispiel 175

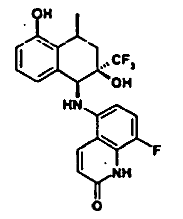

### 5-{[(1α,2α,4α)-2,5-Dihydroxy-4-methyl-2-(trifluormethyl)-1,2,3,4-tetrahydronaphthalin-1-yl]amino}-8-fluor-1H-chinolin-2-on

5-{[4-(2-Methoxyphenyl)-2-hydroxy-2-(trifluormethyl)pentyliden]amino}-8-fluor-1H-chinolin-2-on (455 mg, 1,04 mmol), hergestellt aus 4-(2-Methoxyphenyl)-2-hydroxy-2-(trifluormethyl)-pentanal und 5-Amino-8-fluor-1H-chinotin-2-on nach dem Eisessigverfahren, wird in 10,4 mL Dichlormethan gelöst und bei -50 °C tropfenweise mit 10,4 mL einer 1 M Lösung von Bortribromid in Dichlormethan versetzt. Nach der in Beispiel 172 beschriebenen Reaktionsdurchführung (Temperaturprogramm) und Aufarbeitung wird der Rückstand mehrfach chromatographiert (Flashmaster NH₂ Phase, Laufmittel: Dichlormethan/ Methanol). Es werden 88,3 mg (20,1%) der Titelverbindung und 223,1 mg (50,7%) des in Position 4 epimeren Diastereomers (Charakterisierung in Beispiel 177) erhalten (jeweils als Racemat).

### Beispiele 175A und 175B

### 5-{[(1R,2S,4R)-2,5-Dihydroxy-4-methyl-2-(trifluormethyl)-1,2,3,4-tetrahydronaphthalin-1-yl]amino}-8-fluor-1H-chinolin-2-on und 5-{[(1S,2R,4S)-2,5-Dihydroxy-4-methyl-2-(trifluormethyl)-1,2,3,4-tetrahydronaphthalin-1-yl]amino}-8-fluor-1H-chinolin-2-on

Das in Beispiel 176 beschriebene Diastereomer (71,8 mg) wird mittels chiraler HPLC (Chiralcel OD-H 5 µ, Laufmittel Hexan/ Ethanol) in seine Enantiomeren getrennt. Erhalten werden 35,5 mg (49,4%) des (-)-Enantiomers ([α]_{D} = -27,2°, MeOH) und 35,6 mg (49,6%) des (+)-Enantiomers ([α]_{D} = +26,5°, MeOH). Über die absolute Stereochemie kann natürlich keine Aussage gemacht werden.

### Beispiel 176

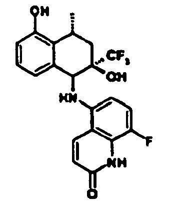

### 5-{[(1α,2α,4β}-2,5-Dihydroxy-4-methyl-2-(trifluormethyl)-1,2,3,4-tetrahydronaphthalin-1-yl]amino}-8-fluor-1H-chinolin-2-on

223,1 mg (50,7%) der Titelverbindung werden durch die in Beispiels 175 beschriebene Reaktion erhalten.

### Beispiele 176A und 176B

### 5-{[(1R,2S,4S)-2,5-Dihydroxy-4-methyl-2-(trifluormethyl)-1,2,3,4-tetrahydronaphthalin-1-yl]amino}-8-fluor-1H-chinolin-2on und 5-{[(1S,2R,4R)-2,5-Dihydroxy-4-methyl-2-(trifluormethyl)-1,2,3,4-tetrahydronaphthalin-1-yl]amino}-8-fluor-1H-chinolin-2-on

Das in Beispiel 177 beschriebene Diastereomer (192,7 mg) wird mittels chiraler HPLC (Chiralcel OD 5 µ, Laufmittel Hexan/ Ethanol) in seine Enantiomeren getrennt. Erhalten werden 97,9 mg (50.8%) des (+)-Enantiomer ([α]_{D} = +74,6°, MeOH) und 104,1 mg (54,1%) des (-)-Enantiomers ([α]_{D} = -78,6°, MeOH). Über die absolute Stereochemie kann natürlich keine Aussage gemacht werden.

### Beispiel 177

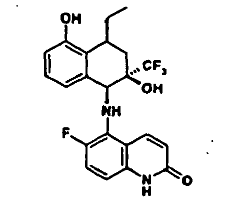

### 5-{[(1α,2α,4α)-4-Ethyl-2,5-dihydroxy-2-(trifluormethyl)-1,2,3,4-tetrahydronaphthalin-1-yl]amino}-6-fluor-1H-chinolin-2-on

5-{[4-(2-Methoxyphenyl)-2-hydroxy-2-trifluormethyl)hexyliden]amino}-6-fluor-1H-chinolin-2-on (271 mg, 0,6 mmol), hergestellt aus 4-(2-Methoxyphenyl)-2-hydroxy-2-(trifluormethyl)-hexanal (synthetisiert in Analogie zu dem in Beispiel 91 beschriebenen Verfahren) und 5-Amino-6-fluor-1H-chinolin-2-on nach der modifizierten Titanatmethode (Zusatz von Eisessig und Dioxan), wird in 6 mL Dichlormethan gelöst und bei -50 °C tropfenweise mit 6 mL einer 1 M Lösung von Bortribromid in Dichlormethan versetzt. Nach zweistündigem Rühren bei -40 °C, einstündigem Rühren zwischen -40 und -20 °C einstündigem Rühren zwischen

-20 und -10°C, einstündigem Rühren zwischen -10 °C und Raumtemperatur werden 18 Stunden bei Raumtemperatur nachgerührt. Der Ansatz wird auf ein Gemisch aus gesättigter Natriumhydrogencarbonatlösung und Eis gegossen, mit 100 mL Ethylacetat verdünnt und anschließend 20 Minuten kräftig gerührt. Die Ethylacetatphase wird abgetrennt und die wässrige Phase ein weiteres Mal mit Ethylacetat (50 mL) extrahiert. Die vereinigten organischen Extrakte werden zweimal mit Wasser (je 20 mL) und einmal mit Sole (20 mL) gewaschen, getrocknet und das Lösungsmittel abrotiert. Nach mehrmaliger Chromatographie (Flashmaster, Laufmittel: Dichlormethan/ Methanol) werden 28,6 mg (10,9%) der Titelverbindung und 59,8 mg (22,8%) des in Position 4 epimeren Diastereomers (Charakterisierung in Beispiel 178) erhalten (jeweils als Racemat).
1H-NMR (300 MHz, DMSO-d6): δ = 1,04 (3H), 1,88 (1H), 2,05 (1H), 2,23 (1H), 2,43 (1H), 5,18 (1H), 5,55 (1H), 6,19 (1H), 6,42 (1H), 6,62-6,80 (2H), 6,85 (1H), 6,99 (1H), 7,28 (1H), 8,19 (1H), 9,44 (1H), 11,65 (1H).

### Beispiel 178

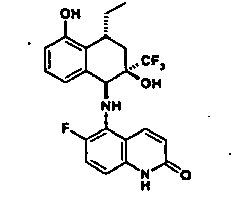

### 5-{[(1α,2α,4β)-4-Ethy)-2,5-dihydroxy-2-(triftuormethyl)1,2,3,4-tetrahydronaphthalin-1-yl]amino}-6-fluor-1H-chinolin-2-on

Es, werden 59,8 mg (22,8%) der Titelverbindung (beschrieben in Beispiel 177) erhalten.
1H-NMR (400 MHz, CD₃OD): δ = 0,89 (3H), 1,69 (1H), 1,82-1,99 (2H), 2,30 (1H), 3,32 (1H, das Signal liegt praktisch unter dem Lösungsmittelsignal), 5,30 (1H), 6,49 (1H), 6.66-6,74 (2H), 7,02 (1H), 7,10 (1H), 7,25 (1H), 8,15 (1H).

### Beispiel 179

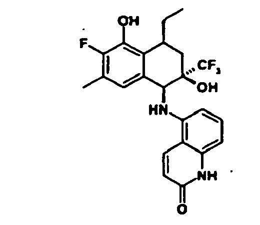

### 5-{[(1α,2α,4α)-4-Ethyl-6-fluor-2,5-dihydroxy-7-methyl-2-(trifluormethyl)-1,2,3,4-tetrahydronaphthalin-1-yl]amino}-1H-chinolin-2-on

Ein Gemisch aus 5-{[4-(3-Fluor-4-methyl-2-methoxyphenyl)-2-hydroxy-2-(trifluormethyl)hexyliden]amino}-1H-chinolin-2-on und 5-{(4-(3-Fluor-4-methyl-2-methoxyphenyl)-2-hydroxy-3-methyl-2-(trifluormethyl)pentyliden]amino)-1H-chinolin-2-on (676,4 mg, 1,46 mmol), hergestellt aus dem Gemisch der entsprechenden Aldehyde und

5-Amino-1H-chinolin-2-on, wird in 7 mL Dichlormethan gelöst und bei -40 °C tropfenweise mit 7,3 mL einer 1M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis gegossen und dreimal mit Ethylacetat extrahiert. Die vereinigten organischen Extrakte werden mit Sole gewaschen, getrocknet und das Lösungsmittel abrotiert. Nach zweimaliger Chromatographie (Flashmaster, Laufmittel: Dichlormethan/ Methanol) und anschließender HPLC (XBridge C18, 5µ, Laufmittel: Wasser/ Acetonitril) werden 23,1 mg (7,1%) der Titelverbindung (enthält noch 11% eines Dimethytderivats) und 30,1 mg (9,2%) des in Position 4 epimeren Diastereomers (Charakterisierung in Beispiel 180) erhalten (jeweits als Racemat).
1H-NMR (400 MHz, CD₃OD): δ = 1,08 (3H), 1,90 (1H), 1,98-2,13 (5H), 2,35 (1H), 2,98 (1H), 5,02 (1H), 6,45 (1H), 6,50-6,70 (3H), 7,34 (1H), 8,19 (1H).

### Beispiel 180

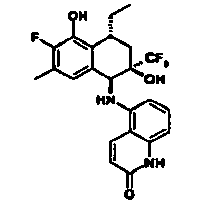

### 5-{[(1α,2α,4β)-4-Ethyl-6-fluor-2,5-dihydroxy-7-methyl-2-(trifluormethyl)1,2,3,4-tetrahydronaphthalin-1-yl]amino}-1H-chinolin-2-on

30,1 mg (9,2%) der Titelverbindung werden aus der in Beispiel 179 beschriebenen Reaktion erhalten.
1H-NMR (400 MHz, CD₃OD): δ = 0,90 (3H), 1,71 (1H), 1,89-2,00 (2H), 2,08 (1H), 2,32 (1H), 3,30 (1H, das Signal liegt praktisch unter dem Lösungsmittelsignal), 4,93 (1H), 6,40-6,50 (2H), 6,56 (1H), 6,65 (1H), 7,32 (1H), 8,18 (1H).

### Beispiel 181

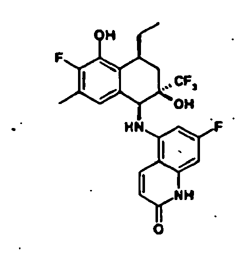

### 5-{[(1α,2α,4α)-4-Ethyl-6-fluor-2,5-dihydroxy-7-methyl-2-(trifluormethyl)-1,2,3,4-tetrahydronaphthalin-1-yl]amino}-7-fluor-1H-chinolin-2-on

Ein Gemisch aus 5-{[4-(3-Fluor-4-methyl-2-methoxyphenyl)-2-hydroxy-2-(trifluormethyl)hexyliden]amino}-7-fluor-1H-chinolin-2-on und 5-{[4-(3-Fluor-4-methyl-2-methoxyphenyl)-2-hydroxy-3-methyl-2-(trifluormethyl)pentyliden]amino}-7-fluor-1H-chinolin-2-on (307,5 mg, 0,64 mmol), hergestellt aus dem Gemisch der entsprechenden Aldehyde und 5-Amino-7-fluor-1H-chinolin-2-on, wird in 4 mL Dichlormethan gelöst und bei -40 °C tropfenweise mit 6,4 mL einer 1 M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis gegossen und dreimal mit Ethylacetat extrahiert. Die vereinigten organischen Extrakte werden mit Sole gewaschen, getrocknet und das Lösungsmittel abrotiert. Nach zweimaliger Chromatographie (Flashmaster, Laufmittel: Dichlormethan/ Methanol) und anschließender HPLC (XBridge C18, 5µ, Laufmittel: Wasser/ Acetonitril) werden 20 mg (13,4%) der Titelverbindung (enthält noch ein Diastereomer der Dimethylverbindung) und 38,3 mg (25,7%) des in Position 4 epimeren Diastereomers (enthält noch ein Diastereomer der Dimethylverbindung; Charakterisierung in Beispiel 182) erhalten (jeweils als Racemat).
1H-NMR (300 MHz, CD₃OD): δ = 1,12 (3H), 1,93 (1H), 2,05-2,20 (5H), 2,39 (1H), 3,04 (1H), 5,05 (1H), 6,38-6,51 (3H), 6,61 (1H), 8,18 (1H).

### Beispiel 182

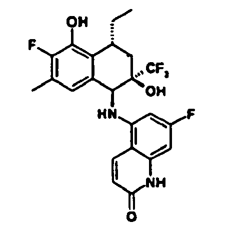

### 5-{[(1α,2α,4β)-4-Ethyl-6-fluor-2,5-dihydroxy-7-methyl-2-(trifluormethyl)-1,2,3,4-tetrahydronaphthalin-1-yl]amino}-7-fluor-1H-chinolin-2-on

38,3 mg (25,7%) der Titelverbindung werden aus der in Beispiel 181 beschriebenen Reaktion erhalten.
1H-NMR (300 MHz, CD₃OD): δ = 0,98 (3H), 1,79 (1H), 1,95-2,05 (2H), 2,18 (3H), 2,40 (1H), 3,32 (1H, das Signal liegt praktisch unter dem Lösungsmittetsignal). 4,99 (1H), 6,29 (1H), 6,42 (1H), 6,48 (1H), 6,60 (1H), 8,19 (1H).

### Beispiel 183

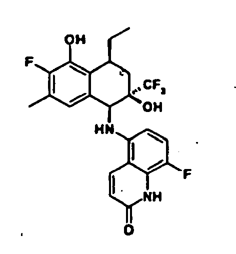

### 5-{[(1α,2α,4α)-4-Ethyl-6-fluor-2,5-dihydroxy-7-methyl-2-(trifluormethyl)-1,2,3,4-tetrahydronaphthalin-1-yl]amino}-8-fluor-1H-chinolin-2-on

Ein Gemisch aus 5-{[4-(3-Fluor-4-methyl-2-methoxyphenyl)-2-hydroxy-2-(trifluormethyl)hexyliden]amino}-8-fluor-1*H*-chinolin-2-on und 5-{[4-(3-Fluor-4-methyl-2-methoxyphenyl)-2-hydroxy-3-methyl-2-(trifluormethyl)pentyliden]amino}-8-fluor-1H-chinolin-2-on (867,4 mg, 1,8 mmol), hergestellt aus dem Gemisch der entsprechenden Aldehyde und 5-Amino-8-fluor-1H-chinolin-2-on, wird in 9 mL Dichlormethan gelöst und bei -40 °C tropfenweise mit 18 mL einer 1M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis gegossen und dreimal mit Ethylacetat extrahiert. Die vereinigten organischen Extrakte werden mit Sole gewaschen, getrocknet und das Lösungsmittel abrotiert. Nach Chromatographie (Flashmaster, Laufmittel: Dichtormethan/ Methanol) und anschließender HPLC (XBridge C18, 5µ, Laufmittel: Wasser/ Acetonitril) werden 10,7 mg (2,5%) der Titelverbindung und 18,8 mg (4,5%) des in Position 4 epimeren Diastereomers (Charakterisierung in Beispiel 184) erhalten (jeweils als Racemat).
1H-NMR (300 MHz, CD₃OD): δ = 1,12 (3H), 1,93 (1H), 2,05-2,19 (5H), 2,39 (1H), 3,04 (1H), 5,00 (1H), 6,51-6,68 (3H), 7,25 (1H), 8,21 (1H),

### Beispiel 184

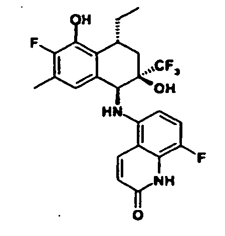

### 5-{[(1α,2α,4β-4-Ethyl-6-fluor-2,5-dihydroxy-7-methyl-2-(trifluormethyl)-1,2,3,4-tetrahydronaphthalin-1-yl]amino}-8-fluor-1H-chinolin-2-on

18.8 mg (4,2%) der Titelverbindung werden aus der in Beispiel 183 beschriebenen Reaktion erhalten. 1H-NMR (300 MHz, CD₃OD): δ = 0,98 (3H), 1,78 (1H), 1,93-2,05 (2H), 2,12 (3H), 2,39 (1H), 3,32 (1H, das Signal liegt praktisch unter dem Lösungsmittelsignal), 4,95 (1H), 6,40 (1H), 6,53-6,65 (2H), 7,22 (1H), 8,20 (1H).

### Beispiel 185

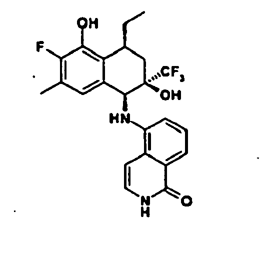

### 5-{[(1α,2α,4α)-4-Ethyl-6-fluor-2,5-dihydroxy-7-methyl-2-(trifluormethyl)-1,2,3,4-tetrahydronaphthalin-1-yl]amino}-2H-chinolin-1-on

Ein Gemisch aus 5-{[4-(3-Fluor-4-methyl-2-methoxyphenyl)-2-hydroxy-2-(trifluormethyl)hexyliden]amino}-2*H-*chinolin-1-on und 5-{[4-(3-Fluor-4-methyl-2-methoxyphenyl)-2-hydroxy-3-methyl-2-(trifluormethyl)pentyliden]amino}2H-chinolin-1-on (845,2 mg, 1,84.mmol), hergestellt aus dem Gemisch der entsprechenden Aldehyde und 5-Amino-2H-chinolin-1-on, wird in 9 mL Dichlormethan gelöst und bei -40°C tropfenweise mit 9,1 mL einer 1 M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis gegossen und dreimal mit Ethylacetat extrahiert. Die vereinigten organischen Extrakte werden mit Sole gewaschen, getrocknet und das Lösungsmittel abrotiert. Nach Chromatographie (Flashmaster, Laufmittel: Dichlormethan/ Methanol) und anschließender HPLC (XBridge C18, 5µ, Laufmittel: Wasser/ Acetonitril) werden 66,5 mg (16,2%) der Titelverbindung und 51,4 mg (12,5%) der in Position 4 epimeren Verbindung (Charakterisierung in Beispiel 186) erhalten (jeweils als Racemat).
1H-NMR (400 MHz, CD₃OD): δ = 1,06 (3H), 1,90 (1H), 1,99-2.11 (5H), 2,36 (1H), 3,01 (1H), 5,00 (1H), 6,54 (1H), 6,78 (1H), 7,06-7,13 (2H), 7,35 (1H), 7,68 (1H).

### Beispiel 186

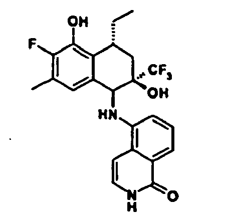

### 5-{[(1α,2α,4β)-4-Ethyl-6-Fluor-2,5-dihydroxy-7-methyl-2-(trifluormethyl)-1,2,3,4-tetrahydronaphthalin-1-yl]amino}-2H-chinolin-1-on

51,4 mg (12,5%) der Titelverbindung werden aus der in Beispiel 185 beschriebenen Reaktion erhalten.
1H-NMR (400 MHz, CD₃OD): δ = 0,92 (3H), 1,72 (1H), 1,89-2,00 (2H), 2,07 (3H), 2,32 (1H), 3,31 (1H), 4,93 (1H), 6,56 (1H), 6,81 (1H), 6,88 (1H), 7,13 (1H), 7,32 (1H), 7,62 (1H).

### Beispiel 187

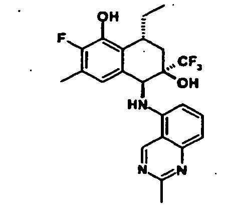

### (5α,6α.8β-8-Ethyl-2-fluor-3-methyl-5-[(2-methylchinazolin-5-yl)amino]-6-(trifluormethyl)-5,6,7,8-tetrahydronaphthalin-1,6-diol

Das Gemisch aus 1,1,1-Trifluor-2-[(2-methylchinazolin-5-ylimino)-methyl-]-4-(3-fluor-4-methyl-2-methoxyphenyl)-hexan-2-ol und 1,1-Trifluor-3-methyl-2-[(2-methylchinazolin-5-ylimino)-methyl-]-4-(3-fluor-4-methyl-2-methoxyphenyl)-pentan-2-ol (536,4 mg, 1,16 mmol), hergestellt aus dem Gemisch der entsprechenden Aldehyde und 5-Amino-2-methylchinazolin, wird in 7 mL Dichlormethan gelöst und bei -40 °C tropfenweise mit 11,6 mL einer 1M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis gegossen und dreimal mit Ethylacetat extrahiert. Die vereinigten organischen Extrakte werden mit Sole gewaschen, getrocknet und das Lösungsmittel abrotiert. Nach mehrfacher Chromatographie (Flashmaster, Laufmittel: Dichlormethan/ Methanol) werden 4,1 mg (1,6%) der Titelverbindung als Racemat erhalten (verunreinigt mit der entsprechenden Dimethylverbindung).
1H-NMR (300 MHz, CD₃OD): δ = 0,98 (3H), 1,80 (1H), 1.95-2.10 (2H), 2,11 (3H), 2,42 (1H), 2,83 (3H), 3,39 (1H, das Signal liegt praktisch unter dem Lösungsmittelsignal), 5,15 (1H), 6,62 (1H), 6,81 (1H), 7,20 (1H), 7,80 (1H), 9,62 (1H).

### Beispiel 188

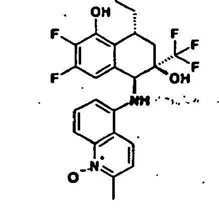

### (5S,6R,8R)-8-Ethyl-2,3-difluor-5-[(2-methyl-1-oxychinolin-5-yl)aminol-6-(trifluormethyl)-5,6,7,8-tetrahydronaphthalin-1,6-diol

Zu 91 mg (0.2 mmol) (5S,6R,8R)-8-Ethy1-2,3-difluor-5-[(2-methyl-chinolin-5-yl)amino]-6-(trifluormethyl)-5,6,7,8-tetrahydronaphthalin-1,6-diol in 20 ml Dichlormethan werden 85 mg (0.49 mmol) 3-Chlorperoxybenzoesäure gegeben. Man rührt zwei Stunden bei Raumtemperatur und gibt gesättigte NaHCO₃ Lösung zu. Nach 15 Minuten verünnt man mit Wasser, extrahiert mehrfach mit Dichlormetan, wäscht mit gesättigter Natriumchlorid Lösung, trocknet über Natriumsulfat und entfernt das Lösungsmittel im Vakuum. Säulenchromatographie an Kieselgel (Hexan / Ethylacetat 0-100%, dann Ethylacetat / Aceton 25%) liefert 51 mg gewünschtes Produkt.
¹H-NMR (300 MHz, CD₃OD); δ = 0.93 (t, 3H), 1.73 (ddq, 1H), 1.96 (m, 1H), 1.99 (dd, 1H), 2.37 (dd, 1H), 2.71 (s, 3H), 3:32 (m, 1H), 5.06 (s, 1H), 6.57 (dd, 1H), 6.83 (d, 1H), 7.47 (d, 1H), 7.66 (t, 1H), 7.94 (d, 1H), 8.22 (d, 1H).

### Beispiel 189

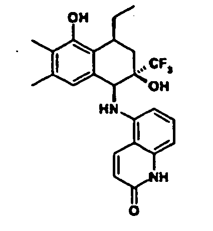

### 5-{[(1α,2α,4α)-4-Ethyl-2,5-dihydroxy-6,7-dimethyl-2-(trifluormethyn-1,2,3,4-tetrahydronaphthalin-1-yl]amino}-1H-chinolin-2-on

Ein Gemisch aus 5-{[4-(3,4-Dimethyl-2-methoxyphenyl)-2-hydroxy-2-(trifluormethyl)hexyliden]amino}-1H-chinolin-2-on und 5-{[4-(3,4-Dimethyl-2-methoxyphenyl)-2-hydroxy-3-methyl-2-(trifluormethyl)pentyliden]amino}-1H-chinolin-2-on (543,5 mg, 1.18 mmol), hergestellt aus dem Gemisch der entsprechenden Aldehyde und 5-Amino-1H-chinolin-2-on, wird in 7 mL Dichlormethan gelöst und bei -40 °C tropfenweise mit 11,8 mL einer 1 M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis (200 mL) gegossen. Nach Zugabe von 150 mL Ethylacetat wird kräftig gerührt. Die organische Phase wird abgetrennt und die wässrige Phase noch zweimal mit Ethylacetat (je 150 mL) extrahiert. Die vereinigten organischen Extrakte werden mit 100 mL Sole gewaschen, getrocknet und das Lösungsmittel abrotiert. Nach mehrmaliger Chromatographie (Flashmaster, Laufmittel: Dichlormethan/ Methanol) und anschließender HPLC (XBridge C18, 5µ, Laufmittel: Wasser/ Acetonitril) werden 7,6 mg (1,5%) der Titelverbindung isoliert 20 mg (3,8%) der in Position 4 epimeren Verbindung (Charakterisierung in Beispiel 190) werden ebenfalls erhalten (jeweils als Racemat).
1H-NMR (300 MHz, DMSO-d6): δ = 1,02 (3H), 1,182-2,14 (9H), 2,25 (1H), 2,95 (1H), 5,12 (1H), 5,92 (1H), 6,05 (1H), 6,38 (1H), 6,45-6,61 (3H), 7,23 (1H), 8,12 (1H), 8,22 (1H), 11,54 (1H).

### Beispiel 190

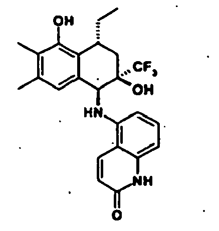

### 5-{[(1α,2α,4β)-2,5-Dihvdroxy-6,7-dimethyl-4-ethyl-2-(trifluormethyl)-1,2,3,4-tetrahydronanhthalin-1-yl]amino}-1H-chinolin-2-on

20 mg (3,8%) der Titelverbindung werden aus der in Beispiel 189 beschriebenen Reaktion erhalten.
1H-NMR (400 MHz; DMSO-d6): δ = 0,82 (3H), 1,52 (1H), 1,69-1,91 (2H), 1,95-2,10 (6H), 2,18 (1H), 3,19 (1H), 4,98 (1H), 5,85 (1H), 6,05 (1H), 6,30-6,41 (2H), 6,48 (1H), 6,52(1H), 7,19(1H), 8,04(1H), 8,12(1H), 11,53(1H).

### Beispiel 191

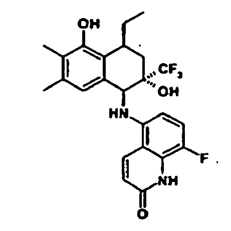

### 5-{[(1α,2α,4α)-2,5-Dihydroxy-6,7-dimethyl-4-ethyl-2-(trifluormethyl)-1,2,3,4-tetrahydronaphthalin-1-yl]amino}-8-fluor-1H-chinolin-2-on

Ein Gemisch aus 5-{[4-(3,4-Dimethyl-2-methoxyphenyl)-2-hydroxy-2-(trifluormethyl)hexyliden]amino}-8-fluor-1H-chinolin-2-on und 5-{[4-(3,4-Dimethyl-2-methoxyphenyl)-2-hydroxy-3-methyl-2-(trifluormethyl)pentyliden]amino}-8-fluor-1H-chinolin-2-on (703,5 mg, 1.47 mmol), hergestellt aus dem Gemisch der entsprechenden Aldehyde und 5-Amino-8-fluor-1H-chinolin-2-on, wird in 8 mL Dichlormethan gelöst und bei -40 °C tropfenweise mit 14,7 mL einer 1M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis (250 mL) gegossen. Nach Zugabe von 200 mL Ethylacetat wird kräftig gerührt. Die organische Phase wird abgetrennt und die wässrige Phase noch zweimal mit Ethylacetat (je 200 mL) extrahiert. Die vereinigten organischen Extrakte werden mit 100 mL Sole gewaschen, getrocknet und das Lösungsmittel abrotiert. Nach mehrmaliger Chromatographie (Flashmaster, verschiedene Phasen, Laufmittel Dichlormethan/ Methanol) und anschließender HPLC (XBridge C18, 5µ, Laufmittel: Wasser/ Acetonitril) werden 18,7 mg (2,7%) der Titelverbindung isoliert. 39,6 mg (5,8%) der in Position 4 epimeren Verbindung (Charakterisierung in Beispiel 193) werden auch erhalten (jeweils als Racemat). 1H-NMR (300 MHz, DMSO-d6): δ = 1,02 (3H), 1,80-2,18 (9H), 2,22 (1H), 2,94 (1H), 5,08 (1H), 5,89 (1H), 5,92 (1H), 6,40-6,50 (2H), 6,53 (1H), 7,20 (1H), 8,13 (1H), 8,25(1H), 11,45 (1H).

### Beispiel 192

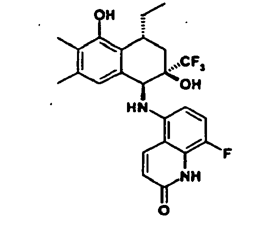

### 5-{[(1α,2α,4β)-2, 5-Dihydroxy-6,7-dimethyl-4-ethyl-2-(tritluormethyl)-1,2,3,4-tetrahydronaphthalin-1-yl]amino}-8-fluor-1H-chinolin-2-on

39,6 mg (5,8%) der Titelverbindung werden aus der in Beispiel 191 beschriebenen Reaktion erhalten.
1H-NMR (300 MHz, CD₃OD): δ = 0,90 (3H), 1,68 (1H), 1,80-2,00 (2H), 2,03-2,16 (6H), 2,30 (1H), 3,30 (1H, das Signal liegt praktisch unter dem Lösungsmittelsignal), 4,89 (1H), 6,32 (1H), 6,52 (1H), 6,61 (1H), 7,18 (1H), 8,19 (1H).

### Beispiel 193

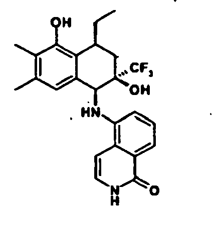

### 5-{[(1α,2α,4α)-2,5-Dihydroxy-6,7-dimethyl-4-ethyl-2-(trifluormethyl)-1,2,3,4-tetrahydronaphthalin-1-yl]amino}-2H-chinolin-1-on

Ein Gemisch aus 5-{[4-(3,4-Dimethyl-2-methoxyphenyl)-2-hydroxy-2-(trifluormethyl)hexyliden]amino}-2H-chinolin-1-on und 5-{[4-(3,4-Dimethyl-2-methoxyphenyl)-2-hydroxy-3-methyl-2-(trifluormethyl)pentyliden]amino}-2H-chinolin-1-on (1,08 g, 2,35 mmol), hergestellt aus dem Gemisch der entsprechenden Aldehyde und 5-Amino-2H-chinolin-1-on, wird in 13 mL Dichlormethan gelöst und bei -40 °C tropfenweise mit 23,5 mL einer 1 M Lösung von Bortribromid in Dichlormethan versetzt. Nach dreistündigem Rühren bei -40 °C wird der Reaktion erlaubt, langsam auf Raumtemperatur zu kommen. Nach dem Rühren über Nacht bei Raumtemperatur wird der Ansatz auf ein Gemisch aus gesättigter Natriumhydrogencarbonatlösung und Eis (350 mL) gegossen. Die Mischung wird dreimal mit Ethylacetat (je 250 mL) extrahiert. Die vereinigten organischen Extrakte werden mit 200 mL Sole gewaschen, getrocknet und das Lösungsmittel abrotiert. Nach mehrmaliger Chromatographie (Flashmaster, verschiedene Phasen, Laufmittel: Dichlormethan/ Methanol) und anschließender HPLC (XBridge C18, 5µ, Laufmittel: Wasser/ Acetonitril) werden
22,4 mg (2,1 %) der Titelverbindung isoliert. 23,2 mg (2,2%) der in Position 4 epimeren Verbindung (Charakterisierung in Beispiel 195) werden ebenfalls erhalten (jeweils als Racemat).
1H-NMR (300 MHz, CD₃OD): δ = 1,08 (3H), 1,82-2,18 (9H), 2,38 (1H), 3,02 (1H), 5,00 (1H), 6,59 (1H), 6,78 (1H), 7,07-7,14 (2H), 7,36 (1H), 7,68 (1H).

### Beispiel 194

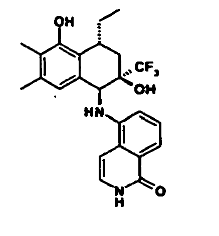

### 5-{[(1α,2α,4β)-2,5-Dihydroxy-6,7-dimethyl-4-ethyl-2-(trifluorrnethyl)-1,2,3,4-tetrahydronaphthalin-1-yl]amino}-2H-chinolin-1-on

23,2 mg (2,2%) der Titelverbindung werden aus der in Beispiel 193 beschriebenen Reaktion erhalten.
1H-NMR (300 MHz, CD₃OD): δ = 0,98 (3H), 1,75 (1H), 1,89-2,19 (8H), 2,37 (1H), 3,38 (1H, das Signal liegt, praktisch unter dem Lösungsmittelsignal), 5,01 (1H), 6,68 (1H), 6;85-6,95 (2H), 7,19 (1H), 7,39 (1H), 7,69 (1H).

### Beispiel 195

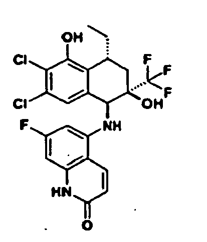

### 5-{[(5S,6R,8R)-2-Chlor-8-ethyl-1,6-dihydroxy-3-fluor-2-6-(trifluormethyl)-5,6,7,8-tetrahydronaphthalin-5-yl]lamino}chinolin-2(1H)-on

### 4-(3-Chlor-4-fluor-2-methoxyphenyl)-2-hydroxy-2-(trifluormethyl)hexanal:

Zu 20 g (122.7 mmol) 2,3-Dichlorphenol in 122 ml Dichlormethan und 13.8 ml Pyridin werden bei 0°C 11.3 ml (128 mmol) Propionsäurechlorid getropft. Man rührt die Mischung 16 Stunden lang und gibt 100 ml einer 2 M Salzsäure zu. Es wird mit Dichlormethan extrahiert und mit Wasser gewaschen. Nach dem Trocknen über Natriumsulfat und dem Entfernen des Lösungsmittels im Vakuum erhält man 25.2 g Propionsäure-2,3-dichlorphenylester. 25.2 g (115.2 mmol) Propionsäure-2,3-dichlorphenylester in 12 ml 1,2-Dichlorbenzol werden zu 15.4 g (115.2 mmol) Aluminiumtrichlorid in 12 ml 1,2-Dichlorbenzol getropft und die Mischung wird im Anschluss 5 Stunden lang bei 100°C gerührt. Man lässt abkühlen, verdünnt mit Dichlormethan und gießt vorsichtig auf eine Mischung von 2 M Salzsäure und Eis. Die Phasen werden getrennt, es wird mit Dichlormethan extrahiert, mit Wasser und gesättigter Natriumchlorid Lösung gewaschen und über Natriumsulfat getrocknet. Das Rohprodukt wird säulenchromatographisch an Kieselgel (Hexan / Ethylacetat 10-25%) gereinigt und man erhält 22.1g 1-(3,4-Dichlor-2-hydroxyphenyl)propan-1-on. 22.1 g (101 mmol) 1-(3,4-Dichlor-2-hydroxyphenyl)propan-1-on werden in 150 ml Aceton gelöst und es werden 25.9 g (187 mmol) Kaliumcarbonat und 11.4 ml (183 mmol) Methyliodid zugegeben. Die Mischung wird 16 Stunden unter Rückfluß gekocht und das Lösungsmittel im Anschluss zu einem großen Teil entfernt. Man gießt den Rückstand in gesättigte Natriumchlorid Lösung und extrahiert mit Diethylether. Es wird über Natriumsulfat getrocknet und nach dem Entfernen des Lösungsmittels im Vakuum erhält man 23.2 g 1-(3,4-Dichlor-2-methoxyphenyl)propan-1-on. 29 g (444 mmol) Zinkstaub und 0.69 g (2.5 mmol) Blei(II)-chlorid werden in 296 ml THF suspendiert und bei 0°C werden 27.8 ml (174 mmol) Dibrommethan zugegeben. Man rührt weitere 30 Minuten bei Raumtemperatur und tropft bei 0°C 49 ml (49 mmol) einer 1 M Titan(IV)-chlorid Lösung in Dichlormethan zu. Das Kühlbad wird entfernt und nach einer Stunde wird die Reaktionsmischung wieder auf 0°C gekühlt. 11.5 g (49 mmol) 1-(3,4-Dichlor-2-methoxyphenyl)propan-1-on in 65 ml THF zugetropft. Man rührt eine weitere Stunde bei Raumtemperatur. Es wird mit Diethylether verdünnt und das Reaktionsgemisch wird vorsichtig auf eine Mischung von 4 M Salzsäure und Eis gegeben wobei die Temperatur 5°C nicht übersteigt. Man trennt die Phasen, extrahiert mit Diethylether, wäscht mit Wasser und gesättigter Natriumchlorid Lösung, trocknet über Natriumsulfat und entfernt das Lösungsmittel. Das Rohprodukt wird säulenchromatographisch an Kieselgel (Hexan / Ethylacetat 0-30%) gereinigt und man erhält 5.8g 2,3-Dichtor-6-(1-methylenpropyl)anisol 1H-NMR (300 MHz, CDCl3); δ = 1.01 (t, 3H), 2.47 (q, 2H), 3.77 (s, 3H), 7.00 (d, 1H), 7.18 (d, 1H),

Zu 5.8 g (25 mmol) 2,3-Dichlor-6-(1-methylenpropyl)anisol, 6.6 ml (50 mmol) Ethyltrifluorpyruvat und 12 g Molekularsieb werden bei 0°C über 30 Minuten 652 mg (0.75 mmol) [Cu(R,R)-2,2-bis(4,5-dihydro-4-tert-butyloxazolin-2-yl)propan)(H₂O)₂]((SbF₆)₂, in 32 ml Dichlormethan getropft. Man rührt die Reaktionsmischung 16 Stunden bei 0°C und reinigt die Reaktionsmischung mittels Säulenchomatographie an Kieselgel (Hexan / Ethylacetat 10-20%). Man erhält 7.23 g (R)-4-(3,4-Dichlor-2-methoxyphenyl)-2-hydroxy-2-(trifluormethyl)-hex-4-ensäureethylester als E/Z Gemisch mit einem Enantiomerenüberschuß von größer 80%. 383 mg (0.37 mmol) (S)-(S)-(3,5-Me-4-MeOPh)₂PPhFc-CH(CH₃)-P(3,5-CF₃Ph)₂ und 130 mg (0.35 mmol) [Rh(nbd)₂]BF₄ werden unter Argon in 25 ml entgastem 2,2,2-Trifluorethanol gelöst und 10 Minuten gerührt. Die so hergestellte Katalysatörlösung und eine Lösung von 3.5 g (26 mmol) (2R, 4E/Z)-4-(3,4-Dichlor-2-methoxyphenyl)-2-hydroxy-2-(trifluormethyl)-hex-4-ensäureethytester in 115 ml entgastem 2,2,2-Trifluorethanol werden unter Argon in einen Stahlautoklaven überführt und über 20 Stunden bei 80°C einem Wasserstoffdruck von 80 bar ausgesetzt. Nach dem Abkühlen wird die Reaktionsmischung eingeengt und mittels Säulenchomatographie an Kieselgel (Hexan / Ethylacetat 10-30%) aufgereinigt. Man erhält 3.0 g 4-(3,4-Dichlor-2-methoxyphenyl)-2-hydroxy-2-(trifluormethyl)-hexansäureethylester. 1.0 g (2.5 mmol) 4-(3,4-Dichlor-2-methoxyphenyl)-2-hydroxy-2-(trifluormethyl)-hexansäureethylester werden in 26 ml Diethylether auf -20°C gekühlt und über 10 Minuten werden portionsweise 188 mg (5.0 mmol) Lithiumaluminiumhydrid fest zugegeben. Man rührt eine Stunde wobei sich die Reaktionsmischung auf -5°C erwärmt, man gibt 2 ml Ethylacetat zu und gießt in gesättigte Ammoniumchlorid Lösung und Eis. Die Phasen werden getrennt und es wird mehfach mit Diethylether extrahiert. Man wäscht mit gesättigter Natriumchlorid Lösung, trocknet über Natriumsulfat, entfernt das Lösungsmittel im Vakuum und erhält so nach Säulenchomatographie an Kieselgel (Hexan / Ethylacetat 0-30%) 370 mg (2R,4R)-4-(3,4-Dichlor-2-methoxyphenyl)-2-hydroxy-2-(trifluormethyl)hexanal.
¹H-NMR (300 MHz, CDCl₃); δ = 0.80 (t, 3H), 1.55 - 1.73 (m, 2H), 2.32 (dd, 1H), 2.52 (dd, 1H), 3.07 (m,1H), 3.91 (s, 3H), 7.00 (d, 1H), 7.14 (d, 1H), 9.04 (s, 1H).

Im Gemisch mit (2*R*,4*R*)-4-(3/4-Chlor-2-methoxyphenyl)-2-hydroxy-2-(trifluormethyl)hexanal.

300 mg (0.88 mmol) (2*R*, 4*R*)-4-(3-Chlor-4-fluor-2-methoxyphenyl)-2-hydroxy-2-(trifluormethyl)hexanal und 140 mg (0.88 mmol) 5-Aminochinolin-2(1H)-on werden in 19 ml Toluol gelöst und 0.55 ml (1.75 mmol) Titantert-butylat und 0.1 ml Essigsäure werden zugegeben. Man erhitzt die Reaktionsmischung 2 Stunden auf 100°C, gießt nach dem Abkühlen in Wasser und rührt heftig. Die Suspension wird durch Celite filtriert wobei gründlich mit Ethylacetat nachgewaschen wird. Die Phasen des Filtrats werden getrennt und es wird nochmals mit Ethylacetat extrahiert. Man wäscht mit gesättigter Natriumchlorid Lösung, trocknet über Natriumsulfat, entfernt das Lösungsmittel im Vakuum und erhält 539 mg (5-{[(2R,4R)-4-(3-Chlor-4-fluor-2-methoxyphenyl)-2-hydroxy-2-(trifluormethyl)hexyliden]amino}chirnolin-2(1H)-on als Rohprodukt Das Imin wird in 44 ml CH₂Cl₂ gelöst und auf -30°C gekühlt. 8.9 ml (8.9 mmol) einer 1 M BBr₃ Lösung in Dichlormethan werden über 15 min langsam zugetropft und man läßt über 22 Stunden auf Raumtemperatur erwärmen. Die Reaktionslösung wird auf eine Mischung aus gesättigter NaHCO₃ Lösung und Eis gegossen. Man extrahiert mehrfach mit Ethylacetat, wäscht mit gesättigter NaCl Lösung und trocknet über Na₂SO₄. Säutenchromatographische Reinigung an Kieselgel (Hexan / Ethylacetaat 0-100%) liefern 134 mg der Titelverbindung.
¹H-NMR (300 MHz, CD₃OD); δ = 0.92 (t, 3H), 1.70 (ddq, 1H), 1.19 (ddq, 1H), 1.99 (dd, 1H), 2.36 (dd, 1H), 3.32 (m, 1H), 4.97 (s, 1H), 6.44 (d, 1H), 6.50 (d, 1H), 6.63 (d, 1H), 6.68 (d, 1H), 7.33 (t, 1H), 8.18 (d, 1H).

### Beispiel 196

### 5-{[(5S,6R,8R)-3-Chlor-8-ethyl-1,6-dihydroxy-2-6-(trifluormethyl)-5,6,7,8-tetrahydronaphthalin-5-yl]amino}-7-fluorchinolin-2(1H)-on

Wird als zusätzliches Produkt neben Beispiel 195 isoliert.
¹H-NMR (300 MHz, CD₃OD); δ = 0.91 (t, 3H), 1.68 (ddq, 1H), 1.95 (d, 1H), 1.96 (m, 1H), 2.35 (dd, 1H), 3.24 (m, 1H), 4.97 (d, 1H), 6.10 (d, 1H), 6.25 (d, 1H), 6.39 (d, 1H), 6.43 (d, 1H), 6.67 (s, 1H), 6.70 (s, 1H), 8.14 (d, 1H).

### Beispiel 197 (5S,6R,8R)-2,3-Dichlor-8-ethyl-5-[(7-fluor-2-methylchinazolin-5-yl)amino]-6-(trifluormethyl)-5,6,7,8-tetrahydronaphthalin-1,6-diol

Analog Beispiel 195 werden 300 mg 0.93 mmol) (*2R*,*4R*)-4-(3,4-Dichlor-2-methoxyphenyl)-2-hydroxy-2-(trifluormethyl)hexanal, 183 mg (1.03 mmol) 5-Amino-7-fluor-2-methylchinazolin und 0.58 ml (1.86 mmol) Titan*tert*-butylat zu 5-{[(*2R*,*4R*)-4-(4-Fluor-2-methoxy-3-methylphenyl)-1-[(7-fluor-2-methylchinazolin-5-yl)imino]-2-(trifluormethyl)hexan-2-ol umgesetzt. 460 mg rohes Imin werden analog Beispiel 30 bei-30°C mit 7.6 mL (7.6 mmol) 1 M Bortribromid-Lösung zum gewünschten Produkt zyklisiert. Säulenchromatographie an Kieselgel (Hexan / Ethylacetat 0-65%) liefert 135 mg gewünschtes Produkt.
¹H-NMR (300 MHz, CD₃OD); δ = 0.94 (t, 3H), 1.71 (ddq, 1H), 1.99 (dd, 1H), 2.00 (m, 1H), 2.41 (dd, 1H), 3.32 (m, 1H), 5.15 (s, 1H), 6.64 (d, 1H), 6.79 (d, 1H), 6.89 (s, 1H), 9.50 (s, 1H).

### Beispiel 198

### (5S,6R,8R)-2-Chlor-8-ethyl-5-[(7-fluor-2-methylchinazolin-5-yl)amino]-6-(trifluormethyl)-5,6,7,8-tetrahydronaphthalin-1,6-diol

Wird als zusätzliches Produkt neben Beispiel 197 isoliert.
¹H-NMR (300 MHz, CD₃OD); δ = 0.93 (t, 3H), 1.72 (ddq, 1H), 2.00 (dd, 1H), 2.01 (m, 1H), 2.40 (dd, 1H), 3.36 (m, 1H), 5.14 (s, 1H), 6.60 (d, 1H), 6.74 (d, 1H), 6.75 (d, 1H), 7.11 (d; 1H), 9.49 (s, 1H).

### Beispiel 199

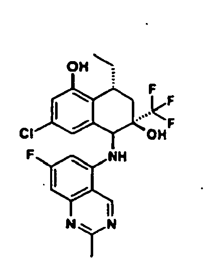

### (5S,6R,8R)-3-Chlor-8-ethyl-5-f(7-fluor-2-methylchinazolin-5-yl)amino] -6-(trifluormethyl)-5,6,7,8-tetrahydronaphthalin-1,6-diol

Wird als zusätzliches Produkt neben Beispiel 197 isoliert.
¹H-NMR (300 MHz, CD₃OD); δ = 0.92 (t, 3H), 1.69 (ddq, 1H), 1.97 (dd, 1H), 1.99 (m, 1H), 2.38 (dd, 1H), 3.25(m, 1H), 5.13(s, 1H), 6:61(d, 1H), 6.69 (s, 1H), 6.71 (s, 1H), 6.78 (d, 1H), 9.50 (s, 1H).

## Patentansprüche

1. Verbindung der allgemeinen Formel (Ia), 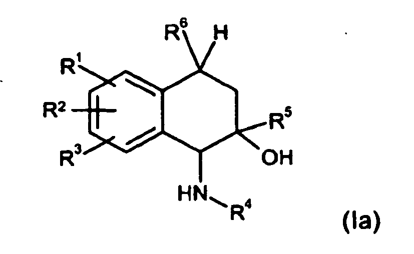 worin
I) die Reste R¹, R² und R³ unabhängig voneinander ausgewählt sind aus
-OH, O-CH₃, Cl, F, H,
II) der Rest R⁴ ausgewählt ist aus
2-Methylchinolin-5-yl
2-Methylchinazolin-5-yl
2-Ethylchinazolin-5-yl
7-Fluor-2-methylchinazolin-5-yl,
8-Fluor-2-methylchinazolin-5-yl,
7,8-Difluor-2-methylchinazolin-5-yl,
Chinolin-2(1H)-on-5-yl,
7-Fluorchinolin-2(1H)-on-5-yl,
8-Fluorchinolin-2(1H)-on-5-yl,
Isochromen-1-on-5-yl
2-Methyl-phthalazin-1-on-5-yl
Isochinolin-2(1*H*)-on-5-yl,
III) der Rest R⁵ für -CF₃ steht
und
IV) der Rest R⁶ ausgewählt ist aus -CH₃, -CH₂-CH₃, -(CH₂)₂-CH₃, oder -CH=CH₂.
sowie deren Stereoisomere oder ihre Salze mit physiologisch verträglichen Anionen,
**dadurch gekennzeichnet, dass** die Verbindungen in der 1α, 2α, 4β-Konfiguration des 1-Amino-1,2,3,4-Tetrahydronaphthalin-2-ol Grundkörpers vorliegen oder dass die Verbindungen in der 5α,6α,8β-Konfiguration des 5,6,7,8-Tetrahydronaphtalin-1,6-diol Grundkörpers vorliegen.

2. Verbindung gemäß Anspruch 1, **dadurch gekennzeichnet, dass** die Verbindungen als absolute Konfiguration
(1S, 2R, 4R) oder (1S, 2R, 4S) am 1-Amino-1,2,3,4-tetrahydronaphthalin-2-ol-Grundkörper
oder
(5S, 6R, 8R) oder (5S, 6R, 8S) am 5,6,7,8-Tetrahydronaphtalin-1,6-diol Grundkörpers aufweisen.

3. Verbindung gemäß Anspruch 1 oder 2, **ausgewählt aus**
(*5*α,*6*α,*8*β)-2-Fluor-8-methyl-5-[(2-methylchinolin-5-yl)amino]-6-(trifluormethyl)-5,6,7,8-tetrahydronaphthalin-1,6-diol
(+)-(*5*α,*6*α,*8*β)-2-Fluor-5-[(7-fluor-2-methylchinazolin-5-yl)amino]-8-methyl-6-(trifluormethyl)-5,6,7,8-tetrahydronaphthalin-1,6-diol
(-)-(*5*α,*6*α,*8*β)-2-Fluor-5-[(7-fluor-2-methylchinazolin-5-yl)amino]-8-methyl-6-(trifluormethyl)-5,6,7,8-tetrahydronaphthalin-1,6-diol
(*5*α,*6*α,*8*β)-2-Fluor-5-[(8-fluor-2-methylchinazolin-5-yl)amino]8-methyl-6-(trifluormethyl)-5,6,7,8-tetrahydronaphthalin-1,6-diol
(*5*α,*6*α,*8*β)-5-[(7,8-Difluor-2-methylchinazolin-5-yl)amino]-2-fluor-8-methyl-6-(trifluormethyl)-5,6,7,8-tetrahydronaphthalin-1,6-diol
(*5*α,*6*α,*8*β)-2-Fluor-8-methyl-5-[-2-methylchinazolin-5-yl)amino]-6-(trifluormethyl)-5,6,7,8-tetrahydronaphthalin-1,6-diol
5-{[(*5*α,*6*α,*8*β)-1,6-Dihydroxy-2-fluor-8-methyl-6-(trifluormethyl)-5,6,7,8-tetrahydronaphthalin-5-yl]amino}chinolin-2(1*H*)-on
8-Fluor-5-{[(*1*α,*2*α,*4*β)-6-fluor-2-hydroxy-5-methoxy-4-methyl-2-(trifluormethyl)-1,2,3,4-tetrahydronaphthalin-1-yl]amino}-chinolin-2(1H)-on
(+)-5-{[(*1*α,*2*α,*4*β)6-fluor-2,5-dihydroxy-4-methyl-2-(trifluormethyl)-1,2,3,4-tetrahydronaphthalin-1-yl]amino}-8-fluorchinolin-2(1H)-on
(-)-5-{[(*1*α,*2*α,*4*β)-6-fluor-2,5-dihydroxy-4-methyl-2-(trifluormethyl)-1,2,3,4-tetrahydronaphthalin-1-yl]amino}-8-fluorchinolin-2(1H)-on
5-{[(*1*α,*2*α,*4*β)-6-fluor-2,5-dihydroxy-4-methyl-2-(trifluormethyl)-1,2,3,4-tetrahydronaphthalin-1-yl]amino}-2-methylphthalazin-1-on
(*5*α,*6*α,*8*β)-3-Chlor-2-fluor-8-methyl-5-[(2-methylchinazolin-5-yl)amino]--6-(trifluormethyl)-5,6,7,8-tetrahydronaphthalin-1,6-diol
(*5*α,*6*α,*8*β)-3-Chlor-2-fluor-5-[(7-fluor-2-methylchinazolin-5-yl)amino]-1-methoxy-8-methyl-6-(trifluormethyl)-5,6,7,8-tetrahydronaphthalin-6-ol
(*5*a,*6*α,*8*β}-3-Chlor-2-fluor-5-[(7-fluor-2-methylchinazolin-5-yl)amino]-8-methyl-6-(trifluormethyl)-5,6,1,8-tetrahydronaphthalin-1,6-diol
(*5*α,*6*α,*8*β)3-Chlor-2-fluor-5-[(8-fluor-2-methylchinazolin-5-yl)amino] 1-methoxy-8-methyl-6-(trifluormethyl)-5,6,7,8-tetrahydronaphthalin-6-ol
(*5*α,*6*α,*8*β)-3-Chlor-2-fluor-5-[(8-fluor-2-methylchinazolin-5-yl)amino] 8-methyl-6-(trifluormethyl)-5,6,7,8-tetrahydronaphthalin-1,6-diol
(5α,6α,8β)-3-Chlor-5-[(7,8-difluor-2-methylchinazolin-5-yl)amino]-2-fluor-8-methyl-6-(trifluormethyl)-5,6,7,8-tetrahydronaphthalin-1,6-diol
(5α,6α,8β)3-Chlor-2-fluor-8-methyl-5-[-2-methylchinolin-5-yl)amino]-6-(trifluormethyl)-5,6,7,8-tetrahydronaphthalin-1,6-diol
(+)-5-{[(*1*α,*2*α,*4*β)-7-Chlor-2,5-dihydroxy-6-fluor-4-methyl-2-(trifluormethyl)-1,2,3,4-tetrahydronaphthalin-1-yl]-amino}-chinolin-2(1*H*)-on
(-)-5-{[(*1*α,*2*α *4*β)-7-Chlor-2,5-dihydroxy-6-fluor-4-methyl-2-(trifluormethyl)-1,2,3,4-tetrahydronaphthalin-1-yl]-amino}-chlnolin-2(1*H*)-on
5-{[(*1*α,*2*α,*4*β)-7-Chlor-2,5-dihydroxy-6-fluor-4-methyl-2-(trifluormethyl)-1,2,3,4-tetrahydronaphthalin-1-yl]-amino}-8-fluorchinolin-2(1*H*)-on
(*5*α,*6*α,*8*β)-8-Methyl-5-[-2-methylchinolin-5-yl)amino]-6-(trifluormethyl)-5,6,7,8-tetrahydronaphthalin-1,6-diol
(*5*α,*6*α,*8*β)-8-Ethyl-5-[2-methylchinazolin-5-yl)amino]-6-(trifluormethyl)-5,6,7,8-tetrahydronaphthalin-1,6-diol
(+)*-*(*5α*,*6α*,*8*β)-8-Ethyl-2-fluor-5-[(2-methylchinazolin-5-yl)amino]-6-(trifluormethyl)-5,6,7,8-tetrahydronaphthalin-1,6-diol
(-)-(*5*α,*6*α*,8*β)-8-Ethyl-2-fluor-5-[(2-methylchinazolin-5-yl)amino]-6-(trifluormethyl)-5,6,7,8-tetrahydronaphthalin-1,6-diol
(*5S*,*6R*,*8R*)-8-Ethyl-2-fluor-5-[(7-fluor-2-methylchinazolin-5-yl)amino]-6-(trifluoromethyl)-5,6,7,8-tetrahydronaphthalen-1,6-diol
(+)-(*5*α,*6*α,*8*β)-8-Ethyl-2-fluor-5-[(8-fluor-2-methylchinazolin-5-yl)amino]-6-(trifluormethyl)-5,6,7,8-tetrahydronaphthalin-1,6-diol
(-)-(*5*α,*6*α,*8*β)-8-Ethyl-2-fluor-5-[(8-fluor-2-methylchinazolin-5-yl)amino]-6-(trifluormethyl)-5,6,7,8-tetrahydronaphthalin-1,6-diol
(*5*α,*6*α,*8*β)-5-[(7,8-Difluor-2-methylchinazolin-5-yl)amino]-8-ethyl-2-fluor-6-(trifluormethyl)-5,6,7,8-tetrahydronaphthalin-1,6-diol
5-{[*1*α,*2*α,*4*β)-4-Ethyl-6-fluor-2,5-dihydroxy-2-(trifluormethyl)1,2,3,4-tetrahydronaphthalin-1-yl]amino}-chinolin-2(1*H*)-on
5-{[(*1*α,*2*α*,4*β)-4-Ethyl-6-fluor-2-hydroxy-5-methoxy-2-(trifluormethyl)-1,2,3,4-tetrahydronaphthalin-1-yl]amino}-8-fluorchinolin-2(1*H*)-on
(+)-5-{[(*1*α,*2*α,*4*β)-4-Ethyl-6-fluor-2,5-dihydroxy-2-(trifluormethyl)1,2,3,4-tetrahydronaphthalin-1-yl]amino}-8-fluorchinolin-2(1*H*)-on
(-)-5-{[(*1*α,*2*α,*4*β)-4-Ethyl-6-fluor-2,5-dihydroxy-2-(trifluormethyl)-1,2,3,4-tetrahydronaphthalin-1-yl]amino}-8-fluorchinolin-2(1*H*)-on
5-{[(*1*α,*2*α,*4*β)4-Ethyl-6-fluor-2,5-dihydroxy-2-(trifluormethyl)-1,2,3,4-tetrahydronaphthalin-1-yl]amino}-2-methylphthalazin-1-on
5-{[(*1*α,*2*α,*4*β)-4-Ethyl-6-fluor-2,5-dihydroxy-2-(trifluormethyl)-1,2,3,4-tetrahydronaphthalin-1-yl]amino}-isochinolin-(2H)-on
5-{[(*1*α,*2*α,*4*β)-4-Ethyl-6-fluor-2,5-dihydroxy-2-(trifluormethyl)-1,2,3,4-tetrahydronaphthalin-1-yl]amino}isochromen-1-on
(*5*α,*6*α,*8*β)-5-[(2-Ethylchinazolin-5-yl)amino]-2-fluor-8-propyl-6-(trifluormethyl)-5,6,7,8-tetrahydronaphthalin-1,6-diol
(*5*S,*6*R,*8*R)-8-Ethyl-2,3-difluor-5-[(2-methylchinazolin-5-yl)amino]-6-(trifluormethyl)-5,6,7,8-tetrahydronaphthalin-1,6-diol
(+)-(*5*α,*6α,8*β)-8-Ethyl-2,3-difluor-5-[(7-fluor-2-methylchinazolin-5-yl)amino]-6-(trifluormethyl)-5,6,7,8-tetrahydronaphthalin-1,6-diol
(-)-(5α,6α,8β)-8-Ethyl-2,3-difluor-5-[(7-fluor-2-methylchinazolin-5-yl)amino]-6-(trifluormethyl)-5,6,7,8-tetrahydronaphthalin-1,6-diol
(*5S*,*6R*,*8R*)-8-Ethyl-2,3-difluor-5-[(8-fluor-2-methylchinazolin-5-yl)amino]-6-(trifluormethyl)-5,6,7,8-tetrahydronaphthalin-1,6-diol
(*5S,6R,*8*R*)-8-Ethyl-2,3-difluor-5-[(7,8-difluor-2-methylchinazolin-5-yl)amino]-6-(trifluormethyl)-5,6,7,8-tetrahydronaphthalin-1,6-diol
(+)-5-{[(*1*α,*2*α,*4β*)-4-ethyl-6,7-difluor-2,5-dihydroxy-2-(trifluormethyl)-1,2,3,4-tetrahydronaphthalin-1-yl]amino}chinolin-2(1*H*)-on
(-)-5-{[(*1*α*,2*α,*4*β)-4-ethyl-6,7-difluor-2,5-dihydroxy-2-(trifluormethyl)-1,2,3,4-tetrahydronaphthalin-1-yl]amino}chinolin-2(1*H*)-on
(5α,6α,8β)-8-Ethyl-2,3-difluor-5-[(2-methylchinolin-5-yl)amino]-6-(trifluormethyl)-5,6,7,8-tetrahydronaphthalin-1,6-diol
5-{[(*1*α,*2*α,*4*β)-4-ethyl-6,7-difluor-2,5-dihydroxy-2-(trifluormethyl)-1,2,3,4-tetrahydronaphthalin-1-yl]amino}phthalazin-1-on
(5α,6α,8β)-2-fluor-5-[(2-methylchinazolin-5-yl)amino]-8-prop-1-yl-6-(trifluormethyl)-5,6,7,8-tetrahydronaphthalin-1,6-diol
(5α,6α,8β)-2-Fluor-5-[(7-fluoro-2-methylquinazolin-5-yl)amino]-8-prop-1-yl-6-(trifluoromethyl)-5,6,7,8-tetrahydronaphthalin-1,6-diol
5-{[(*1*α,*2*α,*4*β)4-Ethyl-6,7-difluor-2,5-dihydroxy-2-(trifluormethyl)-1,2,3,4-tetrahydronaphthalin-1-yl]amino}chinolin-2(1*H*)-on
5-{[(*1*α,*2*α,*4*β)-4-Ethyl-6,7-difluor-2,5-dihydroxy-2-(trifluormethyl)-1,2,3,4-tetrahydronaphthalin-1-yl]amino}isochinolin-1(2*H*)-on
5-{[(*1S*,*2R*,*4S*)-6-Chlor-2,5-dihydroxy-4-methyl-2-(trifluormethyl)-1,2,3,4-tetrahydronaphthalin-1-yl]amino}-1*H*-chinolin-2-on
5-{[(*1S*,*2R*,*4R*)-6-Chlor-2,5-dihydroxy-4-methyl-2-(trifluormethyl)-1,2,3,4-tetrahydronaphthalin-1-yl]amino}-1*H*-chinolin-2-on
5-{[(*1S*,*2R*,*4S*)-6-Chlor-2,5-dihydroxy-4-methyl-2-(trifluormethyl)-1,2,3,4-tetrahydronaphthalin-1-yl]amino}-8-fluor-1*H*-chinolin-2-on
5-{[(*1S*,*2R*,*4R*)-6-Chlor-2,5-dihydroxy-4-methyl-2-(trifluormethyl)-1,2,3,4-tetrahydronaphthalin-1-yl]amino}-8-fluor-1*H*-chinolin-2-on
5-{[(*1S*,*2R*,*4S*)-6-Chlor-2,5-dihydroxy-4-methyl-2-(trifluormethyl)-1,2,3,4-tetrahydronaphthalin-1-yl]amino}-2*H*-chinolin-1-on
5-{[(*1S*,*2R*,*4R*)-6-Chlor-2,5-dihydroxy-4-methyl-2-(trifluormethyl)-1,2,3,4-tetrahydronaphthalin-1-yl]amino}2H-chinolin-1-on
5-{[(*1S*,*2R*,*4S*)-6-Chlor-2,5-dihydroxy-4-methyl-2-(trifluormethyl)-1,2,3,4-tetrahydronaphthalin-1-yl]amino}-isochromen-1-on
5-{[(*1*S,*2R*,*4R*)-6-Chlor-2,5-dihydroxy-4-methyl-2-(trifluormethyl)1,2,3,4-tetrahydronaphthalin-1-yl]amino}-isochromen-1-on
4-{[(*1S*,*2R*,*4S*)-6-Chlor-2,5-dihydroxy-4-methyl-2-(trifluormethyl)-1,2,3,4-tetrahydronaphthalin-1-yl]amino}-1,3-dihydroindol-2-on
4-{[(*1S*,*2R*,*4R*)-6-Chlor-2,5-dihydroxy-4-methyl-2-(trifluormethyl)1,2,3,4-tetrahydronaphthalin-1-yl]amino}-1,3-dihydroindol-2-on
5-([(*1S*,*2R*,*4S*)-Chlor-2,5-dihydroxy-4-ethyl-2-(trifluormethyl)-1,2,3,4-tetrahydronaphthalin-1-yl]amino}-1H-chinolin-2-on
5-{[(*1*α,*2*α,*4*β)-6-Chlor-2,5-dihydroxy-4-ethyl-2-(trifluormethyl)-1,2,3,4-tetrahydronaphthalin-1-yl]amino}-1H-chinolin-2-on
5-{[(*1S*,*2R*,*4S*)-6-Chlor-2,5-dihydroxy-4-ethyl-2-(trifluormethyl)1,2,3,4-tetrahydronaphthalin-1-yl]amino}-8-fluor-1H-chinolin-2-on
5-{[(*1S*,*2R*,*4R*)-6-Chlor-4-ethyl-2,5-dihydroxy-2-(trifluormethyl)-1,2,3,4-tetrahydronaphthalin-1-yl]amino}-8-fluor-1H-chinolin-2-on
5-{[(*1S*,*2R*,*4S*)-6-Chlor-2,5-dihydroxy-4-ethyl-2-(trifluormethyl)-1,2,3,4-tetrahydronaphthalin-1-yl]amino}-2*H*-chinolin-1-on
5-{[(*1S*,*2R*,*4R*)-6-Chlor-4-ethyl-2,5-dihydroxy-2-(trifluormethyl)-1,2,3,4-tetrahydronaphthalin-1-yl]amino}-2*H*-chinolin-1-on
5-{[(*1S*,*2R*,*4S*)-6-Chlor-2,5-dihydroxy-4-ethyl-2-(trifluormethyl)-1,2,3,4-tetrahydronaphthalin-1-yl]amino}-isochromen-1-on
5-{[*(1S*,*2R*,*4R*)-6-Chlor-4-ethyl-2,5-dihydroxy-4-2-(trifluormethyl)-1,2,3,4-tetrahydronaphthalin-1-yl]amino}-isochromen-1-on
4-{[*(1S*,*2R*,*4S)*-6-Chlor-2,5-dihydroxy-4-ethyl-2-(trifluormethyl)-1,2,3,4-tetrahydronaphthalin-1-yl]amino}-1,3-dihydroindol-2-on
4-{[(*1S*,*2R*,*4R*)-6-Chlor-2,5-dihydroxy-4-ethyl-2-(trifluormethyl)1,2,3,4-tetrahydronaphthalin-1-yl]amino}-1,3-dihydroindol-2-on
*5*-*{[(1S*,*2R,4S)*-6-Chlor-2,5-dihydroxy-4-ethyl-2-(trifluormethyl)-1,2,3,4-tetrahydronaphthalin-1-yl]amino}-7-fluor-1H-chinolin-2-on
5-{[(*1S*,*2R*,*4R*)-6-Chlor-4-ethyl-2,5-dihydroxy-2-(trifluormethyl)-1,2,3,4-tetrahydronaphthalin-1-yl]amino}-7-fluor-1H-chinolin-2-on
*5-{[(1S*,*2R,4S)*-6-Chlor-2,5-dihydroxy-4-methyl-2-(trifluormethyl)-1,2,3,4-tetrahydronaphthalin-1-yl]amino}-7-fluor-1*H*-chinolin-2-on
5-{[(*1S*,*2R*,*4R*)-6-Chlor-2,5-dihydroxy-4-methyl-2-(trifluormethyl)-1,2,3,4-tetrahydronaphthalin-1-yl]amino}-7-fluor-1*H*-chinolin-2-on
(*5*α,*6*α,*8*β)-2-Chlor-8-ethyl-5-[(indazol-5-yl)amino]-6-(trifluormethyl)-5,6,7,8-tetrahydronaphthalin-1,6-diol
6-{[(*1*α,*2*α,*4*β)-6-Chlor-2,5-dihydroxy-4-ethyl-2-(trifluormethyl)1,2,3,4-tetrahydronaphthalin-1-yl]amino}-4-methylbenzo[d][1,2]oxazin-1-on
5-{[(*1*α,*2*α,*4*β)-7-Isopropyl-2,5-dihydroxy-4-ethyl-2-(trifluormethyl)-1,2,3,4-tetrahydronaphthalin-1-yl]amino}-1*H*-chinolin-2-on
(*5*α,*6*α,*8*β)-8-Ethyl-3-isopropyl-5-[2-methylchinazolin-5-ylamino]-6-(trifluormethyl)-5,6,7,8-tetrahydronaphthalin-1,6-diol
5-{[(*1*α,*2*α,*4*β)-7-Isopropyl-2,5-dihydroxy-4-ethyl-2-(trifluormethyl)-1,2,3,4-tetrahydronaphthalin-1-yl]amino}-2*H*-chinolin-1-on
4-{[(*1*α,*2*α,*4*β)-7-Isopropyl-2,5-dihydroxy-4-ethyl-2-(trifluormethyl)-1,2,3,4-tetrahydronaphthalin-1-yl]amino}-1,3-dihydroindol-2-on
5-{[(*1*α,*2*α,*4*β)-7-Isopropyl-2,5-dihydroxy-4-ethyl-2-(trifluormethyl)-1,2,3,4-tetrahydronaphthalin-1-yl]amino}-isochromen-1-on
5-{[(*1*α,*2*α,*4*β-7-Isopropyl-2,5-dihydroxy-4-ethyl-2-(trifluormethyl)1,2,3,4-tetrahydronaphthalin-1-yl]amino}-8-fluor-1*H*-chinolin-2-on
5-{[(*1*α,*2*α,*4*β)-7-Isopropyl-2,5-dihydroxy-4-ethyl-2-(trifluormethyl)-1,2,3,4-tetrahydronaphthalin-1-yl]amino}-8-fluor-1*H*-chinolin-2-on
5-{[(*1*α,*2*α,*4*β)-7-Chlor-2,5-dihydroxy-4-ethyl-6-methyl-2-(trifluormethyl)-1,2,3,4-tetrahydronaphthalin-1-yl]amino}-1*H*-chinolin-2-on
5-{[(*1S*,*2R*,*4R*)-7-Chlor-2,5-dihydroxy-6-methyl-4-ethyl-2-(trifluormethyl)-1,2,3,4-tetrahydronaphthalin-1-yl]amino}-2*H*-chinolin-1-on
4-{[(*1*α,*2*α,*4*β)-7-Chlor-2,5-dihydroxy-6-methyl-4-ethyl-2-(trifluormethyl)1,2,3,4-tetrahydronaphthalin-1-yl]amino}-1,3-dihydroindol-2-on
5-{[(*1*α,*2*α,*4*β)-7-Chlor-2,5-dihydroxy-4-ethyl-6-methyl-2-(trifluormethyl)1,2,3,4-tetrahydronaphthalin-1-yl]amino}-8-fluor-1*H*-chinolin-2-on
5-{[(*1S*,*2R*,*4R*)-7-Chlor-2,5-dihydroxy-4-ethyl-6-methyl-2-(trifluormethyl)-1,2,3,4-tetrahydronaphthalin-1-yl]amino}-8-fluor-1*H*-chinolin-2-on
(*5*α,*6*α,*8*β)-3-Chlor-8-ethyl-2-methyl-5-[2-methylchinazolin-5-ylamino]-6-(trifluormethyl)-5,6,7,8-tetrahydronaphthalin-1,6-diol
5-*{[(1S*,*2R*,*4S)-7*-Chlor-2,5-dihydroxy-4-ethyl-6-methyl-2-(trifluormethyl)-1,2,3,4-tetrahydronaphthalin-1-yl]amino}-7-fluor-1*H*-chinolin-2-on
5-{[(*1*α,*2*α,*4*β)-7-Chlor-2,5-dihydroxy-4-ethyl-6-methyl-2-(trifluormethyl)1,2,3,4-tetrahydronaphthalin-1-yl]amino}7-fluor-1*H*-chinolin-2-on
5-{[(*1S*,*2R*,*4R*)-7-Chlor-2,5-dihydroxy-4-ethyl-6-methyl-2-(trifluormethyl)-1,2,3,4-tetrahydronaphthalin-1-yl]amino}-7-fluor-1*H*-chinolin-2-on
(5S,6R,8R)-8-Ethyl-2,3-difluor-5-[(2-methylchinazolin-5-yl)amino]-6-(trifluormethyl)-5,6,7,8-tetrahydronaphthalin-1,6-diol
5-{[(1S,2R,4R)-4-Ethyl-7-fluor-2,5-dihydroxy-6-methyl-2-(trifluormethyl)-1,2,3,4-tetrahydronaphthalin-1-yl]amino}chinolin-2(1H)-on
5-{[(1S,2R,4R)-4-Ethyl-7-fluor-2,5-dihydroxy-6-methyl-2-(trifluormethyl)-1,2,3,4-tetrahydronaphthalin-1-yl]amino}-7-fluorchinolin-2(1H)-on
5-{[(1S,2R,4R)-4-Ethyl-7-fluor-2,5-dihydroxy-6-methyl-2-(trifluormethyl)1,2,3,4-tetrahydronaphthalin-1-yl]amino}-8-fluorchinolin-2(1H)-on
5-{[(1S,2R,4R)-4-Ethyl-7-fluor-2,5-dihydroxy-6-methyl-2-(trifluormethyl)1,2,3,4-tetrahydronaphthalin-1-yl]amino}isochinolin-1 (2H)-on
(5S,6R,8R)-8-Ethyl-3-fluor-5-[(7-fluor-2-methylchinazolin-5-yl)amino]-2-methyl-6-(trifluormethyl)-5,6,7,8-tetrahydronaphthalin-1,6-diol
(5S,6R,8R)-8-Ethyl-3-fluor-5-[(8-fluor-2-methylchinazolin-5-yl)amino]-2-methyl-6-(trifluormethyl)-5,6,7,8-tetrahydronaphthalin-1,6-diol
5-{[(1S,2R,4S)-4,6-Diethyl-7-fluor-2,5-dihydroxy-2-(trifluormethyl)-1,2,3,4-tetrahydronaphthalin-1-yl]amino}chinolin-2(1H)-on
5-{[(1S,2R,4R)-4,6-Diethyl-7-fluor-2,5-dihydroxy-2-(trifluormethyl)-1,2,3,4-tetrahydronaphthatin-1-yl]amino}chinolin-2(1H)-on
5-{[(1S,2R,4S)-4,6-Diethyl-7-fluor-2,5-dihydroxy-2-(trifluormethyl)-1,2,3,4-tetrahydronaphthalin-1-yl]amino}-7-fluorchinolin-2(1H)-on
5-{[(1S,2R,4R)-4,6-Diethyl-7-fluor-2,5-dihydroxy-2-(trifluormethyl}-1,2,3,4-tetrahydronaphthalin-1-yllamino}-7-fluorchinolin-2(1H)-on
5-{[(1S,2R,4S)-4,6-Diethyl-2,5-dihydroxy-2-(trifluormethyl)-1,2,3,4-tetrahydronaphthalin-1-yl]amino}-1H-chinolin-2-on
5-{[(1S,2R,4R)-4,6-Diethyl-2,5-dihydroxy-2-(trifluormethyl)-1,2,3,4-tetrahydronaphthalin-1-yl]amino}-1H-chinolin-2-on
5-{[(*1S,2R,4S*)-4,6-Diethyl-2,5-dihydroxy-2-(trifluormethyl)1,2,3,4-tetrahydronaphthalin-1-yl]amino}-8-fluor-1*H*-chinolin-2-on
5-{[(*1S,2R,4R*)-4,6-Diethyl-2,5-dihydroxy-2-(trifluormethyl)-1,2,3,4-tetrahydronaphthalin-1-yl]amino}-8-fluor-1H-chinolin-2-on
(*5S*,*6R*,*8S*)-2,8-Diethyl-5-[2-methylchinazolin-5-ylamino]-6-(trifluormethy)-5,6,7,8-tetrahydronaphthalin-1,6-diol
5-{[(*1*α,*2*α,*4*β)-7-Chlor-2,5-dihydroxy-4-ethyl-2-(trifluormethyl)-1,2,3,4-tetrahydronaphthalin-1-yl]amino}-1H-chinolin-2-on
5-{[(*1*α,*2*α,*4*β)-7-Chlor-2,5-dihydroxy-4-ethyl-2-(trifluormethyl)-1,2,3,4-tetrahydronaphthalin-1-yl]amino)-8-fluor-1*H*-chinolin-2-on
5-{[(*1S*,*2R*,*4R*)-6-Chlor-4-ethyl-2,5-dihydroxy-7-fluor-2-(trifluormethyl)1,2,3,4-tetrahydronaphthalin-1-yl]amino}chinolin-2(1*H*)-on
5-{[(*1S*,*2R*,*4R*)6-Chlor-4-ethyl-2,5-dihydroxy-7-fluor-2-(trifluormethyl)1,2,3,4-tetrahydronaphthalin-1-yl]amino)-7-fluorchinolin-2(1*H*)-on
5-{[(*1S*,*2R*,*4R*)-6-Chlor-4-ethyl-2,5-dihydroxy-7-fluor-2-(trifluormethyl)1,2,3,4-tetrahydronaphthalin-1-yl]amino}-8-fluorchinolin-2(1*H*)-on
(*5S*,*6R*,*8R*)-2-Chlor-8-ethyl-3-fluor-5-[(2-methylchinazolin-5-yl)amino]-6-(trifluormethyl)-5,6,7,8-tetrahydronaphthalin-1,6-diol
(*5S*,*6R*,*8R*)-2-Chlor-8-ethyl-3-fluor-5-[(7-fluor-2-methylchinazolin-5-yl)amino]-6-(trifluormethyl)-5,6,7,8-tetrahydronaphthalin-1,6-diol
5-{[(*1*α,*2*α*4*β)-7-Fluor-2,5-dihydroxy-4-ethyl-2-(trifluormethyl)1,2,3,4-tetrahydronaphthalin-1-yl]amino}-7-fluor-1*H*-chinolin-2-on
5-{[(*5S*,*6R*,*8R*)-8-Ethyl-3-fluor-1,6-dihydroxy-6-(trifluormethyl)-5,6,7,8-tetrahydronaphthalin-5-yl]amino}-8-fluorchinolin-2(1*H*)-on
5-{[(*1*α,*2*α,*4*β)-7-Fluor-2,5-dihydroxy-4-methyl-2-(trifluormethyl)-1,2,3,4-tetrahydronaphthalin-1-yl]amino}-7-fluor-1*H*-chinolin-2-on
5-{[(*1*α*,2*α*,4*β)-7-Fluor-2,5-dihydroxy-4-methyl-2-(trifluormethyl)-1,2,3,4-tetrahydronaphthalin-1-yl]amino}-8-ftuor-1*H*-chinolin-2-on
5-{[(*1*α,*2*α,*4*β)-7-Brom-2,5-dihydroxy-4-ethyl-2-(trifluormethyl)-1,2,3,4-tetrahydronaphthalin-1-yl]amino}-1*H*-chinolin-2-on
5-{[(*1S*,*2R,4R*)*-7*-Brom-2,5-dihydroxy-4-ethyl-2-(trifluormethyl)-1,2,3,4-tetrahydronaphthalin-1-yl]amino}-7-fluor-1*H*-chinolin-2-on
5-{[(*1*α,*2*α,*4*β)-7-Brom-2,5-dihydroxy-4-ethyl-2-(trifluormethyl)-1,2,3,4-tetrahydronaphthalin-1-yl]amino}-8-fluor-1H-chinolin-2-on
5-{[(*1*α,*2*α,*4*β-6-Fluor-2,7-dihydroxy-4-methyl-2-(trifluormethyl)1,2,3,4-tetrahydronaphthalin-1-yl]amino}-1*H*-chinolin-2-on
(*5*α,*6*α,*8*β)-8-Ethyl-1,6-dihydroxy-5-(2-oxo-1,2-dihydrochinolin-5-ylamino)-6-(trifluormethyl)-5,6,7,8-tetrahydronaphthalin-2-carbonitril
(*5*α,*6*α,*8*β)-8-Ethyl-1,6-dihydroxy-5-(8-fluor-2-oxo-1,2-dihydrochinolin-5-ylamino)-6-(trifluormethyl)-5,6,7,8-tetrahydronaphthalin-2-carbonitril
(*5*α,*6*α,*8*β)-8-Ethyl-1,6-dihydroxy-5-[(1-oxo-1,2-dihydroisochinolin-5-yl)amino]-6-(trifluormethyl)-5,6,7,8-tetrahydronaphthalin-2-carbonitril
(*5*α,*6*α,*8*β-1,6-Dihydroxy-8-methyl-5-[(2-oxo-2,3-dihydro-1*H*-indol-4-yl)amino]-6-(trifluormethyl)5,6,7,8-tetrahydronaphthalin-2-carbonitril
(*5*α,*6*α,*8*β)-5-[(8-Fluor-2-oxo-1,2-dihydrochinolin-5-yl)amino]-1,6-dihydroxy-8-methyl-6-(trifluormethyl)-5,6,7,8-tetrahydronaphthalin-2-carbonitril
5-{[(*5S*,*6R*,*8R*)-8-Ethyl-1,6-dihydroxy-3-fluor-5-[(2-oxo-1,2-dihydrochinolin-5-yl)amino]-6-(trifluormethyl)-5,6,7,8-tetrahydronaphthalin-2-carbonitril
(*5*α,*6*α,*8*β)-8-Ethyl-1,6-dihydroxy-3-fluor-5-[(8-fluor-2-oxo-1,2-dihydrochinolin-5-yl)amino]-6-(trifluormethyl)-5,6,7,8-tetrahydronaphthalin-2-carbonitril
5-{[(1S,2R,4S)-2, 5-Dihydroxy-4-methyl-2-(trifluormethyl)-1,2,3,4-tetrahydronaphthalin-1-yl]amino}-7-fluor-1*H*-chinolin-2-on
5-{[(*1S*,*2R*,*4R*)-2,5-Dihydroxy-4-methyl-2-(trifluormethyl)-1,2,3,4-tetrahydronaphthalin-1-yl]amino}-7-fluor-1H-chinolin-2-on
5-{[(*1S*,*2R*,*4S*)-2,5-Dihydroxy-4-methyl-2-(trifluormethyl)1,2,3,4-tetrahydronaphthalin-1-yl]amino}-8-fluor-1H-chinolin-2-on
5-{[(1*S,2R,4R*)-2,5-Dihydroxy-4-methyl-2-(trifluormethyl)-1,2,3,4-tetrahydronaphthalin-1-yl]amino}-8-fluor-1H-chinolin-2-on
5-{[(*1*α,*2*α,*4*β)-4-Ethyl-2,5-dihydroxy-2-(trifluormethyl)-1,2,3,4-tetrahydronaphthalin-1-yl]amino}-6-fluor-1*H*-chinolin-2-on
5-{[(*1*α,*2*α,*4*β)-4-Ethyl-6-fluor-2,5-dihydroxy-7-methyl-2-(trifluormethyl)-1,2,3,4-tetrahydronaphthalin-1-yl]amino}-1*H*-chinolin-2-on
5-{[(*1*α,*2*α,*4*β)-4-Ethyl-6-Fluor-2,5-dihydroxy-7-methyl-2-(trifluormethyl)-1,2,3,4-tetrahydronaphthalin-1-yl]amino}-7-fluor-1*H*-chinolin-2-on
5-{[(*1*α,*2*α,*4*β)-4-Ethyl-6-fluor-2,5-dihydroxy-7-methyl-2-(trifluormethyl)-1,2,3,4-tetrahydronaphthalin-1-yl]amino}-8-fluor-1*H-*chinolin-2-on
5-{[(*1*α,*2*α,*4*β)-4-Ethyl-6-Fluor-2,5-dihydroxy-7-methyl-2-(trifluormethyl)-1,2,3,4-tetrahydronaphthalin-1-yl]amino}-2*H*-chinolin-1-on
(*5*α,*6*α,*8*β)-8-Ethyl-2-fluor-3-methyl-5-[2-methylchinazolin-5-ylamino]-6-(trifluormethyl)-5,6,7,8-tetrahydronaphthalin-1,6-diol
(*5S*,*6*R,*8*R)-8-Ethyl-2,3-difluor-5-[(2-methyl-1-oxychinolin-5-yl)amino]-6-(trifluormethyl)-5,6,7,8-tetrahydronaphthalin-1,6-diol
5-{[(*1*α,*2*α,*4*β)-2,5-Dihydroxy-6,7-dimethyl-4-ethyl-2-(trifluormethyl)-1,2,3,4-tetrahydronaphthalin-1-yl]amino}-1H-chinolin-2-on
5-{[(*1*α,*2*α,*4*β)-2,5-Dihydroxy-6,7-dimethyl-4-ethyl-2-(trifluormethyl)1,2,3,4-tetrahydronaphthalin-1-yl]amino}-8-fluor-1H-chinolin-2-on
5-{[(*1*α,*2*α,*4*β)-2,5-Dihydroxy-6,7-dimethyl-4-ethyl-2-(trifluormethyl)-1,2,3,4-tetrahydronaphthalin-1-yl]amino}-2H-chinolin-1-on
5-{[(*5S*,*6R*,*8R*)-2-Chlor-8-ethyl-1,6-dihydroxy-3-fluor-2-6-(trifluormethyl)-5,6,7.8-tetrahydronaphthalin-5-yl]amino}chinolin-2(1*H*)-on
5-{[(5S,6R,8R)-3-Chlor-8-ethyl-1,6-dihydroxy-2-6-(trifluormethyt)-5,6,7,8-tetrahydronaphthalin-5-yl]amino}-7-fluorchinolin-2(1H)-on
(*5S*,*6R*,*8R)*-2,3-Dichlor-8-ethyl-5-[(7-fluor-2-methylchinazolin-5-yl)amino]-6-(trifluormethyl)-5,6,7,8-tetrahydronaphthalin-1,6-diol
(*5S*,*6R*,*8R*)-2-Chlor-8-ethyl-5-[(7-fluor-2-methylchinazolin-5-yl)amino]-6-(trifluormethyl)-5,6,7,8-tetrahydronaphthalin-1,6-diol oder
(*5S*,*6R*,*8R*)-3-Ch)or-8-ethyt-5-[(7-ftuor-2-methytchinazo)in-5-yl)amino]-6-(trifluormethyl)-5,6,7,8-tetrahydronaphthalin-1,6-diol.

4. Verwendung von Verbindungen nach Anspruch 1, 2 oder 3 zur Herstellung von Arzneimitteln.

5. Verwendung von Verbindungen nach Anspruch 1, 2 oder 3 zur Herstellung von Arzneimitteln zur Behandlung von entzündlichen Erkrankungen.

## Claims

1. Compound of the general formula (Ia), 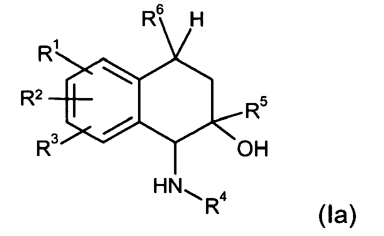 in which
I) the radicals R¹, R² and R³ independently of one another are selected from
-OH, O-CH₃, Cl, F, H,
II) the radical R⁴ is selected from
2-methylquinolin-5-yl
2-methylquinazolin-5-yl
2-ethylquinazolin-5-yl
7-fluoro-2-methylquinazolin-5-yl,
8-fluoro-2-methylquinazolin-5-yl,
7,8-difluoro-2-methylquinazolin-5-yl,
quinolin-2(1H)-on-5-yl,
7-fluoroquinolin-2(1H)-on-5-yl,
8-fluoroquinolin-2(1H)-on-5-yl,
isochromen-1-on-5-yl
2-methylphthalazin-1-on-5-yl
isoquinolin-2(1*H*)-on-5-yl,
III) the radical R⁵ is -CF₃
and
IV) the radical R⁶ is selected from -CH₃, -CH₂-CH₃, -(CH₂)₂-CH₃,
or -CH=CH₂,
and also stereoisomers thereof or its salts with physiologically tolerated anions,
**characterized in that** the compounds are present in the 1α,2α,4β configuration of the 1-amino-1,2,3,4-tetrahydronaphthalen-2-ol parent structure or **in that** the compounds are present in the 5α,6α,8β configuration of the 5,6,7,8-tetrahydronaphthalene-1,6-diol parent structure.

2. Compound according to Claim 1, **characterized in that** the absolute configuration is
(1S,2R,4R) or (1S,2R,4S) on the 1-amino-1,2,3,4-tetrahydronaphthalen-2-ol parent structure
or
(5S,6R,8R) or (5S,6R,8S) on the 5,6,7,8-tetrahydronaphthalene-1,6-diol parent structure.

3. Compound according to Claim 1 or 2, selected from
(*5*α,*6*α,*8*β)-2-fluoro-8-methyl-5-[(2-methylquinolin-5-yl)amino]-6-(trifluoromethyl)-5,6,7,8-tetrahydronaphthalene-1,6-diol
(+)-(*5*α,*6*α,*8*β)-2-fluoro-5-[(7-fluoro-2-methylquinazolin-5-yl)amino]-8-methyl-6-(trifluoromethyl)-5,6,7,8-tetrahydronaphthalene-1,6-diol
(-)-(*5*α,*6*α,*8*β)-2-fluoro-5-[(7-fluoro-2-methylquinazolin-5-yl)amino]-8-methyl-6-(trifluoromethyl)-5,6,7,8-tetrahydronaphthalene-1,6-diol
(*5*α,*6*α,*8*β)-2-fluoro-5-[(8-fluoro-2-methylquinazolin-5-yl)amino]8-methyl-6-(trifluoromethyl)-5,6,7,8-tetrahydronaphthalene-1,6-diol
(*5*α,*6*α,*8*β)-5-[(7,8-difluoro-2-methylquinazolin-5-yl)amino]-2-fluoro-8-methyl-6-(trifluoromethyl)-5,6,7,8-tetrahydronaphthalene-1,6-diol
(*5*α,*6*α,*8*β)-2-fluoro-8-methyl-5-[2-methylquinazolin-5-yl)amino]-6-(trifluoromethyl)-5,6,7,8-tetrahydronaphthalene-1,6-diol
5-{[(*5*α,*6*α,*8*β)-1,6-dihydroxy-2-fluoro-8-methyl-6-(trifluoromethyl)-5,6,7,8-tetrahydronaphthalen-5-yl]amino}quinolin-2(1*H*)-one 8-fluoro-5-{[(*1*α,*2*α,*4*β)-6-fluoro-2-hydroxy-5-methoxy-4-methyl-2-(trifluoromethyl)-1,2,3,4-tetrahydronaphthalen-1-yl]amino}quinolin-2(1H)-one
(+)-5-{[(*1*α,*2*α,*4*β)-6-fluoro-2,5-dihydroxy-4-methyl-2-(trifluoromethyl)-1,2,3,4-tetrahydronaphthalen-1-yl]amino}-8-fluoroquinolin-2(1H)-one
(-)-5-{[(*1*α,*2*α,*4*β)-6-fluoro-2,5-dihydroxy-4-methyl-2-(trifluoromethyl)-1,2,3,4-tetrahydronaphthalen-1-yl]amino}-8-fluoroquinolin-2(1H)-one
5-{[(*1*α,*2*α,*4*β)-6-fluoro-2,5-dihydroxy-4-methyl-2-(trifluoromethyl)-1,2,3,4-tetrahydronaphthalen-1-yl]amino}-2-methylphthalazin-1-one
(*5*α,*6*α,*8*β)-3-chloro-2-fluoro-8-methyl-5-[(2-methylquinazolin-5-yl)amino]--6-(trifluoromethyl)-5,6,7,8-tetrahydronaphthalene-1,6-diol
(*5*α,*6*α,*8*β)-3-chloro-2-fluoro-5-[(7-fluoro-2-methylquinazolin-5-yl)amino]-1-methoxy-8-methyl-6-(trifluoromethyl)-5,6,7,8-tetrahydronaphthalen-6-ol
(*5*α,*6*α,*8*β)-3-chloro-2-fluoro-5-[(7-fluoro-2-methylquinazolin-5-yl)amino]-8-methyl-6-(trifluoromethyl)-5,6,7,8-tetrahydronaphthalene-1,6-diol
(*5*α,*6*α,*8*β)-3-chloro-2-fluoro-5-[(8-fluoro-2-methylquinazolin-5-yl)amino] 1-methoxy-8-methyl-6-(trifluoromethyl)-5,6,7,8-tetrahydronaphthalen-6-ol
(*5*α,*6*α,*8*β)-3-chloro-2-fluoro-5-[(8-fluoro-2-methylquinazolin-5-yl)amino] 8-methyl-6-(trifluoromethyl)-5,6,7,8-tetrahydronaphthalene-1,6-diol
(*5*α,*6*α,*8*β)-3-chloro-5-[(7,8-difluoro-2-methylquinazolin-5-yl)amino]-2-fluoro-8-methyl-6-(trifluoromethyl)-5,6,7,8-tetrahydronaphthalene-1,6-diol
(*5*α,*6*α,*8*β)-3-chloro-2-fluoro-8-methyl-5-[2-methylquinolin-5-yl)amino]-6-(trifluoromethyl)-5,6,7,8-tetrahydronaphthalene-1,6-diol
(+)-5-{[(*1*α,*2*α,*4*β)-7-chloro-2,5-dihydroxy-6-fluoro-4-methyl-2-(trifluoromethyl)-1,2,3,4-tetrahydronaphthalen-1-yl]-amino}quinolin-2(1*H*)-one
(-)-5-{[(*1*α,*2*α,*4*β)-7-chloro-2,5-dihydroxy-6-fluoro-4-methyl-2-(trifluoromethyl)-1,2,3,4-tetrahydronaphthalen-1-yl]-amino}quinolin-2(1*H*)-one
5-{[(*1*α,*2*α,*4*β)-7-chloro-2,5-dihydroxy-6-fluoro-4-methyl-2-(trifluoromethyl) 1,2,3,4-tetrahydronaphthalen-1-yl]-amino}-8-fluoroquinolin-2(1*H*)-one
(*5*α,*6*α,*8*β)-8-methyl-5-[-2-methylquinolin-5-yl)amino]-6-(trifluoromethyl)-5,6,7,8-tetrahydronaphthalene-1,6-diol
(*5*α,*6*α,*8*β)-8-ethyl-5-[2-methylquinazolin-5-yl)amino]-6-(trifluoromethyl)-5,6,7,8-tetrahydronaphthalene-1,6-diol
(+)-(*5*α,*6*α,*8*β)-8-ethyl-2-fluoro-5-[(2-methylquinazolin-5-yl)amino]-6-(trifluoromethyl)-5,6,7,8-tetrahydronaphthalene-1,6-diol
(-)-(*5*α,*6*α,*8*β)-8-ethyl-2-fluoro-5-[(2-methylquinazolin-5-yl)amino]-6-(trifluoromethyl)-5,6,7,8-tetrahydronaphthalene-1,6-diol
(5*S*,*6*R,*8*R)-8-ethyl-2-fluoro-5-[(7-fluoro-2-methylqu inazolin-5-yl)amino]-6-(trifluoromethyl)-5,6,7,8-tetrahydronaphthalene-1,6-diol
(+)-(*5*α,*6*α,*8*β)-8-ethyl-2-fluoro-5-[(8-fluoro-2-methylquinazolin-5-yl)amino]-6-(trifluoromethyl)-5,6,7,8-tetrahydronaphthalene-1,6-diol
(-)-(*5*α,*6*α,*8*β)-8-ethyl-2-fluoro-5-[(8-fluoro-2-methylquinazolin-5-yl)amino]-6-(trifluoromethyl)-5,6,7,8-tetrahydronaphthalene-1,6-diol
(*5*α,*6*α,*8*β)-5-[(7,8-difluoro-2-methylquinazolin-5-yl)amino]-8-ethyl-2-fluoro-6-(trifluoromethyl)-5,6,7,8-tetrahydronaphthalene-1,6-diol
5-{[*1*α,*2*α,*4*β)-4-ethyl-6-fluoro-2,5-dihydroxy-2-(trifluoromethyl)-1,2,3,4-tetrahydronaphthalen-1-yl]amino}quinolin-2(1*H*)-one
5-{[(*1*α,*2*a,*4*β)-4-ethyl-6-fluoro-2-hydroxy-5-methoxy-2-(trifluoromethyl)-1,2,3,4-tetrahydronaphthalen-1-yl]amino}-8-fluoroquinolin-2(1*H*)-one
(+)-5-{[(*1*α,*2*α,*4*β)-4-ethyl-6-fluoro-2,5-dihydroxy-2-(trifluoromethyl)-1,2,3,4-tetrahydronaphthalen-1-yl]amino}-8-fluoroquinolin-2(1*H*)-one
(-)-5-{[(*1*α,*2*α,*4*β)-4-ethyl-6-fluoro-2,5-dihydroxy-2-(trifluoromethyl)-1,2,3,4-tetrahydronaphthalen-1-yl]amino}-8-fluoroquinolin-2(1*H*)-one
5-{[(*1*α,*2*α,*4*β)-4-ethyl-6-fluoro-2,5-dihydroxy-2-(trifluoromethyl)-1,2,3,4-tetrahydronaphthalen-1-yl]amino}-2-methylphthalazin-1-one
5-{[(*1*α,*2*a,*4*β)-4-ethyl-6-fluoro-2,5-dihydroxy-2-(trifluoromethyl)-1,2,3,4-tetrahydronaphthalen-1-yl]amino}isoquinolin-(2H)-one
5-{[(*1*α,*2*α,*4*β)-4-ethyl-6-fluoro-2,5-dihydroxy-2-(trifluoromethyl)-1,2,3,4-tetrahydronaphthalen-1-yl]amino}isochromen-1-one
(*5*α,*6*α,*8*β)-5-[(2-ethylquinazolin-5-yl)amino]-2-fluoro-8-propyl-6-(trifluoromethyl)-5,6,7,8-tetrahydronaphthalene-1,6-diol
(5S, 6R, 8R)-8-ethyl-2,3-difluoro-5-[(2-methylquinazolin-5-yl)amino]-6-(trifluoromethyl)-5,6,7,8-tetrahydronaphthalene-1,6-diol
(+)-(*5*α,*6*α,*8*β)-8-ethyl-2,3-difluoro-5-[(7-fluoro-2-methylquinazolin-5-yl)amino]-6-(trifluoromethyl)-5,6,7,8-tetrahydronaphthalene-1,6-diol
(-)-(*5*α,*6*α,*8*β)-8-ethyl-2,3-difluoro-5-[(7-fluoro-2-methylquinazolin-5-6-(trifluoromethyl)-5,6,7,8-tetrahydronaphthalene-1,6-diol
(*5S*,*6R*,*8R*)-8-ethyl-2,3-difluoro-5-[(8-fluoro-2-methylquinazolin-5-yl)amino]-6-(trifluoromethyl)-5,6,7,8-tetrahydronaphthalene-1,6-diol
(*5S*,*6R*,*8R*)-8-ethyl-2,3-difluoro-5-[(7,8-difluoro-2-methylqu inazolin-5-yl)amino]-6-(trifluoromethyl)-5,6,7,8-tetrahydronaphthalene-1,6-diol
(+)-5-{[(*1*α,*2*α,*4*β)-4-ethyl-6,7-difluoro-2,5-dihydroxy-2-(trifluoromethyl)-1,2,3,4-tetrahydronaphthalen-1-yl]amino}quinolin-2(1*H*)-one
(-)-5-{[(*1*α,*2*α,*4*β)-4-ethyl-6,7-difluoro-2,5-dihydroxy-2-(trifluoromethyl)-1,2,3,4-tetrahydronaphthalen-1-yl]amino}quinolin-2(1*H*)-one
(5α,6α,8β)-8-ethyl-2,3-difluoro-5-[(2-methylquinolin-5-yl)amino]-6-(trifluoromethyl)-5,6,7,8-tetrahydronaphthalene-1,6-diol
5-{[(*1*α,*2*α,*4*β)-4-ethyl-6,7-difluoro-2,5-dihydroxy-2-(trifluoromethyl)-1,2,3,4-tetrahydronaphthalen-1-yl]amino}phthalazin-1-one
(5α,6α,8β)-2-fluoro-5-[(2-methylquinazolin-5-yl)amino]-8-prop-1-yl-6-(trifluoromethyl)-5,6,7,8-tetrahydronaphthalene-1,6-diol
(5α,6α,8β)-2-fluoro-5-[(7-fluoro-2-methylquinazolin-5-yl)amino]-8-prop-1-yl-6-(trifluoromethyl)-5,6,7,8-tetrahydronaphthalene-1,6-diol
5-{[(*1*α,*2*α,*4*β)-4-ethyl-6,7-difluoro-2,5-dihydroxy-2-(trifluoromethyl)-1,2,3,4-tetrahydronaphthalen-1-yl]amino}quinolin-2(1*H*)-one
5-{[(*1*α,*2*α,*4*β)-4-ethyl-6,7-difluoro-2,5-dihydroxy-2-(trifluoromethyl)-1,2,3,4-tetrahydronaphthalen-1-yl]amino}isoquinolin-1(2*H*)-one
5-{[(*1S*,*2R*,*4S*)-6-chloro-2,5-dihydroxy-4-methyl-2-(trifluoromethyl)-1,2,3,4-tetrahydronaphthalen-1-yl]amino}-1*H*-quinolin-2-one
5-{[(*1S*,*2R*,*4R*)-6-chloro-2,5-dihydroxy-4-methyl-2-(trifluoromethyl)-1,2,3,4-tetrahydronaphthalen-1-yl]amino}-1*H*-quinolin-2-one
5-{[(*1S*,*2R*,*4S*)-6-chloro-2,5-dihydroxy-4-methyl-2-(trifluoromethyl)-1,2,3,4-tetrahydronaphthalen-1-yl]amino}-8-fluoro-1*H*-quinolin-2-one
5-{[(*1S*,*2R*,*4R*)-6-chloro-2,5-dihydroxy-4-methyl-2-(trifluoromethyl)-1,2,3,4-tetrahydronaphthalen-1-yl]amino}-8-fluoro-1*H*-quinolin-2-one
5-{[(*1S*,*2R*,*4S*)-6-chloro-2,5-dihydroxy-4-methyl-2-(trifluoromethyl)-1,2,3,4-tetrahydronaphthalen-1-yl]amino}-2*H*-quinolin-1-one
5-{[(*1S*,*2R*,*4R*)-6-chloro-2,5-dihydroxy-4-methyl-2-(trifluoromethyl)-1,2,3,4-tetrahydronaphthalen-1-yl]amino}-2H-quinolin-1-one
5-{[(*1S*,*2R*,*4S*)-6-chloro-2,5-dihydroxy-4-methyl-2-(trifluoromethyl)-1,2,3,4-tetrahydronaphthalen-1-yl]amino}-isochromen-1-one
5-{[(*1S*,*2R*,*4R*)-6-chloro-2,5-dihydroxy-4-methyl-2-(trifluoromethyl)-1,2,3,4-tetrahydronaphthalen-1-yl]amino}-isochromen-1-one
4-{[(*1R*,*2S*,*4R*)-6-chloro-2,5-dihydroxy-4-methyl-2-(trifluoromethyl)-1,2,3,4-tetrahydronaphthalen-1-yl]amino}-1,3-dihydroindol-2-one
4-{[(*1S*,*2R*,*4S*)-6-chloro-2,5-dihydroxy-4-methyl-2-(trifluoromethyl)-1,2,3,4-tetrahydronaphthalen-1-yl]amino}-1,3-dihydroindol-2-one
4-{[(*1S*,*2R*,*4R*)-6-chloro-2,5-dihydroxy-4-methyl-2-(trifluoromethyl)-1,2,3,4-tetrahydronaphthalen-1-yl]amino}-1,3-dihydroindol-2-one
5-{[(*1S*,*2R*,*4S*)-chloro-2,5-dihydroxy-4-ethyl-2-(trifluoromethyl)-1,2,3,4-tetrahydronaphthalen-1-yl]amino}-1H-quinolin-2-one
5-{[(*1*α,*2*α,*4*β)-6-chloro-2,5-dihydroxy-4-ethyl-2-(trifluoromethyl)-1,2,3,4-tetrahydronaphthalen-1-yl]amino}-1H-quinolin-2-one
5-{[(*1S*,*2R*,*4S*)-6-chloro-2,5-dihydroxy-4-ethyl-2-(trifluoromethyl)-1,2,3,4-tetrahydronaphthalen-1-yl]amino}-8-fluoro-1H-quinolin-2-one
5-{[(*1S*,*2R*,*4R*)-6-chloro-4-ethyl-2,5-dihydroxy-2-(trifluoromethyl)-1,2,3,4-tetrahydronaphthalen-1-yl]amino}-8-fluoro-1H-quinolin-2-one
5-{[(*1S*,*2R*,*4S*)-6-chloro-2,5-dihydroxy-4-ethyl-2-(trifluoromethyl)-1,2,3,4-tetrahydronaphthalen-1-yl]amino}-2*H*-quinolin-1-one
5-{[(*1S*,*2R*,*4R*)-6-chloro-4-ethyl-2,5-dihydroxy-2-(trifluoromethyl)-1,2,3,4-tetrahydronaphthalen-1-yl]amino}-2*H*-quinolin-1-one
5-{[(*1S*,*2R*,*4S*)-6-chloro-2,5-dihydroxy-4-ethyl-2-(trifluoromethyl)-1,2,3,4-tetrahydronaphthalen-1-yl]amino}isochromen-1-one
5-{[(*1S*,*2R*,*4R*)-6-chloro-4-ethyl-2,5-dihydroxy-4-2-(trifluoromethyl)-1,2,3,4-tetrahydronaphthalen-1-yl]amino}isochromen-1-one
4-{[(*1S*,*2R*,*4S*)-6-chloro-2,5-dihydroxy-4-ethyl-2-(trifluoromethyl)-1,2,3,4-tetrahydronaphthalen-1-yl]amino}-1,3-dihydroindol-2-one
4-{[(*1S*,*2R*,*4R*)-6-chloro-2,5-dihydroxy-4-ethyl-2-(trifluoromethyl)-1,2,3,4-tetrahydronaphthalen-1-yl]amino}-1,3-dihydroindol-2-one
5-{[(*1S*,*2R*,*4S*)-6-chloro-2,5-dihydroxy-4-ethyl-2-(trifluoromethyl)-1,2,3,4-tetrahydronaphthalen-1-yl]amino}-7-fluoro-1H-quinolin-2-one
5-{[(*1S*,*2R*,*4R*)-6-chloro-4-ethyl-2,5-dihydroxy-2-(trifluoromethyl)-1,2,3,4-tetrahydronaphthalen-1-yl]amino}-7-fluoro-1H-quinolin-2-one
5-{[(*1S*,*2R*,*4S*)-6-chloro-2,5-Dihydroxy-4-methyl-2-(trifluoromethyl)-1,2,3,4-tetrahydronaphthalen-1-yl]amino}-7-fluoro-1*H*-quinolin-2-one
5-{[(*1S*,*2R*,*4R*)-6-chloro-2,5-dihydroxy-4-methyl-2-(trifluoromethyl)-1,2,3,4-tetrahydronaphthalen-1-yl]amino}-7-fluoro-1*H*-quinolin-2-one
(*5*α,*6*a,*8*β)-2-chloro-8-ethyl-5-[(indazol-5-yl)amino]-6-(trifluoromethyl)-5,6,7,8-tetrahydronaphthalene-1,6-diol
6-{[(*1*α,*2*α,*4*β)-6-chloro-2,5-dihydroxy-4-ethyl-2-(trifluoromethyl)-1,2,3,4-tetrahydronaphthalen-1-yl]amino}-4-methylbenzo[d][1,2]oxazin-1-one
5-{[(*1*α,*2*α,*4*β)-*7*-isopropyl-2,5-dihydroxy-4-ethyl-2-(trifluoromethyl)-1,2,3,4-tetrahydronaphthalen-1-yl]amino}-1*H*-quinolin-2-one
(*5*α,*6*α,*8*β)-8-ethyl-3-isopropyl-5-[2-methylquinazolin-5-ylamino]-6-(trifluoromethyl)-5,6,7,8-tetrahydronaphthalene-1,6-diol
5-{[(*1*α,*2*α,*4*β)-*7*-isopropyl-2,5-dihydroxy-4-ethyl-2-(trifluoromethyl)-1,2,3,4-tetrahydronaphthalen-1-yl]amino}-2*H*-quinolin-1-one
4-{[(*1*α,*2*α,*4*β)-*7*-isopropyl-2,5-dihydroxy-4-ethyl-2-(trifluoromethyl)-1,2,3,4-tetrahydronaphthalen-1-yl]amino}-1,3-dihydroindol-2-one
5-{[(*1*α,*2*α,*4*β)-*7*-isopropyl-2,5-dihydroxy-4-ethyl-2-(trifluoromethyl)-1,2,3,4-tetrahydronaphthalen-1-yl]amino}isochromen-1-one
5-{[(*1*α,*2*α,4β)-7-isopropyl-2,5-dihydroxy-4-ethyl-2-(trifluoromethyl)-1,2,3,4-tetrahydronaphthalen-1-yl]amino}-8-fluoro-1*H*-quinolin-2-one
5-{[(*1*α,*2*α,*4*β)-7-isopropyl-2,5-dihydroxy-4-ethyl-2-(trifluoromethyl)-1,2,3,4-tetrahydronaphthalen-1-yl]amino}-8-fluoro-1*H*-quinolin-2-one
5-{[(*1*α,*2*α,*4*β)-7-chloro-2,5-dihydroxy-4-ethyl-6-methyl-2-(trifluoromethyl)-1,2,3,4-tetrahydronaphthalen-1-yl]amino}-1*H*-quinolin-2-one
5-{[(1*S*,2*R*,4*R*)-7-chloro-2,5-dihydroxy-6-methyl-4-ethyl-2-(trifluoromethyl)-1,2,3,4-tetrahydronaphthalen-1-yl]amino}-2*H*-quinolin-1-one
4-{[(*1*α,*2*α,*4*β)-7-chloro-2,5-dihydroxy-6-methyl-4-ethyl-2-(trifluoromethyl)-1,2,3,4-tetrahydronaphthalen-l-yl]amino}-1,3-dihydroindol-2-one
5-{[(*1*α,*2*α,*4*β)-7-chloro-2,5-dihydroxy-4-ethyl-6-methyl-2-(trifluoromethyl)-1,2,3,4-tetrahydronaphthalen-1-yl]amino}-8-fluoro-1*H*-quinolin-2-one
5-{[(*1S*,*2R*,*4R*)-7-chloro-2,5-dihydroxy-4-ethyl-6-methyl-2-(trifluoromethyl)-1,2,3,4-tetrahydronaphthalen-1-yl]amino}-8-fluoro-1*H*-quinolin-2-one
(*5*α,*6*α,*8*β)-3-chloro-8-ethyl-2-methyl-5-[2-methylquinazolin-5-ylamino]-6-(trifluoromethyl)-5,6,7,8-tetrahydronaphthalene-1,6-diol
5-{[(*1S*,*2R*,*4S*)-7-chloro-2,5-dihydroxy-4-ethyl-6-methyl-2-(trifluoromethyl)-1,2,3,4-tetrahydronaphthalen-1-yl]amino}-7-fluoro-1*H*-quinolin-2-one
5-{[(*1*α,*2*α,*4*β)-7-chloro-2,5-dihydroxy-4-ethyl-6-methyl-2-(trifluoromethyl)-1,2,3,4-tetrahydronaphthalen-1-yl]amino}-7-fluoro-1*H*-quinolin-2-one
5-{[(*1S*,*2R*,*4R*)-7-chloro-2,5-dihydroxy-4-ethyl-6-methyl-2-(trifluoromethyl)-1,2,3,4-tetrahydronaphthalen-1-yl]amino}-7-fluoro-1*H*-quinolin-2-one (5S,6R,8R)-8-ethyl-2,3-difluoro-5-[(2-methylquinazolin-5-yl)amino]-6-(trifluoromethyl)-5,6,7,8-tetrahydronaphthalene-1,6-diol
5-{[(1S,2R,4R)-4-ethyl-7-fluoro-2,5-dihydroxy-6-methyl-2-(trifluoromethyl)-1,2,3,4-tetrahydronaphthalen-1-yl]amino}quinolin-2(1H)-one
5-{[(1S,2R,4R)-4-ethyl-7-fluoro-2,5-dihydroxy-6-methyl-2-(trifluoromethyl)-1,2,3,4-tetrahydronaphthalen-1-yl]amino}-7-fluoroquinolin-2(1H)-one
5-{[(1S,2R,4R)-4-ethyl-7-fluoro-2,5-dihydroxy-6-methyl-2-(trifluoromethyl)-1,2,3,4-tetrahydronaphthalen-1-yl]amino)-8-fluoroquinolin-2(1H)-one
5-{[(1S,2R,4R)-4-ethyl-7-fluoro-2,5-dihydroxy-6-methyl-2-(trifluoromethyl)-1,2,3,4-tetrahydronaphthalen-1-yl]amino}isoquinolin-1(2H)-one (5S,6R,8R)-8-ethyl-3-fluoro-5-[(7-fluoro-2-methylquinazolin-5-yl)amino]-2-methyl-6-(trifluoromethyl)-5,6,7,8-tetrahydronaphthalene-1,6-diol (5S,6R,8R)-8-ethyl-3-fluoro-5-[(8-fluoro-2-methylquinazolin-5-yl)amino]-2-methyl-6-(trifluoromethyl)-5,6,7,8-tetrahydronaphthalene-1,6-diol
5-{[(1S,2R,4S)-4,6-diethyl-7-fluoro-2,5-dihydroxy-2-(trifluoromethyl)-1,2,3,4-tetrahydronaphthalen-1-yl]amino}quinolin-2(1H)-one
5-{[(1S,2R,4R)-4,6-diethyl-7-fluoro-2,5-dihydroxy-2-(trifluoromethyl)-1,2,3,4-tetrahydronaphthalen-1-yl]amino}quinolin-2(1H)-one
5-{[(1S,2R,4S)-4,6-diethyl-7-fluoro-2,5-dihydroxy-2-(trifluoromethyl)-1,2,3,4-tetrahydronaphthalen-1-yl]amino}-7-fluoroquinolin-2(1H)-one
5-{[(1S,2R,4R)-4,6-diethyl-7-fluoro-2,5-dihydroxy-2-(trifluoromethyl)-1,2,3,4-tetrahydronaphthalen-1-yl]amino}-7-fluoroquinolin-2(1H)-one
5-{[(1S,2R,4S)-4,6-diethyl-2,5-dihydroxy-2-(trifluoromethyl)-1,2,3,4-tetrahydronaphthalen-1-yl]amino}-1H-quinolin-2-one
5-{[(1S,2R,4R)-4,6-diethyl-2,5-dihydroxy-2-(trifluoromethyl)-1,2,3,4-tetrahydronaphthalen-1-yl]amino}-1H-quinolin-2-one
5-{[(*1S*,*2R*,*4S*)-4,6-diethyl-2,5-dihydroxy-2-(trifluoromethyl)-1,2,3,4-tetrahydronaphthalen-1-yl]amino}-8-fluoro-1*H*-quinolin-2-one
5-{[(*1S*,*2R*,*4R*)-4,6-diethyl-2,5-dihydroxy-2-(trifluoromethyl)-1,2,3,4-tetrahydronaphthaien-1-yl]amino}-8-fluoro-1H-quinolin-2-one
(*5S*,*6R*,*8S*)-2,8-diethyl-5-[2-methylquinazolin-5-ylamino]-6-(trifluoromethyl)-5,6,7,8-tetrahydronaphthalene-1,6-diol
5-{[(*1*α,*2*α,*4*β)-7-chloro-2,5-dihydroxy-4-ethyl-2-(trifluoromethyl)-1,2,3,4-tetrahydronaphthalen-1-yl]amino}-1H-quinolin-2-one
5-{[(*1*α,*2*α,*4*β)-7-chloro-2,5-dihydroxy-4-ethyl-2-(trifluoromethyl)-1,2,3,4-tetrahydronaphthalen-1-yl]amino}-8-fluoro-1H-quinolin-2-one
5-{[(*1S*,*2R*,*4R*)-6-chloro-4-ethyl-2,5-dihydroxy-7-fluoro-2-(trifluoromethyl)-1,2,3,4-tetrahydronaphthalen-1-yl]amino}quinolin-2(1*H*)-one
5-{[(*1S*,*2R*,*4R*)-6-chloro-4-ethyl-2,5-dihydroxy-7-fluoro-2-(trifluoromethyl)-1,2,3,4-tetrahydronaphthalen-1-yl]amino}-7-fluoroquinolin-2(1*H*)-one
5-{[(*1S*,*2R*,*4R*)-6-chloro-4-ethyl-2,5-dihydroxy-7-fluoro-2-(trifluoromethyl)-1,2,3,4-tetrahydronaphthalen-1-yl]amino}-8-fluoroquinolin-2(1*H*)-one
(*5S*,*6R*,*8R*)-2-chloro-8-ethyl-3-fluoro-5-[(2-methylquinazolin-5-yl)amino]-6-(trifluoromethyl)-5,6,7,8-tetrahydronaphthalene-1,6-diol
(*5S*,*6R*,*8R*)-2-chloro-8-ethyl-3-fluoro-5-[(7-fluoro-2-methylquinazolin-5-yl)amino]-6-(trifluoromethyl)-5,6,7,8-tetrahydronaphthalene-1,6-diol
5-{[(*1*α,*2*α,*4*β)-7-fluoro-2,5-dihydroxy-4-ethyl-2-(trifluoromethyl)-1,2,3,4-tetrahydronaphthalen-1-yl]amino}-7-fluoro-1*H*-quinolin-2-one
5-{[(*5S*,*6R*,*8R*)-8-ethyl-3-fluoro-1,6-dihydroxy-6-(trifluoromethyl)-5,6,7,8-tetrahydronaphthalen-5-yl]amino}-8-fluoroquinolin-2(1*H*)-one
5-{[(*1*α,*2*α,*4*β)-7-fluoro-2,5-dihydroxy-4-methyl-2-(trifluoromethyl)-1,2,3,4-tetrahydronaphthalen-1-yl]amino}-7-fluoro-1*H*-quinolin-2-one
5-{[(*1*α,*2*α,*4*β)-7-fluoro-2,5-dihydroxy-4-methyl-2-(trifluoromethyl)-1,2,3,4-tetrahydronaphthalen-1-yl]amino}-8-fluoro-1*H*-quinolin-2-one
5-{[(*1*α,*2*α,*4*β)-7-bromo-2,5-dihydroxy-4-ethyl-2-(trifluoromethyl)-1,2,3,4-tetrahydronaphthalen-1-yl]amino}-1*H*-quinolin-2-one
5-{[(*1S*,*2R*,*4R*)-7-bromo-2,5-dihydroxy-4-ethyl-2-(trifluoromethyl)-1,2,3,4-tetrahydronaphthalen-1-yl]amino}-7-fluoro-1*H*-quinolin-2-one
5-{[(*1*α,*2*α,*4*β)-7-bromo-2,5-dihydroxy-4-ethyl-2-(trifluoromethyl)-1,2,3,4-tetrahydronaphthalen-1-yl]amino}-8-fluoro-1H-quinolin-2-one
5-{[(*1*α,*2*α,*4*β)-6-fluoro-2,7-dihydroxy-4-methyl-2-(trifluoromethyl)-1,2,3,4-tetrahydronaphthalen-1-yl]amino}-1*H*-quinolin-2-one
(*5*α,*6*α,*8*β)-8-ethyl-1,6-dihydroxy-5-(2-oxo-1,2-dihydroquinolin-5-ylamino)-6-(trifluoromethyl)-5,6,7,8-tetrahydronaphthalene-2-carbonitrile
(*5*α,*6*α,*8*β)-8-ethyl-1,6-dihydroxy-5-(8-fluoro-2-oxo-1,2-dihydroquinolin-5-ylamino)-6-(trifluoromethyl)-5,6,7,8-tetrahydronaphthalene-2-carbonitrile
(*5*α,*6*α,*8*β)-8-ethyl-1,6-dihydroxy-5-[(1-oxo-1,2-dihydroisoquinolin-5-yl)amino]-6-(trifluoromethyl)-5,6,7,8-tetrahydronaphthalene-2-carbonitrile
(*5*α,*6*α,*8*β)-1,6-dihydroxy-8-methyl-5-[(2-oxo-2,3-dihydro-1*H*-indol-4-yl)amino]-6-(trifluoromethyl)-5,6,7,8-tetrahydronaphthalene-2-carbonitrile
(*5*α,*6*α,*8*β)-5-[(8-fluoro-2-oxo-1,2-dihydroquinolin-5-yl)amino]-1,6-dihydroxy-8-methyl-6-(trifluoromethyl)-5,6,7,8-tetrahydronaphthalene-2-carbonitrile
5-{[(*5S*,*6R*,*8R*)-8-ethyl-1,6-dihydroxy-3-fluoro-5-[(2-oxo-1,2-dihydroquinolin-5-yl)amino]-6-(trifluoromethyl)-5,6,7,8-tetrahydronaphthalene-2-carbonitrile
(*5*α,*6*α,*8*β)-8-ethyl-1,6-dihydroxy-3-fluoro-5-[(8-fluoro-2-oxo-1,2-dihydroquinolin-5-yl)amino]-6-(trifluoromethyl)-5,6,7,8-tetrahydronaphthalene-2-carbonitrile
5-{[(*1S*,*2R*,*4S*)-2,5-dihydroxy-4-methyl-2-(trifluoromethyl)-1,2,3,4-tetrahydronaphthalen-1-yl]amino}-7-fluoro-1*H*-quinolin-2-one
5-{[(*1S*,*2R*,*4R*)-2,5-dihydroxy-4-methyl-2-(trifluoromethyl)-1,2,3,4-tetrahydronaphthalen-1-yl]amino}-7-fluoro-1H-quinolin-2-one
5-{[(*1S*,*2R*,*4S*)-2,5-dihydroxy-4-methyl-2-(trifluoromethyl)-1,2,3,4-tetrahydronaphthalen-1-yl]aminol-8-fluoro-1H-quinolin-2-one
5-{[(*1S*,*2R*,*4R*)-2,5-dihydroxy-4-methyl-2-(trifluoromethyl)-1,2,3,4-tetrahydronaphthalen-1-yl]amino}-8-fluoro-1H-quinolin-2-one
5-{[(*1*α,*2*α,*4*β)-4-ethyl-2,5-dihydroxy-2-(trifluoromethyl)-1,2,3,4-tetrahydronaphthalen-1-yl]amino}-6-fluoro-1*H*-quinolin-2-one
5-{[(*1*α,*2*α,*4*β)-4-ethyl-6-fluoro-2,5-dihydroxy-7-methyl-2-(trifluoromethyl)-1,2,3,4-tetrahydronaphthalen-1-yl]amino}-1*H*-quinolin-2-one
5-{[(*1*α,*2*α,*4*β)-4-ethyl-6-fluoro-2,5-dihydroxy-7-methyl-2-(trifluoromethyl)-1,2,3,4-tetrahydronaphthalen-1-yl]amino}-7-fluoro-1*H*-quinolin-2-one
5-{[(*1*α,*2*α,*4*β)-4-ethyl-6-fluoro-2,5-dihydroxy-7-methyl-2-(trifluoromethyl)-1,2,3,4-tetrahydronaphthalen-1-yl]amino}-8-fluoro-1*H*-quinolin-2-one
5-{[(*1*α,*2*α,*4*β)-4-ethyl-6-fluoro-2,5-dihydroxy-7-methyl-2-(trifluoromethyl)-1,2,3,4-tetrahydronaphthalen-1-yl]amino}-2*H*-quinolin-1-one
(*5*α,*6*α,*8*β)-8-ethyl-2-fluoro-3-methyl-5-[2-methylquinazolin-5-ylamino]-6-(trifluoromethyl)-5,6,7,8-tetrahydronaphthalene-1,6-diol
(*5S*,*6R*,*8R*)-8-ethyl-2,3-difluoro-5-[(2-methyl-1-oxyquinolin-5-yl)amino]-6-(trifluoromethyl)-5,6,7,8-tetrahydronaphthalene-1,6-diol
5-{[(*1*α,*2*α,*4*β)-2,5-dihydroxy-6,7-dimethyl-4-ethyl-2-(trifluoromethyl)-1,2,3,4-tetrahydronaphthalen-1-yl]amino}-1H-quinolin-2-one
5-{[(*1*α,*2*α,*4*β)-2,5-dihydroxy-6,7-dimethyl-4-ethyl-2-(trifluoromethyl)-1,2,3,4-tetrahydronaphthalen-1-yl]amino}-8-fluoro-1H-quinolin-2-one
5-{[(*1*α,*2*α,*4*β)-2,5-dihydroxy-6,7-dimethyl-4-ethyl-2-(trifluoromethyl)-1,2,3,4-tetrahydronaphthalen-1-yl]amino}-2H-quinolin-1-one
5-{[(*5S*,*6R*,*8R*)-2-chloro-8-ethyl-1,6-dihydroxy-3-fluoro-2-6-(trifluoromethyl)-5,6,7,8-tetrahydronaphthalen-5-yl]amino}quinolin-2(1*H*)-one
5-{[(*5S*,*6R*,*8R*)-3-chloro-8-ethyl-1,6-dihydroxy-2-6-(trifluoromethyl)-5,6,7,8-tetrahydronaphthalen-5-yl]amino}-7-fluoroquinolin-2(1H)-one
(*5S*,*6R*,*8R*)-2,3-d ichloro-8-ethyl-5-[(7-fluoro-2-methylquinazolin-5-yl)amino]-6-(trifluoromethyl)-5,6,7,8-tetrahydronaphthalene-1,6-diol
(*5S*,*6R*,*8R*)-2-chloro-8-ethyl-5-[(7-fluoro-2-methylquinazolin-5-yl)amino]-6-(trifluoromethyl)-5,6,7,8-tetrahydronaphthalene-1,6-diol
or
(*5S*,*6R*,*8R*)-3-chloro-8-ethyl-5-[(7-fluoro-2-methylquinazolin-5-yl)amino]-6-(trifluoromethyl)-5,6,7,8-tetrahydronaphthalene-1,6-diol.

4. Use of compounds according to Claim 1, 2 or 3 for producing medicaments.

5. Use of compounds according to Claim 1, 2 or 3 for producing medicaments for treating inflammatory disorders.

## Revendications

1) Composé de formule générale (Ia), 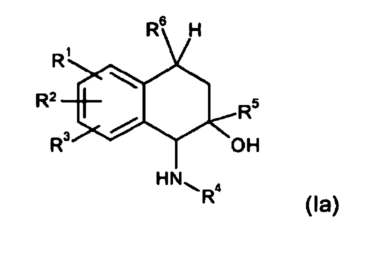 dans laquelle
I) les radicaux R¹, R² et R³ sont choisis, indépendamment les uns des autres, parmi
-OH, O-CH₃, Cl, F, H,
II) le radical R⁴ est choisi parmi les groupes
2-méthylquinolin-5-yle
2-méthylquinazolin-5-yle
2-éthylquinazolin-5-yle
7-fluoro-2-méthylquinazolin-5-yle,
8-fluoro-2-méthylquinazolin-5-yle,
7,8-difluoro-2-méthylquinazolin-5-yle, quinolin-2(1H)-on-5-yle,
7-fluoroquinolin-2(1H)-on-5-yle,
8-fluoroquinolin-2(1H)-on-5-yle, isochromén-1-on-5-yle
2-méthyl-phtalazin-1-on-5-yle isoquinolin-2(1*H*)-on-5-yle,
III) le radical R⁵ représente -CF₃
et
IV) le radical R⁶ est choisi parmi -CH₃, -CH₂-CH₃, -(CH₂)₂-CH₃, ou - CH=CH₂.
ainsi que leurs stéréoisomères ou leurs sels avec des anions physiologiquement acceptables,
**caractérisé en ce que** les composés se trouvent en la configuration 1α,2α,4β du corps de base 1-amino-1,2,3,4-tétrahydronaphtalén-2-ol ou **en ce que** les composés se trouvent en la configuration 5α,6α,8β du corps de base 5,6,7,8-tétrahydronaphtalène-1,6-diol.

2. Composé selon la revendication 1, **caractérisé en ce que** les composés présentent comme configuration absolue (1S,2R,4R) ou (1S,2R,4S) au niveau du corps de base 1-amino-1,2,3,4-tétrahydronaphtalén-2-ol
ou
(5S,6R,8R) ou (5S,6R,8S) au niveau du corps de base 5,6,7,8-tétrahydronaphtalène-1,6-diol.

3. Composé selon la revendication 1 ou 2, choisi parmi les composés suivants
(*5*α,*6*α,*8*β)-2-fluoro-8-méthyl-5-[(2-méthylquinolin-5-yl)amino]-6-(trifluorométhyl)-5,6,7,8-tétrahydronaphtalène-1,6-diol
(+)-(*5*α,*6*α,*8*β)-2-fluoro-5-[(7-fluoro-2-méthyl-quinazolin-5-yl)amino]-8-méthyl-6-(trifluorométhyl)-5,6,7,8-tétrahydronaphtalène-1,6-diol
(-)-(*5*α,*6*α,*8*β)-2-fluoro-5-[(7-fluoro-2-méthyl-quinazolin-5-yl)amino]-8-méthyl-6-(trifluorométhyl)-5,6, 7,8-tétrahydronaphtalène-1,6-diol
(*5*α,*6*α,*8*β)-2-fluoro-5-[(8-fluoro-2-méthylquinazolin-5-yl)amino]8-méthyl-6-(trifluorométhyl)-5,6,7,8-tétrahydronaphtalène-1,6-diol
(*5*α,*6*α,*8*β)-5-[(7,8-difluoro-2-méthylquinazolin-5-yl)-amino]-2-fluoro-8-méthyl-6-(trifluorométhyl)-5,6,7,8-tétrahydronaphtalène-1,6-diol
(*5*α,*6*α,*8*β)-2-fluoro-8-méthyl-5-[(2-méthylquinazolin-5-yl)amino]-6-(trifluorométhyl)-5,6,7,8-tétrahydronaphtalène-1,6-diol
5-{[(*5*α,*6*α,*8*α)-1,6-dihydroxy-2-fluoro-8-méthyl-6-(trifluorométhyl)-5,6,7,8-tétrahydronaphtalén-5-yl]-amino}quinolin-2(1*H*)-one
8-fluoro-5-{[(*1*α,*2*α,*4*β)-6-fluoro-2-hydroxy-5-méthoxy-4-méthyl-2-(trifluorométhyl)-1,2,3,4-tétrahydronaphtalén-1-yl]amino}-quinolin-2(1H)-one
(+)-5-{[(*1*α,*2*α,*4*β)-6-fluoro-2,5-dihydroxy-4-méthyl-2-(trifluorométhyl)-1,2,3,4-tétrahydronaphtalén-1-yl]-amino}-8-fluoroquinolin-2(1H)-one
(-)-5-{[(*1*α,*2*α,*4*β)-6-fluoro-2,5-dihydroxy-4-méthyl-2-(trifluorométhyl)-1,2,3,4-tétrahydronaphtalén-1-yl]-amino}-8-fluoroquinolin-2(1H)-one
5-{[(*1*α,*2*α,*4*β)-6-fluoro-2,5-dihydroxy-4-méthyl-2-(trifluorométhyl)-1,2,3,4-tétrahydronaphtalén-1-yl]-amino}-2-méthylphtalazin-1-one
(*5*α,*6*α,*8*β)-3-chloro-2-fluoro-8-méthyl-5-[(2-méthylquinazolin-5-yl)amino]-6-(trifluorométhyl)-5,6,7,8-tétrahydronaphtalène-1,6-diol
(*5*α,*6*α,*8*β)-3-chloro-2-fluoro-5-[(7-fluoro-2-méthylquinazolin-5-yl)amino]-1-méthoxy-8-méthyl-6-(trifluorométhyl)-5,6,7,8-tétrahydronaphtalén-6-ol
(*5*α,*6*α,*8*β)-3-chloro-2-fluoro-5-[(7-fluoro-2-méthylquinazolin-5-yl)amino]-8-méthyl-6-(trifluorométhyl)-5,6,7,8-tétrahydronaphtalène-1,6-diol
(*5*α,*6*α,*8*β)-3-chloro-2-fluoro-5-[(8-fluoro-2-méthylquinazolin-5-yl)amino]-1-méthoxy-8-méthyl-6-(trifluorométhyl)-5,6,7,8-tétrahydronaphtalén-6-ol
(*5*α,*6*α,*8*β)-3-chloro-2-fluoro-5-[(8-fluoro-2-méthylquinazolin-5-yl)amino]-8-méthyl-6-(trifluorométhyl)-5,6,7,8-tétrahydronaphtalène-1,6-diol
(5α,6α,8β)-3-chloro-5-[(7,8-difluoro-2-méthylquinazolin-5-yl)amino]-2-fluoro-8-méthyl-6-(trifluorométhyl)-5,6,7,8-tétrahydronaphtalène-1,6-diol
(5α,6α,8β)-3-chloro-2-fluoro-8-méthyl-5-[(2-méthylquinolin-5-yl)amino]-6-(trifluorométhyl)-5,6,7,8-tétrahydronaphtalène-1,6-diol
(+)-5-{[(*1*α,*2*α,*4*β)-7-chloro-2,5-dihydroxy-6-fluoro-4-méthyl-2-(trifluorométhyl)-1,2,3,4-tétrahydronaphtalén-1-yl]-amino}-quinolin-2(1*H*)-one
(-)-5-{[(*1*α,*2*α,*4*β)-7-chloro-2,5-dihydroxy-6-fluoro-4-méthyl-2-(trifluorométhyl)-1,2,3,4-tétrahydronaphtalén-1-yl]-amino}-quinolin-2(1*H*)-one
5-{[(*1*α,*2*α,*4*β)-7-chloro-2,5-dihydroxy-6-fluoro-4-méthyl-2-(trifluorométhyl)-1,2,3,4-tétrahydronaphtalén-1-yl]-amino}-8-fluoroquinolin-2(1*H*)-one
(*5*α,*6*α,*8*β)-8-méthyl-5-[(2-méthylquinolin-5-yl)amino]-6-(trifluorométhyl)-5,6,7,8-tétrahydronaphtalène-1,6-diol
(*5*α,*6*α,*8*β)-8-éthyl-5-[(2-méthylquinazolin-5-yl)amino]-6-(trifluorométhyl)-5,6,7,8-tétrahydronaphtalène-1,6-diol
(+)-(*5*α,*6*α,*8*β)-8-éthyl-2-fluoro-5-[(2-méthylquinazolin-5-yl)amino]-6-(trifluorométhyl)-5,6,7,8-tétrahydronaphtalène-1,6-diol
(-)-(*5*α,*6*α,*8*β)-8-éthyl-2-fluoro-5-[(2-méthylquinazolin-5-yl)amino]-6-(trifluorométhyl)-5,6,7,8-tétrahydronaphtalène-1,6-diol
(*5S*,*6R*,*8R*)-8-éthyl-2-fluoro-5-[(7-fluoro-2-méthylquinazolin-5-yl)amino]-6-(trifluorométhyl)-5,6,7,8-tétrahydronaphtalène-1,6-diol
(+)-(*5*α,*6*α,*8*β)-8-éthyl-2-fluoro-5-[(8-fluoro-2-méthylquinazolin-5-yl)amino]-6-(trifluorométhyl)-5,6,7,8-tétrahydronaphtalène-1,6-diol
(-)-(*5*α,*6*α,*8*β)-8-éthyl-2-fluoro-5-[(8-fluoro-2-méthylquinazolin-5-yl)amino]-6-(trifluorométhyl)-5,6,7,8-tétrahydronaphtalène-1,6-diol
(*5*α,*6*α,*8*β)-5-[(7,8-difluoro-2-méthylquinazolin-5-yl)-amino]-8-éthyl-2-fluoro-6-(trifluorométhyl)-5,6,7,8-tétrahydronaphtalène-1,6-diol
5-{[(*1*α,*2*α,*4*β)-4-éthyl-6-fluoro-2,5-dihydroxy-2-(trifluorométhyl)-1,2,3,4-tétrahydronaphtalén-1-yl]amino}-quinolin-2(1*H*)-one
5-{[(*1*α,*2*α,*4*β)-4-éthyl-6-fluoro-2-hydroxy-5-méthoxy-2-(trifluorométhyl)-1,2,3,4-tétrahydronaphtalén-1-yl]-amino}-8-fluoroquinolin-2(1*H*)-one
(+)-5-{[(*1*α,*2*α,*4*β)-4-éthyl-6-fluoro-2,5-dihydroxy-2-(trifluorométhyl)-1,2,3,4-tétrahydronaphtalén-1-yl]-amino}-8-fluoroquinolin-2(1*H*)-one
(-)-5-{[(*1*α,*2*α,*4*β)-4-éthyl-6-fluoro-2,5-dihydroxy-2-(trifluorométhyl)-1,2,3,4-tétrahydronaphtalén-1-yl]-amino}-8-fluoroquinolin-2(1*H*)-one
5-{[(*1*α,*2*α,*4*β)-4-éthyl-6-fluoro-2,5-dihydroxy-2-(trifluorométhyl)-1,2,3,4-tétrahydronaphtalén-1-yl]-amino}-2-méthylphtalazin-1-one
5-{[(*1*α,*2*α,*4*β)-4-éthyl-6-fluoro-2,5-dihydroxy-2-(trifluorométhyl)-1,2,3,4-tétrahydronaphtalén-1-yl]-amino}-isoquinolin-(2H)-one
5-{[(*1*α,*2*α,*4*β)-4-éthyl-6-fluoro-2,5-dihydroxy-2-(trifluorométhyl)-1,2,3,4-tétrahydronaphtalén-1-yl]-amino}isochromén-1-one
(*5*α,*6*α,*8*β)-5-[(2-éthylquinazolin-5-yl)amino]-2-fluoro-8-propyl-6-(trifluorométhyl)-5,6,7,8-tétrahydronaphtalène-1,6-diol
(*5S*,*6R*,*8R*)-8-éthyl-2,3-difluoro-5-[(2-méthylquinazolin-5-yl)amino]-6-(trifluorométhyl)-5,6,7,8-tétrahydronaphtalène-1,6-diol
(+)-(*5*α,*6*α,*8*β)-8-éthyl-2,3-difluoro-5-[(7-fluoro-2-méthylquinazolin-5-yl)amino]-6-(trifluorométhyl)-5,6,7,8-tétrahydronaphtalène-1,6-diol
(-)-(*5*α,*6*α,*8*β)-8-éthyl-2,3-difluoro-5-[(7-fluoro-2-méthylquinazolin-5-yl)amino]-6-(trifluorométhyl)-5,6,7,8-tétrahydronaphtalène-1,6-diol
(*5S*,*6R*,*8R*)-8-éthyl-2,3-difluoro-5-[(8-fluoro-2-méthylquinazolin-5-yl)amino]-6-(trifluorométhyl)-5,6,7,8-tétrahydronaphtalène-1,6-diol
(*5S*,*6R*,*8R*)-8-éthyl-2,3-difluoro-5-[(7,8-difluoro-2-méthylquinazolin-5-yl)amino]-6-(trifluorométhyl)-5,6,7,8-tétrahydronaphtalène-1,6-diol
(+)-5-{[(*1*α,*2*α,*4*β)-4-éthyl-6,7-difluoro-2,5-dihydroxy-2-(trifluorométhyl)-1,2,3,4-tétrahydronaphtalén-1-yl]amino}quinolin-2(1*H*)-one
(-)-5-{[(*1*α,*2*α,*4*β)-4-éthyl-6,7-difluoro-2,5-dihydroxy-2-(trifluorométhyl)-1,2,3,4-tétrahydronaphtalén-1-yl]-amino}quinolin-2(1*H*)-one
(5α,6α,8β)-8-éthyl-2,3-difluoro-5-[(2-méthylquinolin-5-yl)amino]-6-(trifluorométhyl)-5,6,7,8-tétrahydronaphtalène-1,6-diol
5-{[(*1*α,*2*α,*4*β)-4-éthyl-6,7-difluoro-2,5-dihydroxy-2-(trifluorométhyl)-1,2,3,4-tétrahydronaphtalén-1-yl]-amino}phtalazin-1-one
(5α,6α,8β)-2-fluoro-5-[(2-méthylquinazolin-5-yl)amino]-8-prop-1-yl-6-(trifluorométhyl)-5,6,7,8-tétrahydronaphtalène-1,6-diol
(5α,6α,8β)-2-fluoro-5-[(7-fluoro-2-méthylquinazolin-5-yl)amino]-8-prop-1-yl-6-(trifluorométhyl)-5,6,7,8-tétrahydronaphtalène-1,6-diol
5-{[(*1*α,*2*α,*4*β)-4-éthyl-6,7-difluoro-2,5-dihydroxy-2-(trifluorométhyl)-1,2,3,4-tétrahydronaphtalén-1-yl]-amino}quinolin-2(1*H*)-one
5-{[(*1*α,*2*α,*4*β)-4-éthyl-6,7-difluoro-2,5-dihydroxy-2-(trifluorométhyl)-1,2,3,4-tétrahydronaphtalén-1-yl]-amino}isoquinolin-1(2*H*)-one
5-{[(*1S*,*2R*,*4S*)-6-chloro-2,5-dihydroxy-4-méthyl-2-(trifluorométhyl)-1,2,3,4-tétrahydronaphtalén-1-yl]-amino}-1*H*-quinolin-2-one
5-{[(*1S*,*2R*,*4R*)-6-chloro-2,5-dihydroxy-4-méthyl-2-(trifluorométhyl)-1,2,3,4-tétrahydronaphtalén-1-yl]-amino}-1*H*-quinolin-2-one
5-{[(*1S*,*2R*,*4S*)-6-chloro-2,5-dihydroxy-4-méthyl-2-(trifluorométhyl)-1,2,3,4-tétrahydronaphtalén-1-yl]-amino}-8-fluoro-1*H*-quinolin-2-one
5-{[(*1S*,*2R*,*4R*)-6-chloro-2,5-dihydroxy-4-méthyl-2-(trifluorométhyl)-1,2,3,4-tétrahydronaphtalén-1-yl]-amino}-8-fluoro-1*H*-quinolin-2-one
5-{[(*1S*,*2R*,*4S*)-6-chloro-2,5-dihydroxy-4-méthyl-2-(trifluorométhyl)-1,2,3,4-tétrahydronaphtalén-1-yl]-amino}-2*H*-quinolin-1-one
5-{[(1S,2R,4R)-6-chloro-2,5-dihydroxy-4-méthyl-2-(trifluorométhyl)-1,2,3,4-tétrahydronaphtalén-1-yl]-amino}-2*H*-quinolin-1-one 5-{[(1S,2R,4S)-6-chloro-2,5-dihydroxy-4-méthyl-2-(trifluorométhyl)-1,2,3,4-tétrahydronaphtalén-1-yl]-amino}-isochromén-1-one 5-{[(*1S*,*2R*,*4R*)-6-chloro-2,5-dihydroxy-4-méthyl-2-(trifluorométhyl)-1,2,3,4-tétrahydronaphtalén-1-yl]-amino}-isochromén-1-one 4-{[(*1S*,*2R*,*4S*)-6-chloro-2,5-dihydroxy-4-méthyl-2-(trifluorométhyl)-1,2,3,4-tétrahydronaphtalén-1-yl]-amino}-1,3-dihydro-indol-2-one 4-{[(*1S*,*2R*,*4R*)-6-chloro-2,5-dihydroxy-4-méthyl-2-(trifluorométhyl)-1,2,3,4-tétrahydronaphtalén-1-yl]-amino}-1,3-dihydro-indol-2-one 5-{[(*1S*,*2R*,*4S*)-chloro-2,5-dihydroxy-4-éthyl-2-(trifluorométhyl)-1,2,3,4-tétrahydronaphtalén-1-yl]-amino}-1H-quinolin-2-one 5-{[(*1*α,*2*α,*4*β)-6-chloro-2,5-dihydroxy-4-éthyl-2-(trifluorométhyl)-1,2,3,4-tétrahydronaphtalén-1-yl]-amino}-1H-quinolin-2-one 5-{[(*1S*,*2R*,*4S*)-6-chloro-2,5-dihydroxy-4-éthyl-2-(trifluorométhyl)-1,2,3,4-tétrahydronaphtalén-1-yl]-amino}-8-fluoro-1H-quinolin-2-one 5-{[(*1S*,*2R*,*4R*)-6-chloro-4-éthyl-2,5-dihydroxy-2-(trifluorométhyl)-1,2,3,4-tétrahydronaphtalén-1-yl]-amino}-8-fluoro-1H-quinolin-2-one 5-{[(*1S*,*2R*,*4S*)-6-chloro-2,5-dihydroxy-4-éthyl-2-(trifluorométhyl)-1,2,3,4-tétrahydronaphtalén-1-yl]-amino}-2*H*-quinolin-1-one 5-{[(*1S*,*2R*,*4R*)-6-chloro-4-éthyl-2,5-dihydroxy-2-(trifluorométhyl)-1,2,3,4-tétrahydronaphtalén-1-yl]-amino}-2*H*-quinolin-1-one 5-{[(*1S*,*2R*,*4S*)-6-chloro-2,5-dihydroxy-4-éthyl-2-(trifluorométhyl)-1,2,3,4-tétrahydronaphtalén-1-yl]-amino}-isochromén-1-one 5-{[(*1S*,*2R*,*4R*)-6-chloro-4-éthyl-2,5-dihydroxy-4-2-(trifluorométhyl)-1,2,3,4-tétrahydronaphtalén-1-yl]-amino}-isochromén-1-one
4-{[(*1S*,*2R*,*4S*)-6-chloro-2,5-dihydroxy-4-éthyl-2-(trifluorométhyl)-1,2,3,4-tétrahydronaphtalén-1-yl]-amino}-1,3-dihydro-indol-2-one 4-{[(*1S*,*2R*,*4R*)-6-chloro-2,5-dihydroxy-4-éthyl-2-(trifluorométhyl)-1,2,3,4-tétrahydronaphtalén-1-yl]-amino}-1,3-dihydro-indol-2-one 5-{[(*1S*,*2R*,*4S*)-6-chloro-2,5-dihydroxy-4-éthyl-2-(trifluorométhyl)-1,2,3,4-tétrahydronaphtalén-1-yl]-amino}-7-fluoro-1H-quinolin-2-one 5-{[(*1S*,*2R*,*4R*)-6-chloro-4-éthyl-2,5-dihydroxy-2-(trifluorométhyl)-1,2,3,4-tétrahydronaphtalén-1-yl]-amino}-7-fluoro-1H-quinolin-2-one 5-{[(*1S*,*2R*,*4S*)-6-chloro-2,5-dihydroxy-4-méthyl-2-(trifluorométhyl)-1,2,3,4-tétrahydronaphtalén-1-yl]-amino}-7-fluoro-1*H*-quinolin-2-one 5-{[(*1S*,*2R*,*4R*)-6-chloro-2,5-dihydroxy-4-méthyl-2-(trifluorométhyl)-1,2,3,4-tétrahydronaphtalén-1-yl]-amino}-7-fluoro-1*H*-quinolin-2-one (*5*α,*6*α,*8*β)-2-chloro-8-éthyl-5-[(indazol-5-yl)amino]-6-(trifluorométhyl)-5,6,7,8-tétrahydronaphtalène-1,6-diol 6-{[(*1*α,*2*α,*4*β)-6-chloro-2,5-dihydroxy-4-éthyl-2-(trifluorométhyl)-1,2,3,4-tétrahydronaphtalén-1-yl]-amino}-4-méthylbenzo[d][1,2]oxazin-1-one 5-{[(*1*α,*2*α,*4*β)-7-isopropyl-2,5-dihydroxy-4-éthyl-2-(trifluorométhyl)-1,2,3,4-tétrahydronaphtalén-1-yl]-amino}-1*H*-quinolin-2-one (*5*α,*6*α,*8*β)-8-éthyl-3-isopropyl-5-[2-méthylquinazolin-5-ylamino]-6-(trifluorométhyl)-5,6,7,8-tétrahydronaphtalène-1,6-diol 5-{[(*1*α,*2*α,*4*β)-7-isopropyl-2,5-dihydroxy-4-éthyl-2-(trifluorométhyl)-1,2,3,4-tétrahydronaphtalén-1-yl]-amino}-2*H*-quinolin-1-one 4-{[(*1*α,*2*α,*4*β)-7-isopropyl-2,5-dihydroxy-4-éthyl-2-(trifluorométhyl)-1,2,3,4-tétrahydronaphtalén-1-yl]-amino}-1,3-dihydro-indol-2-one 5-{[(*1*α,*2*α,*4*β)-7-isopropyl-2,5-dihydroxy-4-éthyl-2-(trifluorométhyl)-1,2,3,4-tétrahydronaphtalén-1-yl]-amino}-isochromén-1-one
5-{[(*1*α,*2*α,*4*β)-7-isopropyl-2,5-dihydroxy-4-éthyl-2-(trifluorométhyl)-1,2,3,4-tétrahydronaphtalén-1-yl]-amino}-8-fluoro-1*H*-quinolin-2-one 5-{[(*1*α,*2*α,*4*β)-7-isopropyl-2,5-dihydroxy-4-éthyl-2-(trifluorométhyl)-1,2,3,4-tétrahydronaphtalén-1-yl]-amino}-8-fluoro-1*H*-quinolin-2-one 5-{[(*1*α,*2*α,*4*β)-7-chloro-2,5-dihydroxy-4-éthyl-6-méthyl-2-(trifluorométhyl)-1,2,3,4-tétrahydronaphtalén-1-yl]-amino}-1*H*-quinolin-2-one 5-{[(*1S*,*2R*,*4R*)-7-chloro-2,5-dihydroxy-6-méthyl-4-éthyl-2-(trifluorométhyl)-1,2,3,4-tétrahydronaphtalén-1-yl]-amino}-2*H*-quinolin-1-one 4-{[(*1*α,*2*α,*4*β)-7-chloro-2,5-dihydroxy-6-méthyl-4-éthyl-2-(trifluorométhyl)-1,2,3,4-tétrahydronaphtalén-1-yl]-amino}-1,3-dihydro-indol-2-one 5-{[(*1*α,*2*α,*4*β)-7-chloro-2,5-dihydroxy-4-éthyl-6-méthyl-2-(trifluorométhyl)-1,2,3,4-tétrahydronaphtalén-1-yl]-amino}-8-fluoro-1*H*-quinolin-2-one 5-{[(*1S*,*2R*,*4R*)-7-chloro-2,5-dihydroxy-4-éthyl-6-méthyl-2-(trifluorométhyl)-1,2,3,4-tétrahydronaphtalén-1-yl]-amino}-8-fluoro-1*H*-quinolin-2-one (*5*α,*6*α,*8*β)-3-chloro-8-éthyl-2-méthyl-5-[2-méthylquinazolin-5-ylamino]-6-(trifluorométhyl)-5,6,7,8-tétrahydronaphtalène-1,6-diol 5-{[(*1S*,*2R*,*4S*)-7-chloro-2,5-dihydroxy-4-éthyl-6-méthyl-2-(trifluorométhyl)-1,2,3,4-tétrahydronaphtalén-1-yl]-amino}-7-fluoro-1*H*-quinolin-2-one 5-{[(*1*α,*2*α,*4*β)-7-chloro-2,5-dihydroxy-4-éthyl-6-méthyl-2-(trifluorométhyl)-1,2,3,4-tétrahydronaphtalén-1-yl]-amino}-7-fluoro-1*H*-quinolin-2-one 5-{[(*1S*,*2R*,*4R*)-7-chloro-2,5-dihydroxy-4-éthyl-6-méthyl-2-(trifluorométhyl)-1,2,3,4-tétrahydronaphtalén-1-yl]-amino}-7-fluoro-1*H*-quinolin-2-one (5S,6R,8R)-8-éthyl-2,3-difluoro-5-[(2-méthylquinazolin-5-yl)amino]-6-(trifluorométhyl)-5,6,7,8-tétrahydronaphtalène-1,6-diol 5-{[(1S,2R,4R)-4-éthyl-7-fluoro-2,5-dihydroxy-6-méthyl-2-(trifluorométhyl)-1,2,3,4-tétrahydronaphtalén-1-yl]-amino}quinolin-2(1H)-one
5-{[(1S,2R,4R)-4-éthyl-7-fluoro-2,5-dihydroxy-6-méthyl-2-(trifluorométhyl)-1,2,3,4-tétrahydronaphtalén-1-yl]-amino}-7-fluoroquinolin-2(1H)-one 5-{[(1S,2R,4R)-4-éthyl-7-fluoro-2,5-dihydroxy-6-méthyl-2-(trifluorométhyl)-1,2,3,4-tétrahydronaphtalén-1-yl]-amino}-8-fluoroquinolin-2(1H)-one 5-{[(1S,2R,4R)-4-éthyl-7-fluoro-2,5-dihydroxy-6-méthyl-2-(trifluorométhyl)-1,2,3,4-tétrahydronaphtalén-1-yl]-amino}isoquinolin-1(2H)-one (5S,6R,8R)-8-éthyl-3-fluoro-5-[(7-fluoro-2-méthylquinazolin-5-yl)amino]-2-méthyl-6-(trifluorométhyl)-5,6,7,8-tétrahydronaphtalène-1,6-diol (5S,6R,8R)-8-éthyl-3-fluoro-5-[(8-fluoro-2-méthylquinazolin-5-yl)amino]-2-méthyl-6-(trifluorométhyl)-5,6,7,8-tétrahydronaphtalène-1,6-diol 5-{[(1S,2R,4S)-4,6-diéthyl-7-fluoro-2,5-dihydroxy-2-(trifluorométhyl)-1,2,3,4-tétrahydronaphtalén-1-yl]-amino}quinolin-2(1H)-one 5-{[(1S,2R,4R)-4,6-diéthyl-7-fluoro-2,5-dihydroxy-2-(trifluorométhyl)-1,2,3,4-tétrahydronaphtalén-1-yl]-amino}quinolin-2(1H)-one 5-{[(1S,2R,4S)-4,6-diéthyl-7-fluoro-2,5-dihydroxy-2-(trifluorométhyl)-1,2,3,4-tétrahydronaphtalén-1-yl]-amino}-7-fluoroquinolin-2(1H)-one 5-{[(1S,2R,4R)-4,6-diéthyl-7-fluoro-2,5-dihydroxy-2-(trifluorométhyl)-1,2,3,4-tétrahydronaphtalén-1-yl]-amino}-7-fluoroquinolin-2(1H)-one 5-{[(1S,2R,4S)-4,6-diéthyl-2,5-dihydroxy-2-(trifluorométhyl)-1,2,3,4-tétrahydronaphtalén-1-yl]amino}-1H-quinolin-2-one 5-{[(1S,2R,4R)-4,6-diéthyl-2,5-dihydroxy-2-(trifluorométhyl)-1,2,3,4-tétrahydronaphtalén-1-yl]amino}-1H-quinolin-2-one 5-{[(*1S*,*2R*,*4S*)-4,6-diéthyl-2,5-dihydroxy-2-(trifluorométhyl)-1,2,3,4-tétrahydronaphtalén-1-yl]amino}-8-fluoro-1*H*-quinolin-2-one 5-{[(*1S*,*2R*,*4R*)-4,6-diéthyl-2,5-dihydroxy-2-(trifluorométhyl)-1,2,3,4-tétrahydronaphtalén-1-yl]amino}-8-fluoro-1H-quinolin-2-one
(*5S*,*6R*,*8S*)-2,8-diéthyl-5-[2-méthylquinazolin-5-ylamino]-6-(trifluorométhyl)-5,6,7,8-tétrahydronaphtalène-1,6-diol 5-{[(*1*α,*2*α,*4*β)-7-chloro-2,5-dihydroxy-4-éthyl-2-(trifluorométhyl)-1,2,3,4-tétrahydronaphtalén-1-yl]-amino}-1H-quinolin-2-one 5-{[(*1*α,*2*α,*4*β)-7-chloro-2,5-dihydroxy-4-éthyl-2-(trifluorométhyl)-1,2,3,4-tétrahydronaphtalén-1-yl]-amino}-8-fluoro-1H-quinolin-2-one 5-{[(*1S*,*2R*,*4R*)-6-chloro-4-éthyl-2,5-dihydroxy-7-fluoro-2-(trifluorométhyl)-1,2,3,4-tétrahydronaphtalén-1-yl]-amino}quinolin-2(1*H*)-one 5-{[(*1S*,*2R*,*4R*)-6-chloro-4-éthyl-2,5-dihydroxy-7-fluoro-2-(trifluorométhyl)-1,2,3,4-tétrahydronaphtalén-1-yl]-amino}-7-fluoroquinolin-2(1*H*)-one 5-{[(*1S*,*2R*,*4R*)-6-chloro-4-éthyl-2,5-dihydroxy-7-fluoro-2-(trifluorométhyl)-1,2,3,4-tétrahydronaphtalén-1-yl]amino}-8-fluoroquinolin-2(1*H*)-one (*5S*,*6R*,*8R*)-2-chloro-8-éthyl-3-fluoro-5-[(2-méthylquinazolin-5-yl)amino]-6-(trifluorométhyl)-5,6,7,8-tétrahydronaphtalène-1,6-diol (*5S*,*6R*,*8R*)-2-chloro-8-éthyl-3-fluoro-5-[(7-fluoro-2-méthylquinazolin-5-yl)amino]-6-(trifluorométhyl)-5,6,7,8-tétrahydronaphtalène-1,6-diol 5-{[(*1*α,*2*α,*4*β)-7-fluoro-2,5-dihydroxy-4-éthyl-2-(trifluorométhyl)-1,2,3,4-tétrahydronaphtalén-1-yl]amino}-7-fluoro-1*H*-quinolin-2-one 5-{[(*5S*,*6R*,*8R*)-8-éthyl-3-fluoro-1,6-dihydroxy-6-(trifluorométhyl)-5,6,7,8-tétrahydronaphtalén-5-yl]-amino}-8-fluoroquinolin-2(1*H*)-one 5-{[(*1*α,*2*α,*4*β)-7-fluoro-2,5-dihydroxy-4-méthyl-2-(trifluorométhyl)-1,2,3,4-tétrahydronaphtalén-1-yl]-amino}-7-fluoro-1*H*-quinolin-2-one 5-{[(*1*α,*2*α,*4*β)-7-fluoro-2,5-dihydroxy-4-méthyl-2-(trifluorométhyl)-1,2,3,4-tétrahydronaphtalén-1-yl]-amino}-8-fluoro-1*H*-quinolin-2-one 5-{[(*1*α,*2*α,*4*β)-7-bromo-2,5-dihydroxy-4-éthyl-2-(trifluorométhyl)-1,2,3,4-tétrahydronaphtalén-1-yl]-amino}-1*H*-quinolin-2-one
5-{[(*1S*,*2R*,*4R*)-7-bromo-2,5-dihydroxy-4-éthyl-2-(trifluorométhyl)-1,2,3,4-tétrahydronaphtalén-1-yl]-amino}-7-fluoro-1*H*-quinolin-2-one 5-{[(*1*α,*2*α,*4*β)-7-bromo-2,5-dihydroxy-4-éthyl-2-(trifluorométhyl)-1,2,3,4-tétrahydronaphtalén-1-yl]-amino}-8-fluoro-1H-quinolin-2-one 5-{[(*1*α,*2*α,*4*β)-6-fluoro-2,7-dihydroxy-4-méthyl-2-(trifluorométhyl)-1,2,3,4-tétrahydronaphtalén-1-yl]-amino}-1*H*-quinolin-2-one (*5*α,*6*α,*8*β)-8-éthyl-1,6-dihydroxy-5-(2-oxo-1,2-dihydroquinolin-5-ylamino)-6-(trifluorométhyl)-5,6,7,8-tétrahydronaphtalène-2-carbonitrile (*5*α,*6*α,*8*β)-8-éthyl-1,6-dihydroxy-5-(8-fluoro-2-oxo-1,2-dihydroquinolin-5-ylamino)-6-(trifluorométhyl)-5,6,7,8-tétrahydronaphtalène-2-carbonitrile (*5*α,*6*α,*8*β)-8-éthyl-1,6-dihydroxy-5-[(1-oxo-1,2-dihydro-isoquinolin-5-yl)amino]-6-(trifluorométhyl)-5,6,7,8-tétrahydronaphtalène-2-carbonitrile (*5*α,*6*α,*8*β)-1,6-dihydroxy-8-méthyl-5-[(2-oxo-2,3-dihydro-1*H*-indol-4-yl)amino]-6-(trifluorométhyl)-5,6,7,8-tétrahydronaphtalène-2-carbonitrile (*5*α,*6*α,*8*β)-5-[(8-fluoro-2-oxo-1,2-dihydroquinolin-5-yl)amino]-1,6-dihydroxy-8-méthyl-6-(trifluorométhyl)-5,6,7,8-tétrahydronaphtalène-2-carbonitrile 5-{[(*5S*,*6R*,*8R*)-8-éthyl-1,6-dihydroxy-3-fluoro-5-[(2-oxo-1,2-dihydroquinolin-5-yl)amino]-6-(trifluorométhyl)-5,6,7,8-tétrahydronaphtalène-2-carbonitrile (*5*α,*6*α,*8*β)-8-éthyl-1,6-dihydroxy-3-fluoro-5-[(8-fluoro-2-oxo-1,2-dihydroquinolin-5-yl)amino]-6-(trifluorométhyl)-5,6,7,8-tétrahydronaphtalène-2-carbonitrile 5-{[(*1S*,*2R*,*4S*)-2,5-dihydroxy-4-méthyl-2-(trifluorométhyl)-1,2,3,4-tétrahydronaphtalén-1-yl]amino}-7-fluoro-1*H*-quinolin-2-one 5-{[(*1S*,*2R*,*4R*)-2,5-dihydroxy-4-méthyl-2-(trifluorométhyl)-1,2,3,4-tétrahydronaphtalén-1-yl]amino}-7-fluoro-1H-quinolin-2-one 5-{[(1S,2R,4S)-2,5-dihydroxy-4-méthyl-2-(trifluorométhyl)-1,2,3,4-tétrahydronaphtalén-1-yl]amino}-8-fluoro-1H-quinolin-2-one
5-{[(*1S*,*2R*,*4R*)-2,5-dihydroxy-4-méthyl-2-(trifluorométhyl)-1,2,3,4-tétrahydronaphtalén-1-yl]amino}-8-fluoro-1H-quinolin-2-one 5-{[(*1*α,*2*α,*4*β)-4-éthyl-2,5-dihydroxy-2-(trifluorométhyl)-1,2,3,4-tétrahydronaphtalén-1-yl]amino}-6-fluoro-1*H*-quinolin-2-one 5-{[(*1*α,*2*α,*4*β)-4-éthyl-6-fluoro-2,5-dihydroxy-7-méthyl-2-(trifluorométhyl)-1,2,3,4-tétrahydronaphtalén-1-yl]-amino}-1*H*-quinolin-2-one 5-{[(*1*α,*2*α,*4*β)-4-éthyl-6-fluoro-2,5-dihydroxy-7-méthyl-2-(trifluorométhyl)-1,2,3,4-tétrahydronaphtalén-1-yl]-amino}-7-fluoro-1*H*-quinolin-2-one 5-{[(*1*α,*2*α,*4*β)-4-éthyl-6-fluoro-2,5-dihydroxy-7-méthyl-2-(trifluorométhyl)-1,2,3,4-tétrahydronaphtalén-1-yl]-amino}-8-fluoro-1*H*-quinolin-2-one 5-{[(*1*α,*2*α,*4*β)-4-éthyl-6-fluoro-2,5-dihydroxy-7-méthyl-2-(trifluorométhyl)-1,2,3,4-tétrahydronaphtalén-1-yl]-amino}-2*H*-quinolin-1-one (*5*α,*6*α,*8*β)-8-éthyl-2-fluoro-3-méthyl-5-[2-méthylquinazolin-5-ylamino]-6-(trifluorométhyl)-5,6,7,8-tétrahydronaphtalène-1,6-diol (*5S*,*6R*,*8R*)-8-éthyl-2,3-difluoro-5-[(2-méthyl-1-oxyquinolin-5-yl)amino]-6-(trifluorométhyl)-5,6,7,8-tétrahydronaphtalène-1,6-diol 5-{[(*1*α,*2*α,*4*β)-2,5-dihydroxy-6,7-diméthyl-4-éthyl-2-(trifluorométhyl)-1,2,3,4-tétrahydronaphtalén-1-yl]-amino}-1H-quinolin-2-one 5-{[(*1*α,*2*α,*4*β)-2,5-dihydroxy-6,7-diméthyl-4-éthyl-2-(trifluorométhyl)-1,2,3,4-tétrahydronaphtalén-1-yl]-amino}-8-fluoro-1H-quinolin-2-one 5-{[(*1*α,*2*α,*4*β)-2,5-dihydroxy-6,7-diméthyl-4-éthyl-2-(trifluorométhyl)-1,2,3,4-tétrahydronaphtalén-1-yl]-amino}-2H-quinolin-1-one 5-{[(*5S*,*6R*,*8R*)-2-chloro-8-éthyl-1,6-dihydroxy-3-fluoro-2-6-(trifluorométhyl)-5,6,7,8-tétrahydronaphtalén-5-yl]amino}quinolin-2(1*H*)-one 5-{[(*5S*,*6R*,*8R*)-3-chloro-8-éthyl-1,6-dihydroxy-2-6-(trifluorométhyl)-5,6,7,8-tétrahydronaphtalén-5-yl]-amino}-7-fluoroquinolin-2(1H)-one
(*5S*,*6R*,8*R*)-2,3-dichloro-8-éthyl-5-[(7-fluoro-2-méthylquinazolin-5-yl)amino]-6-(trifluorométhyl)-5,6,7,8-tétrahydronaphtalène-1,6-diol
(*5S*,*6R*,*8R*)-2-chloro-8-éthyl-5-[(7-fluoro-2-méthylquinazolin-5-yl)amino]-6-(trifluorométhyl)-5,6,7,8-tétrahydronaphtalène-1,6-diol
ou
(*5S*,*6R*,*8R*)-3-chloro-8-éthyl-5-[(7-fluoro-2-méthylquinazolin-5-yl)amino]-6-(trifluorométhyl)-5,6,7,8-tétrahydronaphtalène-1,6-diol.

4. Utilisation de composés selon la revendication 1, 2 ou 3 pour la fabrication de médicaments.

5. Utilisation de composés selon la revendication 1, 2 ou 3 pour la fabrication de médicaments destinés au traitement maladies inflammatoires.
